# EUROPEAN PATENT SPECIFICATION

(11) **EP 2 147 970 B1**
(45) Date of publication and mention of the grant of the patent: **31.12.2014**
(21) Application number: 08740548.6
(22) Date of filing: 17.04.2008
(51) Int. Cl.: C12N 1/21, C12N 15/09, C12P 7/42, C12P 7/46, C12P 13/14, C12P 13/20, C07K 14/195

(54) **A METHOD FOR PRODUCING AN ACIDIC SUBSTANCE HAVING A CARBOXYL GROUP**
VERFAHREN ZUR HERSTELLUNG EINER SAUREN SUBSTANZ MIT EINER CARBOXYLGRUPPE
PROCÉDÉ DE FABRICATION D'UNE SUBSTANCE ACIDE AYANT UN GROUPE CARBOXYLE

(30) Priority: 17.04.2007 JP 2007108631; 19.09.2007 JP 2007242859
(43) Date of publication of application: 27.01.2010
(73) Proprietor: Ajinomoto Co., Inc., Chuo-ku, Tokyo 104-8315 (JP)
(72) Inventor: HARA, Yoshihiko, Kawasaki-shi Kanagawa 210-8681 (JP); FUKUI, Keita, Kawasaki-shi Kanagawa 210-8681 (JP); TAJIMA, Yoshinori, Kawasaki-shi Kanagawa 210-8681 (JP); KAWAMURA, Kazue, Kawasaki-shi Kanagawa 210-8681 (JP); USUDA, Yoshihiro, Kawasaki-shi Kanagawa 210-8681 (JP); MATSUI, Kazuhiko, Kawasaki-shi Kanagawa 210-8681 (JP)
(74) Representative: Strehl Schübel-Hopf & Partner
(86) International application number: PCT/JP2008/057478
(87) International publication number: WO 2008/133161

(56) References cited:
- WO-A1-2005/085419
- WO-A2-02/22633
- WO-A2-02/22814
- WO-A2-2006/069610
- DATABASE UNIPROT [Online] 'Putative transport protein ybjL', XP003024008 Database accession no. (P60869)
- HAYASHI K. ET AL.: 'Highly accurate genome sequences of Escherichia coli K-12 strains MG1655 and W3110' MOLECULAR SYSTEMS BIOLOGY vol. 2, 2006, XP008109308

### SEQUENCE LISTING

<110> Ajinomoto Co., Inc.
<120> A method for pr oduci ng an acidic compound having carboxyl group
<130> C913-C8021
<150> JP2007-108631
   <151> 2007-04-17
<150> JP2007-242859
   <151> 2007-09-19
<160> 88
<170> Patent In version 3. 1
<210> 1
   <211> 2229
   <212> DNA
   <213> Pant oea ananatis
<220>
   <221> CDS
   <222> (298)..(1986)
<400> 1
   acgccacctg atacgcaggt tgcccagaaa taataacgct gcggcataac tccccactca 60
   ttaaaaacat gccaaaactt cgcctttgac cttgccgagc ctggtggaaa taaaaagaaa 120
   cagtcgtttt gagaacgaat gaaagtcgcc aatggctgag aatgattttt ctgattagaa 180
   tattggccgc gttacctttt cagacatcag tgatagctgg aggtaatctt acggcctgct 240
   caagatagcg aaaaacaggc ctctcttcaa taggttacaa gtaaacaaga agttaaa 297
   atg ttg aat gtt aac atc gca gaa ttg tta aga ggt aat gac att ctg 345
   Met Leu Asn Val Asn Ile Ala Glu Leu Leu Arg Gly Asn Asp Ile Leu
   1 5 10 15
   tta tta ttt ttc gta ctg gca ctg gga ctt tgc ctg gga aaa tta cgt 393
   Leu Leu Phe Phe Val Leu Ala Leu Gly Leu Cys Leu Gly Lys Leu Arg
   20 25 30
   ttt ggc tcg ata caa ctc gga aat tcc att ggc gta tta gtg gtt tca 441
   Phe Gly Ser Ile Gln Leu Gly Asn Ser Ile Gly Val Leu Val Val Ser
   35 40 45
   tta tta tta ggt cag caa cac ttc tcg atg aat acg gat gcc ctg agc 489
   Leu Leu Leu Gly Gln Gln His Phe Ser Met Asn Thr Asp Ala Leu Ser
   50 55 60
   ctg ggt ttc atg ttg ttt att ttc tgc gtc ggc att gaa gca ggc ccc 537
   Leu Gly Phe Met Leu Phe Ile Phe Cys Val Gly Ile Glu Ala Gly Pro
   65 70 75 80
   aac ttt ttt tcc att ttc ttt cgg gat ggc aaa aat tat ttc atg ctc 585
   Asn Phe Phe Ser Ile Phe Phe Arg Asp Gly Lys Asn Tyr Phe Met Leu
   85 90 95
   gcc att gtg atg gtg gtc agc gcc ctg ctg ctg gcc ctg gga ttc ggc 633
   Ala Ile Val Met Val Val Ser Ala Leu Leu Leu Ala Leu Gly Phe Gly
   100 105 110
   aag ctt ttt ggc tgg gac atc ggc ctg acc gca ggc gta ctg gcc ggc 681
   Lys Leu Phe Gly Trp Asp Ile Gly Leu Thr Ala Gly Val Leu Ala Gly
   115 120 125
   tcc atg acg tcc acg ccg gtc ctg gtt ggc gcg ggt gat acg ctg cgc 729
   Ser Met Thr Ser Thr Pro Val Leu Val Gly Ala Gly Asp Thr Leu Arg
   130 135 140
   cag agc atc agc gat ccc aga cag ctg agc atc atg cag gat caa ctc 777
   Gln Ser Ile Ser Asp Pro Arg Gln Leu Ser Ile Met Gln Asp Gln Leu
   145 150 155 160
   agc ctg ggt tat gcc gta acc tat ctt gtc ggg tta gtc agc ctg att 825
   Ser Leu Gly Tyr Ala Val Thr Tyr Leu Val Gly Leu Val Ser Leu Ile
   165 170 175
   ttc ggc gca cgc tat ctg ccc cgt tta caa cat cag gac tta ccc acc 873
   Phe Gly Ala Arg Tyr Leu Pro Arg Leu Gln His Gln Asp Leu Pro Thr
   180 185 190
   tgc gcc cag cag att gcg cgc gaa cgc ggt ctt gac gct gac agc cag 921
   Cys Ala Gln Gln Ile Ala Arg Glu Arg Gly Leu Asp Ala Asp Ser Gln
   195 200 205
   cgc aaa gtg ttt ctg ccg gtg att cgc gct tac cgc gtt ggc ccg gaa 969
   Arg Lys Val Phe Leu Pro Val Ile Arg Ala Tyr Arg Val Gly Pro Glu
   210 215 220
   ctg gtg gcg tgg agc ggc ggc aaa aac ttg cgc gag ctc ggt att tac 1017
   Leu Val Ala Trp Ser Gly Gly Lys Asn Leu Arg Glu Leu Gly Ile Tyr
   225 230 235 240
   cgg cag acc ggc tgc tat atc gaa cga att cgc cgc aat ggc atc ctt 1065
   Arg Gln Thr Gly Cys Tyr Ile Glu Arg Ile Arg Arg Asn Gly Ile Leu
   245 250 255
   gcc agc ccg gat ggt gat gcg gtg ctg caa ctg ggg gac gat att tcc 1113
   Ala Ser Pro Asp Gly Asp Ala Val Leu Gln Leu Gly Asp Asp Ile Ser
   260 265 270
   ctg gtg ggc tat ccg gat gcc cac gcg cgc ctg gat gcc agc ttt cgt 1161
   Leu Val Gly Tyr Pro Asp Ala His Ala Arg Leu Asp Ala Ser Phe Arg
   275 280 285
   aac ggc aaa gaa gtt ttt gac cgc gat ctg ctg gat atg cgg att gtg 1209
   Asn Gly Lys Glu Val Phe Asp Arg Asp Leu Leu Asp Met Arg Ile Val
   290 295 300
   acg gaa gag atc gtg gtg aaa aac cac aat gcc gtc aac aaa cgg cta 1257
   Thr Glu Glu Ile Val Val Lys Asn His Asn Ala Val Asn Lys Arg Leu
   305 310 315 320
   agc aaa ctc aac ctt acc gat cac ggc tgc ttt ctc aac cgc gtg att 1305
   Ser Lys Leu Asn Leu Thr Asp His Gly Cys Phe Leu Asn Arg Val Ile
   325 330 335
   cgc agc cag atc gag atg cct att gat gac aac atc atg ctg aac aaa 1353
   Arg Ser Gln Ile Glu Met Pro Ile Asp Asp Asn Ile Met Leu Asn Lys
   340 345 350
   ggt gat gtg ttg cag gtc agc ggc gag gcg cga cgg gtg aag agc gta 1401
   Gly Asp Val Leu Gln Val Ser Gly Glu Ala Arg Arg Val Lys Ser Val
   355 360 365
   gcg gat cgc atc ggc ttt gtg gcg att cac agt cag atg acc gac ctg 1449
   Ala Asp Arg Ile Gly Phe Val Ala Ile His Ser Gln Met Thr Asp Leu
   370 375 380
   ctg gca ttc tgt gct ttt ttt atc atc ggt ctg atg gtc ggg tta att 1497
   Leu Ala Phe Cys Ala Phe Phe Ile Ile Gly Leu Met Val Gly Leu Ile
   385 390 395 400
   acg ttc cag ttc agc aac ttt agc ttt ggc atc ggt aat gcc gca ggg 1545
   Thr Phe Gln Phe Ser Asn Phe Ser Phe Gly Ile Gly Asn Ala Ala Gly
   405 410 415
   ttg ttg ttt gcc ggc att atg ctg ggc ttc ctg cgc gct aac cat cct 1593
   Leu Leu Phe Ala Gly Ile Met Leu Gly Phe Leu Arg Ala Asn His Pro
   420 425 430
   acc ttt ggc tat att ccg cag ggt gcg ctg acg atg gtg aaa gag ttc 1641
   Thr Phe Gly Tyr Ile Pro Gln Gly Ala Leu Thr Met Val Lys Glu Phe
   435 440 445
   ggg ctg atg gta ttc atg gcg ggc gtc ggg ctg agc gcc ggc agc ggt 1689
   Gly Leu Met Val Phe Met Ala Gly Val Gly Leu Ser Ala Gly Ser Gly
   450 455 460
   atc gac cac ggt ata tcg ggt aac ggc gca ctg atg ctc ttg tgc ggc 1737
   Ile Asp His Gly Ile Ser Gly Asn Gly Ala Leu Met Leu Leu Cys Gly
   465 470 475 480
   ctg ctg gtc agc ctg tta ccg gtg gtg att tgc tac ctg ttt ggc gcc 1785
   Leu Leu Val Ser Leu Leu Pro Val Val Ile Cys Tyr Leu Phe Gly Ala
   485 490 495
   tat gtt ctg cgc atg aac cgc gcg ctg ctg ttt ggc gcc att atg ggt 1833
   Tyr Val Leu Arg Met Asn Arg Ala Leu Leu Phe Gly Ala Ile Met Gly
   500 505 510
   gca cgc acc tgc gcg cca gcc atg gag ata atc agc gac aca gct cgc 1881
   Ala Arg Thr Cys Ala Pro Ala Met Glu Ile Ile Ser Asp Thr Ala Arg
   515 520 525
   agt aac atc ccg gca ctc ggc tac gcc ggg acc tat gct atc gcg aac 1929
   Ser Asn Ile Pro Ala Leu Gly Tyr Ala Gly Thr Tyr Ala Ile Ala Asn
   530 535 540
   gtt ttg cta acg ctg gcg gga aca tta atc gtt att atc tgg ccg tta 1977
   Val Leu Leu Thr Leu Ala Gly Thr Leu Ile Val Ile Ile Trp Pro Leu
   545 550 555 560
   ttg ccc tta taaaattttt ttactgacgt ccagaacttt ttttcccgac 2026
   Leu Pro Leu
   cacagtctga ataagtgcca ctgcttttct ttgaaatccc caaattgtgg agcccgctgg 2086
   tctttttccc agcgggtttt tttatgcctt tctcctgccc gccctgcctg gacactaaaa 2146
   catcattaaa cttaaaccgt tttgcaacct taacgcagat taagaacctt gtgatggact 2206
   ctctgaccgt ctgcacgcag gat 2229
<210> 2
   <211> 563
   <212> PRT
   <213> Pant oea ananatis
<400> 2
   Met Leu Asn Val Asn Ile Ala Glu Leu Leu Arg Gly Asn Asp Ile Leu
   1               5                   10                  15
   Leu Leu Phe Phe Val Leu Ala Leu Gly Leu Cys Leu Gly Lys Leu Arg
               20                  25                  30
   Phe Gly Ser Ile Gln Leu Gly Asn Ser Ile Gly Val Leu Val Val Ser
           35                  40                  45
   Leu Leu Leu Gly Gln Gln His Phe Ser Met Asn Thr Asp Ala Leu Ser
       50                  55                  60
   Leu Gly Phe Met Leu Phe Ile Phe Cys Val Gly Ile Glu Ala Gly Pro
   65                  70                  75                  80
   Asn Phe Phe Ser Ile Phe Phe Arg Asp Gly Lys Asn Tyr Phe Met Leu
                   85                  90                  95
   Ala Ile Val Met Val Val Ser Ala Leu Leu Leu Ala Leu Gly Phe Gly
               100                 105                 110
   Lys Leu Phe Gly Trp Asp Ile Gly Leu Thr Ala Gly Val Leu Ala Gly
           115                 120                 125
   Ser Met Thr Ser Thr Pro Val Leu Val Gly Ala Gly Asp Thr Leu Arg
       130                 135                 140
   Gln Ser Ile Ser Asp Pro Arg Gln Leu Ser Ile Met Gln Asp Gln Leu
   145                 150                 155                 160
   Ser Leu Gly Tyr Ala Val Thr Tyr Leu Val Gly Leu Val Ser Leu Ile
                   165                 170                 175
   Phe Gly Ala Arg Tyr Leu Pro Arg Leu Gln His Gln Asp Leu Pro Thr
               180                 185                 190
   Cys Ala Gln Gln Ile Ala Arg Glu Arg Gly Leu Asp Ala Asp Ser Gln
           195                 200                 205
   Arg Lys Val Phe Leu Pro Val Ile Arg Ala Tyr Arg Val Gly Pro Glu
       210                 215                 220
   Leu Val Ala Trp Ser Gly Gly Lys Asn Leu Arg Glu Leu Gly Ile Tyr
   225                 230                 235                 240
   Arg Gln Thr Gly Cys Tyr Ile Glu Arg Ile Arg Arg Asn Gly Ile Leu
                   245                 250                 255
   Ala Ser Pro Asp Gly Asp Ala Val Leu Gln Leu Gly Asp Asp Ile Ser
               260                 265                 270
   Leu Val Gly Tyr Pro Asp Ala His Ala Arg Leu Asp Ala Ser Phe Arg
           275                 280                 285
   Asn Gly Lys Glu Val Phe Asp Arg Asp Leu Leu Asp Met Arg Ile Val
       290                 295                 300
   Thr Glu Glu Ile Val Val Lys Asn His Asn Ala Val Asn Lys Arg Leu
   305                 310                 315                 320
   Ser Lys Leu Asn Leu Thr Asp His Gly Cys Phe Leu Asn Arg Val Ile
                   325                 330                 335
   Arg Ser Gln Ile Glu Met Pro Ile Asp Asp Asn Ile Met Leu Asn Lys
               340                 345                 350
   Gly Asp Val Leu Gln Val Ser Gly Glu Ala Arg Arg Val Lys Ser Val
           355                 360                 365
   Ala Asp Arg Ile Gly Phe Val Ala Ile His Ser Gln Met Thr Asp Leu
       370                 375                 380
   Leu Ala Phe Cys Ala Phe Phe Ile Ile Gly Leu Met Val Gly Leu Ile
   385                 390                 395                 400
   Thr Phe Gln Phe Ser Asn Phe Ser Phe Gly Ile Gly Asn Ala Ala Gly
                   405                 410                 415
   Leu Leu Phe Ala Gly Ile Met Leu Gly Phe Leu Arg Ala Asn His Pro
               420                 425                 430
   Thr Phe Gly Tyr Ile Pro Gln Gly Ala Leu Thr Met Val Lys Glu Phe
           435                 440                 445
   Gly Leu Met Val Phe Met Ala Gly Val Gly Leu Ser Ala Gly Ser Gly
       450                 455                 460
   Ile Asp His Gly Ile Ser Gly Asn Gly Ala Leu Met Leu Leu Cys Gly
   465                 470                 475                 480
   Leu Leu Val Ser Leu Leu Pro Val Val Ile Cys Tyr Leu Phe Gly Ala
                   485                 490                 495
   Tyr Val Leu Arg Met Asn Arg Ala Leu Leu Phe Gly Ala Ile Met Gly
               500                 505                 510
   Ala Arg Thr Cys Ala Pro Ala Met Glu Ile Ile Ser Asp Thr Ala Arg
           515                 520                 525
   Ser Asn Ile Pro Ala Leu Gly Tyr Ala Gly Thr Tyr Ala Ile Ala Asn
       530                 535                 540
   Val Leu Leu Thr Leu Ala Gly Thr Leu Ile Val Ile Ile Trp Pro Leu
   545                 550                 555                 560
   Leu Pro Leu
<210> 3
   <211> 1886
   <212> DNA
   <213> Escherichia coli
<220>
   <221> CDS
   <222> (101)..(1783)
<400> 3
   ggtgcgctga atgaatctgc gccctgaatt ctggtaaaaa acattatcgt aaattaccat      60
   ttctttcaac agcttactag taaacaagaa gttagcctcc gtg aat ata aac gtc       115
                                               Val Asn Ile Asn Val
                                               1               5
   gcc gaa ttg tta aat ggg aat tac att ctg tta tta ttt gtg gtc ctc       163
   Ala Glu Leu Leu Asn Gly Asn Tyr Ile Leu Leu Leu Phe Val Val Leu
                   10                  15                  20
   gcg ctt ggg cta tgt ctc gga aag tta cga ctt ggt tcg atc caa ctg       211
   Ala Leu Gly Leu Cys Leu Gly Lys Leu Arg Leu Gly Ser Ile Gln Leu
               25                  30                  35
   ggt aat tcc att ggc gtt tta gtc gta tcg ctg tta tta ggc caa caa       259
   Gly Asn Ser Ile Gly Val Leu Val Val Ser Leu Leu Leu Gly Gln Gln
           40                  45                  50
   cat ttc agc att aac acc gat gcg ctt aat ctt ggc ttt atg ctg ttt       307
   His Phe Ser Ile Asn Thr Asp Ala Leu Asn Leu Gly Phe Met Leu Phe
       55                  60                  65
   att ttc tgc gtc ggg gtc gaa gcc gga ccg aac ttt ttt tcc att ttt       355
   Ile Phe Cys Val Gly Val Glu Ala Gly Pro Asn Phe Phe Ser Ile Phe
   70                  75                  80                  85
   ttt cgc gat ggg aaa aat tac cta atg tta gca ctg gtg atg gtt ggc       403
   Phe Arg Asp Gly Lys Asn Tyr Leu Met Leu Ala Leu Val Met Val Gly
                   90                  95                  100
   agt gcg ctg gtg atc gcc tta ggg tta ggt aag ctg ttt ggc tgg gat       451
   Ser Ala Leu Val Ile Ala Leu Gly Leu Gly Lys Leu Phe Gly Trp Asp
               105                 110                 115
   att ggc ctg acg gcc ggt atg tta gca ggc tct atg acg tcg aca ccg       499
   Ile Gly Leu Thr Ala Gly Met Leu Ala Gly Ser Met Thr Ser Thr Pro
           120                 125                 130
   gtt ctg gtc ggt gct ggc gat aca ctg cgt cat tcc ggc atg gaa agc       547
   Val Leu Val Gly Ala Gly Asp Thr Leu Arg His Ser Gly Met Glu Ser
       135                 140                 145
   agg cag ctc tca ctg gca ctg gat aat ctg agc ctc ggg tat gcc tta       595
   Arg Gln Leu Ser Leu Ala Leu Asp Asn Leu Ser Leu Gly Tyr Ala Leu
   150                 155                 160                 165
   acc tat tta atc ggt ctg gtg agt ttg att gtt ggt gcg cgt tac ttg       643
   Thr Tyr Leu Ile Gly Leu Val Ser Leu Ile Val Gly Ala Arg Tyr Leu
                   170                 175                 180
   ccg aaa ttg cag cat cag gac tta cag acc agc gcc cag caa atc gcc       691
   Pro Lys Leu Gln His Gln Asp Leu Gln Thr Ser Ala Gln Gln Ile Ala
   185 190 195
   cgc gaa cgt ggc ctg gac act gat gcc aac cgt aag gtt tat tta ccg 739
   Arg Glu Arg Gly Leu Asp Thr Asp Ala Asn Arg Lys Val Tyr Leu Pro
   200 205 210
   gtg atc cgc gcc tat cgc gtc ggc ccg gaa ctg gtg gcc tgg acc gac 787
   Val Ile Arg Ala Tyr Arg Val Gly Pro Glu Leu Val Ala Trp Thr Asp
   215 220 225
   ggc aaa aat ctg cgt gaa ctg ggt att tat cga caa acc ggc tgc tac 835
   Gly Lys Asn Leu Arg Glu Leu Gly Ile Tyr Arg Gln Thr Gly Cys Tyr
   230 235 240 245
   att gaa cgt att cga cgt aac ggg att ctg gca aat cca gac ggt gat 883
   Ile Glu Arg Ile Arg Arg Asn Gly Ile Leu Ala Asn Pro Asp Gly Asp
   250 255 260
   gcc gtg cta caa atg ggc gat gaa ata gcg ttg gta ggc tat ccc gac 931
   Ala Val Leu Gln Met Gly Asp Glu Ile Ala Leu Val Gly Tyr Pro Asp
   265 270 275
   gcc cat gcc cga ctc gat ccc agc ttc cgt aac ggt aaa gaa gtt ttc 979
   Ala His Ala Arg Leu Asp Pro Ser Phe Arg Asn Gly Lys Glu Val Phe
   280 285 290
   gat cgt gac ctt ctc gac atg cgt atc gtc act gaa gaa gtg gtc gtt 1027
   Asp Arg Asp Leu Leu Asp Met Arg Ile Val Thr Glu Glu Val Val Val
   295 300 305
   aaa aac cat aac gct gta ggt aaa cgt ctc gca caa ctg aag ttg acc 1075
   Lys Asn His Asn Ala Val Gly Lys Arg Leu Ala Gln Leu Lys Leu Thr
   310 315 320 325
   gat cac ggt tgc ttc ctt aac cgc gtc att cgt agc cag att gag atg 1123
   Asp His Gly Cys Phe Leu Asn Arg Val Ile Arg Ser Gln Ile Glu Met
   330 335 340
   ccg ata gat gac aac gtc gtg ctt aac aaa ggt gac gtt tta caa gtc 1171
   Pro Ile Asp Asp Asn Val Val Leu Asn Lys Gly Asp Val Leu Gln Val
   345 350 355
   agc ggc gat gcc cgc cgc gta aaa acc atc gcc gat cgc atc ggc ttt 1219
   Ser Gly Asp Ala Arg Arg Val Lys Thr Ile Ala Asp Arg Ile Gly Phe
   360 365 370
   atc tcg att cac agc cag gtc act gac ctg ctg gca ttc tgc gcc ttc 1267
   Ile Ser Ile His Ser Gln Val Thr Asp Leu Leu Ala Phe Cys Ala Phe
   375 380 385
   ttt gtt att ggg ctg atg atc ggg atg atc acc ttc cag ttc agc aca 1315
   Phe Val Ile Gly Leu Met Ile Gly Met Ile Thr Phe Gln Phe Ser Thr
   390 395 400 405
   ttc agt ttc ggc atg ggg aac gct gcc ggg ttg tta ttc gcc gga att 1363
   Phe Ser Phe Gly Met Gly Asn Ala Ala Gly Leu Leu Phe Ala Gly Ile
   410 415 420
   atg ctg ggc ttt atg cgt gct aac cac ccg acc ttc ggt tac att ccg 1411
   Met Leu Gly Phe Met Arg Ala Asn His Pro Thr Phe Gly Tyr Ile Pro
   425 430 435
   cag ggt gca tta agc atg gtg aaa gag ttc ggc ttg atg gtg ttt atg 1459
   Gln Gly Ala Leu Ser Met Val Lys Glu Phe Gly Leu Met Val Phe Met
   440 445 450
   gca ggc gtt ggt ctg agc gcc ggt agc ggt att aat aac ggc ctg ggc 1507
   Ala Gly Val Gly Leu Ser Ala Gly Ser Gly Ile Asn Asn Gly Leu Gly
   455 460 465
   gcg att ggc ggt cag atg ttg att gcc gga ttg att gtc agt ctg gtg 1555
   Ala Ile Gly Gly Gln Met Leu Ile Ala Gly Leu Ile Val Ser Leu Val
   470 475 480 485
   ccc gtg gtt atc tgt ttc ttg ttc ggt gct tat gta ttg cga atg aac 1603
   Pro Val Val Ile Cys Phe Leu Phe Gly Ala Tyr Val Leu Arg Met Asn
   490 495 500
   cgc gcg ctg ttg ttc ggc gca atg atg ggc gca cgt acc tgc gcg ccg 1651
   Arg Ala Leu Leu Phe Gly Ala Met Met Gly Ala Arg Thr Cys Ala Pro
   505 510 515
   gca atg gag atc atc agt gat aca gct cgc agt aac atc ccg gcg ctg 1699
   Ala Met Glu Ile Ile Ser Asp Thr Ala Arg Ser Asn Ile Pro Ala Leu
   520 525 530
   ggc tat gcg ggc acc tat gca atc gcc aac gtc ctg ctg acg ctg gca 1747
   Gly Tyr Ala Gly Thr Tyr Ala Ile Ala Asn Val Leu Leu Thr Leu Ala
   535 540 545
   ggg aca atc atc gtc atg gta tgg cca gga tta gga taaaactgaa 1793
   Gly Thr Ile Ile Val Met Val Trp Pro Gly Leu Gly
   550 555 560
   gttgccctga aaatgaaatt tttttgcaca accgcagaac ttttccgcag ggcatcagtc 1853
   ttaattagtg ccactgcttt tctttgatgt ccc 1886
<210> 4
   <211> 561
   <212> PRT
   <213> Escherichia coli
<400> 4
   Val Asn Ile Asn Val Ala Glu Leu Leu Asn Gly Asn Tyr Ile Leu Leu
   1 5 10 15
   Leu Phe Val Val Leu Ala Leu Gly Leu Cys Leu Gly Lys Leu Arg Leu
   20 25 30
   Gly Ser Ile Gln Leu Gly Asn Ser Ile Gly Val Leu Val Val Ser Leu
   35 40 45
   Leu Leu Gly Gln Gln His Phe Ser Ile Asn Thr Asp Ala Leu Asn Leu
   50 55 60
   Gly Phe Met Leu Phe Ile Phe Cys Val Gly Val Glu Ala Gly Pro Asn
   65 70 75 80
   Phe Phe Ser Ile Phe Phe Arg Asp Gly Lys Asn Tyr Leu Met Leu Ala
   85 90 95
   Leu Val Met Val Gly Ser Ala Leu Val Ile Ala Leu Gly Leu Gly Lys
   100 105 110
   Leu Phe Gly Trp Asp Ile Gly Leu Thr Ala Gly Met Leu Ala Gly Ser
   115 120 125
   Met Thr Ser Thr Pro Val Leu Val Gly Ala Gly Asp Thr Leu Arg His
   130 135 140
   Ser Gly Met Glu Ser Arg Gln Leu Ser Leu Ala Leu Asp Asn Leu Ser
   145 150 155 160
   Leu Gly Tyr Ala Leu Thr Tyr Leu Ile Gly Leu Val Ser Leu Ile Val
   165 170 175
   Gly Ala Arg Tyr Leu Pro Lys Leu Gln His Gln Asp Leu Gln Thr Ser
   180 185 190
   Ala Gln Gln Ile Ala Arg Glu Arg Gly Leu Asp Thr Asp Ala Asn Arg
   195 200 205
   Lys Val Tyr Leu Pro Val Ile Arg Ala Tyr Arg Val Gly Pro Glu Leu
   210 215 220
   Val Ala Trp Thr Asp Gly Lys Asn Leu Arg Glu Leu Gly Ile Tyr Arg
   225 230 235 240
   Gln Thr Gly Cys Tyr Ile Glu Arg Ile Arg Arg Asn Gly Ile Leu Ala
   245 250 255
   Asn Pro Asp Gly Asp Ala Val Leu Gln Met Gly Asp Glu Ile Ala Leu
   260 265 270
   Val Gly Tyr Pro Asp Ala His Ala Arg Leu Asp Pro Ser Phe Arg Asn
   275 280 285
   Gly Lys Glu Val Phe Asp Arg Asp Leu Leu Asp Met Arg Ile Val Thr
   290 295 300
   Glu Glu Val Val Val Lys Asn His Asn Ala Val Gly Lys Arg Leu Ala
   305 310 315 320
   Gln Leu Lys Leu Thr Asp His Gly Cys Phe Leu Asn Arg Val Ile Arg
   325 330 335
   Ser Gln Ile Glu Met Pro Ile Asp Asp Asn Val Val Leu Asn Lys Gly
   340 345 350
   Asp Val Leu Gln Val Ser Gly Asp Ala Arg Arg Val Lys Thr Ile Ala
   355 360 365
   Asp Arg Ile Gly Phe Ile Ser Ile His Ser Gln Val Thr Asp Leu Leu
   370 375 380
   Ala Phe Cys Ala Phe Phe Val Ile Gly Leu Met Ile Gly Met Ile Thr
   385 390 395 400
   Phe Gln Phe Ser Thr Phe Ser Phe Gly Met Gly Asn Ala Ala Gly Leu
   405 410 415
   Leu Phe Ala Gly Ile Met Leu Gly Phe Met Arg Ala Asn His Pro Thr
   420 425 430
   Phe Gly Tyr Ile Pro Gln Gly Ala Leu Ser Met Val Lys Glu Phe Gly
   435 440 445
   Leu Met Val Phe Met Ala Gly Val Gly Leu Ser Ala Gly Ser Gly Ile
   450 455 460
   Asn Asn Gly Leu Gly Ala Ile Gly Gly Gln Met Leu Ile Ala Gly Leu
   465 470 475 480
   Ile Val Ser Leu Val Pro Val Val Ile Cys Phe Leu Phe Gly Ala Tyr
   485 490 495
   Val Leu Arg Met Asn Arg Ala Leu Leu Phe Gly Ala Met Met Gly Ala
   500 505 510
   Arg Thr Cys Ala Pro Ala Met Glu Ile Ile Ser Asp Thr Ala Arg Ser
   515 520 525
   Asn Ile Pro Ala Leu Gly Tyr Ala Gly Thr Tyr Ala Ile Ala Asn Val
   530 535 540
   Leu Leu Thr Leu Ala Gly Thr Ile Ile Val Met Val Trp Pro Gly Leu
   545 550 555 560
   Gly
<210> 5
   <211> 563
   <212> PRT
   <213> Artificial Sequence
<220>
   <223> Concensus between E. col i and P. ananatis
<220>
   <221> misc_feature
   <222> (2)..(2)
   <223> Xaa can be any naturally occurring amino acid
<220>
   <221> misc_feature
   <222> (4)..(4)
   <223> Xaa can be any naturally occurring amino acid
<220>
   <221> misc_feature
   <222> (6)..(6)
   <223> Xaa can be any naturally occurring amino acid
<220>
   <221> misc_feature
   <222> (11)..(11)
   <223> Xaa can be any naturally occurring amino acid
<220>
   <221> misc_feature
   <222> (14)..(14)
   <223> Xaa can be any naturally occurring amino acid
<220>
   <221> misc_feature
   <222> (20)..(20)
   <223> Xaa can be any naturally occurring amino acid
<220>
   <221> misc_feature
   <222> (33)..(333)
   <223> Xaa can be any naturally occurring amino acid
<220>
   <221> misc_feature
   <222> (58)..(58)
   <223> Xaa can be any naturally occurring amino acid
<220>
   <221> misc_feature
   <222> (64)..(64)
   <223> Xaa can be any naturally occurring amino acid
<220>
   <221> misc_feature
   <222> (76)..(76)
   <223> Xaa can be any naturally occurring amino acid
<220>
   <221> misc_feature
   <222> (94)..(94)
   <223> Xaa can be any naturaly occurring amino acid
<220>
   <221> misc_feature
   <222> (98)..(98)
   <223> Xaa can be any naturally occurring amino acid
<220>
   <221> misc_feature
   <222> (102)..(102)
   <223> Xaa can be any naturally occurring amino acid
<220>
   <221> misc_feature
   <222> (106)..(107)
   <223> Xaa can be any naturally occurring amino acid
<220>
   <221> misc_feature
   <222> (111)..(111)
   <223> Xaa can be any naturally occurring amino acid
<220>
   <221> misc_feature
   <222> (125)..(125)
   <223> Xaa can be any naturally occurring amino acid
<220>
   <221> misc_feature
   <222> (145)..(145)
   <223> Xaa can be any naturally occurring amino acid
<220>
   <221> misc_feature
   <222> (147)..(150)
   <223> Xaa can be any naturally occurring amino acid
<220>
   <221> misc_feature
   <222> (155)..(157)
   <223> Xaa can be any naturally occurring amino acid
<220>
   <221> m sc_f eat ur e
   <222> (159)..(159)
   <223> Xaa can be any naturally occurring amino acid
<220>
   <221> misc_feature
   <222> (166)..(166)
   <223> Xaa can be any naturally occurring amino acid
<220>
   <221> misc_feature
   <222> (170)..(170)
   <223> Xaa can be any naturally occurring amino acid
<220>
   <221> misc_feature
   <222> (177)..(177)
   <223> Xaa can be any naturally occurring amino acid
<220>
   <221> misc_feature
   <222> (184)..(184)
   <223> Xaa can be any naturally occurring amino acid
<220>
   <221> misc_feature
   <222> (191)..(191)
   <223> Xaa can be any naturally occurring amino acid
<220>
   <221> misc_feature
   <222> (193)..(193)
   <223> Xaa can be any naturally occurring amino acid
<220>
   <221> misc_feature
   <222> (205)..(205)
   <223> Xaa can be any naturally occurring amino acid
<220>
   <221> misc_feature
   <222> (207)..(208)
   <223> Xaa can be any naturally occurring amino acid
<220>
   <221> misc_feature
   <222> (212).. (212)
   <223> Xaa can be any naturally occur ring amino acid
<220>
   <221> misc_feature
   <222> (229)..(230)
   <223> Xaa can be any naturally occurring amino acid
<220>
   <221> misc_feature
   <222> (258).. (258)
   <223> Xaa can be any naturally occurring amino acid
<220>
   <221> misc_feature
   <222> (267).. (267)
   <223> Xaa can be any naturally occurring amino acid
<220>
   <221> misc_feature
   <222> (270).. (270)
   <223> Xaa can be any naturally occurring amino acid
<220>
   <221> misc_feature
   <222> (272)..(272)
   <223> Xaa can be any naturally occurring amino acid
<220>
   <221> misc_feature
   <222> (285)..(285)
   <223> Xaa can be any naturally occurring amino acid
<220>
   <221> misc_feature
   <222> (308)..(308)
   <223> Xaa can be any naturally occurring amino acid
<220>
   <221> misc_feature
   <222> (317)..(317)
   <223> Xaa can be any naturally occurring amino acid
<220>
   <221> misc_feature
   <222> (321)..(322)
   <223> Xaa can be any naturally occurring amino acid
<220>
   <221> misc_feature
   <222> (324)..(324)
   <223> Xaa can be any naturally occurring amino acid
<220>
   <221> misc_feature
   <222> (348)..(349)
   <223> Xaa can be any naturally occur r i ng amino acid
<220>
   <221> misc_feature
   <222> (361)..(361)
   <223> Xaa can be any naturally occurring amino acid
<220>
   <221> misc_feature
   <222> (367)..(368)
   <223> Xaa can be any naturally occurring amino acid
<220>
   <221> misc_feature
   <222> (375)..(376)
   <223> Xaa can be any naturally occur r i ng amino acid
<220>
   <221> misc_feature
   <222> (381)..(381)
   <223> Xaa can be any naturally occurring amino acid
<220>
   <221> misc_feature
   <222> (392)..(392)
   <223> Xaa can be any naturally occurring amino acid
<220>
   <221> misc_feature
   <222> (397)..(397)
   <223> Xaa can be any naturally occurring amino acid
<220>
   <221> misc_feature
   <222> (399)..(399)
   <223> Xaa can be any naturally occur r i ng amino acid
<220>
   <221> misc_feature
   <222> (406)..(406)
   <223> Xaa can be any naturally occur r i ng amino acid
<220>
   <221> misc_feature
   <222> (411)..(411)
   <223> Xaa can be any naturally occurring amino acid
<220>
   <221> misc_feature
   <222> (427)..(427)
   <223> Xaa can be any naturally occurring amino acid
<220>
   <221> misc_feature
   <222> (443)..(443)
   <223> Xaa can be any naturally occurring amino acid
<220>
   <221> misc_feature
   <222> (466)..(467)
   <223> Xaa can be any naturally occurring amino acid
<220>
   <221> misc_feature
   <222> (469)..(472)
   <223> Xaa can be any naturally occurring amino acid
<220>
   <221> misc_feature
   <222> (474)..(475)
   <223> Xaa can be any naturally occurring amino acid
<220>
   <221> misc_feature
   <222> (478)..(479)
   <223> Xaa can be any naturally occurring amino acid
<220>
   <221> misc_feature
   <222> (482)..(482)
   <223> Xaa can be any naturally occurring amino acid
<220>
   <221> misc_feature
   <222> (486)..(486)
   <223> Xaa can be any naturally occurring amino acid
<220>
   <221> misc_feature
   <222> (492)..(492)
   <223> Xaa can be any naturally occurring amino acid
<220>
   <221> misc_feature
   <222> (510)..(510)
   <223> Xaa can be any naturally occurring amino acid
<220>
   <221> misc_feature
   <222> (553)..(553)
   <223> Xaa can be any naturally occurring amino acid
<220>
   <221> misc_feature
   <222> (556)..(557)
   <223> Xaa can be any naturally occurring amino acid
<220>
   <221> misc_feature
   <222> (560)..(560)
   <223> Xaa can be any naturally occurring amino acid
<220>
   <221> misc_feature
   <222> (562)..(562)
   <223> Xaa can be any naturally occurring amino acid
<400> 5
   Met Xaa Asn Xaa Asn Xaa Ala Glu Leu Leu Xaa Gly Asn Xaa Ile Leu
   1               5                   10                  15
   Leu Leu Phe Xaa Val Leu Ala Leu Gly Leu Cys Leu Gly Lys Leu Arg
               20                  25                  30
   Xaa Gly Ser Ile Gln Leu Gly Asn Ser Ile Gly Val Leu Val Val Ser
           35                          40                          45
   Leu Leu Leu Gly Gln Gln His Phe Ser Xaa Asn Thr Asp Ala Leu Xaa
       50                      55                      60
   Leu Gly Phe Met Leu Phe Ile Phe Cys Val Gly Xaa Glu Ala Gly Pro
   65                  70                      75                  80
   Asn Phe Phe Ser Ile Phe Phe Arg Asp Gly Lys Asn Tyr Xaa Met Leu
                   85                      90                      95
   Ala Xaa Val Met Val Xaa Ser Ala Leu Xaa Xaa Ala Leu Gly Xaa Gly
               100                     105                     110
   Lys Leu Phe Gly Trp Asp Ile Gly Leu Thr Ala Gly Xaa Leu Ala Gly
           115                     120                     125
   Ser Met Thr Ser Thr Pro Val Leu Val Gly Ala Gly Asp Thr Leu Arg
       130                     135                     140
   Xaa Ser Xaa Xaa Xaa Xaa Arg Gln Leu Ser Xaa Xaa Xaa Asp Xaa Leu
   145                 150                     155                     160
   Ser Leu Gly Tyr Ala Xaa Thr Tyr Leu Xaa Gly Leu Val Ser Leu Ile
                   165                     170                     175
   Xaa Gly Ala Arg Tyr Leu Pro Xaa Leu Gln His Gln Asp Leu Xaa Thr
               180                     185                     190
   Xaa Ala Gln Gln Ile Ala Arg Glu Arg Gly Leu Asp Xaa Asp Xaa Xaa
           195                     200                     205
   Arg Lys Val Xaa Leu Pro Val Ile Arg Ala Tyr Arg Val Gly Pro Glu
       210                     215                     220
   Leu Val Ala Trp Xaa Xaa Gly Lys Asn Leu Arg Glu Leu Gly Ile Tyr
   225                 230                     235                     240
   Arg Gln Thr Gly Cys Tyr Ile Glu Arg Ile Arg Arg Asn Gly Ile Leu
                   245                     250                     255
   Ala Xaa Pro Asp Gly Asp Ala Val Leu Gln Xaa Gly Asp Xaa Ile Xaa
               260                     265                     270
   Leu Val Gly Tyr Pro Asp Ala His Ala Arg Leu Asp Xaa Ser Phe Arg
           275                     280                     285
   Asn Gly Lys Glu Val Phe Asp Arg Asp Leu Leu Asp Met Arg Ile Val
       290                     295                     300
   Thr Glu Glu Xaa Val Val Lys Asn His Asn Ala Val Xaa Lys Arg Leu
   305                 310                     315                     320
   Xaa Xaa Leu Xaa Leu Thr Asp His Gly Cys Phe Leu Asn Arg Val Ile
                   325                     330                     335
   Arg Ser Gln Ile Glu Met Pro Ile Asp Asp Asn Xaa Xaa Leu Asn Lys
               340                     345                     350
   Gly Asp Val Leu Gln Val Ser Gly Xaa Ala Arg Arg Val Lys Xaa Xaa
           355                     360                     365
   Ala Asp Arg Ile Gly Phe Xaa Xaa Ile His Ser Gln Xaa Thr Asp Leu
       370                     375                     380
   Leu Ala Phe Cys Ala Phe Phe Xaa Ile Gly Leu Met Xaa Gly Xaa Ile
   385                 390                     395                     400
   Thr Phe Gln Phe Ser Xaa Phe Ser Phe Gly Xaa Gly Asn Ala Ala Gly
                   405                     410                     415
   Leu Leu Phe Ala Gly Ile Met Leu Gly Phe Xaa Arg Ala Asn His Pro
               420                     425                     430
   Thr Phe Gly Tyr Ile Pro Gln Gly Ala Leu Xaa Met Val Lys Glu Phe
           435                     440                     445
   Gly Leu Met Val Phe Met Ala Gly Val Gly Leu Ser Ala Gly Ser Gly
       450                     455                     460
   Ile Xaa Xaa Gly Xaa Xaa Xaa Xaa Gly Xaa Xaa Met Leu Xaa Xaa Gly
   465                 470                     475                     480
   Leu Xaa Val Ser Leu Xaa Pro Val Val Ile Cys Xaa Leu Phe Gly Ala
                   485                     490                     495
   Tyr Val Leu Arg Met Asn Arg Ala Leu Leu Phe Gly Ala Xaa Met Gly
               500                     505                     510
   Ala Arg Thr Cys Ala Pro Ala Met Glu Ile Ile Ser Asp Thr Ala Arg
           515                     520                     525
   Ser Asn Ile Pro Ala Leu Gly Tyr Ala Gly Thr Tyr Ala Ile Ala Asn
       530                     535                     540
   Val Leu Leu Thr Leu Ala Gly Thr Xaa Ile Val Xaa Xaa Trp Pro Xaa
   545                 550                     555                     560
   Leu Xaa Leu
<210> 6
   <211> 80
   <212> DNA
   <213> Artificial Sequence
<220>
   <223> primer
<400> 6
   agcggcgt at t cgcct ct at caaacat t aa ct gat agcga agat ct aaat t gaagcct gc 60
   t t t t t t at ac t aagt t ggca 80
<210> 7
   <211> 80
   <212> DNA
   <213> Artificial Sequence
<220>
   <223> primer
<400> 7
   ccaccagat a acct t ccggt agcgt t aaaa cgt cacgaca t t caat agaa cgct caagt t 60
   agt at aaaaa agct gaacga 80
<210> 8
   <211> 45
   <212> DNA
   <213> Artificial Sequence
<220>
   <223> primer
<400> 8
   gatcggtacc cagacatcag tgatagctgg aggt aat ct t acggc 45
<210> 9
   <211> 50
   <212> DNA
   <213> Artificial Sequence
<220>
   <223> primer
<400> 9
   gat cgcat gc gacgt cagt a aaaaaat t t t at aagggcaa t aacggccag 50
<210> 10
   <211> 16214
   <212> DNA
   <213> Artificial Sequence
<220>
   <223> RSFCPG
<400> 10
   gaattccgcc agaaccttca tcagcagcat aaacaggtgc agtgaacagc agagatacgg    60
   ccagtgcggc caatgttttt tgtcctttaa acataacaga gtcctttaag gatatagaat   120
   aggggtatag ctacgccaga atatcgtatt tgattattgc tagtttttag ttttgcttaa   180
   aaaatattgt tagttttatt aaattggaaa actaaattat tggtatcatg aattgttgta   240
   tgatgataaa tatagggggg atatgataga cgtcattttc atagggttat aaaatgcgac   300
   taccatgaag tttttaattc aaagtattgg gttgctgata atttgagctg ttctattctt   360
   tttaaatatc tatataggtc tgttaatgga ttttattttt acaagttttt tgtgtttagg   420
   catataaaaa tcaagcccgc catatgaacg gcgggttaaa atatttacaa cttagcaatc   480
   gaaccattaa cgcttgatat cgcttttaaa gtcgcgtttt tcatatcctg tatacagctg   540
   acgcggacgg gcaatcttca taccgtcact gtgcatttcg ctccagtggg cgatccagcc   600
   aacggtacgt gccattgcga aaatgacggt gaacatggaa gacggaatac ccatcgcttt 660
   caggatgata ccagagtaga aatcgacgtt cgggtacagt ttcttctcga taaagtacgg 720
   gtcgttcagc gcgatgtttt ccagctccat agccacttcc agcaggtcat ccttcgtgcc 780
   cagctctttc agcacttcat ggcaggtttc acgcattacg gtggcgcgcg ggtcgtaatt 840
   tttgtacacg cggtgaccga agcccatcag gcggaaagaa tcatttttgt ctttcgcacg 900
   acgaaaaaat tccggaatgt gtttaacgga gctgatttct tccagcattt tcagcgccgc 960
   ttcgttagca ccgccgtgcg caggtcccca cagtgaagca atacctgctg cgatacaggc 1020
   aaacgggttc gcacccgaag agccagcggt acgcacggtg gaggtagagg cgttctgttc 1080
   atggtcagcg tgcaggatca gaatacggtc catagcacgt tccagaatcg gattaacttc 1140
   atacggttcg cacggcgtgg agaacatcat attcaggaag ttaccggcgt aggagagatc 1200
   gttgcgcggg taaacaaatg gctgaccaat ggaatacttg taacacatcg cggccatggt 1260
   cggcattttc gacagcaggc ggaacgcggc aatttcacgg tgacgaggat tgttaacatc 1320
   cagcgagtcg tgatagaacg ccgccagcgc gccggtaata ccacacatga ctgccattgg 1380
   atgcgagtcg cgacggaaag catggaacag acgggtaatc tgctcgtgga tcatggtatg 1440
   acgggtcacc gtagttttaa attcgtcata ctgttcctga gtcggttttt caccattcag 1500
   caggatgtaa caaacttcca ggtagttaga atcggtcgcc agctgatcga tcgggaaacc 1560
   gcggtgcagc aaaatacctt catcaccatc aataaaagta attttagatt cgcaggatgc 1620
   ggttgaagtg aagcctgggt caaaggtgaa cacacctttt gaaccgagag tacggatatc 1680
   aataacatct tgacccagcg tgcctttcag cacatccagt tcaacagctg tatccccgtt 1740
   gagggtgagt tttgcttttg tatcagccat ttaaggtctc cttagcgcct tattgcgtaa 1800
   gactgccgga acttaaattt gccttcgcac atcaacctgg ctttacccgt tttttatttg 1860
   gctcgccgct ctgtgaaaga ggggaaaacc tgggtacaga gctctgggcg cttgcaggta 1920
   aaggatccat tgatgacgaa taaatggcga atcaagtact tagcaatccg aattattaaa 1980
   cttgtctacc actaataact gtcccgaatg aattggtcaa tactccacac tgttacataa 2040
   gttaatctta ggtgaaatac cgacttcata acttttacgc attatatgct tttcctggta 2100
   atgtttgtaa caactttgtt gaatgattgt caaattagat gattaaaaat taaataaatg 2160
   ttgttatcgt gacctggatc actgttcagg ataaaacccg acaaactata tgtaggttaa 2220
   ttgtaatgat tttgtgaaca gcctatactg ccgccagtct ccggaacacc ctgcaatccc 2280
   gagccaccca gcgttgtaac gtgtcgtttt cgcatctgga agcagtgttt tgcatgacgc 2340
   gcagttatag aaaggacgct gtctgacccg caagcagacc ggaggaagga aatcccgacg 2400
   tcggggatcc tctagagctt tagcgtctga ggttatcgca atttggttat gagattactc 2460
   tcgttattaa tttgctttcc tgggtcattt ttttcttgct taccgtcaca ttcttgatgg 2520
   tatagtcgaa aactgcaaaa gcacatgaca taaacaacat aagcacaatc gtattaatat 2580
   ataagggttt tatatctatg gatcagacat attctctgga gtcattcctc aaccatgtcc 2640
   aaaagcgcga cccgaatcaa accgagttcg cgcaagccgt tcgtgaagta atgaccacac 2700
   tctggccttt tcttgaacaa aatccaaaat atcgccagat gtcattactg gagcgtctgg 2760
   ttgaaccgga gcgcgtgatc cagtttcgcg tggtatgggt tgatgatcgc aaccagatac 2820
   aggtcaaccg tgcatggcgt gtgcagttca gctctgccat cggcccgtac aaaggcggta 2880
   tgcgcttcca tccgtcagtt aacctttcca ttctcaaatt cctcggcttt gaacaaacct 2940
   tcaaaaatgc cctgactact ctgccgatgg gcggtggtaa aggcggcagc gatttcgatc 3000
   cgaaaggaaa aagcgaaggt gaagtgatgc gtttttgcca ggcgctgatg actgaactgt 3060
   atcgccacct gggcgcggat accgacgttc cggcaggtga tatcggggtt ggtggtcgtg 3120
   aagtcggctt tatggcgggg atgatgaaaa agctctccaa caataccgcc tgcgtcttca 3180
   ccggtaaggg cctttcattt ggcggcagtc ttattcgccc ggaagctacc ggctacggtc 3240
   tggtttattt cacagaagca atgctaaaac gccacggtat gggttttgaa gggatgcgcg 3300
   tttccgtttc tggctccggc aacgtcgccc agtacgctat cgaaaaagcg atggaatttg 3360
   gtgctcgtgt gatcactgcg tcagactcca gcggcactgt agttgatgaa agcggattca 3420
   cgaaagagaa actggcacgt cttatcgaaa tcaaagccag ccgcgatggt cgagtggcag 3480
   attacgccaa agaatttggt ctggtctatc tcgaaggcca acagccgtgg tctctaccgg 3540
   ttgatatcgc cctgccttgc gccacccaga atgaactgga tgttgacgcc gcgcatcagc 3600
   ttatcgctaa tggcgttaaa gccgtcgccg aaggggcaaa tatgccgacc accatcgaag 3660
   cgactgaact gttccagcag gcaggcgtac tatttgcacc gggtaaagcg gctaatgctg 3720
   gtggcgtcgc tacatcgggc ctggaaatgc cacaaaacgc tgcgcgcctg ggctggaaag 3780
   ccgagaaagt tgacgcacgt ttgcatcaca tcatgctgga tatccaccat gcctgtgttg 3840
   agcatggtgg tgaaggtgag caaaccaact acgtgcaggg cgcgaacatt gccggttttg 3900
   tgaaggttgc cgatgcgatg ctggcgcagg gtgtgattta agttgtaaat gcctgatggc 3960
   gctacgctta tcaggcctac aaatgggcac aattcattgc agttacgctc taatgtaggc 4020
   cgggcaagcg cagcgccccc ggcaaaattt caggcgttta tgagtattta acggatgatg 4080
   ctccccacgg aacatttctt atgggccaac ggcatttctt actgtagtgc tcccaaaact 4140
   gcttgtcgta acgataacac gcttcaagtt cagcatccgt taactttctg cggactcacg 4200
   cgcgcagcac tatgccagta aagaaatccc atttgactat ttttttgata atcttcttcg 4260
   ctttcgaaca actcgtgcgc ctttcgagaa gctagagtcg actcgccaat caccagcact 4320
   aaagtgcgcg gttcgttacc cgattcatct ttgaaattag ccagtggcgg caaggcatta 4380
   ttttcattca gtaactttgt tagcgagttt agttgctgac gatactgata atagccggtc 4440
   aggaattgcc acggtgcggc aggctccata cgcgaggcca ggttatccaa cgttttctca 4500
   aacggcttgt ttttgataaa cgtattcatg gcgatcggat gcagaatcaa gccataaagc 4560
   agggcaaaag agacaacata acgccacggc tttggaatat agaccgggcg caggcgtgtc 4620
   cacagcagaa ctgccaccgc cgtataggcc agcgcgataa gcacaatttt caggctgaaa 4680
   tactggctta aatactcgct ggcttcgttg gtgttggttt cgaacatcac aaacagaacg 4740
   ctctgcgaga actcctgacc gtagatgacg tagtagcaca gcgccgccag agaggccgcc 4800
   catagcacca cgccgattac tgcggcaata attttaatcc gcttcggaaa gaggaatacc 4860
   gggatcaacc acagcgaact gaataacagc gagtcgcgaa tgccgttagt gccactataa 4920
   ccactgatgt aaataatggc ctgtagcaga gtagagaaaa accaaaagta gagcagtgcc 4980
   caacccaggg ctttccagct aaaaagaggt ttagcctgga cttctgtgga atgcatagta 5040
   agaacctgtc ttgaaaaaat atcgccgaat gtaacgacaa ttccttaagg atatctgaag 5100
   gtatattcag aatttgaata aaatgcagac agaaatatat tgaaaacgag ggtgttagaa 5160
   cagaagtatt tcagaaaacc ctcgcgcaaa agcacgaggg tttgcagaag aggaagatta 5220
   gccggtatta cgcatacctg ccgcaatccc ggcaatagtg accattaacg cttgttcgac 5280
   gcgaggatcc ggttcctggc cttctttttc tgcctggcgg gagcggtgca gcaactcggc 5340
   ctgcaatacg ttcagcgggt cggtgtaaat attccgtagc tgaatagact ctgcaatcca 5400
   cggcagatcg gccatcagat gggaatcgtt ggcaatcgcc agcaccactt tgatgtcttc 5460
   ttcttgcagg ttgcgtaact ctttacctaa cggccacagt gctttgtcta ccaggcgttg 5520
   gtcatagtat tccgccagcc acaggtctgc tttggcgaag accatctcca gcatgccgag 5580
   acgcgtcgag aagaatggcc aatcgcggca catagcctcc agctcgctct gtttgccgtc 5640
   ttcgaccact ttttgcagcg ccgtacctgc acccagccag gcggggagca tcagacggtt 5700
   ttgcgtccag gcgaagatcc acggaatggc gcgtagtgac tcgacgccgc cggttgggcg 5760
   acgtttcgcc ggacgtgaac ccaacggcag tttgcccagt tcttgttccg gcgtagcgga 5820
   gcggaagtaa ggcacaaaat ctttgttttc acgtacgtag ccgcggtaga catcgcagga 5880
   gatgactgac agttcatcca taatgcgacg ccagctctct ttcggctccg gcggtggcag 5940
   caggttggct tccagaatcg ccccggtata aagcgacagg ctgctgacgg tgatttctgg 6000
   cagaccatat ttaaagcgga tcatctcgcc ctgttcggtt acgcgcaggc cgcctttcag 6060
   gcttcctggc ggttgtgaca gcagcgccgc atgagcaggt gcgccgccgc gaccaatgga 6120
   accgccgcga ccgtggaaca acgtcagctc aatacccgct ttttcgcagg ttttgattaa 6180
   tgcatcctgt gcctgatatt gcgcccagga agctgccatc actcccgcat cttttgctga 6240
   gtcggaatag ccaatcatca ccatctgttt gccctgaatc aggccacgat accagtcaat 6300
   attgagcagc tgggtcatga catcgttggc gttgttcaga tcatcgaggg tttcaaacag 6360
   cggagcaacc ggcatcgcaa acccgatacc cgcttctttc agcagcaggt ggacagccag 6420
   tacgtcggac ggcgttttcg ccatcgagat cacgtaggcg gcaatggagc cttgcggtgc 6480
   ttcggcaatc acctggcagg tatcgagcac ttcgcgcgtt tcggcgcttg gttgccagtt 6540
   gcgcggcaga agcggacgtt tggagttcag ttcgcggatc aggaacgcct gtttgtcggc 6600
   ctctgaccag ctttcgtagt cgccgatacc gaggtagcgg gtcagctcgc ccagcgcttc 6660
   ggtatgacgc gtgctctcct gacggatatc aatacggacc agcggtacgc cgaaacattt 6720
   cacgcggcgc agggtgtcga gcagatcgcc gttggcgata atacccatgc cacacgcctg 6780
   aagtgactgg tagcaagcgt agagcggttc ccacagttct tcgttttgtg tcagcaggcc 6840
   ttctggtttt ggcagttctt cgcctttcag gcgcgcttcc agccatgcct gtgtcgccat 6900
   caggcgagaa cgcaggtttt tcatcagata gcgatacggt tctgcggcac cttcttcgcc 6960
   aaccagcgcc agcagttcag ggtcgcttcc aaccatcgac agttcagaaa ccagcacctg 7020
   aatatctttc aggaacaaat cggtggcttt ccagcggctg agtagcagga cgtggcgggt 7080
   gatatcggca gtgacgttcg ggttgccgtc gcggtcgccg cccatccacg aagtaaaacg 7140
   gaccggaaca aattcgacgg gcagtttgta gccgaggttc tcttccagtt gttcgttcag 7200
   ttcgcgcagg taatttggta cgccttgcca caggctgttt tccactacgg caaagcccca 7260
   tttggcttca tctaccgggc ttggacgcag cttacggatt tcatcggtat gccatgactg 7320
   ggcgatcaac tggcgcaggc gacgcatcag ctggttgtgt tcgtagtcag cgatatcttt 7380
   gttatcgagc tgttttaaac aggcgttcac ttccaccatt ttgtggatca gtgtacgacg 7440
   ggtaatttcg gttgggtgag ccgtgaggac cagttccagc gacagcgatt ccactgcttt 7500
   tttgatggtg tcttcgctca gttccggctg gtttttcagt ttacgcaggg tgcgggcgat 7560
   cacttccggg ttgctggcag cttcgccttt cggcgaaatg ctgtggtatt gctcggcggt 7620
   gttggccagg ttcaggaact gactaaacgc acgcgcaacg ggcagcagct cgtcgttcga 7680
   caaattttgt aaggtggtga gcaactcctg gcggttagca tcattgccag cgcgtgaaga 7740
   tttcgacaac ttacggatag tttctacgcg ttcaagaatg tgttctccca acgcatcctt 7800
   gatggtttct cccagcactt tgccgagcat actgacatta ctacgcaatg cggaatattg 7860
   ttcgttcata ttaccccaga caccccatct tatcgtttga tagccctgta tccttcacgt 7920
   cgcattggcg cgaatatgct cgggctttgc ttttcgtcgt cttttataaa gccacgtaaa 7980
   agcggtgacg tcaaatgctg cgaaatcgct tcagcaaacg aataaatagc aggaatttac 8040
   gtcattaaat tcacgacgct ttaaataagc gtaacttatg gaaatgttaa aaaatcgccc 8100
   caagtaacac caaaggtgta ggtcggataa gatgcgcaag tatcgcatcc gacattattg 8160
   cggcactgga gtttggcaac agtgccggat gcggcgcgag cgccttatcc ggcctacagt 8220
   tgggcatcgt ttgagtcact gtcggtcgga taagatgcgc aagtatcgca tccgacatta 8280
   ttgcggcact ggagtttggc aacagtgccg gatgcggcgc gagcgcctta tccggcctac 8340
   ggttgggcat cgtttgagtc actgtaggtc ggataagatg cgcaagcatc gcatccgaca 8400
   ttattgcggc actggagttt ggcaacagcg ccggatgcgg cgcgagcgcc ttatccggcc 8460
   tacgttttaa tgccagcaaa aatggtgaat tacctgggtt atcagttcgc gggtgggctt 8520
   gataaaccgt gtttccagat attcatcagg ttgatgagcc tgattaattg agccaggccc 8580
   caacaccagc gtcgggcata acgtttgaat aaacggcgct tcggtacagt agttcaccac 8640
   ttcggttttt gctccgagca atttctcaac cacttcaacc agttgatgat tcggtgggca 8700
   ttcatagcca gggatcggcg gatgcagctc gtcgacctgc aggagcagaa gagcatacat 8760
   ctggaagcaa agccaggaaa gcggcctatg gagctgtgcg gcagcgctca gtaggcaatt 8820
   tttcaaaata ttgttaagcc ttttctgagc atggtatttt tcatggtatt accaattagc 8880
   aggaaaataa gccattgaat ataaaagata aaaatgtctt gtttacaata gagtgggggg 8940
   ggtcagcctg ccgccttggg ccgggtgatg tcgtacttgc ccgccgcgaa ctcggttacc 9000
   gtccagccca gcgcgaccag ctccggcaac gcctcgcgca cccgctggcg gcgcttgcgc 9060
   atggtcgaac cactggcctc tgacggccag acatagccgc acaaggtatc tatggaagcc 9120
   ttgccggttt tgccggggtc gatccagcca cacagccgct ggtgcagcag gcgggcggtt 9180
   tcgctgtcca gcgcccgcac ctcgtccatg ctgatgcgca catgctggcc gccacccatg 9240
   acggcctgcg cgatcaaggg gttcagggcc acgtacaggc gcccgtccgc ctcgtcgctg 9300
   gcgtactccg acagcagccg aaacccctgc cgcttgcggc cattctgggc gatgatggat 9360
   accttccaaa ggcgctcgat gcagtcctgt atgtgcttga gcgccccacc actatcgacc 9420
   tctgccccga tttcctttgc cagcgcccga tagctacctt tgaccacatg gcattcagcg 9480
   gtgacggcct cccacttggg ttccaggaac agccggagct gccgtccgcc ttcggtcttg 9540
   ggttccgggc caagcactag gccattaggc ccagccatgg ccaccagccc ttgcaggatg 9600
   cgcagatcat cagcgcccag cggctccggg ccgctgaact cgatccgctt gccgtcgccg 9660
   tagtcatacg tcacgtccag cttgctgcgc ttgcgctcgc cccgcttgag ggcacggaac 9720
   aggccggggg ccagacagtg cgccgggtcg tgccggacgt ggctgaggct gtgcttgttc 9780
   ttaggcttca ccacggggca cccccttgct cttgcgctgc ctctccagca cggcgggctt 9840
   gagcaccccg ccgtcatgcc gcctgaacca ccgatcagcg aacggtgcgc catagttggc 9900
   cttgctcaca ccgaagcgga cgaagaaccg gcgctggtcg tcgtccacac cccattcctc 9960
   ggcctcggcg ctggtcatgc tcgacaggta ggactgccag cggatgttat cgaccagtac 10020
   cgagctgccc cggctggcct gctgctggtc gcctgcgccc atcatggccg cgcccttgct 10080
   ggcatggtgc aggaacacga tagagcaccc ggtatcggcg gcgatggcct ccatgcgacc 10140
   gatgacctgg gccatggggc cgctggcgtt ttcttcctcg atgtggaacc ggcgcagcgt 10200
   gtccagcacc atcaggcggc ggccctcggc ggcgcgcttg aggccgtcga accactccgg 10260
   ggccatgatg ttgggcaggc tgccgatcag cggctggatc agcaggccgt cagccacggc 10320
   ttgccgttcc tcggcgctga ggtgcgcccc aagggcgtgc aggcggtgat gaatggcggt 10380
   gggcgggtct tcggcgggca ggtagatcac cgggccggtg ggcagttcgc ccacctccag 10440
   cagatccggc ccgcctgcaa tctgtgcggc cagttgcagg gccagcatgg atttaccggc 10500
   accaccgggc gacaccagcg ccccgaccgt accggccacc atgttgggca aaacgtagtc 10560
   cagcggtggc ggcgctgctg cgaacgcctc cagaatattg ataggcttat gggtagccat 10620
   tgattgcctc ctttgcaggc agttggtggt taggcgctgg cggggtcact acccccgccc 10680
   tgcgccgctc tgagttcttc caggcactcg cgcagcgcct cgtattcgtc gtcggtcagc 10740
   cagaacttgc gctgacgcat ccctttggcc ttcatgcgct cggcatatcg cgcttggcgt 10800
   acagcgtcag ggctggccag caggtcgccg gtctgcttgt ccttttggtc tttcatatca 10860
   gtcaccgaga aacttgccgg ggccgaaagg cttgtcttcg cggaacaagg acaaggtgca 10920
   gccgtcaagg ttaaggctgg ccatatcagc gactgaaaag cggccagcct cggccttgtt 10980
   tgacgtataa ccaaagccac cgggcaacca atagcccttg tcacttttga tcaggtagac 11040
   cgaccctgaa gcgctttttt cgtattccat aaaaccccct tctgtgcgtg agtactcata 11100
   gtataacagg cgtgagtacc aacgcaagca ctacatgctg aaatctggcc cgcccctgtc 11160
   catgcctcgc tggcggggtg ccggtgcccg tgccagctcg gcccgcgcaa gctggacgct 11220
   gggcagaccc atgaccttgc tgacggtgcg ctcgatgtaa tccgcttcgt ggccgggctt 11280
   gcgctctgcc agcgctgggc tggcctcggc catggccttg ccgatttcct cggcactgcg 11340
   gccccggctg gccagcttct gcgcggcgat aaagtcgcac ttgctgaggt catcaccgaa 11400
   gcgcttgacc agcccggcca tctcgctgcg gtactcgtcc agcgccgtgc gccggtggcg 11460
   gctaagctgc cgctcgggca gttcgaggct ggccagcctg cgggccttct cctgctgccg 11520
   ctgggcctgc tcgatctgct ggccagcctg ctgcaccagc gccgggccag cggtggcggt 11580
   cttgcccttg gattcacgca gcagcaccca cggctgataa ccggcgcggg tggtgtgctt 11640
   gtccttgcgg ttggtgaagc ccgccaagcg gccatagtgg cggctgtcgg cgctggccgg 11700
   gtcggcgtcg tactcgctgg ccagcgtccg ggcaatctgc ccccgaagtt caccgcctgc 11760
   ggcgtcggcc accttgaccc atgcctgata gttcttcggg ctggtttcca ctaccagggc 11820
   aggctcccgg ccctcggctt tcatgtcatc caggtcaaac tcgctgaggt cgtccaccag 11880
   caccagacca tgccgctcct gctcggcggg cctgatatac acgtcattgc cctgggcatt 11940
   catccgcttg agccatggcg tgttctggag cacttcggcg gctgaccatt cccggttcat 12000
   catctggccg gtggtggcgt ccctgacgcc gatatcgaag cgctcacagc ccatggcctt 12060
   gagctgtcgg cctatggcct gcaaagtcct gtcgttcttc atcgggccac caagcgcagc 12120
   cagatcgagc cgtcctcggt tgtcagtggc gtcaggtcga gcaagagcaa cgatgcgatc 12180
   agcagcacca ccgtaggcat catggaagcc agcatcacgg ttagccatag cttccagtgc 12240
   cacccccgcg acgcgctccg ggcgctctgc gcggcgctgc tcacctcggc ggctacctcc 12300
   cgcaactctt tggccagctc cacccatgcc gcccctgtct ggcgctgggc tttcagccac 12360
   tccgccgcct gcgcctcgct ggcctgctgg gtctggctca tgacctgccg ggcttcgtcg 12420
   gccagtgtcg ccatgctctg ggccagcggt tcgatctgct ccgctaactc gttgatgcct 12480
   ctggatttct tcactctgtc gattgcgttc atggtctatt gcctcccggt attcctgtaa 12540
   gtcgatgatc tgggcgttgg cggtgtcgat gttcagggcc acgtctgccc ggtcggtgcg 12600
   gatgccccgg ccttccatct ccaccacgtt cggccccagg tgaacaccgg gcaggcgctc 12660
   gatgccctgc gcctcaagtg ttctgtggtc aatgcgggcg tcgtggccag cccgctctaa 12720
   tgcccggttg gcatggtcgg cccatgcctc gcgggtctgc tcaagccatg ccttgggctt 12780
   gagcgcttcg gtcttctgtg ccccgccctt ctccggggtc ttgccgttgt accgcttgaa 12840
   ccactgagcg gcgggccgct cgatgccgtc attgatccgc tcggagatca tcaggtggca 12900
   gtgcgggttc tcgccgccac cggcatggat ggccagcgta tacggcaggc gctcggcacc 12960
   ggtcaggtgc tgggcgaact cggacgccag cgccttctgc tggtcgaggg tcagctcgac 13020
   cggcagggca aattcgacct ccttgaacag ccgcccattg gcgcgttcat acaggtcggc 13080
   agcat cccag t agt cggcgg gccgct cgac gaact ccggc at gt gcccgg at t cggcgt g 13140
   caagact t ca t ccat gt cgc gggcat act t gcct t cgcgc t ggat gt agt cggcct t ggc 13200
   cct ggccgat t ggccgcccg acct gct gcc ggt t t t cgcc gt aaggt gat aaat cgccat 13260
   gctgcctcgc tgttgctttt gcttttcggc tccatgcaat ggccctcgga gagcgcaccg 13320
   cccgaagggt ggccgttagg ccagtttctc gaagagaaac cggtaagtgc gccctcccct 13380
   acaaagtagg gtcgggattg ccgccgctgt gcctccatga tagcctacga gacagcacat 13440
   taacaatggg gtgtcaagat ggttaagggg agcaacaagg cggcggatcg gctggccaag 13500
   ctcgaagaac aacgagcgcg aatcaatgcc gaaattcagc gggtgcgggc aagggaacag 13560
   cagcaagagc gcaagaacga aacaaggcgc aaggtgctgg tgggggccat gattttggcc 13620
   aaggtgaaca gcagcgagtg gccggaggat cggctcatgg cggcaatgga tgcgtacctt 13680
   gaacgcgacc acgaccgcgc cttgttcggt ctgccgccac gccagaagga tgagccgggc 13740
   tgaatgatcg accgagacag gccctgcggg gctgcacacg cgcccccacc cttcgggtag 13800
   ggggaaaggc cgctaaagcg gctaaaagcg ctccagcgta tttctgcggg gtttggtgtg 13860
   gggt t t agcg ggct t t gccc gcct t t cccc ct gccgcgca gcggt ggggc ggt gt gt agc 13920
   ctagcgcagc gaatagacca gctatccggc ctctggccgg gcatattggg caagggcagc 13980
   agcgccccac aagggcgctg ataaccgcgc ctagtggatt attcttagat aatcatggat 14040
   ggatttttcc aacaccccgc cagcccccgc ccctgctggg tttgcaggtt tgggggcgtg 14100
   acagt t at t g caggggt t cg t gacagt t at t gcagggggg cgt gacagt t at t gcagggg 14160
   ttcgtgacag ttagtacggg agtgacgggc actggctggc aatgtctagc aacggcaggc 14220
   at t t cggct g agggt aaaag aact t t ccgc t aagcgat ag act gt at gt a aacacagt at 14280
   tgcaaggacg cggaacatgc ctcatgtggc ggccaggacg gccagccggg atcgggatac 14340
   tggtcgttac cagagccacc gacccgagca aacccttctc tatcagatcg ttgacgagta 14400
   ttacccggca ttcgctgcgc ttatggcaga gcagggaaag gaattgccgg gctatgtgca 14460
   acgggaat t t gaagaat t t c t ccaat gcgg gcggct ggag cat ggct t t c t acgggt t cg 14520
   ct gcgagt ct t gccacgccg agcacct ggt cgct t t cagc t gt aagcgt c gcggt t t ct g 14580
   cccgagctgt ggggcgcggc ggatggccga aagtgccgcc ttgctggttg atgaagtact 14640
   gcct gaacaa cccat gcgt c agt gggt gt t gagct t cccg t t t cagct gc gt t t cct gt t 14700
   t ggggt cgt t t gcgggaagg ggcggaat cc t acgct aagg ct t t ggccag cgat at t ct c 14760
   cggt gagat t gat gt gt t cc caggggat ag gagaagt cgc t t gat at ct a gt at gacgt c 14820
   tgtcgcacct gcttgatcgc ggcccaaggg ttggtttgcg cattcacagt tctccgcaag 14880
   aat t gat t gg ct ccaat t ct t ggagt ggt g aat ccgt t ag cgaggt gccg ccggct t cca 14940
   ttcaggtcga ggtggcccgg ctccatgcac cgcgacgcaa cgcggggagg cagacaaggt 15000
   at agggcggc gcct acaat c cat gccaacc cgt t ccat gt gct cgccgag gcggcat aaa 15060
   t cgccgt gac gat cagcggt ccagt gat cg aagt t aggct ggt aagagcc gcgagcgat c 15120
   ct t gaagct g t ccct gat gg t cgt cat ct a cct gcct gga cagcat ggcc t gcaacgcgg 15180
   gcatcccgat gccgccggaa gcgagaagaa tcataatggg gaaggccatc cagcctcgcg 15240
   tcgcgaacgc cagcaagacg tagcccagcg cgtcggccgc catgccggcg ataatggcct 15300
   gct t ct cgcc gaaacgt t t g gt ggcgggac cagt gacgaa ggct t gagcg agggcgt gca 15360
   agattccgaa taccgcaagc gacaggccga tcatcgtcgc gctccagcga aagcggtcct 15420
   cgccgaaaat gacccagagc gctgccggca cctgtcctac gagttgcatg ataaagaaga 15480
   cagtcataag tgcggcgacg atagtcatgc cccgcgccca ccggaaggag ctgactgggt 15540
   t gaaggct ct caagggcat c ggt cgacgct ct ccct t at g cgact cct gc at t aggaagc 15600
   agcccagtag taggttgagg ccgttgagca ccgccgccgc aaggaatggt gcatgcaagg 15660
   agatggcgcc caacagtccc ccggccacgg ggcctgccac catacccacg ccgaaacaag 15720
   cgctcatgag cccgaagtgg cgagcccgat cttccccatc ggtgatgtcg gcgatatagg 15780
   cgccagcaac cgcacctgtg gcgccggtga tgccggccac gatgcgtccg gcgtagagga 15840
   t ccacaggac gggt gt ggt c gccat gat cg cgt agt cgat agt ggct cca agt agcgaag 15900
   cgagcaggac tgggcggcgg ccaaagcggt cggacagtgc tccgagaacg ggtgcgcata 15960
   gaaat t gcat caacgcat at agcgct agca gcacgccat a gt gact ggcg at gct gt cgg 16020
   aatggacgat atcccgcaag aggcccggca gtaccggcat aaccaagcct atgcctacag 16080
   cat ccagggt gacggt gccg aggat gacga t gagcgcat t gt t agat t t c at acacggt g 16140
   cct gact gcg t t agcaat t t aact gt gat a aact accgca t t aaagct t a t cgat gat aa 16200
   gct gt caaac at ga 16214
<210> 11
   <211> 40
   <212> DNA
   <213> Artificial Sequence
<220>
   <223> primer
<400> 11
   gat cggt acc ggt gcgct ga at gaat ct gc gccctgaatt t 40
<210> 12
   <211> 40
   <212> DNA
   <213> Artificial Sequence
<220>
   <223> primer
<400> 12
   gat cgcat gc ggcaact t ca gt t t t at cct aat cct ggcc 40
<210> 13
   <211> 55
   <212> DNA
   <213> Artificial Sequence
<220>
   <223> primer
<400> 13
   gt gat t caac at cact ggag aaagt ct t at gaaact ct ga agcct gct t t t t t at 55
<210> 14
   <211> 55
   <212> DNA
   <213> Artificial Sequence
<220>
   <223> primer
<400> 14
   cct ggaat gc aggggagcgg caagat t aaa ccagt t ccgc t caagt t agt at aaa 55
<210> 15
   <211> 22
   <212> DNA
   <213> Artificial Sequence
<220>
   <223> primer
<400> 15
   gcgcct acac t aagcat agt t g 22
<210> 16
   <211> 22
   <212> DNA
   <213> Artificial Sequence
<220>
   <223> Artificial Sequence
<400> 16
   ccatcagcag gct t agcgca ac 22
<210> 17
   <211> 55
   <212> DNA
   <213> Artificial Sequence
<220>
   <223> primer
<400> 17
   gagggagaaa aacgcat gat t at t t ccgca gccagcgt ga agcct gct t t t t t at 55
<210> 18
   <211> 54
   <212> DNA
   <213> Artificial Sequence
<220>
   <223> primer
<400> 18
   gcgcaaacga ct at gccgca t t ccct t t cg ccat ggcgct caagt t agt a taaa 54
<210> 19
   <211> 23
   <212> DNA
   <213> Artificial Sequence
<220>
   <223> primer
<400> 19
   gcgt aaagca at gat ggcgc acc 23
<210> 20
   <211> 23
   <212> DNA
   <213> Artificial Sequence
<220>
   <223> primer
<400> 20
   gcggtgtcgt t t cagagt ga ggg 23
<210> 21
   <211> 348
   <212> DNA
   <213> Escherichia coli
<400> 21
   aagctttacg cgaacgagcc atgacattgc tgacgactct ggcagtggca gatgacataa 60
   aactggtcga ctggttacaa caacgcctgg ggcttttaga gcaacgagac acggcaatgt 120
   tgcaccgttt gctgcatgat attgaaaaaa atatcaccaa ataaaaaacg ccttagtaag 180
   tatttttcag cttttcattc tgactgcaac gggcaatatg tctctgtgtg gattaaaaaa 240
   agagtgtctg atagcagctt ctgaactggt tacctgccgt gagtaaatta aaatttattt 300
   gacttaggtc actaaatact ttaaccaata taggcgactc taggatcc 348
<210> 22
   <211> 24
   <212> DNA
   <213> Artificial Sequence
<220>
   <223> primer
<400> 22
   cgggat ccca gct t act agt aaac 24
<210> 23
   <211> 32
   <212> DNA
   <213> Artificial Sequence
<220>
   <223> primrer
<400> 23
   gggaaaggat ccgat gggct ccacaaaatg gg 32
<210> 24
   <211> 1686
   <212> DNA
   <213> Salmonella typhimurium
<220>
   <221> CDS
   <222> (1)..(1686)
<400> 24
   gtg aat ata aac gtc gca gat ttg tta aat ggg aat tac atc ctg tta    48
   Val Asn Ile Asn Val Ala Asp Leu Leu Asn Gly Asn Tyr Ile Leu Leu
   1               5                   10                  15
   tta ttt gtg gtc ctg gct ctt ggc ctg tgt ctt ggc aag tta cgt ctg    96
   Leu Phe Val Val Leu Ala Leu Gly Leu Cys Leu Gly Lys Leu Arg Leu
               20                  25                  30
   ggt tca gtc caa ctt ggt aat tcc att ggc gtt tta gtg gtc tct cta   144
   Gly Ser Val Gln Leu Gly Asn Ser Ile Gly Val Leu Val Val Ser Leu
           35                  40                  45
   tta tta ggg cag caa cac ttc agt att aac acc gac gcg cta aat ctg   192
   Leu Leu Gly Gln Gln His Phe Ser Ile Asn Thr Asp Ala Leu Asn Leu
       50                  55                  60
   gga ttt atg ctg ttt att ttt tgt gtc ggc gtc gag gct ggc ccc aac   240
   Gly Phe Met Leu Phe Ile Phe Cys Val Gly Val Glu Ala Gly Pro Asn
   65                  70                  75                  80
   ttt ttt tcg att ttt ttt cgc gac ggt aaa aat tat ctg atg ctt gcc   288
   Phe Phe Ser Ile Phe Phe Arg Asp Gly Lys Asn Tyr Leu Met Leu Ala
                   85                  90                  95
   ctg gtc atg gtc ggt agc gct ctg cta atc gcc tta ggc ctt ggt aag   336
   Leu Val Met Val Gly Ser Ala Leu Leu Ile Ala Leu Gly Leu Gly Lys
               100                 105                 110
   cta ttc ggc tgg gat atc ggc ctg acg gcc ggt atg ctg gcc ggc tca   384
   Leu Phe Gly Trp Asp Ile Gly Leu Thr Ala Gly Met Leu Ala Gly Ser
           115                 120                 125
   atg acc tcc acg ccc gtg ctg gtt ggc gca ggc gat acc ctg cga cat   432
   Met Thr Ser Thr Pro Val Leu Val Gly Ala Gly Asp Thr Leu Arg His
       130                 135                 140
   tcc ggc atc gcc agc acc caa ctc tcc tcc gct ctt gat aat ctg agc   480
   Ser Gly Ile Ala Ser Thr Gln Leu Ser Ser Ala Leu Asp Asn Leu Ser
   145                 150                 155                 160
   ctg ggc tat gcc ctg acc tat ctg att ggg ttg gtc agt ctg atc gtg   528
   Leu Gly Tyr Ala Leu Thr Tyr Leu Ile Gly Leu Val Ser Leu Ile Val
                   165                 170                 175
   ggc gcg cgc tac ttg cct aaa cta cag cat cag gat ttg caa acc agc   576
   Gly Ala Arg Tyr Leu Pro Lys Leu Gln His Gln Asp Leu Gln Thr Ser
               180                 185                 190
   gcc cag caa att gct cgc gag cgc ggt ctg gat acc gat gct aat cgc   624
   Ala Gln Gln Ile Ala Arg Glu Arg Gly Leu Asp Thr Asp Ala Asn Arg
           195                 200                 205
   aag gtt tat ttg ccg gtt atc cgc gcc tat cgg gtt ggc ccg gaa ctg   672
   Lys Val Tyr Leu Pro Val Ile Arg Ala Tyr Arg Val Gly Pro Glu Leu
       210                 215                 220
   gtg gcc tgg act gac ggt aaa aac ctg cgc gag ctg ggt att tac cgc   720
   Val Ala Trp Thr Asp Gly Lys Asn Leu Arg Glu Leu Gly Ile Tyr Arg
   225                 230                 235                 240
   cag aca ggc tgc tat atc gaa cgt att cgc cgt aac ggt att ctg gcg   768
   Gln Thr Gly Cys Tyr Ile Glu Arg Ile Arg Arg Asn Gly Ile Leu Ala
                   245                 250                 255
   aac ccg gat ggc gat gcg gtg ctg caa atg ggc gat gag att gcg ctg   816
   Asn Pro Asp Gly Asp Ala Val Leu Gln Met Gly Asp Glu Ile Ala Leu
               260                 265                 270
   gtc ggc tat ccg gac gcg cac gcc agg ctc gat ccc agc ttc cgt aat   864
   Val Gly Tyr Pro Asp Ala His Ala Arg Leu Asp Pro Ser Phe Arg Asn
           275                 280                 285
   ggt aag gaa gta ttt gat cgc gat ctg ctt gat atg cgc atc gtg acg     912
   Gly Lys Glu Val Phe Asp Arg Asp Leu Leu Asp Met Arg Ile Val Thr
       290                 295                 300
   gaa gaa att gtg gtg aaa aac cac aat gcg gtc ggt cgc cgc ctc gct     960
   Glu Glu Ile Val Val Lys Asn His Asn Ala Val Gly Arg Arg Leu Ala
   305                 310                 315                 320
   cag ctc aag ctg acc gat cat ggc tgc ttc ctt aac cgc gtc atc cgc    1008
   Gln Leu Lys Leu Thr Asp His Gly Cys Phe Leu Asn Arg Val Ile Arg
                   325                 330                 335
   agc cag atc gaa atg cct atc gac gat aac gtc gtg ctg aat aaa ggc    1056
   Ser Gln Ile Glu Met Pro Ile Asp Asp Asn Val Val Leu Asn Lys Gly
               340                 345                 350
   gac gtg tta cag gtg agc ggc gac gcc aga cgc gta aaa acc atc gcc    1104
   Asp Val Leu Gln Val Ser Gly Asp Ala Arg Arg Val Lys Thr Ile Ala
           355                 360                 365
   gat cgc att ggt ttt att tcg att cat agt cag gtg acg gac ctg ctt    1152
   Asp Arg Ile Gly Phe Ile Ser Ile His Ser Gln Val Thr Asp Leu Leu
       370                 375                 380
   gcc ttc tgc gct ttt ttt atc atc ggg tta atg atc ggg atg att acc    1200
   Ala Phe Cys Ala Phe Phe Ile Ile Gly Leu Met Ile Gly Met Ile Thr
   385                 390                 395                 400
   ttc cag ttc agc aat ttt agt ttc ggt atc ggc aac gcg gcc gga ttg    1248
   Phe Gln Phe Ser Asn Phe Ser Phe Gly Ile Gly Asn Ala Ala Gly Leu
                   405                 410                 415
   ctg ttc gcc ggg atc atg ctc ggt ttc ctg cgc gct aac cat cct acc    1296
   Leu Phe Ala Gly Ile Met Leu Gly Phe Leu Arg Ala Asn His Pro Thr
               420                 425                 430
   ttt ggt tat att ccg cag ggc gcg ctt aat atg gtg aaa gaa ttc ggc    1344
   Phe Gly Tyr Ile Pro Gln Gly Ala Leu Asn Met Val Lys Glu Phe Gly
           435                 440                 445
   ttg atg gtg ttt atg gca ggc gtt ggt tta agc gcc ggc agc ggt atc    1392
   Leu Met Val Phe Met Ala Gly Val Gly Leu Ser Ala Gly Ser Gly Ile
       450                 455                 460
   agc aac ggt ctg ggc gcg gtc ggc gga caa atg ctg atc gcc ggg ctt    1440
   Ser Asn Gly Leu Gly Ala Val Gly Gly Gln Met Leu Ile Ala Gly Leu
   465                 470                 475                 480
   gtc gtc agc ctg gta ccg gtg gtg atc tgc ttc ttg ttt ggc gct tat    1488
   Val Val Ser Leu Val Pro Val Val Ile Cys Phe Leu Phe Gly Ala Tyr
                   485                 490                 495
   gtg ctg cgc atg aac cgg gcg ctg ctg ttc ggt gcc atg atg ggc gcg    1536
   Val Leu Arg Met Asn Arg Ala Leu Leu Phe Gly Ala Met Met Gly Ala
               500                 505                 510
   cgt acc tgc gcc ccg gcg atg gaa atc att agc gat acg gcg cgc agt    1584
   Arg Thr Cys Ala Pro Ala Met Glu Ile Ile Ser Asp Thr Ala Arg Ser
           515                 520                 525
   aat att cca gcg ctg ggt tat gcg ggg act tac gcc atc gcc aac gtt    1632
   Asn Ile Pro Ala Leu Gly Tyr Ala Gly Thr Tyr Ala Ile Ala Asn Val
       530                 535                 540
   ctg cta acg ctg gcg ggg acg ctt att gtc atc atc tgg ccg ggg tta    1680
   Leu Leu Thr Leu Ala Gly Thr Leu Ile Val Ile Ile Trp Pro Gly Leu
   545                 550                 555                 560
   gga taa                                                            1686
   Gly

<210> 25
   <211> 561
   <212> PRT
   <213> Salmonella typhimurium
<400> 25
   Val Asn Ile Asn Val Ala Asp Leu Leu Asn Gly Asn Tyr Ile Leu Leu
   1               5                   10                  15
   Leu Phe Val Val Leu Ala Leu Gly Leu Cys Leu Gly Lys Leu Arg Leu
               20                  25                  30
   Gly Ser Val Gln Leu Gly Asn Ser Ile Gly Val Leu Val Val Ser Leu
   35 40 45
   Leu Leu Gly Gln Gln His Phe Ser Ile Asn Thr Asp Ala Leu Asn Leu
   50 55 60
   Gly Phe Met Leu Phe Ile Phe Cys Val Gly Val Glu Ala Gly Pro Asn
   65 70 75 80
   Phe Phe Ser Ile Phe Phe Arg Asp Gly Lys Asn Tyr Leu Met Leu Ala
   85 90 95
   Leu Val Met Val Gly Ser Ala Leu Leu Ile Ala Leu Gly Leu Gly Lys
   100 105 110
   Leu Phe Gly Trp Asp Ile Gly Leu Thr Ala Gly Met Leu Ala Gly Ser
   115 120 125
   Met Thr Ser Thr Pro Val Leu Val Gly Ala Gly Asp Thr Leu Arg His
   130 135 140
   Ser Gly Ile Ala Ser Thr Gln Leu Ser Ser Ala Leu Asp Asn Leu Ser
   145 150 155 160
   Leu Gly Tyr Ala Leu Thr Tyr Leu Ile Gly Leu Val Ser Leu Ile Val
   165 170 175
   Gly Ala Arg Tyr Leu Pro Lys Leu Gln His Gln Asp Leu Gln Thr Ser
   180 185 190
   Ala Gln Gln Ile Ala Arg Glu Arg Gly Leu Asp Thr Asp Ala Asn Arg
   195 200 205
   Lys Val Tyr Leu Pro Val Ile Arg Ala Tyr Arg Val Gly Pro Glu Leu
   210 215 220
   Val Ala Trp Thr Asp Gly Lys Asn Leu Arg Glu Leu Gly Ile Tyr Arg
   225 230 235 240
   Gln Thr Gly Cys Tyr Ile Glu Arg Ile Arg Arg Asn Gly Ile Leu Ala
   245 250 255
   Asn Pro Asp Gly Asp Ala Val Leu Gln Met Gly Asp Glu Ile Ala Leu
   260 265 270
   Val Gly Tyr Pro Asp Ala His Ala Arg Leu Asp Pro Ser Phe Arg Asn
   275 280 285
   Gly Lys Glu Val Phe Asp Arg Asp Leu Leu Asp Met Arg Ile Val Thr
   290 295 300
   Glu Glu Ile Val Val Lys Asn His Asn Ala Val Gly Arg Arg Leu Ala
   305 310 315 320
   Gln Leu Lys Leu Thr Asp His Gly Cys Phe Leu Asn Arg Val Ile Arg
   325 330 335
   Ser Gln Ile Glu Met Pro Ile Asp Asp Asn Val Val Leu Asn Lys Gly
   340 345 350
   Asp Val Leu Gln Val Ser Gly Asp Ala Arg Arg Val Lys Thr Ile Ala
   355 360 365
   Asp Arg Ile Gly Phe Ile Ser Ile His Ser Gln Val Thr Asp Leu Leu
   370 375 380
   Ala Phe Cys Ala Phe Phe Ile Ile Gly Leu Met Ile Gly Met Ile Thr
   385 390 395 400
   Phe Gln Phe Ser Asn Phe Ser Phe Gly Ile Gly Asn Ala Ala Gly Leu
   405 410 415
   Leu Phe Ala Gly Ile Met Leu Gly Phe Leu Arg Ala Asn His Pro Thr
   420 425 430
   Phe Gly Tyr Ile Pro Gln Gly Ala Leu Asn Met Val Lys Glu Phe Gly
   435 440 445
   Leu Met Val Phe Met Ala Gly Val Gly Leu Ser Ala Gly Ser Gly Ile
   450 455 460
   Ser Asn Gly Leu Gly Ala Val Gly Gly Gln Met Leu Ile Ala Gly Leu
   465 470 475 480
   Val Val Ser Leu Val Pro Val Val Ile Cys Phe Leu Phe Gly Ala Tyr
   485 490 495
   Val Leu Arg Met Asn Arg Ala Leu Leu Phe Gly Ala Met Met Gly Ala
   500 505 510
   Arg Thr Cys Ala Pro Ala Met Glu Ile Ile Ser Asp Thr Ala Arg Ser
   515 520 525
   Asn Ile Pro Ala Leu Gly Tyr Ala Gly Thr Tyr Ala Ile Ala Asn Val
   530 535 540
   Leu Leu Thr Leu Ala Gly Thr Leu Ile Val Ile Ile Trp Pro Gly Leu
   545 550 555 560
   Gly
<210> 26
   <211> 1689
   <212> DNA
   <213> Yersiniaa pestis
<220>
   <221> CDS
   <222> (1)..(1689)
<400> 26
   gtg aat ata aac gtc gca aat ttg tta aac ggc aac tat atc ttg ctg 48
   Val Asn Ile Asn Val Ala Asn Leu Leu Asn Gly Asn Tyr Ile Leu Leu
   1 5 10 15
   tta ttc gtc gtc tta gcg ttg ggg tta tgc tta ggt aaa ttg cgc ctt 96
   Leu Phe Val Val Leu Ala Leu Gly Leu Cys Leu Gly Lys Leu Arg Leu
   20 25 30
   ggc tct atc caa tta ggt aac gct att ggt gta ctg gtg gta tca ctg 144
   Gly Ser Ile Gln Leu Gly Asn Ala Ile Gly Val Leu Val Val Ser Leu
   35 40 45
   ttg cta ggg caa cag cat ttc gcc atc aat act gaa gcg cta aat ctg 192
   Leu Leu Gly Gln Gln His Phe Ala Ile Asn Thr Glu Ala Leu Asn Leu
   50 55 60
   ggc ttt atg ttg ttt att ttt tgt gtc ggt gtt gaa gcc ggt cca aac 240
   Gly Phe Met Leu Phe Ile Phe Cys Val Gly Val Glu Ala Gly Pro Asn
   65 70 75 80
   ttc ttt tct att ttc ttc cgt gat ggc aaa aat tac ctg atg ctc gcc 288
   Phe Phe Ser Ile Phe Phe Arg Asp Gly Lys Asn Tyr Leu Met Leu Ala
   85 90 95
   ttg gtt atg gtg ggc agc gcg atg att tta gcg ttg ggg ctt ggc aaa 336
   Leu Val Met Val Gly Ser Ala Met Ile Leu Ala Leu Gly Leu Gly Lys
   100 105 110
   tta ttt ggt tgg gat atc ggt ctg acc gcc ggg atg ctg gca ggg tca 384
   Leu Phe Gly Trp Asp Ile Gly Leu Thr Ala Gly Met Leu Ala Gly Ser
   115 120 125
   atg aca tcg acc ccc gtt ttg gtt ggg gcc ggt gat acg cta cgc cat 432
   Met Thr Ser Thr Pro Val Leu Val Gly Ala Gly Asp Thr Leu Arg His
   130 135 140
   acc atg gct aat ggt tcg tca ctg caa caa gca cag gat aat ttg agc 480
   Thr Met Ala Asn Gly Ser Ser Leu Gln Gln Ala Gln Asp Asn Leu Ser
   145 150 155 160
   ctc ggc tat gcc ctg act tat ctt atc ggg ctg gtt agc ctg att tta 528
   Leu Gly Tyr Ala Leu Thr Tyr Leu Ile Gly Leu Val Ser Leu Ile Leu
   165 170 175
   ggt gcg cgc tat tta cct aag ctt cag cat cag gat tta ccg acc agc 576
   Gly Ala Arg Tyr Leu Pro Lys Leu Gln His Gln Asp Leu Pro Thr Ser
   180 185 190
   gcc caa caa att gcc cgc gaa cga ggg ctg gac acc gat agc cag cgt 624
   Ala Gln Gln Ile Ala Arg Glu Arg Gly Leu Asp Thr Asp Ser Gln Arg
   195 200 205
   aaa gtg tat ttg cct gtc atc cgt gct tat cgt gta ggc ccg gag ctg 672
   Lys Val Tyr Leu Pro Val Ile Arg Ala Tyr Arg Val Gly Pro Glu Leu
   210 215 220
   gtt gcc tgg gca gat ggt aaa aac ttg cgt gaa tta ggg att tac cgc 720
   Val Ala Trp Ala Asp Gly Lys Asn Leu Arg Glu Leu Gly Ile Tyr Arg
   225 230 235 240
   caa acc ggt tgc tat atc gag cgt atc cgc cgc aac ggt att ctc gcg 768
   Gln Thr Gly Cys Tyr Ile Glu Arg Ile Arg Arg Asn Gly Ile Leu Ala
   245 250 255
   aat ccg gat ggt gac gcc gtg cta caa gtc ggt gat gaa atc tcg ttg 816
   Asn Pro Asp Gly Asp Ala Val Leu Gln Val Gly Asp Glu Ile Ser Leu
   260 265 270
   gtg ggt tac cca gat gcc cat tcc cgc ctt gac ccc agc ttc cgt aat 864
   Val Gly Tyr Pro Asp Ala His Ser Arg Leu Asp Pro Ser Phe Arg Asn
   275 280 285
   ggc aaa gaa gtt ttc gac cgt gat ctg ttg gat atg cgt atc gtc acc 912
   Gly Lys Glu Val Phe Asp Arg Asp Leu Leu Asp Met Arg Ile Val Thr
   290 295 300
   gaa gag atc gtg gtc aaa aat agc aat gct gtc ggt aaa cgc ctc agc 960
   Glu Glu Ile Val Val Lys Asn Ser Asn Ala Val Gly Lys Arg Leu Ser
   305                     310                     315                     320
   cat tta aaa ctc acc gat cac ggt tgc ttc ctt aat cgg gtc att cgt        1008
   His Leu Lys Leu Thr Asp His Gly Cys Phe Leu Asn Arg Val Ile Arg
                   325                     330                     335
   agc caa att gaa atg ccc att gat gat aac gtg gtg ttg aat aaa ggt        1056
   Ser Gln Ile Glu Met Pro Ile Asp Asp Asn Val Val Leu Asn Lys Gly
               340                     345                     350
   gat gtg ctg caa gtc agc ggt gat gcc cgg cga gtc aaa agc gtg gca        1104
   Asp Val Leu Gln Val Ser Gly Asp Ala Arg Arg Val Lys Ser Val Ala
           355                     360                     365
   gaa aaa att ggc ttt atc tct att cac agt cag gtc act gac ctg ctg        1152
   Glu Lys Ile Gly Phe Ile Ser Ile His Ser Gln Val Thr Asp Leu Leu
       370                     375                     380
   gcc ttc tgc tca ttc ttt att ctt ggg tta atg att ggc ctg att act        1200
   Ala Phe Cys Ser Phe Phe Ile Leu Gly Leu Met Ile Gly Leu Ile Thr
   385                     390                     395                     400
   ttc cag ttc agc aat ttc agc ttc ggt att ggc aat gcc gca ggg ctg        1248
   Phe Gln Phe Ser Asn Phe Ser Phe Gly Ile Gly Asn Ala Ala Gly Leu
                   405                     410                     415
   tta tta gca ggg atc atg ctg gga ttt tta cgt gcc aac cac cct act        1296
   Leu Leu Ala Gly Ile Met Leu Gly Phe Leu Arg Ala Asn His Pro Thr
               420                     425                     430
   ttt ggc tat atc ccg caa ggc gcc ttg aat atg gtg aaa gag ttc ggg        1344
   Phe Gly Tyr Ile Pro Gln Gly Ala Leu Asn Met Val Lys Glu Phe Gly
           435                     440                     445
   tta atg gtc ttt atg gca ggt gtc ggg tta agt gcc ggg ggc ggc att        1392
   Leu Met Val Phe Met Ala Gly Val Gly Leu Ser Ala Gly Gly Gly Ile
       450                     455                     460
   aat agc agc ctg ggc gcc gtc ggt gga caa atg ttg att tcc ggt ttg        1440
   Asn Ser Ser Leu Gly Ala Val Gly Gly Gln Met Leu Ile Ser Gly Leu
   465                     470                     475                     480
   ata gtc agt ttg gtt ccg gtg gtg atc tgc ttt gtt ttt ggt gct tat        1488
   Ile Val Ser Leu Val Pro Val Val Ile Cys Phe Val Phe Gly Ala Tyr
                   485                     490                     495
   gtt ctg cgt atg aac cgt gca ctg ctt ttt ggt gcc att atg ggg gcc        1536
   Val Leu Arg Met Asn Arg Ala Leu Leu Phe Gly Ala Ile Met Gly Ala
               500                     505                     510
   aga acc tgt gca ccc gcc atg gac atc atc agt gat acc gcg cgt agc        1584
   Arg Thr Cys Ala Pro Ala Met Asp Ile Ile Ser Asp Thr Ala Arg Ser
           515                     520                     525
   aat att ccc gca tta ggc tac gcg ggg act tac gct atc gcc aac gtc        1632
   Asn Ile Pro Ala Leu Gly Tyr Ala Gly Thr Tyr Ala Ile Ala Asn Val
       530                     535                     540
   tta ctg acc ttg gct ggc tcg ttg att gtc atc ctc tgg cca ggg ata        1680
   Leu Leu Thr Leu Ala Gly Ser Leu Ile Val Ile Leu Trp Pro Gly Ile
   545                     550                     555                     560
   tta ggt tag                                                            1689
   Leu Gly
<210> 27
   <211> 562
   <212> PRT
   <213> Yersinia pestis
<400> 27
   Val Asn Ile Asn Val Ala Asn Leu Leu Asn Gly Asn Tyr Ile Leu Leu
   1               5                   10                  15
   Leu Phe Val Val Leu Ala Leu Gly Leu Cys Leu Gly Lys Leu Arg Leu
               20                  25                  30
   Gly Ser Ile Gln Leu Gly Asn Ala Ile Gly Val Leu Val Val Ser Leu
           35                  40                  45
   Leu Leu Gly Gln Gln His Phe Ala Ile Asn Thr Glu Ala Leu Asn Leu
       50                  55                  60
   Gly Phe Met Leu Phe Ile Phe Cys Val Gly Val Glu Ala Gly Pro Asn
   65                  70                  75                  80
   Phe Phe Ser Ile Phe Phe Arg Asp Gly Lys Asn Tyr Leu Met Leu Ala
                   85                  90                  95
   Leu Val Met Val Gly Ser Ala Met Ile Leu Ala Leu Gly Leu Gly Lys
   100 105 110
   Leu Phe Gly Trp Asp Ile Gly Leu Thr Ala Gly Met Leu Ala Gly Ser
   115 120 125
   Met Thr Ser Thr Pro Val Leu Val Gly Ala Gly Asp Thr Leu Arg His
   130 135 140
   Thr Met Ala Asn Gly Ser Ser Leu Gln Gln Ala Gln Asp Asn Leu Ser
   145 150 155 160
   Leu Gly Tyr Ala Leu Thr Tyr Leu Ile Gly Leu Val Ser Leu Ile Leu
   165 170 175
   Gly Ala Arg Tyr Leu Pro Lys Leu Gln His Gln Asp Leu Pro Thr Ser
   180 185 190
   Ala Gln Gln Ile Ala Arg Glu Arg Gly Leu Asp Thr Asp Ser Gln Arg
   195 200 205
   Lys Val Tyr Leu Pro Val Ile Arg Ala Tyr Arg Val Gly Pro Glu Leu
   210 215 220
   Val Ala Trp Ala Asp Gly Lys Asn Leu Arg Glu Leu Gly Ile Tyr Arg
   225 230 235 240
   Gln Thr Gly Cys Tyr Ile Glu Arg Ile Arg Arg Asn Gly Ile Leu Ala
   245 250 255
   Asn Pro Asp Gly Asp Ala Val Leu Gln Val Gly Asp Glu Ile Ser Leu
   260 265 270
   Val Gly Tyr Pro Asp Ala His Ser Arg Leu Asp Pro Ser Phe Arg Asn
   275 280 285
   Gly Lys Glu Val Phe Asp Arg Asp Leu Leu Asp Met Arg Ile Val Thr
   290 295 300
   Glu Glu Ile Val Val Lys Asn Ser Asn Ala Val Gly Lys Arg Leu Ser
   305 310 315 320
   His Leu Lys Leu Thr Asp His Gly Cys Phe Leu Asn Arg Val Ile Arg
   325 330 335
   Ser Gln Ile Glu Met Pro Ile Asp Asp Asn Val Val Leu Asn Lys Gly
   340 345 350
   Asp Val Leu Gln Val Ser Gly Asp Ala Arg Arg Val Lys Ser Val Ala
   355 360 365
   Glu Lys Ile Gly Phe Ile Ser Ile His Ser Gln Val Thr Asp Leu Leu
   370 375 380
   Ala Phe Cys Ser Phe Phe Ile Leu Gly Leu Met Ile Gly Leu Ile Thr
   385 390 395 400
   Phe Gln Phe Ser Asn Phe Ser Phe Gly Ile Gly Asn Ala Ala Gly Leu
   405 410 415
   Leu Leu Ala Gly Ile Met Leu Gly Phe Leu Arg Ala Asn His Pro Thr
   420 425 430
   Phe Gly Tyr Ile Pro Gln Gly Ala Leu Asn Met Val Lys Glu Phe Gly
   435 440 445
   Leu Met Val Phe Met Ala Gly Val Gly Leu Ser Ala Gly Gly Gly Ile
   450 455 460
   Asn Ser Ser Leu Gly Ala Val Gly Gly Gln Met Leu Ile Ser Gly Leu
   465 470 475 480
   Ile Val Ser Leu Val Pro Val Val Ile Cys Phe Val Phe Gly Ala Tyr
   485 490 495
   Val Leu Arg Met Asn Arg Ala Leu Leu Phe Gly Ala Ile Met Gly Ala
   500 505 510
   Arg Thr Cys Ala Pro Ala Met Asp Ile Ile Ser Asp Thr Ala Arg Ser
   515 520 525
   Asn Ile Pro Ala Leu Gly Tyr Ala Gly Thr Tyr Ala Ile Ala Asn Val
   530 535 540
   Leu Leu Thr Leu Ala Gly Ser Leu Ile Val Ile Leu Trp Pro Gly Ile
   545 550 555 560
   Leu Gly
<210> 28
   <211> 1689
   <212> DNA
   <213> Erwinia carotovora
<220>
   <221> CDS
   <222> (1)..(1689)
<400> 28
   atg aat ata aac gtc gct gat ttg tta aac ggg aat tac att ctg ctt   48
   Met Asn Ile Asn Val Ala Asp Leu Leu Asn Gly Asn Tyr Ile Leu Leu
   1               5                   10                  15
   tta ttc gtg gtt ctt tca tta gga ctc tgt ctg ggg aaa ttg cgc ctc   96
   Leu Phe Val Val Leu Ser Leu Gly Leu Cys Leu Gly Lys Leu Arg Leu
               20                  25                  30
   ggg cca gta caa ctc ggt aat tct att ggc gtt tta gtg gtt tct tta  144
   Gly Pro Val Gln Leu Gly Asn Ser Ile Gly Val Leu Val Val Ser Leu
           35                  40                  45
   tta ctc ggc caa caa cat ttt tcg att aat acc gaa gca ctg agc ctc  192
   Leu Leu Gly Gln Gln His Phe Ser Ile Asn Thr Glu Ala Leu Ser Leu
       50                  55                  60
   ggt ttt atg tta ttt att ttt tgc gtg gga gta gaa gcc ggg ccg aat  240
   Gly Phe Met Leu Phe Ile Phe Cys Val Gly Val Glu Ala Gly Pro Asn
   65                  70                  75                  80
   ttc ttt tct att ttc ttc cgc gac ggg aaa aat tat ttc atg ctg gcg  288
   Phe Phe Ser Ile Phe Phe Arg Asp Gly Lys Asn Tyr Phe Met Leu Ala
                   85                  90                  95
   ctg gtg atg gtc ggc agc gcc atg tta ctg gcg tta ggg tta ggc aaa  336
   Leu Val Met Val Gly Ser Ala Met Leu Leu Ala Leu Gly Leu Gly Lys
               100                 105                 110
   ctt ttc ggc tgg gga atc ggc ctg acc gcg ggg atg ctg gca gga tcc  384
   Leu Phe Gly Trp Gly Ile Gly Leu Thr Ala Gly Met Leu Ala Gly Ser
           115                 120                 125
   atg aca tcg aca ccg gtg ctg gtc ggt gca ggc gac aca cta cgc aat  432
   Met Thr Ser Thr Pro Val Leu Val Gly Ala Gly Asp Thr Leu Arg Asn
       130                 135                 140
   acc gcc agt ttg ggt ggt caa ctc ggt gtt gaa caa gat cat ttg agc  480
   Thr Ala Ser Leu Gly Gly Gln Leu Gly Val Glu Gln Asp His Leu Ser
   145                 150                 155                 160
   ctg ggc tat gcg ctg acg tat ctg gtc ggg ctg gtc agt ctc att ttt  528
   Leu Gly Tyr Ala Leu Thr Tyr Leu Val Gly Leu Val Ser Leu Ile Phe
                   165                 170                 175
   ggt gca cgc tat ctg ccc aaa ctt cag cat cag gat ctt ccc acc agt  576
   Gly Ala Arg Tyr Leu Pro Lys Leu Gln His Gln Asp Leu Pro Thr Ser
               180                 185                 190
   gcg cag caa att gca cgc gag cgc ggg ctg gat gta gac agc caa aga  624
   Ala Gln Gln Ile Ala Arg Glu Arg Gly Leu Asp Val Asp Ser Gln Arg
           195                 200                 205
   aaa gtg tat ctg cca gtg att cgt gcc tat cgc gtc ggg cca gag ttg  672
   Lys Val Tyr Leu Pro Val Ile Arg Ala Tyr Arg Val Gly Pro Glu Leu
       210                 215                 220
   gtt gac tgg gcg gct ggc aaa aac ttg cgc gaa ctg gga atc tat cgc  720
   Val Asp Trp Ala Ala Gly Lys Asn Leu Arg Glu Leu Gly Ile Tyr Arg
   225                 230                 235                 240
   cag acc ggt tgc tac atc gag cgc att cga cgc aac gga att ctg gca  768
   Gln Thr Gly Cys Tyr Ile Glu Arg Ile Arg Arg Asn Gly Ile Leu Ala
                   245                 250                 255
   aat ccc gac ggg gat gcc gtc ttg caa atc ggc gac gag atc gcg ctg  816
   Asn Pro Asp Gly Asp Ala Val Leu Gln Ile Gly Asp Glu Ile Ala Leu
               260                 265                 270
   gtc ggt tat ccc gat gca cac tct cgt ctg aac tcc aat ttc cgc gat  864
   Val Gly Tyr Pro Asp Ala His Ser Arg Leu Asn Ser Asn Phe Arg Asp
           275                 280                 285
   ggc aaa gaa gtt ttc gac cgt aat ttg ttg gac atg cgc atc gtg acg  912
   Gly Lys Glu Val Phe Asp Arg Asn Leu Leu Asp Met Arg Ile Val Thr
       290                 295                 300
   gaa gag att gtc gtc aag aat cac aac gcc gtc ggt aag cgc ctc agc  960
   Glu Glu Ile Val Val Lys Asn His Asn Ala Val Gly Lys Arg Leu Ser
   305                 310                 315                 320
   caa ttg aag ctg act gac cac ggc tgt ttc ctt aac cgt att gtt cgc 1008
   Gln Leu Lys Leu Thr Asp His Gly Cys Phe Leu Asn Arg Ile Val Arg
                   325                 330                 335
   agc cag ata gaa atg ccg ctc gac gat aac gtc gtg ctg aat aaa ggc 1056
   Ser Gln Ile Glu Met Pro Leu Asp Asp Asn Val Val Leu Asn Lys Gly
               340                 345                 350
   gat gtc tta cag gtc agc ggt gac gcc cgt cgg gta aaa agc att gct      1104
   Asp Val Leu Gln Val Ser Gly Asp Ala Arg Arg Val Lys Ser Ile Ala
           355                 360                 365
   gag cga atc ggg ttc att tct att cac agt cag gtc acc gat tta ctg      1152
   Glu Arg Ile Gly Phe Ile Ser Ile His Ser Gln Val Thr Asp Leu Leu
       370                 375                 380
   gcc ttc tgc gcc ttt ttt gtt atc ggc gtg atg gtc ggg ctg atc acc      1200
   Ala Phe Cys Ala Phe Phe Val Ile Gly Val Met Val Gly Leu Ile Thr
   385                 390                 395                 400
   atc cag ttc agc aac ttc acc ttt ggc atc ggt aac gcg gct ggg ttg      1248
   Ile Gln Phe Ser Asn Phe Thr Phe Gly Ile Gly Asn Ala Ala Gly Leu
                   405                 410                 415
   ctc ttc gcc ggt atc atg ctg ggc ttc ctg cgc gcc aac cac ccg acc      1296
   Leu Phe Ala Gly Ile Met Leu Gly Phe Leu Arg Ala Asn His Pro Thr
               420                 425                 430
   ttc ggc tat att ccg caa ggc gcg ctt aac atg gtc aaa gag ttt ggc      1344
   Phe Gly Tyr Ile Pro Gln Gly Ala Leu Asn Met Val Lys Glu Phe Gly
           435                 440                 445
   ctg atg gtc ttt atg gcg ggc gta gga ctg agt gcc ggt agc acc atc      1392
   Leu Met Val Phe Met Ala Gly Val Gly Leu Ser Ala Gly Ser Thr Ile
       450                 455                 460
   aac agt agc cta gga gaa gtc ggt atc cag atg ctg gct tca ggg ctg      1440
   Asn Ser Ser Leu Gly Glu Val Gly Ile Gln Met Leu Ala Ser Gly Leu
   465                 470                 475                 480
   att gtc agt ctc gtg cca gtg gtg att tgt ttt ctg ttc ggc gcg tat      1488
   Ile Val Ser Leu Val Pro Val Val Ile Cys Phe Leu Phe Gly Ala Tyr
                   485                 490                 495
   gtg ctg aaa atg aat cga gcg ctg ctg ttt ggt gcc atg atg ggc gct      1536
   Val Leu Lys Met Asn Arg Ala Leu Leu Phe Gly Ala Met Met Gly Ala
               500                 505                 510
   cgg acc tgc gcg cca gcc atg gag atc atc agc gat acc gct cgc agt      1584
   Arg Thr Cys Ala Pro Ala Met Glu Ile Ile Ser Asp Thr Ala Arg Ser
           515                 520                 525
   aat att cct gct ctc ggc tat gcg ggt acc tac gcc atc gct aac gtt      1632
   Asn Ile Pro Ala Leu Gly Tyr Ala Gly Thr Tyr Ala Ile Ala Asn Val
       530                 535                 540
   ctg ctc aca tta gca ggt tca ctt atc gtg gtt atc tgg cct gaa ttg      1680
   Leu Leu Thr Leu Ala Gly Ser Leu Ile Val Val Ile Trp Pro Glu Leu
   545                 550                 555                 560
   ccc ggc tga                                                          1689
   Pro Gly
<210> 29
   <211> 562
   <212> PRT
   <213> Erwinia car ot ovor a
<400> 29
   Met Asn Ile Asn Val Ala Asp Leu Leu Asn Gly Asn Tyr Ile Leu Leu
   1               5                   10                  15
   Leu Phe Val Val Leu Ser Leu Gly Leu Cys Leu Gly Lys Leu Arg Leu
               20                  25                  30
   Gly Pro Val Gln Leu Gly Asn Ser Ile Gly Val Leu Val Val Ser Leu
           35                  40                  45
   Leu Leu Gly Gln Gln His Phe Ser Ile Asn Thr Glu Ala Leu Ser Leu
       50                  55                  60
   Gly Phe Met Leu Phe Ile Phe Cys Val Gly Val Glu Ala Gly Pro Asn
   65                  70                  75                  80
   Phe Phe Ser Ile Phe Phe Arg Asp Gly Lys Asn Tyr Phe Met Leu Ala
                   85                  90                  95
   Leu Val Met Val Gly Ser Ala Met Leu Leu Ala Leu Gly Leu Gly Lys
               100                 105                 110
   Leu Phe Gly Trp Gly Ile Gly Leu Thr Ala Gly Met Leu Ala Gly Ser
           115                 120                 125
   Met Thr Ser Thr Pro Val Leu Val Gly Ala Gly Asp Thr Leu Arg Asn
       130                 135                 140
   Thr Ala Ser Leu Gly Gly Gln Leu Gly Val Glu Gln Asp His Leu Ser
   145                 150                 155                 160
   Leu Gly Tyr Ala Leu Thr Tyr Leu Val Gly Leu Val Ser Leu Ile Phe
                   165                 170                 175
   Gly Ala Arg Tyr Leu Pro Lys Leu Gln His Gln Asp Leu Pro Thr Ser
               180                 185                 190
   Ala Gln Gln Ile Ala Arg Glu Arg Gly Leu Asp Val Asp Ser Gln Arg
           195                 200                 205
   Lys Val Tyr Leu Pro Val Ile Arg Ala Tyr Arg Val Gly Pro Glu Leu
       210                 215                 220
   Val Asp Trp Ala Ala Gly Lys Asn Leu Arg Glu Leu Gly Ile Tyr Arg
   225                 230                 235                 240
   Gln Thr Gly Cys Tyr Ile Glu Arg Ile Arg Arg Asn Gly Ile Leu Ala
                   245                 250                 255
   Asn Pro Asp Gly Asp Ala Val Leu Gln Ile Gly Asp Glu Ile Ala Leu
               260                 265                 270
   Val Gly Tyr Pro Asp Ala His Ser Arg Leu Asn Ser Asn Phe Arg Asp
           275                 280                 285
   Gly Lys Glu Val Phe Asp Arg Asn Leu Leu Asp Met Arg Ile Val Thr
       290                 295                 300
   Glu Glu Ile Val Val Lys Asn His Asn Ala Val Gly Lys Arg Leu Ser
   305                 310                 315                 320
   Gln Leu Lys Leu Thr Asp His Gly Cys Phe Leu Asn Arg Ile Val Arg
                   325                 330                 335
   Ser Gln Ile Glu Met Pro Leu Asp Asp Asn Val Val Leu Asn Lys Gly
               340                 345                 350
   Asp Val Leu Gln Val Ser Gly Asp Ala Arg Arg Val Lys Ser Ile Ala
           355                 360                 365
   Glu Arg Ile Gly Phe Ile Ser Ile His Ser Gln Val Thr Asp Leu Leu
       370                 375                 380
   Ala Phe Cys Ala Phe Phe Val Ile Gly Val Met Val Gly Leu Ile Thr
   385                 390                 395                 400
   Ile Gln Phe Ser Asn Phe Thr Phe Gly Ile Gly Asn Ala Ala Gly Leu
                   405                 410                 415
   Leu Phe Ala Gly Ile Met Leu Gly Phe Leu Arg Ala Asn His Pro Thr
               420                 425                 430
   Phe Gly Tyr Ile Pro Gln Gly Ala Leu Asn Met Val Lys Glu Phe Gly
           435                 440                 445
   Leu Met Val Phe Met Ala Gly Val Gly Leu Ser Ala Gly Ser Thr Ile
       450                 455                 460
   Asn Ser Ser Leu Gly Glu Val Gly Ile Gln Met Leu Ala Ser Gly Leu
   465                 470                 475                 480
   Ile Val Ser Leu Val Pro Val Val Ile Cys Phe Leu Phe Gly Ala Tyr
                   485                 490                 495
   Val Leu Lys Met Asn Arg Ala Leu Leu Phe Gly Ala Met Met Gly Ala
               500                 505                 510
   Arg Thr Cys Ala Pro Ala Met Glu Ile Ile Ser Asp Thr Ala Arg Ser
           515                 520                 525
   Asn Ile Pro Ala Leu Gly Tyr Ala Gly Thr Tyr Ala Ile Ala Asn Val
       530                 535                 540
   Leu Leu Thr Leu Ala Gly Ser Leu Ile Val Val Ile Trp Pro Glu Leu
   545                 550                 555                 560
   Pro Gly
<210> 30
   <211> 1677
   <212> DNA
   <213> Vibrio cholerae
<220>
   <221> CDS
   <222> (1)..(1677)
<400> 30
   gtg aac atc gac gtc gtt ttg ttg cta gag caa aat cca att ttg ctg      48
   Val Asn Ile Asp Val Val Leu Leu Leu Glu Gln Asn Pro Ile Leu Leu
   1               5                   10                  15
   ata ttt gtt gtc ctc gcc atc ggt ctc tct ttc ggg aaa att cgc ttt      96
   Ile Phe Val Val Leu Ala Ile Gly Leu Ser Phe Gly Lys Ile Arg Phe
               20                  25                  30
   ggc agc ttc caa ttg ggt aat tcg att gga gtg ctc atc acc tct ctg      144
   Gly Ser Phe Gln Leu Gly Asn Ser Ile Gly Val Leu Ile Thr Ser Leu
           35                  40                  45
   atc atg ggg cat ctt ggt ttt tcc ttt aca ccg gaa gca tta acc atc      192
   Ile Met Gly His Leu Gly Phe Ser Phe Thr Pro Glu Ala Leu Thr Ile
       50                  55                  60
   ggc ttt atg ctc ttt att tat tgt gtc ggc att gaa gcg ggt cct aac      240
   Gly Phe Met Leu Phe Ile Tyr Cys Val Gly Ile Glu Ala Gly Pro Asn
   65                  70                  75                  80
   ttc ttt ggt att ttc ttc cgc gat ggt aaa cac tac tta att cta agc      288
   Phe Phe Gly Ile Phe Phe Arg Asp Gly Lys His Tyr Leu Ile Leu Ser
                   85                  90                  95
   tta gtg gtc ttg atc act gcg act tgg att gcc tac ttt ggt ggt tac      336
   Leu Val Val Leu Ile Thr Ala Thr Trp Ile Ala Tyr Phe Gly Gly Tyr
               100                 105                 110
   tat ctt aat ctg gat tat ggc tta gcc gcc ggg atg atg gcg ggc gca      384
   Tyr Leu Asn Leu Asp Tyr Gly Leu Ala Ala Gly Met Met Ala Gly Ala
           115                 120                 125
   tta acc tca aca ccc gta ctg gtt ggt gct cag gat gcg ctc aat tct      432
   Leu Thr Ser Thr Pro Val Leu Val Gly Ala Gln Asp Ala Leu Asn Ser
       130                 135                 140
   ggg ctt gcc gcg gta cca aga cat atg gat tta tca ctg att tta gat      480
   Gly Leu Ala Ala Val Pro Arg His Met Asp Leu Ser Leu Ile Leu Asp
   145                 150                 155                 160
   aat gtc tcg gtc ggt tat gcg atg gcc tac ttg atc ggc ctt atc agc      528
   Asn Val Ser Val Gly Tyr Ala Met Ala Tyr Leu Ile Gly Leu Ile Ser
                   165                 170                 175
   atg atc atg ttt gct aaa tta ctc cct aag ttg caa aag caa aac ctg      576
   Met Ile Met Phe Ala Lys Leu Leu Pro Lys Leu Gln Lys Gln Asn Leu
               180                 185                 190
   tcc gat tcg gct caa caa atc gcg caa gaa cga ggg tta ggc agt cag      624
   Ser Asp Ser Ala Gln Gln Ile Ala Gln Glu Arg Gly Leu Gly Ser Gln
           195                 200                 205
   cgt aaa gtc tat ctg ccg atc atc cgt gcc tat cga gtc gga cct gaa      672
   Arg Lys Val Tyr Leu Pro Ile Ile Arg Ala Tyr Arg Val Gly Pro Glu
       210                 215                 220
   ctg att aac tgg atc gac ggt cgt aat ctg cgc gag ctg ggt atc tac      720
   Leu Ile Asn Trp Ile Asp Gly Arg Asn Leu Arg Glu Leu Gly Ile Tyr
   225                 230                 235                 240
   cgt caa aca ggc tgt tat att gag cgt atc cgc cga cat ggc att ttg      768
   Arg Gln Thr Gly Cys Tyr Ile Glu Arg Ile Arg Arg His Gly Ile Leu
                   245                 250                 255
   gcc cat cct gat ggt gat gcc att ttg caa gaa ggg gat gaa att gct      816
   Ala His Pro Asp Gly Asp Ala Ile Leu Gln Glu Gly Asp Glu Ile Ala
               260                 265                 270
   ctc gta ggt ttc cca gat agt cac gct cgt ctt gac ccg agc ttt cgc      864
   Leu Val Gly Phe Pro Asp Ser His Ala Arg Leu Asp Pro Ser Phe Arg
           275                 280                 285
   aat ggc aaa gaa gtg ttt gac cgt aac tta ctc gac cta cga att tca      912
   Asn Gly Lys Glu Val Phe Asp Arg Asn Leu Leu Asp Leu Arg Ile Ser
       290                 295                 300
   gaa gaa gag atc gtg gta aaa agt gac agc att gcg gga aaa cgt ctc      960
   Glu Glu Glu Ile Val Val Lys Ser Asp Ser Ile Ala Gly Lys Arg Leu
   305                 310                 315                 320
   tcc gat ctt aat ctt tcg gaa tac ggc tgc ttc ctt aac cga gtc gtt     1008
   Ser Asp Leu Asn Leu Ser Glu Tyr Gly Cys Phe Leu Asn Arg Val Val
                   325                 330                 335
   cgt gcg caa atc gaa atg ccg atg gat ctg gat atc gtg ctc gcc aaa     1056
   Arg Ala Gln Ile Glu Met Pro Met Asp Leu Asp Ile Val Leu Ala Lys
               340                 345                 350
   ggc gat gtc ctg caa gtc agc ggc gag aaa agc aaa gta aag ggg ctg     1104
   Gly Asp Val Leu Gln Val Ser Gly Glu Lys Ser Lys Val Lys Gly Leu
           355                 360                 365
   gca gat aaa atc ggt ttt atc tct gta cac agc caa atg gct gat ctg     1152
   Ala Asp Lys Ile Gly Phe Ile Ser Val His Ser Gln Met Ala Asp Leu
       370                 375                 380
   ctt gct ttc tgt agc ttt ttt att ctt ggc att ctg ttt ggt ttg gtc     1200
   Leu Ala Phe Cys Ser Phe Phe Ile Leu Gly Ile Leu Phe Gly Leu Val
   385                 390                 395                 400
   acc atg acc ttt ggc caa gtg tcg ttt agc tta ggt aat gcg gtg ggg      1248
   Thr Met Thr Phe Gly Gln Val Ser Phe Ser Leu Gly Asn Ala Val Gly
                   405                 410                 415
   tta ctg ctc tct ggt atc act ttg ggc ttt ttg cga gcc aac cac ccg      1296
   Leu Leu Leu Ser Gly Ile Thr Leu Gly Phe Leu Arg Ala Asn His Pro
               420                 425                 430
   act ttc ggt tat gtt ccg caa ggc gca ctc aat atg gtc aaa gac ctt      1344
   Thr Phe Gly Tyr Val Pro Gln Gly Ala Leu Asn Met Val Lys Asp Leu
           435                 440                 445
   ggt cta gcc atc ttc atg gtc ggt att ggg ctc aat gca ggt agc aaa      1392
   Gly Leu Ala Ile Phe Met Val Gly Ile Gly Leu Asn Ala Gly Ser Lys
       450                 455                 460
   atg ttc cag cat ctc tcc gaa gta ggt gtg caa gtg atc ggt tta gcc      1440
   Met Phe Gln His Leu Ser Glu Val Gly Val Gln Val Ile Gly Leu Ala
   465                 470                 475                 480
   ttt tta gtg agc gtt gta cct gtc gtg ttc gct tac ttg gtt ggc gcc      1488
   Phe Leu Val Ser Val Val Pro Val Val Phe Ala Tyr Leu Val Gly Ala
                   485                 490                 495
   tac att cta aag atg aac cgc gct cta ttg ttt ggt gcg att att ggc      1536
   Tyr Ile Leu Lys Met Asn Arg Ala Leu Leu Phe Gly Ala Ile Ile Gly
               500                 505                 510
   gcg cgc acc tgt gct ccg gcg atg gat gtg gtt aat gaa tac gct aag      1584
   Ala Arg Thr Cys Ala Pro Ala Met Asp Val Val Asn Glu Tyr Ala Lys
           515                 520                 525
   tct acc att cca gca ctg ggt tat gcg ggc acc tac gcg att gcc aat      1632
   Ser Thr Ile Pro Ala Leu Gly Tyr Ala Gly Thr Tyr Ala Ile Ala Asn
       530                 535                 540
   atc tta atg acc tta acg ggt acc atc ttt atc ttg ctc agt taa          1677
   Ile Leu Met Thr Leu Thr Gly Thr Ile Phe Ile Leu Leu Ser
   545                 550                 555
<210> 31
   <211> 558
   <212> PRT
   <213> Vibrio cholerae
<400> 31
   Val Asn Ile Asp Val Val Leu Leu Leu Glu Gln Asn Pro Ile Leu Leu
   1 5 10 15
   Ile Phe Val Val Leu Ala Ile Gly Leu Ser Phe Gly Lys Ile Arg Phe
   20 25 30
   Gly Ser Phe Gln Leu Gly Asn Ser Ile Gly Val Leu Ile Thr Ser Leu
   35 40 45
   Ile Met Gly His Leu Gly Phe Ser Phe Thr Pro Glu Ala Leu Thr Ile
   50 55 60
   Gly Phe Met Leu Phe Ile Tyr Cys Val Gly Ile Glu Ala Gly Pro Asn
   65 70 75 80
   Phe Phe Gly Ile Phe Phe Arg Asp Gly Lys His Tyr Leu Ile Leu Ser
   85 90 95
   Leu Val Val Leu Ile Thr Ala Thr Trp Ile Ala Tyr Phe Gly Gly Tyr
   100 105 110
   Tyr Leu Asn Leu Asp Tyr Gly Leu Ala Ala Gly Met Met Ala Gly Ala
   115 120 125
   Leu Thr Ser Thr Pro Val Leu Val Gly Ala Gln Asp Ala Leu Asn Ser
   130 135 140
   Gly Leu Ala Ala Val Pro Arg His Met Asp Leu Ser Leu Ile Leu Asp
   145 150 155 160
   Asn Val Ser Val Gly Tyr Ala Met Ala Tyr Leu Ile Gly Leu Ile Ser
   165 170 175
   Met Ile Met Phe Ala Lys Leu Leu Pro Lys Leu Gln Lys Gln Asn Leu
   180 185 190
   Ser Asp Ser Ala Gln Gln Ile Ala Gln Glu Arg Gly Leu Gly Ser Gln
   195 200 205
   Arg Lys Val Tyr Leu Pro Ile Ile Arg Ala Tyr Arg Val Gly Pro Glu
   210 215 220
   Leu Ile Asn Trp Ile Asp Gly Arg Asn Leu Arg Glu Leu Gly Ile Tyr
   225 230 235 240
   Arg Gln Thr Gly Cys Tyr Ile Glu Arg Ile Arg Arg His Gly Ile Leu
   245 250 255
   Ala His Pro Asp Gly Asp Ala Ile Leu Gln Glu Gly Asp Glu Ile Ala
   260 265 270
   Leu Val Gly Phe Pro Asp Ser His Ala Arg Leu Asp Pro Ser Phe Arg
   275 280 285
   Asn Gly Lys Glu Val Phe Asp Arg Asn Leu Leu Asp Leu Arg Ile Ser
   290 295 300
   Glu Glu Glu Ile Val Val Lys Ser Asp Ser Ile Ala Gly Lys Arg Leu
   305 310 315 320
   Ser Asp Leu Asn Leu Ser Glu Tyr Gly Cys Phe Leu Asn Arg Val Val
   325 330 335
   Arg Ala Gln Ile Glu Met Pro Met Asp Leu Asp Ile Val Leu Ala Lys
   340 345 350
   Gly Asp Val Leu Gln Val Ser Gly Glu Lys Ser Lys Val Lys Gly Leu
   355 360 365
   Ala Asp Lys Ile Gly Phe Ile Ser Val His Ser Gln Met Ala Asp Leu
   370 375 380
   Leu Ala Phe Cys Ser Phe Phe Ile Leu Gly Ile Leu Phe Gly Leu Val
   385 390 395 400
   Thr Met Thr Phe Gly Gln Val Ser Phe Ser Leu Gly Asn Ala Val Gly
   405 410 415
   Leu Leu Leu Ser Gly Ile Thr Leu Gly Phe Leu Arg Ala Asn His Pro
   420 425 430
   Thr Phe Gly Tyr Val Pro Gln Gly Ala Leu Asn Met Val Lys Asp Leu
   435 440 445
   Gly Leu Ala Ile Phe Met Val Gly Ile Gly Leu Asn Ala Gly Ser Lys
   450 455 460
   Met Phe Gln His Leu Ser Glu Val Gly Val Gln Val Ile Gly Leu Ala
   465 470 475 480
   Phe Leu Val Ser Val Val Pro Val Val Phe Ala Tyr Leu Val Gly Ala
   485 490 495
   Tyr Ile Leu Lys Met Asn Arg Ala Leu Leu Phe Gly Ala Ile Ile Gly
   500 505 510
   Ala Arg Thr Cys Ala Pro Ala Met Asp Val Val Asn Glu Tyr Ala Lys
   515 520 525
   Ser Thr Ile Pro Ala Leu Gly Tyr Ala Gly Thr Tyr Ala Ile Ala Asn
   530 535 540
   Ile Leu Met Thr Leu Thr Gly Thr Ile Phe Ile Leu Leu Ser
   545 550 555
<210> 32
   <211> 1686
   <212> DNA
   <213> Aeromonas hydrophila
<220>
   <221> CDS
   <222> (1)..(1686)
<400> 32
   atg acc ata gat ttt gtg aca ctg cta cat caa agc gac tcc ctg ctg      48
   Met Thr Ile Asp Phe Val Thr Leu Leu His Gln Ser Asp Ser Leu Leu
   1               5                   10                  15
   ctg ttc gtg gta ctg gcc ttc ggc ctg ctg ctc ggt aaa gtc agg ttc      96
   Leu Phe Val Val Leu Ala Phe Gly Leu Leu Leu Gly Lys Val Arg Phe
               20                  25                  30
   ggc aac ttc cag atc ggc aac acc atc ggc gtg ctg ttt acc gcc ctg      144
   Gly Asn Phe Gln Ile Gly Asn Thr Ile Gly Val Leu Phe Thr Ala Leu
           35                  40                  45
   ctg ttc ggc cag atg ggc ttc gaa ttc acc gcc acc acg gag aac gtc      192
   Leu Phe Gly Gln Met Gly Phe Glu Phe Thr Ala Thr Thr Glu Asn Val
       50                  55                  60
   ggc ttc atg ctg ttc atc ttc tgc gtg ggg ata gaa gcg ggg ccg cac      240
   Gly Phe Met Leu Phe Ile Phe Cys Val Gly Ile Glu Ala Gly Pro His
   65                  70                  75                  80
   ttc ttc agc gtc ttc ctg cgt gac ggc atc cac tac atc acc ctc acc      288
   Phe Phe Ser Val Phe Leu Arg Asp Gly Ile His Tyr Ile Thr Leu Thr
   85 90 95
   ctg gtg atc ctg ctg acc gcc ctc ttc ctc acc gtg ggg ctc gcc aag 336
   Leu Val Ile Leu Leu Thr Ala Leu Phe Leu Thr Val Gly Leu Ala Lys
   100 105 110
   ttc ttc aac ctg ggg ccg ggc atg gcg gcc ggc atc ctg gcc ggc tcg 384
   Phe Phe Asn Leu Gly Pro Gly Met Ala Ala Gly Ile Leu Ala Gly Ser
   115 120 125
   ctc acc tcg acc ccg gcg ctg gtg ggt gcg cag gat gcc ctg cgc agc 432
   Leu Thr Ser Thr Pro Ala Leu Val Gly Ala Gln Asp Ala Leu Arg Ser
   130 135 140
   ggc ctg ctc aac ctg ccc cat cag acc gac atg cag tcg gtg ctc gac 480
   Gly Leu Leu Asn Leu Pro His Gln Thr Asp Met Gln Ser Val Leu Asp
   145 150 155 160
   aac atg ggc atc ggc tat gcg ttg acc tat ctg gtg ggg ctg gtc ggc 528
   Asn Met Gly Ile Gly Tyr Ala Leu Thr Tyr Leu Val Gly Leu Val Gly
   165 170 175
   ctg atg ctg gtg gtg cgc tac ctg cct tcg ctg gca cgg ctc gac ctc 576
   Leu Met Leu Val Val Arg Tyr Leu Pro Ser Leu Ala Arg Leu Asp Leu
   180 185 190
   aac acc gag gcg cag aag atc gcc cgt gag cgc ggc ctc tcc gac aac 624
   Asn Thr Glu Ala Gln Lys Ile Ala Arg Glu Arg Gly Leu Ser Asp Asn
   195 200 205
   gag agc cgc aag acc tac ctg ccc atc atc cgt gcc tac cgg gtt ggt 672
   Glu Ser Arg Lys Thr Tyr Leu Pro Ile Ile Arg Ala Tyr Arg Val Gly
   210 215 220
   ccc gag ctc gcc gcc tgg atc ggc ggc cgc acc ctg cgc gag acc ggc 720
   Pro Glu Leu Ala Ala Trp Ile Gly Gly Arg Thr Leu Arg Glu Thr Gly
   225 230 235 240
   atc tac ccc cac acc ggc tgc tac gtg gag cgg atc cgc cgc aac ggc 768
   Ile Tyr Pro His Thr Gly Cys Tyr Val Glu Arg Ile Arg Arg Asn Gly
   245 250 255
   atc ctg gcg agc ccg gat ggc gac gcc gtc atc cag gaa ggg gac gag 816
   Ile Leu Ala Ser Pro Asp Gly Asp Ala Val Ile Gln Glu Gly Asp Glu
   260 265 270
   atc gcc ctg gtg ggt tac ccc gag agc cac gag aag ctc gac gtc aac 864
   Ile Ala Leu Val Gly Tyr Pro Glu Ser His Glu Lys Leu Asp Val Asn
   275 280 285
   tac cgc aac ggc aag gag gtg ttc gac cgc aac ctg ctt gac ctg cag 912
   Tyr Arg Asn Gly Lys Glu Val Phe Asp Arg Asn Leu Leu Asp Leu Gln
   290 295 300
   atc gtc acc gaa gag ata gtg gtg aaa aac gat gcg gtg gtg ggt cgc 960
   Ile Val Thr Glu Glu Ile Val Val Lys Asn Asp Ala Val Val Gly Arg
   305 310 315 320
   cat ctg gtg gag ctc aac ctc acc gag aag ggc tgc ttc ctc aac cgg 1008
   His Leu Val Glu Leu Asn Leu Thr Glu Lys Gly Cys Phe Leu Asn Arg
   325 330 335
   gtg gtg cgc tcc cag atc gag atg ccg ttc gat cgc aac atc atg ctg 1056
   Val Val Arg Ser Gln Ile Glu Met Pro Phe Asp Arg Asn Ile Met Leu
   340 345 350
   caa aag ggc gac gtg ctg cag atc agc ggc gag aag cag cgg gtc aag 1104
   Gln Lys Gly Asp Val Leu Gln Ile Ser Gly Glu Lys Gln Arg Val Lys
   355 360 365
   ctg ctg gcc aac aag att ggc ttc atc agc atc cac agc cag acc acg 1152
   Leu Leu Ala Asn Lys Ile Gly Phe Ile Ser Ile His Ser Gln Thr Thr
   370 375 380
   gat ctg gtg gcc ttc acc acc ttc ttc gtg ctg ggg ctg ctg ata ggc 1200
   Asp Leu Val Ala Phe Thr Thr Phe Phe Val Leu Gly Leu Leu Ile Gly
   385 390 395 400
   tcc gtc tcc ctg gtg ttc ggc cag ctc gag ttt ggg ctg ggc aac gcc 1248
   Ser Val Ser Leu Val Phe Gly Gln Leu Glu Phe Gly Leu Gly Asn Ala
   405 410 415
   gtc ggc ctg ctg ctg gcg ggg atc ctg atg ggc tac ctg cgc gcc aac 1296
   Val Gly Leu Leu Leu Ala Gly Ile Leu Met Gly Tyr Leu Arg Ala Asn
   420 425 430
   cac ccc acc gtc ggc tac gtg ccg ccg ggc gcc ctg cgt ctg gcc aag 1344
   His Pro Thr Val Gly Tyr Val Pro Pro Gly Ala Leu Arg Leu Ala Lys
   435 440 445
   gat ctc ggt ctg gcg gtg ttc atg gtg agc acc ggc ctc aaa gct ggc 1392
   Asp Leu Gly Leu Ala Val Phe Met Val Ser Thr Gly Leu Lys Ala Gly
       450                 455                 460
   ggc ggc att ctg gac cac ctg agc cag gtc ggt gcc gtg gtg ctc ttt      1440
   Gly Gly Ile Leu Asp His Leu Ser Gln Val Gly Ala Val Val Leu Phe
   465                 470                 475                 480
   tcc ggc atg ctg gtg acc acc ctg ccg gtg ctg gtg ggc tac ctg ttc      1488
   Ser Gly Met Leu Val Thr Thr Leu Pro Val Leu Val Gly Tyr Leu Phe
                   485                 490                 495
   gga gtc tgg gtg ctg aag atg aac ccg gcc ctg ctg ctc ggc gcc atc      1536
   Gly Val Trp Val Leu Lys Met Asn Pro Ala Leu Leu Leu Gly Ala Ile
               500                 505                 510
   acg ggg gcc cgc acc tgc gcc ccg gcc atg gac gtg gtg aac gag gcg      1584
   Thr Gly Ala Arg Thr Cys Ala Pro Ala Met Asp Val Val Asn Glu Ala
           515                 520                 525
   gcc aac agc tcc atc ccg gcg ctc ggc tat gcc ggc acc tat gcg gtg      1632
   Ala Asn Ser Ser Ile Pro Ala Leu Gly Tyr Ala Gly Thr Tyr Ala Val
       530                 535                 540
   gcc aac gtc atg ctg acc ttg gcg ggc tcc ttc atc atc ggc ttc tgg      1680
   Ala Asn Val Met Leu Thr Leu Ala Gly Ser Phe Ile Ile Gly Phe Trp
   545                 550                 555                 560
   ttc tag                                                              1686
   Phe
<210> 33
   <211> 561
   <212> PRT
   <213> Aeromonas hydrophila
<400> 33
   Met Thr Ile Asp Phe Val Thr Leu Leu His Gln Ser Asp Ser Leu Leu
   1               5                   10                  15
   Leu Phe Val Val Leu Ala Phe Gly Leu Leu Leu Gly Lys Val Arg Phe
               20                  25                  30
   Gly Asn Phe Gln Ile Gly Asn Thr Ile Gly Val Leu Phe Thr Ala Leu
           35                  40                  45
   Leu Phe Gly Gln Met Gly Phe Glu Phe Thr Ala Thr Thr Glu Asn Val
       50                  55                  60
   Gly Phe Met Leu Phe Ile Phe Cys Val Gly Ile Glu Ala Gly Pro His
   65                  70                  75                  80
   Phe Phe Ser Val Phe Leu Arg Asp Gly Ile His Tyr Ile Thr Leu Thr
                   85                  90                  95
   Leu Val Ile Leu Leu Thr Ala Leu Phe Leu Thr Val Gly Leu Ala Lys
               100                 105                 110
   Phe Phe Asn Leu Gly Pro Gly Met Ala Ala Gly Ile Leu Ala Gly Ser
           115                 120                 125
   Leu Thr Ser Thr Pro Ala Leu Val Gly Ala Gln Asp Ala Leu Arg Ser
       130                 135                 140
   Gly Leu Leu Asn Leu Pro His Gln Thr Asp Met Gln Ser Val Leu Asp
   145                 150                 155                 160
   Asn Met Gly Ile Gly Tyr Ala Leu Thr Tyr Leu Val Gly Leu Val Gly
                   165                 170                 175
   Leu Met Leu Val Val Arg Tyr Leu Pro Ser Leu Ala Arg Leu Asp Leu
               180                 185                 190
   Asn Thr Glu Ala Gln Lys Ile Ala Arg Glu Arg Gly Leu Ser Asp Asn
           195                 200                 205
   Glu Ser Arg Lys Thr Tyr Leu Pro Ile Ile Arg Ala Tyr Arg Val Gly
       210                 215                 220
   Pro Glu Leu Ala Ala Trp Ile Gly Gly Arg Thr Leu Arg Glu Thr Gly
   225                 230                 235                 240
   Ile Tyr Pro His Thr Gly Cys Tyr Val Glu Arg Ile Arg Arg Asn Gly
                   245                 250                 255
   Ile Leu Ala Ser Pro Asp Gly Asp Ala Val Ile Gln Glu Gly Asp Glu
               260                 265                 270
   Ile Ala Leu Val Gly Tyr Pro Glu Ser His Glu Lys Leu Asp Val Asn
           275                 280                 285
   Tyr Arg Asn Gly Lys Glu Val Phe Asp Arg Asn Leu Leu Asp Leu Gln
       290                 295                 300
   Ile Val Thr Glu Glu Ile Val Val Lys Asn Asp Ala Val Val Gly Arg
   305                 310                 315                 320
   His Leu Val Glu Leu Asn Leu Thr Glu Lys Gly Cys Phe Leu Asn Arg
                   325                 330                 335
   Val Val Arg Ser Gln Ile Glu Met Pro Phe Asp Arg Asn Ile Met Leu
               340                 345                 350
   Gln Lys Gly Asp Val Leu Gln Ile Ser Gly Glu Lys Gln Arg Val Lys
           355                 360                 365
   Leu Leu Ala Asn Lys Ile Gly Phe Ile Ser Ile His Ser Gln Thr Thr
       370                 375                 380
   Asp Leu Val Ala Phe Thr Thr Phe Phe Val Leu Gly Leu Leu Ile Gly
   385                 390                 395                 400
   Ser Val Ser Leu Val Phe Gly Gln Leu Glu Phe Gly Leu Gly Asn Ala
                   405                 410                 415
   Val Gly Leu Leu Leu Ala Gly Ile Leu Met Gly Tyr Leu Arg Ala Asn
               420                 425                 430
   His Pro Thr Val Gly Tyr Val Pro Pro Gly Ala Leu Arg Leu Ala Lys
           435                 440                 445
   Asp Leu Gly Leu Ala Val Phe Met Val Ser Thr Gly Leu Lys Ala Gly
       450                 455                 460
   Gly Gly Ile Leu Asp His Leu Ser Gln Val Gly Ala Val Val Leu Phe
   465                 470                 475                 480
   Ser Gly Met Leu Val Thr Thr Leu Pro Val Leu Val Gly Tyr Leu Phe
                   485                 490                 495
   Gly Val Trp Val Leu Lys Met Asn Pro Ala Leu Leu Leu Gly Ala Ile
               500                 505                 510
   Thr Gly Ala Arg Thr Cys Ala Pro Ala Met Asp Val Val Asn Glu Ala
           515                 520                 525
   Ala Asn Ser Ser Ile Pro Ala Leu Gly Tyr Ala Gly Thr Tyr Ala Val
       530                 535                 540
   Ala Asn Val Met Leu Thr Leu Ala Gly Ser Phe Ile Ile Gly Phe Trp
   545                 550                 555                 560
   Phe
<210> 34
   <211> 1638
   <212> DNA
   <213> Photobacterium pr of undum
<220>
   <221> CDS
   <222> (1)..(1638)
<400> 34
   ttg ctt ttt gtt gtt ctc gct gtt ggt cta agt ttt ggt aaa att cgt      48
   Leu Leu Phe Val Val Leu Ala Val Gly Leu Ser Phe Gly Lys Ile Arg
   1               5                   10                  15
   ttt gct aag atg caa gta ggc aat tct att ggt gtt tta ctg act gcc      96
   Phe Ala Lys Met Gln Val Gly Asn Ser Ile Gly Val Leu Leu Thr Ala
               20                  25                  30
   ata ctg ttc ggt aat gcc ggg ttt acc ttt aat act gaa gca tta aat     144
   Ile Leu Phe Gly Asn Ala Gly Phe Thr Phe Asn Thr Glu Ala Leu Asn
           35                  40                  45
   att ggc ttt atg ctg ttt att ttt tgt gtg ggt ata gag gct ggc ccc     192
   Ile Gly Phe Met Leu Phe Ile Phe Cys Val Gly Ile Glu Ala Gly Pro
       50                  55                  60
   aac ttt ttt ggt att ttc ttc cga gat ggt aaa cat tac ctt tta ctc     240
   Asn Phe Phe Gly Ile Phe Phe Arg Asp Gly Lys His Tyr Leu Leu Leu
   65                  70                  75                  80
   gcc ctt gtt gtg ctg ctt tcc gcc ata gct atc act tta gcc atg act     288
   Ala Leu Val Val Leu Leu Ser Ala Ile Ala Ile Thr Leu Ala Met Thr
                   85                  90                  95
   cat tat ctt ggg ctt gat ata ggt tta gcg aca ggc tta atg gct ggt     336
   His Tyr Leu Gly Leu Asp Ile Gly Leu Ala Thr Gly Leu Met Ala Gly
               100                 105                 110
   tca ttg aca gca aca cct gtt ctt gta ggt gca aaa gac gca ttg aat     384
   Ser Leu Thr Ala Thr Pro Val Leu Val Gly Ala Lys Asp Ala Leu Asn
               115                     120                     125
   tcc gga tta tct ggc att agc gat cct gat atc att aaa caa acc att      432
   Ser Gly Leu Ser Gly Ile Ser Asp Pro Asp Ile Ile Lys Gln Thr Ile
       130                     135                     140
   gat agc cta agc gtt ggc tat gct atg tct tat tta atg ggc tta atc      480
   Asp Ser Leu Ser Val Gly Tyr Ala Met Ser Tyr Leu Met Gly Leu Ile
   145                     150                     155                 160
   agt ctt att ttt ctc gct aaa cta atg cct aaa tta cag aaa caa gac      528
   Ser Leu Ile Phe Leu Ala Lys Leu Met Pro Lys Leu Gln Lys Gln Asp
                       165                     170                 175
   ctg gct gaa tcg tcg caa caa att gcc cgt gaa cgc ggt att ggt gaa      576
   Leu Ala Glu Ser Ser Gln Gln Ile Ala Arg Glu Arg Gly Ile Gly Glu
               180                     185                     190
   gtg ggg caa cgt aaa gta tat ttg cca atc att cgc gct tac cgt gtt      624
   Val Gly Gln Arg Lys Val Tyr Leu Pro Ile Ile Arg Ala Tyr Arg Val
           195                     200                     205
   ggc cct gag ctt att aac tgg att gat agt cgt aac tta cgt gag ttg      672
   Gly Pro Glu Leu Ile Asn Trp Ile Asp Ser Arg Asn Leu Arg Glu Leu
       210                     215                     220
   ggc atc tac cgc caa aca ggc tgc tac atc gaa cgt ata cgg cga aac      720
   Gly Ile Tyr Arg Gln Thr Gly Cys Tyr Ile Glu Arg Ile Arg Arg Asn
   225                     230                     235                 240
   ggt ata tta gct aac cca gat ggt gat gct att tta caa gaa ggt gat      768
   Gly Ile Leu Ala Asn Pro Asp Gly Asp Ala Ile Leu Gln Glu Gly Asp
                       245                     250                 255
   gag att gcc tta gtg ggt tac cca gac agc cat gca cgt ttg gat ccc      816
   Glu Ile Ala Leu Val Gly Tyr Pro Asp Ser His Ala Arg Leu Asp Pro
               260                     265                     270
   agc ttt cga aac ggc aaa gaa gtt ttc gac cgt gat ctt ctc gac ctt      864
   Ser Phe Arg Asn Gly Lys Glu Val Phe Asp Arg Asp Leu Leu Asp Leu
           275                     280                     285
   cgt att gtt gaa gaa gag att gta gtt aag aat gac aac atc tct ggc      912
   Arg Ile Val Glu Glu Glu Ile Val Val Lys Asn Asp Asn Ile Ser Gly
       290                     295                     300
   aaa cgc cta tct gag cta aat tta tca gaa tac ggt tgt ttc ttg aac      960
   Lys Arg Leu Ser Glu Leu Asn Leu Ser Glu Tyr Gly Cys Phe Leu Asn
   305                     310                     315                 320
   cgt gtt gtt cgc gca caa ata gaa atg ccg atg gac cat aat att ctt     1008
   Arg Val Val Arg Ala Gln Ile Glu Met Pro Met Asp His Asn Ile Leu
                       325                     330                 335
   ttg aat aaa ggg gat att ttg caa gtc agt gga gaa aaa agt cga gta     1056
   Leu Asn Lys Gly Asp Ile Leu Gln Val Ser Gly Glu Lys Ser Arg Val
               340                     345                     350
   ctt ggt ctt gct gaa cgt att ggt ttt atc tcg att cac agt caa att     1104
   Leu Gly Leu Ala Glu Arg Ile Gly Phe Ile Ser Ile His Ser Gln Ile
           355                     360                     365
   gcc gat cta tta gca ttc tgc tgt ttt ttt atc atc ggg tta ctt atc     1152
   Ala Asp Leu Leu Ala Phe Cys Cys Phe Phe Ile Ile Gly Leu Leu Ile
       370                     375                     380
   ggt tcg att aca tta gca ttt ggt cac gtt gca ttt ggt tta ggt agt     1200
   Gly Ser Ile Thr Leu Ala Phe Gly His Val Ala Phe Gly Leu Gly Ser
   385                     390                     395                 400
   gct gca ggg ctt ctg att gcc ggt att aca tta gga ttc cta cgt gct     1248
   Ala Ala Gly Leu Leu Ile Ala Gly Ile Thr Leu Gly Phe Leu Arg Ala
                       405                     410                 415
   aac cac cct act ttt ggc tat gta ccc caa ggt gcg ctt aat atg gcg     1296
   Asn His Pro Thr Phe Gly Tyr Val Pro Gln Gly Ala Leu Asn Met Ala
               420                     425                     430
   aaa gat ctt ggc tta atg gtc ttc atg gta gga att ggt ttg agt gcg     1344
   Lys Asp Leu Gly Leu Met Val Phe Met Val Gly Ile Gly Leu Ser Ala
           435                     440                     445
   ggt tct aat tta ttc gat tca ttt gca cat att ggc cct atg gtc tta     1392
   Gly Ser Asn Leu Phe Asp Ser Phe Ala His Ile Gly Pro Met Val Leu
       450                     455                     460
   gtc acc agc tta atg gtg agt gtg atc cct gtt gta ttg gca tac ctg     1440
   Val Thr Ser Leu Met Val Ser Val Ile Pro Val Val Leu Ala Tyr Leu
   465                     470                     475                 480
   ttt ggt gct tac gta ctg aag atg aac cgt gca ctt tta ttt ggt gcc     1488
   Phe Gly Ala Tyr Val Leu Lys Met Asn Arg Ala Leu Leu Phe Gly Ala
                       485                     490                     495
   atc att ggt gct cga act tgt gca cct gcg atg gat atg atc aat gaa        1536
   Ile Ile Gly Ala Arg Thr Cys Ala Pro Ala Met Asp Met Ile Asn Glu
                   500                     505                     510
   cat gct cga agc aca att cca gct tta ggc tat gct ggt act tat gcc        1584
   His Ala Arg Ser Thr Ile Pro Ala Leu Gly Tyr Ala Gly Thr Tyr Ala
               515                     520                     525
   att gcc aac gta ctg tta acg att gcc ggt acg tta atc atc atc atg        1632
   Ile Ala Asn Val Leu Leu Thr Ile Ala Gly Thr Leu Ile Ile Ile Met
           530                     535                     540
   aat taa                                                                1638
   Asn
   545
<210> 35
   <211> 545
   <212> PRT
   <213> Photobacterium profundum
<400> 35
   Leu Leu Phe Val Val Leu Ala Val Gly Leu Ser Phe Gly Lys Ile Arg
   1 5 10 15
   Phe Ala Lys Met Gln Val Gly Asn Ser Ile Gly Val Leu Leu Thr Ala
   20 25 30
   Ile Leu Phe Gly Asn Ala Gly Phe Thr Phe Asn Thr Glu Ala Leu Asn
   35 40 45
   Ile Gly Phe Met Leu Phe Ile Phe Cys Val Gly Ile Glu Ala Gly Pro
   50 55 60
   Asn Phe Phe Gly Ile Phe Phe Arg Asp Gly Lys His Tyr Leu Leu Leu
   65 70 75 80
   Ala Leu Val Val Leu Leu Ser Ala Ile Ala Ile Thr Leu Ala Met Thr
   85 90 95
   His Tyr Leu Gly Leu Asp Ile Gly Leu Ala Thr Gly Leu Met Ala Gly
   100 105 110
   Ser Leu Thr Ala Thr Pro Val Leu Val Gly Ala Lys Asp Ala Leu Asn
   115 120 125
   Ser Gly Leu Ser Gly Ile Ser Asp Pro Asp Ile Ile Lys Gln Thr Ile
   130 135 140
   Asp Ser Leu Ser Val Gly Tyr Ala Met Ser Tyr Leu Met Gly Leu Ile
   145 150 155 160
   Ser Leu Ile Phe Leu Ala Lys Leu Met Pro Lys Leu Gln Lys Gln Asp
   165 170 175
   Leu Ala Glu Ser Ser Gln Gln Ile Ala Arg Glu Arg Gly Ile Gly Glu
   180 185 190
   Val Gly Gln Arg Lys Val Tyr Leu Pro Ile Ile Arg Ala Tyr Arg Val
   195 200 205
   Gly Pro Glu Leu Ile Asn Trp Ile Asp Ser Arg Asn Leu Arg Glu Leu
   210 215 220
   Gly Ile Tyr Arg Gln Thr Gly Cys Tyr Ile Glu Arg Ile Arg Arg Asn
   225 230 235 240
   Gly Ile Leu Ala Asn Pro Asp Gly Asp Ala Ile Leu Gln Glu Gly Asp
   245 250 255
   Glu Ile Ala Leu Val Gly Tyr Pro Asp Ser His Ala Arg Leu Asp Pro
   260 265 270
   Ser Phe Arg Asn Gly Lys Glu Val Phe Asp Arg Asp Leu Leu Asp Leu
   275 280 285
   Arg Ile Val Glu Glu Glu Ile Val Val Lys Asn Asp Asn Ile Ser Gly
   290 295 300
   Lys Arg Leu Ser Glu Leu Asn Leu Ser Glu Tyr Gly Cys Phe Leu Asn
   305 310 315 320
   Arg Val Val Arg Ala Gln Ile Glu Met Pro Met Asp His Asn Ile Leu
   325 330 335
   Leu Asn Lys Gly Asp Ile Leu Gln Val Ser Gly Glu Lys Ser Arg Val
   340 345 350
   Leu Gly Leu Ala Glu Arg Ile Gly Phe Ile Ser Ile His Ser Gln Ile
   355 360 365
   Ala Asp Leu Leu Ala Phe Cys Cys Phe Phe Ile Ile Gly Leu Leu Ile
   370 375 380
   Gly Ser Ile Thr Leu Ala Phe Gly His Val Ala Phe Gly Leu Gly Ser
   385                 390                 395                 400
   Ala Ala Gly Leu Leu Ile Ala Gly Ile Thr Leu Gly Phe Leu Arg Ala
                   405                 410                 415
   Asn His Pro Thr Phe Gly Tyr Val Pro Gln Gly Ala Leu Asn Met Ala
               420                 425                 430
   Lys Asp Leu Gly Leu Met Val Phe Met Val Gly Ile Gly Leu Ser Ala
           435                 440                 445
   Gly Ser Asn Leu Phe Asp Ser Phe Ala His Ile Gly Pro Met Val Leu
       450                 455                 460
   Val Thr Ser Leu Met Val Ser Val Ile Pro Val Val Leu Ala Tyr Leu
   465                 470                 475                 480
   Phe Gly Ala Tyr Val Leu Lys Met Asn Arg Ala Leu Leu Phe Gly Ala
                   485                 490                 495
   Ile Ile Gly Ala Arg Thr Cys Ala Pro Ala Met Asp Met Ile Asn Glu
               500                 505                 510
   His Ala Arg Ser Thr Ile Pro Ala Leu Gly Tyr Ala Gly Thr Tyr Ala
           515                 520                 525
   Ile Ala Asn Val Leu Leu Thr Ile Ala Gly Thr Leu Ile Ile Ile Met
       530                 535                 540
   Asn
   545
<210> 36
   <211> 990
   <212> DNA
   <213> Escherichia coli
<220>
   <221> CDS
   <222> (1)..(990)
<400> 36
   atg aaa ctc gcc gtt tat agc aca aaa cag tac gac aag aag tac ctg      48
   Met Lys Leu Ala Val Tyr Ser Thr Lys Gln Tyr Asp Lys Lys Tyr Leu
   1               5                   10                  15
   caa cag gtg aac gag tcc ttt ggc ttt gag ctg gaa ttt ttt gac ttt      96
   Gln Gln Val Asn Glu Ser Phe Gly Phe Glu Leu Glu Phe Phe Asp Phe
               20                  25                  30
   ctg ctg acg gaa aaa acc gct aaa act gcc aat ggc tgc gaa gcg gta     144
   Leu Leu Thr Glu Lys Thr Ala Lys Thr Ala Asn Gly Cys Glu Ala Val
           35                  40                  45
   tgt att ttc gta aac gat gac ggc agc cgc ccg gtg ctg gaa gag ctg     192
   Cys Ile Phe Val Asn Asp Asp Gly Ser Arg Pro Val Leu Glu Glu Leu
       50                  55                  60
   aaa aag cac ggc gtt aaa tat atc gcc ctg cgc tgt gcc ggt ttc aat     240
   Lys Lys His Gly Val Lys Tyr Ile Ala Leu Arg Cys Ala Gly Phe Asn
   65                  70                  75                  80
   aac gtc gac ctt gac gcg gca aaa gaa ctg ggg ctg aaa gta gtc cgt     288
   Asn Val Asp Leu Asp Ala Ala Lys Glu Leu Gly Leu Lys Val Val Arg
                   85                  90                  95
   gtt cca gcc tat gat cca gag gcc gtt gct gaa cac gcc atc ggt atg     336
   Val Pro Ala Tyr Asp Pro Glu Ala Val Ala Glu His Ala Ile Gly Met
               100                 105                 110
   atg atg acg ctg aac cgc cgt att cac cgc gcg tat cag cgt acc cgt     384
   Met Met Thr Leu Asn Arg Arg Ile His Arg Ala Tyr Gln Arg Thr Arg
           115                 120                 125
   gat gct aac ttc tct ctg gaa ggt ctg acc ggc ttt act atg tat ggc     432
   Asp Ala Asn Phe Ser Leu Glu Gly Leu Thr Gly Phe Thr Met Tyr Gly
       130                 135                 140
   aaa acg gca ggc gtt atc ggt acc ggt aaa atc ggt gtg gcg atg ctg     480
   Lys Thr Ala Gly Val Ile Gly Thr Gly Lys Ile Gly Val Ala Met Leu
   145                 150                 155                 160
   cgc att ctg aaa ggt ttt ggt atg cgt ctg ctg gcg ttc gat ccg tat     528
   Arg Ile Leu Lys Gly Phe Gly Met Arg Leu Leu Ala Phe Asp Pro Tyr
                   165                 170                 175
   cca agt gca gcg gcg ctg gaa ctc ggt gtg gag tat gtc gat ctg cca     576
   Pro Ser Ala Ala Ala Leu Glu Leu Gly Val Glu Tyr Val Asp Leu Pro
               180                 185                 190
   acc ctg ttc tct gaa tca gac gtt atc tct ctg cac tgc ccg ctg aca     624
   Thr Leu Phe Ser Glu Ser Asp Val Ile Ser Leu His Cys Pro Leu Thr
           195                 200                 205
   ccg gaa aac tat cat ctg ttg aac gaa gcc gcc ttc gaa cag atg aaa     672
   Pro Glu Asn Tyr His Leu Leu Asn Glu Ala Ala Phe Glu Gln Met Lys
       210                 215                 220
   aat ggc gtg atg atc gtc aat acc agt cgc ggt gca ttg att gat tct     720
   Asn Gly Val Met Ile Val Asn Thr Ser Arg Gly Ala Leu Ile Asp Ser
   225                 230                 235                 240
   cag gca gca att gaa gcg ctg aaa aat cag aaa att ggt tcg ttg ggt     768
   Gln Ala Ala Ile Glu Ala Leu Lys Asn Gln Lys Ile Gly Ser Leu Gly
                   245                 250                 255
   atg gac gtg tat gag aac gaa cgc gat cta ttc ttt gaa gat aaa tcc     816
   Met Asp Val Tyr Glu Asn Glu Arg Asp Leu Phe Phe Glu Asp Lys Ser
               260                 265                 270
   aac gac gtg atc cag gat gac gta ttc cgt cgc ctg tct gcc tgc cac     864
   Asn Asp Val Ile Gln Asp Asp Val Phe Arg Arg Leu Ser Ala Cys His
           275                 280                 285
   aac gtg ctg ttt acc ggg cac cag gca ttc ctg aca gca gaa gct ctg     912
   Asn Val Leu Phe Thr Gly His Gln Ala Phe Leu Thr Ala Glu Ala Leu
       290                 295                 300
   acc agt att tct cag act acg ctg caa aac tta agc aat ctg gaa aaa     960
   Thr Ser Ile Ser Gln Thr Thr Leu Gln Asn Leu Ser Asn Leu Glu Lys
   305                 310                 315                 320
   ggc gaa acc tgc ccg aac gaa ctg gtt taa                             990
   Gly Glu Thr Cys Pro Asn Glu Leu Val
                   325
<210> 37
   <211> 329
   <212> PRT
   <213> Escherichia coli
<400> 37
   Met Lys Leu Ala Val Tyr Ser Thr Lys Gln Tyr Asp Lys Lys Tyr Leu
   1 5 10 15
   Gln Gln Val Asn Glu Ser Phe Gly Phe Glu Leu Glu Phe Phe Asp Phe
   20 25 30
   Leu Leu Thr Glu Lys Thr Ala Lys Thr Ala Asn Gly Cys Glu Ala Val
   35 40 45
   Cys Ile Phe Val Asn Asp Asp Gly Ser Arg Pro Val Leu Glu Glu Leu
   50 55 60
   Lys Lys His Gly Val Lys Tyr Ile Ala Leu Arg Cys Ala Gly Phe Asn
   65 70 75 80
   Asn Val Asp Leu Asp Ala Ala Lys Glu Leu Gly Leu Lys Val Val Arg
   85 90 95
   Val Pro Ala Tyr Asp Pro Glu Ala Val Ala Glu His Ala Ile Gly Met
   100 105 110
   Met Met Thr Leu Asn Arg Arg Ile His Arg Ala Tyr Gln Arg Thr Arg
   115 120 125
   Asp Ala Asn Phe Ser Leu Glu Gly Leu Thr Gly Phe Thr Met Tyr Gly
   130 135 140
   Lys Thr Ala Gly Val Ile Gly Thr Gly Lys Ile Gly Val Ala Met Leu
   145 150 155 160
   Arg Ile Leu Lys Gly Phe Gly Met Arg Leu Leu Ala Phe Asp Pro Tyr
   165 170 175
   Pro Ser Ala Ala Ala Leu Glu Leu Gly Val Glu Tyr Val Asp Leu Pro
   180 185 190
   Thr Leu Phe Ser Glu Ser Asp Val Ile Ser Leu His Cys Pro Leu Thr
   195 200 205
   Pro Glu Asn Tyr His Leu Leu Asn Glu Ala Ala Phe Glu Gln Met Lys
   210 215 220
   Asn Gly Val Met Ile Val Asn Thr Ser Arg Gly Ala Leu Ile Asp Ser
   225 230 235 240
   Gln Ala Ala Ile Glu Ala Leu Lys Asn Gln Lys Ile Gly Ser Leu Gly
   245 250 255
   Met Asp Val Tyr Glu Asn Glu Arg Asp Leu Phe Phe Glu Asp Lys Ser
   260 265 270
   Asn Asp Val Ile Gln Asp Asp Val Phe Arg Arg Leu Ser Ala Cys His
   275 280 285
   Asn Val Leu Phe Thr Gly His Gln Ala Phe Leu Thr Ala Glu Ala Leu
   290 295 300
   Thr Ser Ile Ser Gln Thr Thr Leu Gln Asn Leu Ser Asn Leu Glu Lys
   305 310 315 320
   Gly Glu Thr Cys Pro Asn Glu Leu Val
   325
<210> 38
   <211> 1191
   <212> DNA
   <213> Escheri chi a coli
<220>
   <221> CDS
   <222> (1)..(1191)
<400> 38
   atg att att tcc gca gcc agc gat tat cgc gcc gca gcg caa cgc att      48
   ctg ccg ccg ttc ctg ttc cac tat atg gat ggt ggt gca tat tct gaa      96
   tac acg ctg cgc cgc aac gtg gaa gat ttg tca gaa gtg gcg ctg cgc     144
   cag cgt att ctg aaa aac atg tcc gac tta agc ctg gaa acg acg ctg     192
   ttt aat gag aaa ttg tcg atg ccg gtg gca ctg gct ccg gtg ggt ttg     240
   tgt ggc atg tat gcg cgt cgt ggc gaa gtt cag gca gcc aaa gcg gcg     288
   gac gcg cat ggt att ccg ttt act ctc tcg acg gtt tcc gtt tgc ccg     336
   att gaa gaa gtc gcg cca gcc atc aag cgc cca atg tgg ttc cag ctt     384
   tat gta ctg cgc gat cgc ggc ttt atg cgt aac gcg ctg gag cga gca     432
   aaa gca gcg ggt tgt tcg acg ctg gtt ttc acc gtg gat atg ccg aca     480
   ccg ggc gca cgc tac cgt gat gcg cat tca ggt atg agc ggc ccg aac     528
   gcg gca atg cgc cgc tac ttg caa gcg gtg aca cat ccg caa tgg gcg     576
   tgg gat gtg ggc ctg aac ggt cgt cca cat gat tta ggt aat atc tca     624
   gct tat ctc ggc aaa ccg acc gga ctg gaa gat tac atc ggc tgg ctg     672
   ggg aat aac ttc gat ccg tcc atc tca tgg aaa gac ctt gaa tgg atc     720
   cgc gat ttc tgg gat ggc ccg atg gtg atc aaa ggg atc ctc gat ccg     768
   gaa gat gcg cgc gat gca gta cgt ttt ggt gct gat gga att gtg gtt      816
   tct aac cac ggt ggc cgc cag ctg gac ggt gta ctc tct tcc gcc cgt      864
   gca ctg cct gct att gca gat gcg gtg aaa ggt gat ata gcc att ctg      912
   gcg gat agc gga att cgt aac ggg ctt gat gtc gtg cgt atg att gcg      960
   ctc ggt gcc gac acc gta ctg ctg ggt cgt gct ttc ttg tat gcg ctg     1008
   gca aca gcg ggc cag gcg ggt gta gct aac ctg cta aat ctg atc gaa     1056
   aaa gag atg aaa gtg gcg atg acg ctg act ggc gcg aaa tcg atc agc     1104
   gaa att acg caa gat tcg ctg gtg cag ggg ctg ggt aaa gag ttg cct     1152
   gcg gca ctg gct ccc atg gcg aaa ggg aat gcg gca tag                 1191
<210> 39
   <211> 396
   <212> PRT
   <213> Escherichia coli
<400> 39
<210> 40
   <211> 1308
   <212> DNA
   <213> Pant oea ananatis
<400> 40
   atgagcagaa tcatgacgcc cgtgaactgg gaagcctgca gcagcgaggc gcagcaggcg 60
   ctgttggcac gccctgcgct cgcctcgtct gacagcatca gccagatcgt gcgcgatgtg 120
   ttggtcagag tgaaagagga aggcgatgcg gctttacgag aattcagcgc gcgctttgac 180
   aaggttgaaa cagacgacct gcgcgttacg ccacagcaga tgcaggcggc cagcgatcgc 240
   cttggtgacg agctgaaaca ggcgatggcc gtggccattg gcaatattga aacctttcac 300
   cgtgcgcaga tcctgccgcc ggtggatgtg gaaacgcagc ccggcgtgcg ctgtcagcaa 360
   attacgcgcc cgatgaaatc ggtgggcttg tatattccgg gcggttctgc cccgctgttt 420
   tctaccgttc tgatgctggc taccccggcg cggattgcgg gctgtggtcg cgtggtgctg 480
   tgctcgcccc cgccgattgc tgatgaaatt ctctacgcgg ccaaactttg cggtgtggaa 540
   gaagtgttcc aggtgggtgg atcacaggcg attgccgccc tggcttttgg caccgaaagc 600
   atccctaagg tagataaaat ttttggtccg ggcaacgcgt gggttaccga agccaaacgt 660
   caggtcagcc agcgccttga tggcgcggcg attgatatgc ccgctggccc gtcggaagtg 720
   ctggtgattg ccgatgaagg tgccacaccg gccttcgttg cctctgatct gctgtcgcag 780
   gcggaacacg gccctgactc gcaggtgatt ttactgacgc cttcgctggc gctggccgag 840
   cgcgtcgccg aggcggtgga ggatcagctg gcccagttgc cacgtgcggc gacagcccgc 900
   caggcactgg aaagcagccg cctgatcgtc gcccgggata tgcagcaatg cattgcgatc 960
   tccaaccgct atggtccgga gcacctgatt ctgcaaaccc gcacgccacg ggatctggtg 1020
   gaacagatta ccagcgccgg ttcggttttc ctgggcgact ggtcaccgga atccgcagga 1080
   gattatgctt cgggcaccaa ccacgtgctg ccgacctacg gctataccgc gacatgctcc 1140
   agcctgggcc tggccgactt tcagaaacgc atgacggtac aggagctgac gccgcagggc 1200
   ttcctgaacc tggcggcgac catcgaaacc ctggcggccg ctgaacagct gcacgcccac 1260
   aaaaatgccg tcacgttgcg cgttgccgca ctcaaggagc aagcatga 1308
<210> 41
   <211> 68
   <212> DNA
   <213> Artificial Sequence
<220>
   <223> primer
<400> 41
   ccatagcggt tggagatcgc aatgcattgc tgcatatccc tgaagcctgc ttttttatac 60
   taagttgg 68
<210> 42
   <211> 68
   <212> DNA
   <213> Artificial Sequence
<220>
   <223> primer
<400> 42
   gcccgccagg cactggaaag cagccgcctg atcgtcgccc cgctcaagtt agtataaaaa 60
   agctgaac 68
<210> 43
   <211> 33
   <212> DNA
   <213> Artificial Sequence
<220>
   <223> primer
<400> 43
   t agcgagat c t ct gat gt cc ggcggt gct t t t g 33
<210> 44
   <211> 32
   <212> DNA
   <213> Artificial Sequence
<220>
   <223> primer
<400> 44
   aaaaagagct cttacgcccc gccctgccac t c 32
<210> 45
   <211> 34
   <212> DNA
   <213> Artificial Sequence
<220>
   <223> primer
<400> 45
   caggat ct ag aaggagacat gaacgat gaa cat c 34
<210> 46
   <211> 36
   <212> DNA
   <213> Artificial Sequence
<220>
   <223> primer
<400> 46
   gataaggatc c cgaaat aaaa gaaaatgcca atagga 36
<210> 47
   <211> 31
   <212> DNA
   <213> Artificial Sequence
<220>
   <223> primer
<400> 47
   cct t t gagct cgcgggcagt gagcgcaacg c 31
<210> 48
   <211> 48
   <212> DNA
   <213> Artificial Sequence
<220>
   <223> primer
<400> 48
   ctagagcggc cgccgatcgg gatcctcctg tgtgaaattg t t at ccgc 48
<210> 49
   <211> 42
   <212> DNA
   <213> Artiticial Sequence
<220>
   <223> primer
<400> 49
   ct ct acgat c gaggaggt t a t aaaaaat gg at at t aat ac t g 42
<210> 50
   <211> 36
   <212> DNA
   <213> Artificial Sequence
<220>
   <223> primer
<400> 50
   t caaagcggc cgct t ct t cg t ct gt t t ct a ct ggt a 36
<210> 51
   <211> 31
   <212> DNA
   <213> Artificial Sequence
<220>
   <223> primer
<400> 51
   cctttggtac cgcgggcagt gagcgcaacg c 31
<210> 52
   <211> 34
   <212> DNA
   <213> Artificial Sequence
<220>
   <223> primer
<400> 52
   aacaggaat t ct t t gcct gg cggcagt agc gcgg 34
<210> 53
   <211> 40
   <212> DNA
   <213> Artificial Sequence
<220>
   <223> primer P1
<400> 53
   ct agt aagat ct t gaagcct gct t t t t t at act aagt t gg 40
<210> 54
   <211> 41
   <212> DNA
   <213> Artificial Sequence
<220>
   <223> primer P2
<400> 54
   at gat cgaat t cgaaat caa at aat gat t t t at t t t gact g 41
<210> 55
   <211> 120
   <212> DNA
   <213> Artificial Sequence
<220>
   <223> DNA fragment cont ai ni ng at t L
<400> 55
   agatcttgaa gcctgctttt ttatactaag ttggcattat aaaaaagcat tgcttatcaa      60
   t t t gt t gcaa cgaacaggt c act at cagt c aaaat aaaat cat t at t t ga t t t cgaat t c 120
<210> 56
   <211> 41
   <212> DNA
   <213> Artificial Sequence
<220>
   <223> primer P3
<400> 56
   at gccact gc agt ct gt t ac aggt cact aa t accat ct aa g 41
<210> 57
   <211> 46
   <212> DNA
   <213> Artificial Sequence
<220>
   <223> primer P4
<400> 57
   accgt t aagc t t t ct agacg ct caagt t ag t at aaaaaag ct gaac 46
<210> 58
   <211> 184
   <212> DNA
   <213> Artificial Sequence
<220>
   <223> DNA fragment cont ai ni ng at t R
<400> 58
   ct gcagt ct g t t acaggt ca ct aat accat ct aagt agt t gat t cat agt gact gcat at 60
   gt t gt gt t t t acagt at t at gt agt ct gt t t t t t at gcaa aat ct aat t t aat at at t ga 120
   t at t t at at c at t t t acgt t t ct cgt t cag ct t t t t t at a ct aact t gag cgt ct agaaa 180
   gct t 184
<210> 59
   <211> 38
   <212> DNA
   <213> Artificial Sequence
<220>
   <223> primer P5
<400> 59
   ttcttagacg t caggt ggca cttttcgggg aaat gt gc 38
<210> 60
   <211> 37
   <212> DNA
   <213> Artificial Sequence
<220>
   <223> primer P6
<400> 60
   t aacagagat ct cgcgcaga aaaaaaggat ct caaga 37
<210> 61
   <211> 46
   <212> DNA
   <213> Artificial Sequence
<220>
   <223> primer P7
<400> 61
   aacagagat c t aagct t aga t cct t t gcct ggcggcagt a gcgcgg 46
<210> 62
   <211> 35
   <212> DNA
   <213> Artificial Sequence
<220>
   <223> primer P8
<400> 62
   at aaact gca gcaaaaagag t t t gt agaaa cgcaa 35
<210> 63
   <211> 1388
   <212> DNA
   <213> Artificial Sequence
<220>
   <223> DNA fragment cont ai ni ng Tc gene and ter_thrL
<400> 63
   gaat t ct cat  gt t t gacagc  t t at cat cga  t aagct t t aa  t gcggt agt t  t at cacagt t      60
   aaat t gct aa  cgcagt cagg  caccgt gt at  gaaat ct aac  aat gcgct ca  t cgt cat cct     120
   cggcaccgt c  accct ggat g  ct gt aggcat  aggct t ggt t  at gccggt ac  t gccgggcct     180
   cttgcgggat atcgtccatt ccgacagcat cgccagtcac tatggcgtgc tgctagcgct     240
   at at gcgt t g  at gcaat t t c  t at gcgcacc  cgt t ct cgga  gcact gt ccg  accgct t t gg     300
   ccgccgccca gtcctgctcg cttcgctact tggagccact atcgactacg cgatcatggc     360
   gaccacaccc gtcctgtgga tcctctacgc cggacgcatc gtggccggca tcaccggcgc     420
   cacaggt gcg  gt t gct ggcg  cct at at cgc  cgacat cacc  gat ggggaag  at cgggct cg     480
   ccact t cggg  ct cat gagcg  ct t gt t t cgg  cgt gggt at g  gt ggcaggcc  ccgt ggccgg     540
   gggactgttg ggcgccatct ccttgcatgc accattcctt gcggcggcgg tgctcaacgg     600
   cct caacct a  ct act gggct  gct t cct aat  gcaggagt cg  cat aagggag  agcgt cgacc     660
   gat gccct t g  agagcct t ca  acccagt cag  ct cct t ccgg  t gggcgcggg  gcat gact at     720
   cgt cgccgca  ct t at gact g  t ct t ct t t at  cat gcaact c  gt aggacagg  t gccggcagc     780
   gct ct gggt c  at t t t cggcg  aggaccgct t  t cgct ggagc  gcgacgat ga  t cggcct gt c     840
   gct t gcggt a  t t cggaat ct  t gcacgccct  cgct caagcc  t t cgt cact g  gt cccgccac     900
   caaacgtttc ggcgagaagc aggccattat cgccggcatg gcggccgacg cgctgggcta     960
   cgt ct t gct g  gcgt t cgcga  cgcgaggct g  gat ggcct t c  cccat t at ga  t t ct t ct cgc    1020
   t t ccggcggc  at cgggat gc  ccgcgt t gca  ggccat gct g  t ccaggcagg  t agat gacga    1080
   ccat caggga  cagct t caag  gat cgct cgc  ggct ct t acc  agcct aact t  cgat cact gg    1140
   accgct gat c  gt cacggcga  t t t at gccgc  ct cggcgagc  acat ggaacg  ggt t ggcat g    1200
   gat t gt aggc  gccgccct at  acct t gt ct g  cct ccccgcg  t t gcgt cgcg  gt gcat ggag    1260
   ccgggccacc tcgacctgaa tggaagccgg cggcacctcg ctaacggatt caccactcca    1320
   actagaaagc ttaacacaga aaaaagcccg cacctgacag tgcgggcttt ttttttcgac    1380
   cact gcag                                                                1388

<210> 64
   <211> 36
   <212> DNA
   <213> Artificial Sequence
<220>
   <223> primer P9
<400> 64
   agt aat t ct a gaaagct t aa cacagaaaaa agcccg 36
<210> 65
   <211> 43
   <212> DNA
   <213> Artificial Sequence
<220>
   <223> primer P10
<400> 65
   ct agt aggat ccct gcagt g gt cgaaaaaa aaagcccgca ct g 43
<210> 66
   <211> 6550
   <212> DNA
   <213> Pant oea ananat i s
<220>
   <221> CDS
   <222> (231)..(1370)
<220>
   <221> CDS
   <222> (1380)..(1814)
<220>
   <221> CDS
   <222> (1827)..(4004)
<220>
   <221> CDS
   <222> (4260)..(5396)
<220>
   <221> CDS
   <222> (5526)..(6443)
<400> 66
   aggttccggc gggcagattt ctctaaaaaa aagcggtacg gcattgataa accgaaatgt     60
   tctgctttgc tttattccgt tccgggtaaa gcgcgtgatg ggtacaagac aggagctaag    120
   aataatgaac gacatgttca gggcctgagt caggatagct tacttttttc gagcgcttac    180
   acagcggcgt attcgcctct atcaaacatt aactgatagc gaagatctaa atg att       236
                                                          Met Ile
                                                          1
   aca atg aaa atg aag atg ata cct gtt ctg gta tct gca act ttt atg       284
   Thr Met Lys Met Lys Met Ile Pro Val Leu Val Ser Ala Thr Phe Met
           5                   10                  15
   gcc ggg tgt act gtc gtg ccg ggg caa aat tta tcc act tcg gga aaa       332
   Ala Gly Cys Thr Val Val Pro Gly Gln Asn Leu Ser Thr Ser Gly Lys
   20 25 30
   gat gtc att gag cag cag gac agc aat ttt gac atc gac aag tac gtt 380
   Asp Val Ile Glu Gln Gln Asp Ser Asn Phe Asp Ile Asp Lys Tyr Val
   35 40 45 50
   aac gtt tat ccg tta acg cca ggc ctg gtg gag aaa atg cgt cct aag 428
   Asn Val Tyr Pro Leu Thr Pro Gly Leu Val Glu Lys Met Arg Pro Lys
   55 60 65
   cca ctt gtg gca cag gct aat ccg tcg tta caa acc gaa att cag aac 476
   Pro Leu Val Ala Gln Ala Asn Pro Ser Leu Gln Thr Glu Ile Gln Asn
   70 75 80
   tac gaa tac cgc atc ggc atc ggt gat gtt ctc acc gtc acc gtg tgg 524
   Tyr Glu Tyr Arg Ile Gly Ile Gly Asp Val Leu Thr Val Thr Val Trp
   85 90 95
   gat cac ccc gaa ctc acc acg cct gcc ggt cag tac cgt agc gcc agt 572
   Asp His Pro Glu Leu Thr Thr Pro Ala Gly Gln Tyr Arg Ser Ala Ser
   100 105 110
   gac acc ggt aac tgg gtc cat tcc gac ggc acg att ttc tat cct tac 620
   Asp Thr Gly Asn Trp Val His Ser Asp Gly Thr Ile Phe Tyr Pro Tyr
   115 120 125 130
   atc ggc cgc gtc cgt gtt gcc ggc cgt acc gta ggt gac gtg cgt aat 668
   Ile Gly Arg Val Arg Val Ala Gly Arg Thr Val Gly Asp Val Arg Asn
   135 140 145
   gaa gtg gcg cgt cgt ctg gcg cag tac atc gaa agc ccc cag gtt gac 716
   Glu Val Ala Arg Arg Leu Ala Gln Tyr Ile Glu Ser Pro Gln Val Asp
   150 155 160
   gtc agc att gcc tcg ttt aag tca cag aag act tac gtg acc ggt gcc 764
   Val Ser Ile Ala Ser Phe Lys Ser Gln Lys Thr Tyr Val Thr Gly Ala
   165 170 175
   gtg acg acc tca ggc cag cag cct atc acc aac gta ccg ctg act atc 812
   Val Thr Thr Ser Gly Gln Gln Pro Ile Thr Asn Val Pro Leu Thr Ile
   180 185 190
   ctg gat gcg att aac gca gcg ggc ggc tta gcg gcg gac gca gac tgg 860
   Leu Asp Ala Ile Asn Ala Ala Gly Gly Leu Ala Ala Asp Ala Asp Trp
   195 200 205 210
   cgc aac gtt atc ctg acc cac aac ggc cgt gag cag cgc gtg tca ctg 908
   Arg Asn Val Ile Leu Thr His Asn Gly Arg Glu Gln Arg Val Ser Leu
   215 220 225
   cag gcc ctg atg caa aac ggc gat ctg agc cag aac cac ctg ctt tat 956
   Gln Ala Leu Met Gln Asn Gly Asp Leu Ser Gln Asn His Leu Leu Tyr
   230 235 240
   ccg ggt gac atc ctg tat gtg ccg cgt aac gac gac ctg aaa gtg ttt 1004
   Pro Gly Asp Ile Leu Tyr Val Pro Arg Asn Asp Asp Leu Lys Val Phe
   245 250 255
   gtc atg ggc gaa gtg aaa cag cag gcc acc ctg aaa atg gac cgc agc 1052
   Val Met Gly Glu Val Lys Gln Gln Ala Thr Leu Lys Met Asp Arg Ser
   260 265 270
   ggt atg acc ctg gct gaa gcg ctg ggt aat gcg gca ggc atg gat caa 1100
   Gly Met Thr Leu Ala Glu Ala Leu Gly Asn Ala Ala Gly Met Asp Gln
   275 280 285 290
   acc acg tcc gac gcg acc ggg gtc ttt gtt atc cga ccg acg cgc ggc 1148
   Thr Thr Ser Asp Ala Thr Gly Val Phe Val Ile Arg Pro Thr Arg Gly
   295 300 305
   tcc gat cgc agc aaa atc gcc aat atc tac cag ctg aat acc aaa gat 1196
   Ser Asp Arg Ser Lys Ile Ala Asn Ile Tyr Gln Leu Asn Thr Lys Asp
   310 315 320
   gcg gca tcc atg gtg atg ggt acc gaa ttc caa ctt gaa cct tac gat 1244
   Ala Ala Ser Met Val Met Gly Thr Glu Phe Gln Leu Glu Pro Tyr Asp
   325 330 335
   atc gtc tac gtg aca tcg acg ccg ctg acg cgt tgg aac cgc gtg ata 1292
   Ile Val Tyr Val Thr Ser Thr Pro Leu Thr Arg Trp Asn Arg Val Ile
   340 345 350
   tcg cag ttg ata cca acc ata gcc ggt gtt aac gat gcg acg cag gct 1340
   Ser Gln Leu Ile Pro Thr Ile Ala Gly Val Asn Asp Ala Thr Gln Ala
   355 360 365 370
   gca cag cgc atc cgc aac tgg tcg aac taa ggtttcgcc atg att aag tcc 1391
   Ala Gln Arg Ile Arg Asn Trp Ser Asn Met Ile Lys Ser
   375 380
   gtg tta gtc gtc tgc gta ggc aac atc tgc cgc tct cct acg gga gag 1439
   Val Leu Val Val Cys Val Gly Asn Ile Cys Arg Ser Pro Thr Gly Glu
       385                     390                     395
   cgc ctg ttc aaa cgc gcg ctg ccg cac ctg cct gtg gcc tcc gcc gga     1487
   Arg Leu Phe Lys Arg Ala Leu Pro His Leu Pro Val Ala Ser Ala Gly
   400                 405                     410                 415
   ctc ggc gcc ctg gtt ggg cac gcg gcg gat aaa acg gcg acg gag gtg     1535
   Leu Gly Ala Leu Val Gly His Ala Ala Asp Lys Thr Ala Thr Glu Val
                   420                 425                 430
   gca gcc gaa aaa ggc tta tcg ctg gaa ggt cat gag gct cag cag cta     1583
   Ala Ala Glu Lys Gly Leu Ser Leu Glu Gly His Glu Ala Gln Gln Leu
               435                 440                 445
   acg gcc agc ctg tgc cgg gag tat gac ctg att ctg gtg atg gaa aag     1631
   Thr Ala Ser Leu Cys Arg Glu Tyr Asp Leu Ile Leu Val Met Glu Lys
           450                 455                 460
   cgc cac att gaa cag gta aac cgc atc gat ccg gct gca cgc ggc aaa     1679
   Arg His Ile Glu Gln Val Asn Arg Ile Asp Pro Ala Ala Arg Gly Lys
       465                 470                 475
   acg atg ctg ctt ggg cac tgg ctc aat cag aag gaa att gcg gat ccg     1727
   Thr Met Leu Leu Gly His Trp Leu Asn Gln Lys Glu Ile Ala Asp Pro
   480                 485                 490                 495
   tac aga aaa agc cgt gag gcc ttt gaa gag gtt tac ggg tta ctg gaa     1775
   Tyr Arg Lys Ser Arg Glu Ala Phe Glu Glu Val Tyr Gly Leu Leu Glu
                   500                 505                 510
   cac gcc act cag aaa tgg gtc agc gta cta agc cga tag ttgggattat cc   1826
   His Ala Thr Gln Lys Trp Val Ser Val Leu Ser Arg
               515                     520
   atg aat ata aaa aac aaa gtc atg acc gcg ccg cgc gag gaa tcg aat     1874
   Met Asn Ile Lys Asn Lys Val Met Thr Ala Pro Arg Glu Glu Ser Asn
       525                 530                 535
   ggg tgg gat ctg gcg cat ctt gtg gga cag ctt att gat cac cgc tgg     1922
   Gly Trp Asp Leu Ala His Leu Val Gly Gln Leu Ile Asp His Arg Trp
   540                 545                 550                 555
   gtt atc gtc gct gtc acc gct ttt ttc atg ctg gta gca acg ctc tac     1970
   Val Ile Val Ala Val Thr Ala Phe Phe Met Leu Val Ala Thr Leu Tyr
                   560                 565                 570
   aca ctg ttt gcg acg cct att tac agc gcc gat gcc atg gtt cag gtg     2018
   Thr Leu Phe Ala Thr Pro Ile Tyr Ser Ala Asp Ala Met Val Gln Val
               575                 580                 585
   gag cag aag aat acc agc acc gtc ctg aat gaa tta cag acg ctg atg     2066
   Glu Gln Lys Asn Thr Ser Thr Val Leu Asn Glu Leu Gln Thr Leu Met
           590                 595                 600
   ccc acg acg cca gca tcg gat acc gaa atc cag att ctt gaa tca cgc     2114
   Pro Thr Thr Pro Ala Ser Asp Thr Glu Ile Gln Ile Leu Glu Ser Arg
       605                 610                 615
   atg gtg ctg gga aaa acc gtt cag gat ctt ggc ctg gat gca gtg gtt     2162
   Met Val Leu Gly Lys Thr Val Gln Asp Leu Gly Leu Asp Ala Val Val
   620                 625                 630                 635
   tca cag aac tat ttc ccg gtt atc ggc aaa ggt ctg tcg cgc ctg atg     2210
   Ser Gln Asn Tyr Phe Pro Val Ile Gly Lys Gly Leu Ser Arg Leu Met
                   640                 645                 650
   ggc aac aag cct ggc aat gtt gcg atc tcc cgt ctg gag att ccg cgt     2258
   Gly Asn Lys Pro Gly Asn Val Ala Ile Ser Arg Leu Glu Ile Pro Arg
               655                 660                 665
   acg atc gag aag cgc agt gtt gag ctc gag gtt acg ggt aaa gac agc     2306
   Thr Ile Glu Lys Arg Ser Val Glu Leu Glu Val Thr Gly Lys Asp Ser
           670                 675                 680
   tat acc gtt tct gcc gat ggc gat gag ctg ttc aaa ggc aaa gtg ggg     2354
   Tyr Thr Val Ser Ala Asp Gly Asp Glu Leu Phe Lys Gly Lys Val Gly
       685                 690                 695
   cag ttc gaa aaa cac ggc gat gtg agc atg ctg gtc agc gat atc aat     2402
   Gln Phe Glu Lys His Gly Asp Val Ser Met Leu Val Ser Asp Ile Asn
   700                 705                 710                 715
   gcg gag ccg ggc acc acg ttt acc gtc acc aaa ctc aac gat ctg cag     2450
   Ala Glu Pro Gly Thr Thr Phe Thr Val Thr Lys Leu Asn Asp Leu Gln
                   720                 725                 730
   gca att aac agc atc ctg tct aac ctg acc gtt gca gac aaa ggg aaa     2498
   Ala Ile Asn Ser Ile Leu Ser Asn Leu Thr Val Ala Asp Lys Gly Lys
               735                 740                 745
   gac acc ggc gta ctg gga ctg cag tat gtg ggt gaa gat cag gag cag     2546
   Asp Thr Gly Val Leu Gly Leu Gln Tyr Val Gly Glu Asp Gln Glu Gln
           750                     755                     760
   atc agc aaa gtt ctg aac cag atc gtg aat aac tac ctg ttg cag aac     2594
   Ile Ser Lys Val Leu Asn Gln Ile Val Asn Asn Tyr Leu Leu Gln Asn
       765                     770                     775
   gtg cag cgt aaa tcc gaa cag gcc gag aaa agc ctg gag ttc ctg aaa     2642
   Val Gln Arg Lys Ser Glu Gln Ala Glu Lys Ser Leu Glu Phe Leu Lys
   780                     785                     790                 795
   cag caa ctg ccg gaa gtg cgt ggc aaa ctg gat cag gcg gaa gac aaa     2690
   Gln Gln Leu Pro Glu Val Arg Gly Lys Leu Asp Gln Ala Glu Asp Lys
                       800                     805                 810
   ctt aac gcc ttc cgc cgt gag aac gag tcc gtt gac ctg tcg ctt gaa     2738
   Leu Asn Ala Phe Arg Arg Glu Asn Glu Ser Val Asp Leu Ser Leu Glu
                   815                     820                 825
   gct aag tct gcg ttg gat tca tcc gtt agc gtg cag agc cag ctt aac     2786
   Ala Lys Ser Ala Leu Asp Ser Ser Val Ser Val Gln Ser Gln Leu Asn
               830                     835                 840
   gag ctg acg ttc cgc gaa gcc gaa gtt tcg cag ctg ttc acc aaa gat     2834
   Glu Leu Thr Phe Arg Glu Ala Glu Val Ser Gln Leu Phe Thr Lys Asp
       845                     850                     855
   cac cct acc tat cgg gcc ctg tta gaa aag cgt aaa acg ctg gaa gat     2882
   His Pro Thr Tyr Arg Ala Leu Leu Glu Lys Arg Lys Thr Leu Glu Asp
   860                     865                     870                 875
   gag cag gcg cag ctg aac aag aaa att gcg cag atg ccg aaa act cag     2930
   Glu Gln Ala Gln Leu Asn Lys Lys Ile Ala Gln Met Pro Lys Thr Gln
                       880                     885                 890
   cag gag att ctg cgt ctg acc cgc gat gtg cag tca ggt cag gaa atc     2978
   Gln Glu Ile Leu Arg Leu Thr Arg Asp Val Gln Ser Gly Gln Glu Ile
                   895                     900                 905
   tac atg cag tta ctg aac cgc cag caa gag ctg agc atc agt aag gcc     3026
   Tyr Met Gln Leu Leu Asn Arg Gln Gln Glu Leu Ser Ile Ser Lys Ala
               910                     915                 920
   agc acc gtg ggt gat gtt cgc atc atc gat cag gcc gcc acg gca aac     3074
   Ser Thr Val Gly Asp Val Arg Ile Ile Asp Gln Ala Ala Thr Ala Asn
       925                     930                     935
   tcg ccg gtt gcg ccg aaa aag ctg ctg atc atc gca gcc agc ctg att     3122
   Ser Pro Val Ala Pro Lys Lys Leu Leu Ile Ile Ala Ala Ser Leu Ile
   940                     945                     950                 955
   ctg ggg ctg atg gtg tcc gtc ggt gtg gtt ctg ctt aaa gcg ctg ttg     3170
   Leu Gly Leu Met Val Ser Val Gly Val Val Leu Leu Lys Ala Leu Leu
                       960                     965                 970
   cat cac gga atc gag aac ccg gaa cag ctg gaa gag ctg ggc atg aac     3218
   His His Gly Ile Glu Asn Pro Glu Gln Leu Glu Glu Leu Gly Met Asn
                   975                     980                 985
   gtc tat gcc agc gta ccg ctc tct gag tgg cag cgt aag aag gat acc     3266
   Val Tyr Ala Ser Val Pro Leu Ser Glu Trp Gln Arg Lys Lys Asp Thr
               990                     995                 1000
   gaa gca ctg gcg cgt cgc ggt cac aaa ggc aaa acc gat ccg cat     3311
   Glu Ala Leu Ala Arg Arg Gly His Lys Gly Lys Thr Asp Pro His
       1005                    1010                    1015
   gag acg ctg ttg gca ctg ggt aat cca acc gac ctc tct att gaa     3356
   Glu Thr Leu Leu Ala Leu Gly Asn Pro Thr Asp Leu Ser Ile Glu
       1020                    1025                    1030
   gcg att cgt agc ctg cgt acc agc ctg cac ttc gcc atg atg gaa     3401
   Ala Ile Arg Ser Leu Arg Thr Ser Leu His Phe Ala Met Met Glu
       1035                    1040                    1045
   gcg aaa aac aac att ctg atg atc acc ggc gcc agc ccg ggt att     3446
   Ala Lys Asn Asn Ile Leu Met Ile Thr Gly Ala Ser Pro Gly Ile
       1050                    1055                    1060
   ggt aaa acc ttt atc tgc gtt aac ctc gct acc ctg gtg gcc aag     3491
   Gly Lys Thr Phe Ile Cys Val Asn Leu Ala Thr Leu Val Ala Lys
       1065                    1070                    1075
   gcc ggt cag aag gtg ttg ttt atc gat ggt gac atg cgc cgc ggt     3536
   Ala Gly Gln Lys Val Leu Phe Ile Asp Gly Asp Met Arg Arg Gly
       1080                    1085                    1090
   tac acc cac gaa ctg ctg ggg gcg gac aat aaa tcc ggt ctg tct     3581
   Tyr Thr His Glu Leu Leu Gly Ala Asp Asn Lys Ser Gly Leu Ser
       1095                    1100                    1105
   aac gtc ctg tct ggc aaa acc gag ttt acg ccg acc atg att cag     3626
   Asn Val Leu Ser Gly Lys Thr Glu Phe Thr Pro Thr Met Ile Gln
   1110 1115 1120
   caa ggg cca tac ggt ttt gat ttc ctg ccg cgc gga cag gtt cca 3671
   Gln Gly Pro Tyr Gly Phe Asp Phe Leu Pro Arg Gly Gln Val Pro
   1125 1130 1135
   ccg aac ccg tca gag ctg ttg atg cac cgc cgc atg ggc gag ctg 3716
   Pro Asn Pro Ser Glu Leu Leu Met His Arg Arg Met Gly Glu Leu
   1140 1145 1150
   ctg gag tgg gca agc aaa aac tat gat tta gtc ctg att gat aca 3761
   Leu Glu Trp Ala Ser Lys Asn Tyr Asp Leu Val Leu Ile Asp Thr
   1155 1160 1165
   cca ccg att ctg gcc gta acg gat gcg tcc atc atc ggt aag ctg 3806
   Pro Pro Ile Leu Ala Val Thr Asp Ala Ser Ile Ile Gly Lys Leu
   1170 1175 1180
   gcc ggt acc tcg ctg atg gtg gcg cgt ttt gaa acc aac acc aca 3851
   Ala Gly Thr Ser Leu Met Val Ala Arg Phe Glu Thr Asn Thr Thr
   1185 1190 1195
   aaa gaa gtg gat gtc agc ttc aaa cgc ttc gcg cag aac ggt atc 3896
   Lys Glu Val Asp Val Ser Phe Lys Arg Phe Ala Gln Asn Gly Ile
   1200 1205 1210
   gaa atc aaa ggg gtt atc ctg aac gcc gtg gta cgt aaa gcg gct 3941
   Glu Ile Lys Gly Val Ile Leu Asn Ala Val Val Arg Lys Ala Ala
   1215 1220 1225
   aac tcg tac ggt tat ggt tat gac tac tac gac tac gag tac ggt 3986
   Asn Ser Tyr Gly Tyr Gly Tyr Asp Tyr Tyr Asp Tyr Glu Tyr Gly
   1230 1235 1240
   aaa cca acg aaa agc taa gagctgaaag aaaaggggcg ggtttgaccg 4034
   Lys Pro Thr Lys Ser
   1245
   ccccttttga tccctggctc atgataacga cacgtaacga tttgcacagg tgattctgga 4094
   tacaacgcac aataagcgac gatctaacgt caatcatcag aattcatcat caacacgctt 4154
   ttgtctgtcg cgatgtcggc gcagacctgc tgagagatca acccggaagg taatcagcac 4214
   tggccactgt tggcatgaga acttgtggat ttaaggaatc acacc atg gct cca 4268
   Met Ala Pro
   1250
   tat tgg tat ata tca ggg ttt ttg ctg ctg att tcg ctg ttc gaa 4313
   Tyr Trp Tyr Ile Ser Gly Phe Leu Leu Leu Ile Ser Leu Phe Glu
   1255 1260 1265
   atc ctg gtg aaa aaa gac gaa cgc aca aat tat att ttg acc tgg 4358
   Ile Leu Val Lys Lys Asp Glu Arg Thr Asn Tyr Ile Leu Thr Trp
   1270 1275 1280
   ctg ctg tgc ttt gcg gcc att att ctg atc gta ttc ggg ggg att 4403
   Leu Leu Cys Phe Ala Ala Ile Ile Leu Ile Val Phe Gly Gly Ile
   1285 1290 1295
   cgt ggc ctc ggc acc ggc atg gat gac ttc cag tac cgc agt ttc 4448
   Arg Gly Leu Gly Thr Gly Met Asp Asp Phe Gln Tyr Arg Ser Phe
   1300 1305 1310
   ttt gag gac ttt gta cgg cgt att cag atc aac ggt ttt ttc aat 4493
   Phe Glu Asp Phe Val Arg Arg Ile Gln Ile Asn Gly Phe Phe Asn
   1315 1320 1325
   acc gtc gcg ttt ttc cgc tac gaa ccg ctg att ttt gcg atg gcg 4538
   Thr Val Ala Phe Phe Arg Tyr Glu Pro Leu Ile Phe Ala Met Ala
   1330 1335 1340
   tgg ata acg agt ctg ttt tcg cat aac gcc agc gtg ttt ctg ttc 4583
   Trp Ile Thr Ser Leu Phe Ser His Asn Ala Ser Val Phe Leu Phe
   1345 1350 1355
   gtc ttc tgt gcg att gcg gta tcc att aac gcc ttc ttt ttc aga 4628
   Val Phe Cys Ala Ile Ala Val Ser Ile Asn Ala Phe Phe Phe Arg
   1360 1365 1370
   aag atg tcg cct tat ccg gta ctg gcg ctg gcg ctg tat tcg gcg 4673
   Lys Met Ser Pro Tyr Pro Val Leu Ala Leu Ala Leu Tyr Ser Ala
   1375 1380 1385
   cac atc ttt att aac aaa gac atc aac cag ata cgt ttt ggc tta 4718
   His Ile Phe Ile Asn Lys Asp Ile Asn Gln Ile Arg Phe Gly Leu
   1390 1395 1400
   agc tcc gcc ctg ttc ctc ggc gtg ctg tgg acg atc tac ctg aag 4763
   Ser Ser Ala Leu Phe Leu Gly Val Leu Trp Thr Ile Tyr Leu Lys
   1405 1410 1415
   cgt tac tgg tgg gcc ttt acc ttt ttt atc ctg tcg ttc atg agt 4808
   Arg Tyr Trp Trp Ala Phe Thr Phe Phe Ile Leu Ser Phe Met Ser
                   1420                          1425                        1430
   cac aac acc gcc   gtt atg gtg ata acg   ctg gtg cct ttc ctg   ttt      4853
   His Asn Thr Ala   Val Met Val Ile Thr   Leu Val Pro Phe Leu   Phe
                   1435                          1440                        1445
   att cgt gac tgg   cgc tgg tgg ccg gtg   gtg atc atc gtt gtc   agc      4898
   Ile Arg Asp Trp   Arg Trp Trp Pro Val   Val Ile Ile Val Val   Ser
                   1450                          1455                        1460
   ctg ccg ctc tcg   gtc gtc ggg ggt tca   agc ttc atc gcc ctg   att      4943
   Leu Pro Leu Ser   Val Val Gly Gly Ser   Ser Phe Ile Ala Leu   Ile
                   1465                          1470                        1475
   gcc gga cac ctc   gga tcg ctg ggg gaa   agg gca tcg ggc tat   aac      4988
   Ala Gly His Leu   Gly Ser Leu Gly Glu   Arg Ala Ser Gly Tyr   Asn
                   1480                          1485                        1490
   aac gat ccc tcc   tat gca atg gat ggc   agc atc ctg tca gtc   tcg      5033
   Asn Asp Pro Ser   Tyr Ala Met Asp Gly   Ser Ile Leu Ser Val   Ser
                   1495                          1500                        1505
   aac ctg aag aac   atc atg ctg gtg ttt   att ttc ttc tat ttc   atg      5078
   Asn Leu Lys Asn   Ile Met Leu Val Phe   Ile Phe Phe Tyr Phe   Met
                   1510                          1515                        1520
   ctg act gaa gaa   ctg aag cgc gaa aac   tat gcc cag tat cgt   ctg      5123
   Leu Thr Glu Glu   Leu Lys Arg Glu Asn   Tyr Ala Gln Tyr Arg   Leu
                   1525                          1530                        1535
   aac tac ttg ctg   att tta agt ttt gct   atc ggc ggg gcg atc   cgc      5168
   Asn Tyr Leu Leu   Ile Leu Ser Phe Ala   Ile Gly Gly Ala Ile   Arg
                   1540                          1545                        1550
   atc ttc ctg tat   aac ttc ccg tca ggt   tcg cgt ctt tcc aac   tat      5213
   Ile Phe Leu Tyr   Asn Phe Pro Ser Gly   Ser Arg Leu Ser Asn   Tyr
                   1555                          1560                        1565
   ttg caa cag gtt   gaa ccc atc att ctg   acc tcg ctt atc tac   cag      5258
   Leu Gln Gln Val   Glu Pro Ile Ile Leu   Thr Ser Leu Ile Tyr   Gln
                   1570                          1575                        1580
   gcc aga cgc gtc   tgg aag ccg gcg ctg   ttt ggc atg ctg ttt   ttc      5303
   Ala Arg Arg Val   Trp Lys Pro Ala Leu   Phe Gly Met Leu Phe   Phe
                   1585                          1590                        1595
   ttc ctg ctc tat   tac ctc tac tac aac   acc atc tca acc aag   cag      5348
   Phe Leu Leu Tyr   Tyr Leu Tyr Tyr Asn   Thr Ile Ser Thr Lys   Gln
                   1600                          1605                        1610
   gcg gtt gta ggg   tac gaa gtg gcc cag   gag ttc tgg ctg att   cgg      5393
   Ala Val Val Gly   Tyr Glu Val Ala Gln   Glu Phe Trp Leu Ile   Arg
                   1615                          1620                        1625
   taa cggacccggc ggcgcgcccc cagtgaacgg cttcacttcg cggggcggtc            5446
   gcctgtaaca acacaggttt ttcttcggcg tttgtcgatt gaattgttta tttttgaata     5506
   ctttaatgag gtgctggca atg aag gac att   cgt ttc tcc atc gta   att      5555
                        Met Lys Asp Ile   Arg Phe Ser Ile Val   Ile
                                         1630                        1635
   ccg gct tat aac   gcg tca gaa tcg att   gtc acg acg ctg gat   tgc      5600
   Pro Ala Tyr Asn   Ala Ser Glu Ser Ile   Val Thr Thr Leu Asp   Cys
                   1640                          1645                        1650
   gtt aaa gca caa   act tat cgc cat ttc   gaa gtc atc atc gtg   gat      5645
   Val Lys Ala Gln   Thr Tyr Arg His Phe   Glu Val Ile Ile Val   Asp
                   1655                          1660                        1665
   gac aaa tct gcc   gat gcc gcc gcg ctg   gcg gaa gtg gtg cgc   agt      5690
   Asp Lys Ser Ala   Asp Ala Ala Ala Leu   Ala Glu Val Val Arg   Ser
                   1670                          1675                        1680
   gag cgc tat cag   gac ctg gac atc aat   ctg gtc ttg tct gag   gtt      5735
   Glu Arg Tyr Gln   Asp Leu Asp Ile Asn   Leu Val Leu Ser Glu   Val
                   1685                          1690                        1695
   aaa ctc aac ggt   gcc ggc gcg cgt aac   aaa ggc att gag ctg   gca      5780
   Lys Leu Asn Gly   Ala Gly Ala Arg Asn   Lys Gly Ile Glu Leu   Ala
                   1700                          1705                        1710
   acg ggc gac tat   gtc agt ttt ctg gat   gcc gat gac gag tgg   cat      5825
   Thr Gly Asp Tyr   Val Ser Phe Leu Asp   Ala Asp Asp Glu Trp   His
                   1715                          1720                        1725
   gcc gac aag ctt   ctg cag gtc agt gaa   aag att gcg gag cta   gag      5870
   Ala Asp Lys Leu   Leu Gln Val Ser Glu   Lys Ile Ala Glu Leu   Glu
                   1730                          1735                        1740
   gcg cag ggt cag   cac aat acc att atc   ttc agc cag gta aat   atc      5915
   Ala Gln Gly Gln   His Asn Thr Ile Ile   Phe Ser Gln Val Asn   Ile
                   1745                          1750                        1755
   tat cag gac ggt gcg ttc ctg aag gtt atg ccg atg cag cct ccg      5960
   Tyr Gln Asp Gly Ala Phe Leu Lys Val Met Pro Met Gln Pro Pro
               1760                1765                1770
   gca aaa aac gaa act gtc gca gag tat ctg ttt ggc tgt tat ggc      6005
   Ala Lys Asn Glu Thr Val Ala Glu Tyr Leu Phe Gly Cys Tyr Gly
               1775                1780                1785
   ttt att caa acc agc acc atc gtg ctg aag cgt gaa gat gcc gct      6050
   Phe Ile Gln Thr Ser Thr Ile Val Leu Lys Arg Glu Asp Ala Ala
               1790                1795                1800
   aaa att cag ttc gat gcg cgt ttt atc cgc cat cag gac tat gac      6095
   Lys Ile Gln Phe Asp Ala Arg Phe Ile Arg His Gln Asp Tyr Asp
               1805                1810                1815
   ttc tgc atc cgt gcc gac cgc atg ggt tac cgg ttt gaa atg atc      6140
   Phe Cys Ile Arg Ala Asp Arg Met Gly Tyr Arg Phe Glu Met Ile
               1820                1825                1830
   gcc gcc ccg ctg gcc aac tac cac ctg gtg acg aag ttt ggc tcc      6185
   Ala Ala Pro Leu Ala Asn Tyr His Leu Val Thr Lys Phe Gly Ser
               1835                1840                1845
   aaa cac aaa ggt gaa tcg gtc aag tac tcc ctg ttc tgg ctg gat      6230
   Lys His Lys Gly Glu Ser Val Lys Tyr Ser Leu Phe Trp Leu Asp
               1850                1855                1860
   acg atg aaa ccg cac ctt acc gca cga gat att cac acc tac aaa      6275
   Thr Met Lys Pro His Leu Thr Ala Arg Asp Ile His Thr Tyr Lys
               1865                1870                1875
   gca ttc aag ctg ccc ctg cgc tac aaa atg gac ggt aac tcg ctg      6320
   Ala Phe Lys Leu Pro Leu Arg Tyr Lys Met Asp Gly Asn Ser Leu
               1880                1885                1890
   atg gcg agc gtc agc ttc gcg cgc tac ttc ctg ctc acc aat aaa      6365
   Met Ala Ser Val Ser Phe Ala Arg Tyr Phe Leu Leu Thr Asn Lys
               1895                1900                1905
   gat aat cgc acc tac ttc ctg aac cgc gtg aaa gac aaa ctg aaa      6410
   Asp Asn Arg Thr Tyr Phe Leu Asn Arg Val Lys Asp Lys Leu Lys
               1910                1915                1920
   gcg cgg ttg ggt ggc gaa aag gca ctc tcc tga ttctattgaa           6453
   Ala Arg Leu Gly Gly Glu Lys Ala Leu Ser
               1925                1930
   tgtcgtgacg ttttaacgct accggaaggt tatctggtgg ccccgtctat tttatttatt 6513
   caggtatgaa aatgaataat cactctggta ccgtcgg                          6550
<210> 67
   <211> 379
   <212> PRT
   <213> Pant oea ananatis
<400> 67
   Met Ile Thr Met Lys Met Lys Met Ile Pro Val Leu Val Ser Ala Thr
   1 5 10 15
   Phe Met Ala Gly Cys Thr Val Val Pro Gly Gln Asn Leu Ser Thr Ser
   20 25 30
   Gly Lys Asp Val Ile Glu Gln Gln Asp Ser Asn Phe Asp Ile Asp Lys
   35 40 45
   Tyr Val Asn Val Tyr Pro Leu Thr Pro Gly Leu Val Glu Lys Met Arg
   50 55 60
   Pro Lys Pro Leu Val Ala Gln Ala Asn Pro Ser Leu Gln Thr Glu Ile
   65 70 75 80
   Gln Asn Tyr Glu Tyr Arg Ile Gly Ile Gly Asp Val Leu Thr Val Thr
   85 90 95
   Val Trp Asp His Pro Glu Leu Thr Thr Pro Ala Gly Gln Tyr Arg Ser
   100 105 110
   Ala Ser Asp Thr Gly Asn Trp Val His Ser Asp Gly Thr Ile Phe Tyr
   115 120 125
   Pro Tyr Ile Gly Arg Val Arg Val Ala Gly Arg Thr Val Gly Asp Val
   130 135 140
   Arg Asn Glu Val Ala Arg Arg Leu Ala Gln Tyr Ile Glu Ser Pro Gln
   145 150 155 160
   Val Asp Val Ser Ile Ala Ser Phe Lys Ser Gln Lys Thr Tyr Val Thr
   165 170 175
   Gly Ala Val Thr Thr Ser Gly Gln Gln Pro Ile Thr Asn Val Pro Leu
   180 185 190
   Thr Ile Leu Asp Ala Ile Asn Ala Ala Gly Gly Leu Ala Ala Asp Ala
   195 200 205
   Asp Trp Arg Asn Val Ile Leu Thr His Asn Gly Arg Glu Gln Arg Val
   210 215 220
   Ser Leu Gln Ala Leu Met Gln Asn Gly Asp Leu Ser Gln Asn His Leu
   225 230 235 240
   Leu Tyr Pro Gly Asp Ile Leu Tyr Val Pro Arg Asn Asp Asp Leu Lys
   245 250 255
   Val Phe Val Met Gly Glu Val Lys Gln Gln Ala Thr Leu Lys Met Asp
   260 265 270
   Arg Ser Gly Met Thr Leu Ala Glu Ala Leu Gly Asn Ala Ala Gly Met
   275 280 285
   Asp Gln Thr Thr Ser Asp Ala Thr Gly Val Phe Val Ile Arg Pro Thr
   290 295 300
   Arg Gly Ser Asp Arg Ser Lys Ile Ala Asn Ile Tyr Gln Leu Asn Thr
   305 310 315 320
   Lys Asp Ala Ala Ser Met Val Met Gly Thr Glu Phe Gln Leu Glu Pro
   325 330 335
   Tyr Asp Ile Val Tyr Val Thr Ser Thr Pro Leu Thr Arg Trp Asn Arg
   340 345 350
   Val Ile Ser Gln Leu Ile Pro Thr Ile Ala Gly Val Asn Asp Ala Thr
   355 360 365
   Gln Ala Ala Gln Arg Ile Arg Asn Trp Ser Asn
   370 375
<210> 68
   <211> 144
   <212> PRT
   <213> Pant oea ananat i s
<400> 68
   Met Ile Lys Ser Val Leu Val Val Cys Val Gly Asn Ile Cys Arg Ser
   1               5                   10                  15
   Pro Thr Gly Glu Arg Leu Phe Lys Arg Ala Leu Pro His Leu Pro Val
               20                  25                  30
   Ala Ser Ala Gly Leu Gly Ala Leu Val Gly His Ala Ala Asp Lys Thr
           35                  40                  45
   Ala Thr Glu Val Ala Ala Glu Lys Gly Leu Ser Leu Glu Gly His Glu
       50                  55                  60
   Ala Gln Gln Leu Thr Ala Ser Leu Cys Arg Glu Tyr Asp Leu Ile Leu
   65                  70                  75                  80
   Val Met Glu Lys Arg His Ile Glu Gln Val Asn Arg Ile Asp Pro Ala
                   85                  90                  95
   Ala Arg Gly Lys Thr Met Leu Leu Gly His Trp Leu Asn Gln Lys Glu
               100                 105                 110
   Ile Ala Asp Pro Tyr Arg Lys Ser Arg Glu Ala Phe Glu Glu Val Tyr
           115                 120                 125
   Gly Leu Leu Glu His Ala Thr Gln Lys Trp Val Ser Val Leu Ser Arg
       130                 135                 140
<210> 69
   <211> 725
   <212> PRT
   <213> Pant oea ananat i s
<400> 69
<210> 70
   <211> 378
   <212> PRT
   <213> Pant oea ananat i s
<400> 70
   Met Ala Pro Tyr Trp Tyr Ile Ser Gly Phe Leu Leu Leu Ile Ser Leu
   1 5 10 15
   Phe Glu Ile Leu Val Lys Lys Asp Glu Arg Thr Asn Tyr Ile Leu Thr
   20 25 30
   Trp Leu Leu Cys Phe Ala Ala Ile Ile Leu Ile Val Phe Gly Gly Ile
   35 40 45
   Arg Gly Leu Gly Thr Gly Met Asp Asp Phe Gln Tyr Arg Ser Phe Phe
   50 55 60
   Glu Asp Phe Val Arg Arg Ile Gln Ile Asn Gly Phe Phe Asn Thr Val
   65 70 75 80
   Ala Phe Phe Arg Tyr Glu Pro Leu Ile Phe Ala Met Ala Trp Ile Thr
   85 90 95
   Ser Leu Phe Ser His Asn Ala Ser Val Phe Leu Phe Val Phe Cys Ala
   100 105 110
   Ile Ala Val Ser Ile Asn Ala Phe Phe Phe Arg Lys Met Ser Pro Tyr
   115 120 125
   Pro Val Leu Ala Leu Ala Leu Tyr Ser Ala His Ile Phe Ile Asn Lys
   130 135 140
   Asp Ile Asn Gln Ile Arg Phe Gly Leu Ser Ser Ala Leu Phe Leu Gly
   145 150 155 160
   Val Leu Trp Thr Ile Tyr Leu Lys Arg Tyr Trp Trp Ala Phe Thr Phe
   165 170 175
   Phe Ile Leu Ser Phe Met Ser His Asn Thr Ala Val Met Val Ile Thr
   180 185 190
   Leu Val Pro Phe Leu Phe Ile Arg Asp Trp Arg Trp Trp Pro Val Val
   195 200 205
   Ile Ile Val Val Ser Leu Pro Leu Ser Val Val Gly Gly Ser Ser Phe
   210 215 220
   Ile Ala Leu Ile Ala Gly His Leu Gly Ser Leu Gly Glu Arg Ala Ser
   225 230 235 240
   Gly Tyr Asn Asn Asp Pro Ser Tyr Ala Met Asp Gly Ser Ile Leu Ser
   245 250 255
   Val Ser Asn Leu Lys Asn Ile Met Leu Val Phe Ile Phe Phe Tyr Phe
   260 265 270
   Met Leu Thr Glu Glu Leu Lys Arg Glu Asn Tyr Ala Gln Tyr Arg Leu
   275 280 285
   Asn Tyr Leu Leu Ile Leu Ser Phe Ala Ile Gly Gly Ala Ile Arg Ile
   290 295 300
   Phe Leu Tyr Asn Phe Pro Ser Gly Ser Arg Leu Ser Asn Tyr Leu Gln
   305 310 315 320
   Gln Val Glu Pro Ile Ile Leu Thr Ser Leu Ile Tyr Gln Ala Arg Arg
   325 330 335
   Val Trp Lys Pro Ala Leu Phe Gly Met Leu Phe Phe Phe Leu Leu Tyr
   340 345 350
   Tyr Leu Tyr Tyr Asn Thr Ile Ser Thr Lys Gln Ala Val Val Gly Tyr
   355 360 365
   Glu Val Ala Gln Glu Phe Trp Leu Ile Arg
   370 375
<210> 71
   <211> 305
   <212> PRT
   <213> Pant oea ananat i s
<400> 71
   Met Lys Asp Ile Arg Phe Ser Ile Val Ile Pro Ala Tyr Asn Ala Ser
   1               5                   10                  15
   Glu Ser Ile Val Thr Thr Leu Asp Cys Val Lys Ala Gln Thr Tyr Arg
               20                  25                  30
   His Phe Glu Val Ile Ile Val Asp Asp Lys Ser Ala Asp Ala Ala Ala
           35                  40                  45
   Leu Ala Glu Val Val Arg Ser Glu Arg Tyr Gln Asp Leu Asp Ile Asn
       50                  55                  60
   Leu Val Leu Ser Glu Val Lys Leu Asn Gly Ala Gly Ala Arg Asn Lys
   65                  70                  75                  80
   Gly Ile Glu Leu Ala Thr Gly Asp Tyr Val Ser Phe Leu Asp Ala Asp
                   85                  90                  95
   Asp Glu Trp His Ala Asp Lys Leu Leu Gln Val Ser Glu Lys Ile Ala
               100                 105                 110
   Glu Leu Glu Ala Gln Gly Gln His Asn Thr Ile Ile Phe Ser Gln Val
           115                 120                 125
   Asn Ile Tyr Gln Asp Gly Ala Phe Leu Lys Val Met Pro Met Gln Pro
       130                 135                 140
   Pro Ala Lys Asn Glu Thr Val Ala Glu Tyr Leu Phe Gly Cys Tyr Gly
   145                 150                 155                 160
   Phe Ile Gln Thr Ser Thr Ile Val Leu Lys Arg Glu Asp Ala Ala Lys
                   165                 170                 175
   Ile Gln Phe Asp Ala Arg Phe Ile Arg His Gln Asp Tyr Asp Phe Cys
               180                 185                 190
   Ile Arg Ala Asp Arg Met Gly Tyr Arg Phe Glu Met Ile Ala Ala Pro
           195                 200                 205
   Leu Ala Asn Tyr His Leu Val Thr Lys Phe Gly Ser Lys His Lys Gly
       210                 215                 220
   Glu Ser Val Lys Tyr Ser Leu Phe Trp Leu Asp Thr Met Lys Pro His
   225                 230                 235                 240
   Leu Thr Ala Arg Asp Ile His Thr Tyr Lys Ala Phe Lys Leu Pro Leu
                   245                 250                 255
   Arg Tyr Lys Met Asp Gly Asn Ser Leu Met Ala Ser Val Ser Phe Ala
               260                 265                 270
   Arg Tyr Phe Leu Leu Thr Asn Lys Asp Asn Arg Thr Tyr Phe Leu Asn
           275                 280                 285
   Arg Val Lys Asp Lys Leu Lys Ala Arg Leu Gly Gly Glu Lys Ala Leu
       290                 295                 300
   Ser
   305
<210> 72
   <211> 89
   <212> DNA
   <213> Artificial Sequence
<220>
   <223> primer
<400> 72
   caagttgata agatcttccg tgccgccgct ctggccgctg cagatgctcg aatccctctc    60
   tgaagcctgc ttttttatac taagttggc                                      89
<210> 73
   <211> 89
   <212> DNA
   <213> Artificial Sequence
<220>
   <223> primer
<400> 73
   acgtgagcca ggaaatcagc ttcctgaacg ccggcttcgc gaatagattt cggaataccc    60
   gctcaagtta gtataaaaaa gctgaacga                                      89
<210> 74
   <211> 2688
   <212> DNA
   <213> Ent er obact er aerogenes
<220>
   <221> misc_feature
   <222> (663)..(663)
   <223> n i s a, c, g, or t
<220>
   <221> misc_feature
   <222> (1303)..(1303)
   <223> n i s a, c, g, or t
<220>
   <221> misc_feature
   <222> (1869)..(1869)
   <223> n i s a, c, g, or t
<220>
   <221> misc_feature
   <222> (2609)..(2610)
   <223> n i s a, c, g, or t
<220>
   <221> misc_feature
   <222> (2618).. (2618)
   <223> n i s a, c, g, or t
<400> 74
   tcgcatgctc ccggccgcca tggccgcggg attctcgttg agcgtgtaaa aaaagcccag 60
   cat t gaat at gccagt t t ca ct caagaaca agt t gat aag at ct t ccgt g ccgccgct ct 120
   ggccgct gca gat gct cgaa t ccct ct cgc t aaaat ggct gt t gccgaat ccggcat ggg 180
   t at t at t gaa gat aaagt ga t caaaaacca ct t cgct t cc gaat at at ct acaacgcct a 240
   t aaagat gaa aagacct gt g gcgt cct gt c t gaagacgac act t t cggt a ccat caccat 300
   tgctgagcca atcggtatta tttgcggtat cgtcccgact accaacccga cttctactgc 360
   t at ct t caaa t cgct gat ca gcct gaagac gcgt aacgcc at cat ct t ct ct ccgcaccc 420
   gcgtgctgct aaagacgcga ctaacaaagc ggcggacatc gtattgcagg cagctattgc 480
   cgcaggcgcg ccgaaagatc tgatcggttg gatcgaccag ccttccgtag aactgtctaa 540
   cgcactgatg catcatcctg acatcaacct gatcctcgcc accggcggcc caggtatggt 600
   taaggccgct tatagctccg gtaaaccagc tatcggcgtt ggcgcgggca acacgccggt 660
   t gnt at t gat gaaacggct g at at caaacg cgct gt ggcg t ccgt act ga t gt caaaaac 720
   ct t cgat aac ggcgt t at ct gt gct t ct ga acaat ccgt g gt ggt t gt cg act ccgt ct a 780
   cgacgccgt c cgcgagcgt t t t gccagcca t ggcggct ac ct gct gcagg gt aaagagct 840
   gaaagccgt t caggacat ca t cct gaaaaa t ggcgcgct g aat gcggcga t cgt t ggt ca 900
   accagcggca aaaatcgctg aactggcagg cttcaccgtg ccagccacca ctaagattct 960
   gat cggcgaa gt t accgacg t t gacgaaag cgaaccgt t c gct cacgaaa aact gt ct cc 1020
   gacgctggca atgtaccgtg cgaaagattt cgaagacgcg gtcaataaag cagaaaaact 1080
   ggtcgccatg ggcggtatcg gtcacacctc ttgcctgtac accgaccagg acaaccagcc 1140
   ggctcgcgtg gcctacttcg gccagatgat gaaaaccgcg cgtatcctga tcaacacccc 1200
   ggct t cccag ggt ggt at cg gcgacct gt a t aact t caag ct cgcacct t ccct gact ct 1260
   gggt t gt ggt t cct ggggt g gt aact ccat ct ct gaaaac gt nggt ccga aacacct gat 1320
   caacaagaaa accgttgcta agcgagctga aaacatgttg tggcataaac ttccgaaatc 1380
   t at ct act t c cgt cgt ggct cact gccaat cgcact ggat gaagt gat t a ct gat ggt ca 1440
   caaacgcgcg ctgattgtga ctgaccgctt cctgttcaac aacggttacg cggaccagat 1500
   cact t ccgt a ct gaaagcgg ct ggcgt aga aaccgaagt g t t ct t t gaag t t gaagct ga 1560
   cccaacgctg actatcgtgc gtaaaggcgc ggatctggcc aactccttca aaccagacgt 1620
   aat cat cgcc ct gggcggcg gt t ccccgat ggat gcggca aaaat cat gt gggt cat gt a 1680
   cgagcacccg gaaacccact tcgaagaact ggcgctgcgc tttatggata tccgtaaacg 1740
   t at ct acaag t t cccgaaaa t gggcgt caa agccaagat g gt t gccat t a ccaccacct c 1800
   cggt accggt t ct gaagt t a ccccgt t cgc cgt agt aacc gacgat gcaa ct ggacagaa 1860
   at at ccgcng gct gact acg ct ct gact cc ggat at ggcg at t gt cgat g ccaacct ggt 1920
   cat ggat at g ccgaagt ct c t ct gt gcct t cggt ggt ct g gat gccgt ga ct cacgct ct 1980
   ggaagct t ac gt ct ccgt ac t ggct t ct ga gt t ct ccgat ggt caggct c t gcaggcgct 2040
   gaaact gct g aaagagt at c t gccggcct c t t accat gaa ggt t ct aaga acccggt agc 2100
   ccgcgaacgt gtgcacagcg ccgccactat cgccggtatc gcgtttgcta acgccttcct 2160
   cggcgtgtgc cactcaatgg cgcacaaact gggctcgcag ttccacattc ctcacggtct 2220
   ggcaaacgcc ctgctgatca gcaacgttat ccgctataac gcgaatgaca acccgaccaa 2280
   gcagaccgca ttcagccagt atgaccgtcc gcaggcgcgc cgtcgctacg ctgagatcgc 2340
   agaccacct g ggcct gagcg ct ccgggcga ccgcact gcg gcgaaaat cg agaaact gct 2400
   ggcat ggct g gaaagcct ca aagct gaact gggt at t ccg aaat ct at cc gt gaagct gg 2460
   cgt t caggaa gcagact t cc t ggcgaacgt ggat aaact g t ct gaagat g cat t cgat ga 2520
   ccagt gcacc ggcgct aacc cgcgt t accc gct gat ct cc gagct gaaac agat t ct gct 2580
   ggatacctac tacggtcgtg atttatgtnn agaaggtnga aactgcagcg aagaaagaag 2640
   ctgctccggc taaagctgag aaaaaagcga aaaaatccgc ttaatcag                 2688
<210> 75
   <211> 30
   <212> DNA
   <213> Artificial Sequence
<220>
   <223> primer
<400> 75
   aagagct ccg cgaacgagcc at gacat t gc 30
<210> 76
   <211> 43
   <212> DNA
   <213> Artificial Sequence
<220>
   <223> primer
<400> 76
   gt cgacacac gt cat t cct c ct t gt cgcct at at t ggt t a aag 43
<210> 77
   <211> 361
   <212> DNA
   <213> Enterobacter aer ogenes
<400> 77
   aagagctccg cgaacgagcc atgacattgc tgacgactct ggcagtggca gatgacataa      60
   aactggtcga ctggttacaa caacgcctgg ggcttttaga gcaacgagac acggcaatgt     120
   t gcaccgt t t  gct gcat gat  at t gaaaaaa  at at caccaa  at aaaaaacg  cct t agt aag   180
   t at t t t t cag  ct t t t cat t c  t gact gcaac  gggcaat at g  t ct ct gt gt g  gat t aaaaaa  240
   agagt gt ct g  at agcagct t  ct gaact ggt  t acct gccgt  gagt aaat t a  aaat t t t at t   300
   gact t aggt c  act aaat act  t t aaccaat a  t aggcgacaa  ggaggaat ga  cgt gt gt cga   360
   c                                                                    361
<210> 78
   <211> 44
   <212> DNA
   <213> Artificial Sequence
<220>
   <223> primer
<400> 78
   aaggaggaat gacgt gt gt c gact cacaca t ct t caacgc t t cc 44
<210> 79
   <211 > 28
   <212> DNA
   <213> Artificial Sequence
<220>
   <223> primer
<400> 79
   t t gagct ct t t t t acagaaa ggt t t agg 28
<210> 80
   <211> 3461
   <212> DNA
   <213> Corynebacterium glutamicum
<400> 80
   aaggaggaat gacgtgtgtc gactcacaca tcttcaacgc ttccagcatt caaaaagatc 60
   ttggtagcaa accgcggcga aatcgcggtc cgtgctttcc gtgcagcact cgaaaccggt 120
   gcagccacgg tagctattta cccccgtgaa gatcggggat cattccaccg ctcttttgct 180
   tctgaagctg tccgcattgg taccgaaggc tcaccagtca aggcgtacct ggacatcgat 240
   gaaattatcg gtgcagctaa aaaagttaaa gcagatgcca tttacccggg atacggcttc 300
   ctgtctgaaa atgcccagct tgcccgcgag tgtgcggaaa acggcattac ttttattggc 360
   ccaaccccag aggttcttga tctcaccggt gataagtctc gcgcggtaac cgccgcgaag 420
   aaggctggtc tgccagtttt ggcggaatcc accccgagca aaaacatcga tgagatcgtt 480
   aaaagcgctg aaggccagac ttaccccatc tttgtgaagg cagttgccgg tggtggcgga 540
   cgcggtatgc gttttgttgc ttcacctgat gagcttcgca aattagcaac agaagcatct 600
   cgtgaagctg aagcggcttt cggcgatggc gcggtatatg tcgaacgtgc tgtgattaac 660
   cctcagcata ttgaagtgca gatccttggc gatcacactg gagaagttgt acacctttat 720
   gaacgtgact gctcactgca gcgtcgtcac caaaaagttg tcgaaattgc gccagcacag 780
   catttggatc cagaactgcg tgatcgcatt tgtgcggatg cagtaaagtt ctgccgctcc 840
   attggttacc agggcgcggg aaccgtggaa ttcttggtcg atgaaaaggg caaccacgtc 900
   ttcatcgaaa tgaacccacg tatccaggtt gagcacaccg tgactgaaga agtcaccgag 960
   gtggacctgg tgaaggcgca gatgcgcttg gctgctggtg caaccttgaa ggaattgggt 1020
   ctgacccaag ataagatcaa gacccacggt gcagcactgc agtgccgcat caccacggaa 1080
   gatccaaaca acggcttccg cccagatacc ggaactatca ccgcgtaccg ctcaccaggc 1140
   ggagctggcg ttcgtcttga cggtgcagct cagctcggtg gcgaaatcac cgcacacttt 1200
   gactccatgc tggtgaaaat gacctgccgt ggttccgact ttgaaactgc tgttgctcgt 1260
   gcacagcgcg cgttggctga gttcaccgtg tctggtgttg caaccaacat tggtttcttg 1320
   cgtgcgttgc tgcgggaaga ggacttcact tccaagcgca tcgccaccgg attcattgcc 1380
   gatcacccgc acctccttca ggctccacct gctgatgatg agcagggacg catcctggat 1440
   tacttggcag atgtcaccgt gaacaagcct catggtgtgc gtccaaagga tgttgcagct 1500
   cctatcgata agctgcctaa catcaaggat ctgccactgc cacgcggttc ccgtgaccgc 1560
   ctgaagcagc ttggcccagc cgcgtttgct cgtgatctcc gtgagcagga cgcactggca 1620
   gttactgata ccaccttccg cgatgcacac cagtctttgc ttgcgacccg agtccgctca 1680
   ttcgcactga agcctgcggc agaggccgtc gcaaagctga ctcctgagct tttgtccgtg 1740
   gaggcctggg gcggcgcgac ctacgatgtg gcgatgcgtt tcctctttga ggatccgtgg 1800
   gacaggctcg acgagctgcg cgaggcgatg ccgaatgtaa acattcagat gctgcttcgc 1860
   ggccgcaaca ccgtgggata caccccgtac ccagactccg tctgccgcgc gtttgttaag 1920
   gaagctgcca gctccggcgt ggacatcttc cgcatcttcg acgcgcttaa cgacgtctcc 1980
   cagatgcgtc cagcaatcga cgcagtcctg gagaccaaca ccgcggtagc cgaggtggct 2040
   atggcttatt ctggtgatct ctctgatcca aatgaaaagc tctacaccct ggattactac 2100
   ctaaagatgg cagaggagat cgtcaagtct ggcgctcaca tcttggccat taaggatatg 2160
   gctggtctgc ttcgcccagc tgcggtaacc aagctggtca ccgcactgcg ccgtgaattc 2220
   gatctgccag tgcacgtgca cacccacgac actgcgggtg gccagctggc aacctacttt 2280
   gctgcagctc aagctggtgc agatgctgtt gacggtgctt ccgcaccact gtctggcacc 2340
   acctcccagc catccctgtc tgccattgtt gctgcattcg cgcacacccg tcgcgatacc 2400
   ggtttgagcc tcgaggctgt ttctgacctc gagccgtact gggaagcagt gcgcggactg 2460
   tacctgccat ttgagtctgg aaccccaggc ccaaccggtc gcgtctaccg ccacgaaatc 2520
   ccaggcggac agttgtccaa cctgcgtgca caggccaccg cactgggcct tgcggatcgt 2580
   ttcgaactca tcgaagacaa ctacgcagcc gttaatgaga tgctgggacg cccaaccaag 2640
   gtcaccccat cctccaaggt tgttggcgac ctcgcactcc acctcgttgg tgcgggtgtg 2700
   gatccagcag actttgctgc cgatccacaa aagtacgaca tcccagactc tgtcatcgcg 2760
   ttcctgcgcg gcgagcttgg taaccctcca ggtggctggc cagagccact gcgcacccgc 2820
   gcactggaag gccgctccga aggcaaggca ccctctgacg aagttcctga ggaagagcag 2880
   gcgcacctcg acgctgatga ttccaaggaa cgtcgcaata gcctcaaccg cctgctgttc 2940
   ccgaagccaa ccgaagagtt cctcgagcac cgtcgccgct tcggcaacac ctctgcgctg 3000
   gatgatcgtg aattcttcta cggcctggtc gaaggccgcg agactttgat ccgcctgcca 3060
   gatgtgcgca ccccactgct tgttcgcctg gatgcgatct ctgagccaga cgataagggt 3120
   atgcgcaatg ttgtggccaa cgtcaacggc cagatccgcc caatgcgtgt gcgtgaccgc 3180
   tccgttgagt ctgtcaccgc aaccgcagaa aaggcagatt cctccaacaa gggccatgtt 3240
   gctgcaccat tcgctggtgt tgtcaccgtg actgttgctg aaggtgatga ggtcaaggct 3300
   ggagatgcag tcgcaatcat cgaggctatg aagatggaag caacaatcac tgcttctgtt 3360
   gacggcaaaa tcgatcgcgt tgtggttcct gctgcaacga aggtggaagg tggcgacttg 3420
   atcgtcgtcg tttcctaaac ctttctgtaa aaagagctca a 3461
<210> 81
   <211> 32
   <212> DNA
   <213> Artificial Sequence
<220>
   <223> primer
<400> 81
   aagcatgcta cgcgaacgag ccatgacatt t gc 32
<210> 82
   <211> 38
   <212> DNA
   <213> Artificial Sequence
<220>
   <223> primer
<400> 82
   cat acgt cat t cct cct t gt cgcct at at t ggt t aaag 38
<210> 83
   <211> 357
   <212> DNA
   <213> Eschericia coli
<400> 83
   aagcatgctc gcgaacgagc catgacattg ctgacgactc tggcagtggc agatgacata 60
   aaactggtcg actggttaca acaacgcctg gggcttttag agcaacgaga cacggcaatg 120
   t t gcaccgt t t gct gcat ga t at t gaaaaa aat at cacca aat aaaaaac gcct t agt aa 180
   gt at t t t t ca gct t t t cat t ct gact gcaa cgggcaat at gt ct ct gt gt ggat t aaaaa 240
   aagagt gt ct gat agcagct t ct gaact gg t t acct gccg t gagt aaat t aaaat t t t at 300
   t gact t aggt cact aaat ac t t t aaccaat at aggcgaca aggaggaat g acgt at g 357
<210> 84
   <211> 37
   <212> DNA
   <213> Artificial Sequence
<220>
   <223> primer
<400> 84
   gacaaggagg aat gacgt at gaat at aaac gt cgcag 37
<210> 85
   <211> 30
   <212> DNA
   <213> Artificial Sequence
<220>
   <223> pr i rrer
<400> 85
   gggaaggat c cat cct ggga aaaagt t ct g 30
<210> 86
   <211> 1781
   <212> DNA
   <213> Enterobacter aer ogenes
<220>
   <221> CDS
   <222> (19)..(1704)
<400> 86
   gacaaggagg aatgacgt atg aat ata aac gtc gca gat ttg tta aac ggg      51
                       Met Asn Ile Asn Val Ala Asp Leu Leu Asn Gly
                       1               5                   10
   aat tac atc ctg tta ttg ttt gtt gta ctc gca ctg ggt ctt tgc ctg      99
   Asn Tyr Ile Leu Leu Leu Phe Val Val Leu Ala Leu Gly Leu Cys Leu
               15                  20                  25
   ggg aaa ctt cgc ctc ggg tca gta caa ctt ggt aat tcc att ggc gtt     147
   Gly Lys Leu Arg Leu Gly Ser Val Gln Leu Gly Asn Ser Ile Gly Val
           30                  35                  40
   tta gtc gtt tct tta tta tta ggt cag cag cac ttc gcc att aac aca     195
   Leu Val Val Ser Leu Leu Leu Gly Gln Gln His Phe Ala Ile Asn Thr
       45                  50                  55
   gat gcg cta aat ctt ggc ttt atg ctg ttt att ttc tgc gtc ggc gtc     243
   Asp Ala Leu Asn Leu Gly Phe Met Leu Phe Ile Phe Cys Val Gly Val
   60                  65                  70                  75
   gaa gcg ggc ccc aac ttt ttt tcg att ttt ttc cgc gac ggc aaa aac     291
   Glu Ala Gly Pro Asn Phe Phe Ser Ile Phe Phe Arg Asp Gly Lys Asn
                   80                  85                  90
   tat cta atg ctg gcg ctg gtg atg gtg gcc agc gcg atg tta atc gct     339
   Tyr Leu Met Leu Ala Leu Val Met Val Ala Ser Ala Met Leu Ile Ala
               95                  100                 105
   atg ggg ctg ggc aaa ttg ttt ggc tgg gac atc ggc ctg acc gcc ggg     387
   Met Gly Leu Gly Lys Leu Phe Gly Trp Asp Ile Gly Leu Thr Ala Gly
           110                 115                 120
   atg ctg gcg ggc gct atg acc tcc acc ccg gtg ctg gtg ggc gct ggc     435
   Met Leu Ala Gly Ala Met Thr Ser Thr Pro Val Leu Val Gly Ala Gly
       125                 130                 135
   gat acc cta cgc cac ttt ggc ctg ccc agc gat cag ctg gcg cag tcg     483
   Asp Thr Leu Arg His Phe Gly Leu Pro Ser Asp Gln Leu Ala Gln Ser
   140                 145                 150                 155
   ctt gac cac ctg agc ctg ggt tac gcc ctg act tac ctg gtt ggc ctg     531
   Leu Asp His Leu Ser Leu Gly Tyr Ala Leu Thr Tyr Leu Val Gly Leu
                   160                 165                 170
   gtg agc ctg atc gtc ggc gcg cgc tat atg ccg aag ctg cag cat cag     579
   Val Ser Leu Ile Val Gly Ala Arg Tyr Met Pro Lys Leu Gln His Gln
               175                 180                 185
   gat ctg cag acc agc gcc cag cag atc gcc cgc gag cgc ggt ctg gat     627
   Asp Leu Gln Thr Ser Ala Gln Gln Ile Ala Arg Glu Arg Gly Leu Asp
           190                 195                 200
   acc gac tcc aag cgt aaa gtc tat ctg ccg gtt atc cgc gcc tac cgc     675
   Thr Asp Ser Lys Arg Lys Val Tyr Leu Pro Val Ile Arg Ala Tyr Arg
       205                 210                 215
   gtc ggc ccg gaa ttg gtc gcg tgg gcg gac ggc aaa aat ctt cgt gag     723
   Val Gly Pro Glu Leu Val Ala Trp Ala Asp Gly Lys Asn Leu Arg Glu
   220                 225                 230                 235
   ctg gga att tac cgc cag acc ggc tgc tat atc gag cgt att cgt cgt     771
   Leu Gly Ile Tyr Arg Gln Thr Gly Cys Tyr Ile Glu Arg Ile Arg Arg
                   240                 245                 250
   aac ggt atc ctg gcg aac ccg gat ggc gac gcg gta ctg cag atg ggc     819
   Asn Gly Ile Leu Ala Asn Pro Asp Gly Asp Ala Val Leu Gln Met Gly
               255                 260                 265
   gac gat atc gcg ctg gtt ggc tac ccg gac gcc cat gcc cgc ctc gac     867
   Asp Asp Ile Ala Leu Val Gly Tyr Pro Asp Ala His Ala Arg Leu Asp
           270                 275                 280
   cca agc ttc cgt aac ggt aaa gag gtg ttt gac cgc gac ctg ctc gat     915
   Pro Ser Phe Arg Asn Gly Lys Glu Val Phe Asp Arg Asp Leu Leu Asp
       285                 290                 295
   atg cgt atc gtc acc gaa gag att gtg gtt aag aat cat aac gcc gtc     963
   Met Arg Ile Val Thr Glu Glu Ile Val Val Lys Asn His Asn Ala Val
   300 305 310 315
   ggc cgc cgc ctg gcg cag ctg aag ctg acc gat cac ggc tgt ttc tta 1011
   Gly Arg Arg Leu Ala Gln Leu Lys Leu Thr Asp His Gly Cys Phe Leu
   320 325 330
   aac cgg gtg atc cgc agc cag att gag atg cca atc gac gat aac gtg 1059
   Asn Arg Val Ile Arg Ser Gln Ile Glu Met Pro Ile Asp Asp Asn Val
   335 340 345
   gtg ctc aat aaa ggc gac gtg ctg cag gtg agc ggc gac gcc cgc cgc 1107
   Val Leu Asn Lys Gly Asp Val Leu Gln Val Ser Gly Asp Ala Arg Arg
   350 355 360
   gta aaa acc gtc gct gac cgc atc ggc ttt att tcg att cac agc cag 1155
   Val Lys Thr Val Ala Asp Arg Ile Gly Phe Ile Ser Ile His Ser Gln
   365 370 375
   gtg acc gat ctg ctg gct ttc tgc gcc ttc ttt atc gtc ggc ctg atg 1203
   Val Thr Asp Leu Leu Ala Phe Cys Ala Phe Phe Ile Val Gly Leu Met
   380 385 390 395
   atc ggc atg atc act ttc cag ttc agc tcg ttc agc ttc ggc atc ggt 1251
   Ile Gly Met Ile Thr Phe Gln Phe Ser Ser Phe Ser Phe Gly Ile Gly
   400 405 410
   aac gcc gcc ggc ctg ctg ttc gcc ggc att atg ctt ggc ttc ctg cgc 1299
   Asn Ala Ala Gly Leu Leu Phe Ala Gly Ile Met Leu Gly Phe Leu Arg
   415 420 425
   gcc aac cat ccg acc ttc ggc tat atc ccg cag ggc gca ttg aac atg 1347
   Ala Asn His Pro Thr Phe Gly Tyr Ile Pro Gln Gly Ala Leu Asn Met
   430 435 440
   gtt aag gag ttc ggt ctg atg gtg ttt atg gcc ggg gtc ggc ctg agc 1395
   Val Lys Glu Phe Gly Leu Met Val Phe Met Ala Gly Val Gly Leu Ser
   445 450 455
   gcc ggc gcg ggt att aat aac ggt ctg ggc gcc att ggc ggg caa atg 1443
   Ala Gly Ala Gly Ile Asn Asn Gly Leu Gly Ala Ile Gly Gly Gln Met
   460 465 470 475
   ctg gct gcc ggt ctt atc gtc agc ctg gtg ccg gtg gtg atc tgc ttc 1491
   Leu Ala Ala Gly Leu Ile Val Ser Leu Val Pro Val Val Ile Cys Phe
   480 485 490
   ctg ttc ggc gcc tat gtg cta cgc atg aac cgc gcg atg ctg ttc ggc 1539
   Leu Phe Gly Ala Tyr Val Leu Arg Met Asn Arg Ala Met Leu Phe Gly
   495 500 505
   gcg atg atg ggc gcg cgc acc tgc gcc ccg gcg atg gaa att atc agc 1587
   Ala Met Met Gly Ala Arg Thr Cys Ala Pro Ala Met Glu Ile Ile Ser
   510 515 520
   gat acc gcg cgc agc aac att ccc gca ctc ggc tat gct ggc act tac 1635
   Asp Thr Ala Arg Ser Asn Ile Pro Ala Leu Gly Tyr Ala Gly Thr Tyr
   525 530 535
   gct atc gcc aac gtg ctg ctc acc ctt gcc ggt acg cta atc gtc atc 1683
   Ala Ile Ala Asn Val Leu Leu Thr Leu Ala Gly Thr Leu Ile Val Ile
   540 545 550 555
   atc tgg ccg ggc ctt ggc tag ccccggccga aaatttttaa ctttttcgca         1734
   Ile Trp Pro Gly Leu Gly
                   560
   aaaaaatcgg tgatgaacag aactttttcc caggatggat ccttccc 1781
<210> 87
   <211> 561
   <212> PRT
   <213> Ent er obact er aerogenes
<400> 87
   Met Asn Ile Asn Val Ala Asp Leu Leu Asn Gly Asn Tyr Ile Leu Leu
   1               5                   10                  15
   Leu Phe Val Val Leu Ala Leu Gly Leu Cys Leu Gly Lys Leu Arg Leu
               20                  25                  30
   Gly Ser Val Gln Leu Gly Asn Ser Ile Gly Val Leu Val Val Ser Leu
           35                  40                  45
   Leu Leu Gly Gln Gln His Phe Ala Ile Asn Thr Asp Ala Leu Asn Leu
       50                  55                  60
   Gly Phe Met Leu Phe Ile Phe Cys Val Gly Val Glu Ala Gly Pro Asn
   65                  70                  75                  80
   Phe Phe Ser Ile Phe Phe Arg Asp Gly Lys Asn Tyr Leu Met Leu Ala
   85 90 95
   Leu Val Met Val Ala Ser Ala Met Leu Ile Ala Met Gly Leu Gly Lys
   100 105 110
   Leu Phe Gly Trp Asp Ile Gly Leu Thr Ala Gly Met Leu Ala Gly Ala
   115 120 125
   Met Thr Ser Thr Pro Val Leu Val Gly Ala Gly Asp Thr Leu Arg His
   130 135 140
   Phe Gly Leu Pro Ser Asp Gln Leu Ala Gln Ser Leu Asp His Leu Ser
   145 150 155 160
   Leu Gly Tyr Ala Leu Thr Tyr Leu Val Gly Leu Val Ser Leu Ile Val
   165 170 175
   Gly Ala Arg Tyr Met Pro Lys Leu Gln His Gln Asp Leu Gln Thr Ser
   180 185 190
   Ala Gln Gln Ile Ala Arg Glu Arg Gly Leu Asp Thr Asp Ser Lys Arg
   195 200 205
   Lys Val Tyr Leu Pro Val Ile Arg Ala Tyr Arg Val Gly Pro Glu Leu
   210 215 220
   Val Ala Trp Ala Asp Gly Lys Asn Leu Arg Glu Leu Gly Ile Tyr Arg
   225 230 235 240
   Gln Thr Gly Cys Tyr Ile Glu Arg Ile Arg Arg Asn Gly Ile Leu Ala
   245 250 255
   Asn Pro Asp Gly Asp Ala Val Leu Gln Met Gly Asp Asp Ile Ala Leu
   260 265 270
   Val Gly Tyr Pro Asp Ala His Ala Arg Leu Asp Pro Ser Phe Arg Asn
   275 280 285
   Gly Lys Glu Val Phe Asp Arg Asp Leu Leu Asp Met Arg Ile Val Thr
   290 295 300
   Glu Glu Ile Val Val Lys Asn His Asn Ala Val Gly Arg Arg Leu Ala
   305 310 315 320
   Gln Leu Lys Leu Thr Asp His Gly Cys Phe Leu Asn Arg Val Ile Arg
   325 330 335
   Ser Gln Ile Glu Met Pro Ile Asp Asp Asn Val Val Leu Asn Lys Gly
   340 345 350
   Asp Val Leu Gln Val Ser Gly Asp Ala Arg Arg Val Lys Thr Val Ala
   355 360 365
   Asp Arg Ile Gly Phe Ile Ser Ile His Ser Gln Val Thr Asp Leu Leu
   370 375 380
   Ala Phe Cys Ala Phe Phe Ile Val Gly Leu Met Ile Gly Met Ile Thr
   385 390 395 400
   Phe Gln Phe Ser Ser Phe Ser Phe Gly Ile Gly Asn Ala Ala Gly Leu
   405 410 415
   Leu Phe Ala Gly Ile Met Leu Gly Phe Leu Arg Ala Asn His Pro Thr
   420 425 430
   Phe Gly Tyr Ile Pro Gln Gly Ala Leu Asn Met Val Lys Glu Phe Gly
   435 440 445
   Leu Met Val Phe Met Ala Gly Val Gly Leu Ser Ala Gly Ala Gly Ile
   450 455 460
   Asn Asn Gly Leu Gly Ala Ile Gly Gly Gln Met Leu Ala Ala Gly Leu
   465 470 475 480
   Ile Val Ser Leu Val Pro Val Val Ile Cys Phe Leu Phe Gly Ala Tyr
   485 490 495
   Val Leu Arg Met Asn Arg Ala Met Leu Phe Gly Ala Met Met Gly Ala
   500 505 510
   Arg Thr Cys Ala Pro Ala Met Glu Ile Ile Ser Asp Thr Ala Arg Ser
   515 520 525
   Asn Ile Pro Ala Leu Gly Tyr Ala Gly Thr Tyr Ala Ile Ala Asn Val
   530 535 540
   Leu Leu Thr Leu Ala Gly Thr Leu Ile Val Ile Ile Trp Pro Gly Leu
   545 550 555 560
   Gly
<210> 88
   <211> 561
   <212> PRT
   <213> Artificial Sequence
<220>
   <223> conserved sequence
<220>
   <221> misc_feature
   <222> (1)..(1)
   <223> Xaa can be any naturally occurring amino acid
<220>
   <221> misc_feature
   <222> (3)..(3)
   <223> Xaa can be any naturally occurring amino acid
<220>
   <221> misc_feature
   <222> (5)..(5)
   <223> Xaa can be any naturally occurring amino acid
<220>
   <221> misc_feature
   <222> (7)..(7)
   <223> Xaa can be any naturally occuring amino acid
<220>
   <221> misc_feature
   <222> (10)..(10)
   <223> Xaa can be any naturally occurring amino acid
<220>
   <221> misc_feature
   <222> (13)..(13)
   <223> Xaa can be any naturally occurring amino acid
<220>
   <221> misc_feature
   <222> (19)..(19)
   <223> Xaa can be any naturally occurring amino acid
<220>
   <221> misc_feature
   <222> (32)..(32)
   <223> Xaa can be any naturally occurring amino acid
<220>
   <221> misc_feature
   <222> (35)..(35)
   <223> Xaa can be any naturally occurring amino acid
<220>
   <221> misc_feature
   <222> (56)..(57)
   <223> Xaa can be any naturally occurring amino acid
<220>
   <221> misc_feature
   <222> (63)..(63)
   <223> Xaa can be any naturally occurring amino acid
<220>
   <221> misc_feature
   <222> (75)..(75)
   <223> Xaa can be any naturally occurring amino acid
<220>
   <221> misc_feature
   <222> (93)..(93)
   <223> Xaa can be any naturally occurring amino acid
<220>
   <221> misc_feature
   <222> (97)..(97)
   <223> Xaa can be any naturally occurring amino acid
<220>
   <221> misc feature
   <222> (101)..(101)
   <223> Xaa can be any naturally occurring amino acid
<220>
   <221> misc_feature
   <222> (104)..(106)
   <223> Xaa can be any naturally occurring amino acid
<220>
   <221> misc_feature
   <222> (108)..(108)
   <223> Xaa can be any naturally occurring amino acid
<220>
   <221> misc_feature
   <222> (110)..(110)
   <223> Xaa can be any naturally occur r i ng amino acid
<220>
   <221> misc_feature
   <222> (124)..(124)
   <223> Xaa can be any naturally occurring amino acid
<220>
   <221> misc_feature
   <222> (128)..(128)
   <223> Xaa can be any naturally occurring amino acid
<220>
   <221> misc_feature
   <222> (144)..(146)
   <223> Xaa can be any naturally occurring amino acid
<220>
   <221> misc_feature
   <222> (149)..(150)
   <223> Xaa can be any naturally occurring amino acid
<220>
   <221> misc_feature
   <222> (153)..(156)
   <223> Xaa can be any naturally occur r i ng amino acid
<220>
   <221> misc_feature
   <222> (158)..(158)
   <223> Xaa can be any naturally occurring amino acid
<220>
   <221> misc_feature
   <222> (165)..(165)
   <223> Xaa can be any naturally occurring amino acid
<220>
   <221> misc_feature
   <222> (169)..(169)
   <223> Xaa can be any naturally occurring amino acid
<220>
   <221> misc_feature
   <222> (176)..(176)
   <223> Xaa can be any naturally occurring amino acid
<220>
   <221> misc_feature
   <222> (181)..(181)
   <223> Xaa can be any naturally occurring amino aci d
<220>
   <221> misc_feature
   <222> (183)..(183)
   <223> Xaa can be any naturally occurring amino acid
<220>
   <221> misc_feature
   <222> (190)..(190)
   <223> Xaa can be any naturally occurring amino acid
<220>
   <221> misc_feature
   <222> (192)..(192)
   <223> Xaa can be any naturally occurring amino acid
<220>
   <221> misc_feature
   <222> (204)..(204)
   <223> Xaa can be any naturally occurring amino acid
<220>
   <221> misc_feature
   <222> (206)..(207)
   <223> Xaa can be any naturally occurring amino acid
<220>
   <221> misc_feature
   <222> (211)..(211)
   <223> Xaa can be any naturally occurring amino acid
<220>
   <221> misc_feature
   <222> (228).. (229)
   <223> Xaa can be any naturally occurring amino acid
<220>
   <221> misc_feature
   <222> (257)..(257)
   <223> Xaa can be any naturally occurring amino acid
<220>
   <221> misc_feature
   <222> (266)..(266)
   <223> Xaa can be any naturally occurring amino acid
<220>
   <221> misc_feature
   <222> (269)..(269)
   <223> Xaa can be any naturally occurring amino acid
<220>
   <221> misc_feature
   <222> (271)..(271)
   <223> Xaa can be any naturally occurring amino acid
<220>
   <221> misc_feature
   <222> (284)..(284)
   <223> Xaa can be any naturally occurring amino acid
<220>
   <221> misc_feat ur e
   <222> (307)..(307)
   <223> Xaa can be any naturally occurring amino acid
<220>
   <221> misc_feature
   <222> (316)..(317)
   <223> Xaa can be any naturally occurring amino acid
<220>
   <221> misc_feature
   <222> (320)..(321)
   <223> Xaa can be any naturally occurring amino acid
<220>
   <221> misc_feature
   <222> (323)..(323)
   <223> Xaa can be any naturally occurring amino aci d
<220>
   <221> misc_feature
   <222> (347)..(348)
   <223> Xaa can be any naturally occurring amino aci d
<220>
   <221> misc_feature
   <222> (360)..(360)
   <223> Xaa can be any naturally occurring amino acid
<220>
   <221> misc_feature
   <222> (366)..(367)
   <223> Xaa can be any naturally occurring amino aci d
<220>
   <221> misc_featur e
   <222> (374)..(375)
   <223> Xaa can be any naturally occurring amino aci d
<220>
   <221> misc_feature
   <222> (380)..(380)
   <223> Xaa can be any naturally occurring amino acid
<220>
   <221> misc_feature
   <222> (391)..(392)
   <223> Xaa can be any naturally occurring amino acid
<220>
   <221> misc_feature
   <222> (396).. (396)
   <223> Xaa can be any naturally occurring amino acid
<220>
   <221> misc_feat ur e
   <222> (398)..(398)
   <223> Xaa can be any naturally occurring amino acid
<220>
   <221> misc_feature
   <222> (405).. (405)
   <223> Xaa can be any naturally occurring amino acid
<220>
   <221> misc_feature
   <222> (410)..(410)
   <223> Xaa can be any naturally occurring amino acid
<220>
   <221> misc_feature
   <222> (426).. (426)
   <223> Xaa can be any naturally occurring amino acid
<220>
   <221> misc_feature
   <222> (442)..(442)
   <223> Xaa can be any naturally occurring amno acid
<220>
   <221> misc_feature
   <222> (462)..(462)
   <223> Xaa can be any naturally occurring amino acid
<220>
   <221> misc_feature
   <222> (465).. (466)
   <223> Xaa can be any naturally occurring amino acid
<220>
   <221> misc_feature
   <222> (468)..(471)
   <223> Xaa can be any naturally occurring amino acid
<220>
   <221> misc_featur e
   <222> (473)..(474)
   <223> Xaa can be any naturally occur r i ng amino acid
<220>
   <221> misc_feature
   <222> (477)..(478)
   <223> Xaa can be any naturally occurring amino acid
<220>
   <221> misc_feature
   <222> (481)..(481)
   <223> Xaa can be any naturally occurring amino acid
<220>
   <221> misc_feature
   <222> (485).. (485)
   <223> Xaa can be any naturally occurring amino acid
<220>
   <221> misc_feature
   <222> (491)..(491)
   <223> Xaa can be any naturally occurring amino acid
<220>
   <221> misc_feature
   <222> (504)..(504)
   <223> Xaa can be any naturally occurring amino acid
<220>
   <221> misc_feature
   <222> (509)..(509)
   <223> Xaa can be any naturally occurring amino acid
<220>
   <221> misc feature
   <222> (552)..(552)
   <223> Xaa can be any naturally occurring amino acid
<220>
   <221> misc_feature
   <222> (555)..(556)
   <223> Xaa can be any naturally occurring amino acid
<220>
   <221> misc_feature
   <222> (559)..(559)
   <223> Xaa can be any naturally occurring amino acid
<220>
   <221> misc_feature
   <222> (561)..(561)
   <223> Xaa can be any naturally occurring amino acid
<400> 88
   Xaa Asn Xaa Asn Xaa Ala Xaa Leu Leu Xaa Gly Asn Xaa Ile Leu Leu
   1               5                   10                  15
   Leu Phe Xaa Val Leu Ala Leu Gly Leu Cys Leu Gly Lys Leu Arg Xaa
               20                  25                  30
   Gly Ser Xaa Gln Leu Gly Asn Ser Ile Gly Val Leu Val Val Ser Leu
           35                  40                  45
   Leu Leu Gly Gln Gln His Phe Xaa Xaa Asn Thr Asp Ala Leu Xaa Leu
       50                  55                  60
   Gly Phe Met Leu Phe Ile Phe Cys Val Gly Xaa Glu Ala Gly Pro Asn
   65                  70                  75                  80
   Phe Phe Ser Ile Phe Phe Arg Asp Gly Lys Asn Tyr Xaa Met Leu Ala
                   85                  90                  95
   Xaa Val Met Val Xaa Ser Ala Xaa Xaa Xaa Ala Xaa Gly Xaa Gly Lys
               100                 105                 110
   Leu Phe Gly Trp Asp Ile Gly Leu Thr Ala Gly Xaa Leu Ala Gly Xaa
           115                 120                 125
   Met Thr Ser Thr Pro Val Leu Val Gly Ala Gly Asp Thr Leu Arg Xaa
       130                 135                 140
   Xaa Xaa Met Glu Xaa Xaa Gln Leu Xaa Xaa Xaa Xaa Asp Xaa Leu Ser
   145                 150                 155                 160
   Leu Gly Tyr Ala Xaa Thr Tyr Leu Xaa Gly Leu Val Ser Leu Ile Xaa
                   165                 170                 175
   Gly Ala Arg Tyr Xaa Pro Xaa Leu Gln His Gln Asp Leu Xaa Thr Xaa
               180                 185                 190
   Ala Gln Gln Ile Ala Arg Glu Arg Gly Leu Asp Xaa Asp Xaa Xaa Arg
           195                 200                 205
   Lys Val Xaa Leu Pro Val Ile Arg Ala Tyr Arg Val Gly Pro Glu Leu
       210                 215                 220
   Val Ala Trp Xaa Xaa Gly Lys Asn Leu Arg Glu Leu Gly Ile Tyr Arg
   225                 230                 235                 240
   Gln Thr Gly Cys Tyr Ile Glu Arg Ile Arg Arg Asn Gly Ile Leu Ala
                   245                 250                 255
   Xaa Pro Asp Gly Asp Ala Val Leu Gln Xaa Gly Asp Xaa Ile Xaa Leu
               260                 265                 270
   Val Gly Tyr Pro Asp Ala His Ala Arg Leu Asp Xaa Ser Phe Arg Asn
           275                 280                 285
   Gly Lys Glu Val Phe Asp Arg Asp Leu Leu Asp Met Arg Ile Val Thr
       290                 295                 300
   Glu Glu Xaa Val Val Lys Asn His Asn Ala Val Xaa Xaa Arg Leu Xaa
   305                 310                 315                 320
   Xaa Leu Xaa Leu Thr Asp His Gly Cys Phe Leu Asn Arg Val Ile Arg
                   325                 330                 335
   Ser Gln Ile Glu Met Pro Ile Asp Asp Asn Xaa Xaa Leu Asn Lys Gly
               340                 345                 350
   Asp Val Leu Gln Val Ser Gly Xaa Ala Arg Arg Val Lys Xaa Xaa Ala
           355                 360                 365
   Asp Arg Ile Gly Phe Xaa Xaa Ile His Ser Gln Xaa Thr Asp Leu Leu
       370                 375                 380
   Ala Phe Cys Ala Phe Phe Xaa Xaa Gly Leu Met Xaa Gly Xaa Ile Thr
   385                 390                 395                 400
   Phe Gln Phe Ser Xaa Phe Ser Phe Gly Xaa Gly Asn Ala Ala Gly Leu
                   405                 410                 415
   Leu Phe Ala Gly Ile Met Leu Gly Phe Xaa Arg Ala Asn His Pro Thr
               420                 425                 430
   Phe Gly Tyr Ile Pro Gln Gly Ala Leu Xaa Met Val Lys Glu Phe Gly
           435                 440                 445
   Leu Met Val Phe Met Ala Gly Val Gly Leu Ser Ala Gly Xaa Gly Ile
       450                 455                 460
   Xaa Xaa Gly Xaa Xaa Xaa Xaa Gly Xaa Xaa Met Leu Xaa Xaa Gly Leu
   465                 470                 475                 480
   Xaa Val Ser Leu Xaa Pro Val Val Ile Cys Xaa Leu Phe Gly Ala Tyr
                   485                 490                 495
   Val Leu Arg Met Asn Arg Ala Xaa Leu Phe Gly Ala Xaa Met Gly Ala
               500                 505                 510
   Arg Thr Cys Ala Pro Ala Met Glu Ile Ile Ser Asp Thr Ala Arg Ser
           515                 520                 525
   Asn Ile Pro Ala Leu Gly Tyr Ala Gly Thr Tyr Ala Ile Ala Asn Val
       530                 535                 540
   Leu Leu Thr Leu Ala Gly Thr Xaa Ile Val Xaa Xaa Trp Pro Xaa Leu
   545                 550                 555                 560
   Xaa

## Description

### Technical Field

The present invention relates to a method for producing an acidic substance having a carboxyl group. L-Glutamic acid and L-aspartic acid are widely used as raw materials of seasonings and to forth. Succinic acid is widely used as a raw material of seasonings and biodegradable plastics.

### Background Art

L-Glutamic acid is mainly produced by fermentation utilizing L-glutamic acid-producing bacteria of the so-called coryneform bacteria belonging to the genus *Brevibacterium, Corynebacterium* or *Microbacterium* or mutant strains thereof (see, for example, Non-patent document 1). As methods for producing L-glutamic acid by fermentation using other bacterial strains, methods using a microorganism belonging to the genus *Bacillus, Streptomyces, Penicillium* or the like (see, for example, Patent document 1), methods using a microorganism belonging to the genus *Pseudomonas, Arthrobacter, Serratia, Candida* or the like (see, for example, Patent document 2), methods using a microorganism belonging to the genus *Bacillus, Pseudomonas, Serratia, Aerobacter aerogenes* (currently referred to as *Enterobacter aerogenes*) or the like (see, for example, Patent document 3), methods using a mutant strain of *Escherichia coli* (see, for example, Patent document 4), and the like are known. In addition, methods for producing L-glutamic acid using a microorganism belonging to the genus *Klebsiella, Erwinia, Pantoea* or *Enterobacter* have also been disclosed (see, for example, Patent documents 5 to 7).

Furthermore, various techniques for increasing L-glutamic acid-producing ability by enhancing L-glutamic acid biosynthetic enzymes using recombinant DNA techniques have been disclosed. For example, it has been reported for *Corynebacterium* or *Brevibacterium* bacteria that introduction of a gene coding for citrate synthase derived from *Escherichia coli* or *Corynebacterium glutamicum* was effective for enhancement of L-glutamic acid-producing ability of coryneform bacteria (see, for example, Patent document 8). Furthermore, it has also been reported that introduction of a citrate synthase gene derived from a coryneform bacterium into enterobacteria belonging to the genus *Enterobacter, Klebsiella, Serratia, Erwinia* or *Escherichia* was effective for enhancement of L-glutamic acid-producing ability thereof (see, for example, Patent document 7).

As methods for improving abilities to produce target substances such as amino acids, methods of modifying uptake or excretion systems of substances are known. As methods for modifying an uptake system for a substance, for example, methods of deleting or attenuating a system for uptake of a target substance into cells to enhance ability to produce the target substance are known. Specifically, methods of improving an ability to produce L-glutamic acid by deleting the *gluABCD* operon or a part thereof to eliminate or attenuate uptake of L-glutamic acid into cells (see, for example, Patent document 9), methods of enhancing an ability to produce purine nucleotides by attenuating uptake of purine nucleotides into cells (see, for example, Patent document 10), and the like are known.

Furthermore, as methods for modifying an excretion system, methods of enhancing an excretion system for a target substance and methods of deleting or attenuating an excretion system for an intermediate or substrate in a biosynthesis system of a target substance are known. As the methods of enhancing an excretion system of a target substance, for example, methods for producing L-lysine by utilizing a *Corynebacterium* bacterium strain in which L-lysine excretion gene (*lysE*) is enhanced (see, for example, Patent document 11), and methods for producing L-glutamic acid by using an enterobacterium in which L-glutamic acid excretion system gene (*yhfK*) is enhanced (see, for example, Patent document 18) are known. Furthermore, methods for producing an L-amino acid using the *rhtA*, *B*, *C* genes, which are suggested to be involved in excretion of L-amino acids, have also been reported (see, for example, Patent document 12). As the methods for, for example, deleting an excretion system for an intermediate or substrate in a biosynthesis system of a target substance, for the case where the target substance is L-glutamic acid, methods of mutating or disrupting the 2-oxoglutarate permease gene to attenuate excretion of 2-oxoglutarate, which is an intermediate of the target substance (see, for example, Patent document 13) are known.

Furthermore, use of the gene coding for the ATP binding cassette superfamily (ABC transporter) involved in transportation of substances through cell membranes in breeding of microorganisms in which transmembrane transportation of amino acids is modified has been suggested (see, for example, Patent document 14).

Furthermore, it has also been reported that L-glutamic acid production efficiency was improved in *Escherichia* bacteria by enhancing expression of genes expected to participate in excretion of L-amino acids such as *yfiK* (see, for example, Patent document 15). Moreover, it has also been reported that L-glutamic acid-producing ability can be improved by enhancing expression of the *yhfK* gene (see, for example, Patent document 18).

Moreover, as methods for producing L-glutamic acid, methods of culturing a microorganism under acidic conditions to produce L-glutamic acid by fermentation with precipitating the L-glutamic acid are known (see, for example, Patent document 16). Under a low pH condition where L-glutamic acid is precipitated, existence ratio of L-glutamic acid in free form having no electrical charge increases, and the L-glutamic acid easily penetrates cell membranes. When L-glutamic acid is taken up into cells, it is converted into an intermediate of the TCA cycle, 2-oxoglutaric acid, in one step by glutamate dehydrogenase, and therefore it is generally considered that L-glutamic acid taken up into cells is easily metabolized. However, an *Enterobacter* bacterium or *Escherichia* bacterium in which 2-oxoglutarate dehydrogenase activity is deleted or attenuated or the like is used in the fermentative production of L-glutamic acid (for example, Patent documents 16 and 17), but such a microorganism cannot degrade 2-oxoglutaric acid, therefore intracellular 2-oxoglutaric acid concentration increases as a result to inhibit growth of the microorganism, and thus culture cannot be performed. Therefore, in Patent document 16 described above, a strain that is deficient in the 2-oxoglutarate dehydrogenase activity and can produce L-glutamic acid with precipitating L-glutamic acid was bred by a mutation treatment and used for the production of L-glutamic acid.

For the production of non-amino organic acids including succinic acid by fermentation, anaerobic bacteria including those belonging to the genus *Anaerobiospirillum* or *Actinobacillus* are usually used (Patent documents 19 and 20, Non-patent document 2). Although use of such anaerobic bacteria provides high yields of products, many nutrients are required for their proliferation, and therefore, it is necessary to add a large amount of organic nitrogen sources such as corn steep liquor (CSL) into culture medium. Addition of large amounts of organic nitrogen sources results in not only increase in culture cost, but also increase in purification cost for isolating the product, and therefore it is not economical.

In addition, known methods in which aerobic bacteria such as coryneform bacteria are once cultured under aerobic conditions to proliferate bacterial cells, then harvested, washed, and allowed as resting cells to produce a non-amino organic acid without supplying oxygen (Patent documents 21 and 22) are also known. These methods are economical, since organic nitrogen may be added in a smaller amount for proliferating the bacterial cells, and the bacteria can sufficiently grow in a simple culture medium. However, there is still a room for improvement in terms of production amount, concentration, and production rate per cell of the target organic acids as well as simplification of production process, and the like. Furthermore, production of a non-amino organic acid by fermentation using a bacterium in which phosphoenolpyruvate carboxylase activity is enhanced (for example, Patent document 23), and the like have also been reported.

Furthermore, as for *Escherichia coli,* which is a facultative anaerobic gram negative bacterium, methods for producing a non-amino organic acid by culturing it once under an aerobic condition to allow growth of cells, and then culturing it as resting cells without supplying oxygen to anaerobically produce the non-amino organic acid (Non-patent document 3), like the methods using coryneform bacteria, or aerobically culturing it to aerobically produce the non-amino organic acid (Patent document 24) are known. However, since *Escherichia coli* is a gram negative bacterium, it is vulnerable to osmotic pressure, and there remains room for improvement in productivity per cell etc.

The *ybjL* gene is a gene located on the genome of *Escherichia coli* (see, for example, Non-patent document 4), and it is also considered to code for a certain transporter on the basis of the motives, topology etc. of the deduced amino acid sequence. However, cloning of the gene as well as expression of the gene and analysis of the expression product have not been reported, and actual functions of the gene have still remained unknown.
WO 2006/069610 A2 discloses a process for the control of the production of fine chemicals including carboxyl containing chemicals, in a microorganism.
Patent document 1: U.S. Patent No. 3,220,929
Patent document 2: U.S. Patent No. 3,563,857
Patent document 3: Japanese Patent Publication (KOKOKU) No. 32-9393
Patent document 4: Japanese Patent Laid-open (KOKAI) No. 5-244970
Patent document 5: Japanese Patent Laid-open No. 2000-106869 (U.S. Patent No. 6,682,912)
Patent document 6: Japanese Patent Laid-open No. 2000-189169 (U.S. Patent Published Application No. 2001009836)
Patent document 7: Japanese Patent Laid-open No. 2000-189175 (U.S. Patent No. 7,247,459)
Patent document 8: Japanese Patent Publication No. 7-121228
Patent document 9: European Patent Application Laid-open No. 1038970
Patent document 10: European Patent Application Laid-open No. 1004663
Patent document 11: International Patent Publication WO97/23597
Patent document 12: Japanese Patent Laid-open No. 2000-189177 (U.S. Patent Published Application No. 2005239177)
Patent document 13: International Patent Publication WO 2001/5959
Patent document 14: International Patent Publication WO00/37647
Patent document 15: Japanese Patent Laid-open No. 2000-189180 (U.S. Patent No. 6,979,560).
Patent document 16: Japanese Patent Laid-open No. 2001-333769 (U.S. Patent Published Application No. 2007134773)
Patent document 17: Japanese Patent Laid-open No. 7-203980 (U.S. Patent No. 5,573,945)
Patent document 18: Japanese Patent Laid-open No. 2005-278643 (U.S. Patent Published Application No. 2005196846)
Patent document 19: U.S. Patent No. 5,142,834
Patent document 20: U.S. Patent No. 5,504,004
Patent document 21: Japanese Patent Laid-open No. 11-113588
Patent document 22: Japanese Patent Laid-open No. 11-196888
Patent document 23: Japanese Patent Laid-open No. 11-196887
Patent document 24: U.S. Patent Published Application No. 20050170482
Non-patent document 1: Kunihiko Akashi et al., Amino Acid Fermentation, Japan Scientific Societies Press (Gakkai Shuppan Center), pp.195-215, 1986
Non-patent document 2: Guettler, M.V. et al., 1999, International Journal of Systematic Bacteriology, 49:207-216
Non-patent document 3: Vemuri G.N. et al., 2002, Journal of Industrial Microbiology and Biotechnology, 28(6):325-332
Non-patent document 4: Blattner, F.R. et al., 1997, Science, 277(5331):1453-74

### Disclosure of the Invention

### Object to be Achieved by the Invention

An object of the present invention is to provide a method for efficiently producing an acidic substance having a carboxyl group by using a bacterial strain that can efficiently produce an acidic substance having a carboxyl group, especially L-glutamic acid, L-aspartic acid, and succinic acid.

### Means for Achieving the Object

The inventors of the present invention conducted various researches in order to achieve the aforementioned object, and as a result, they isolated *ybjL* as a gene involved in L-glutamic acid resistance and found that in a strain in which expression of the *ybjL* gene has been enhanced, L-glutamic acid fermentation yield is improved. They also found that in a strain in which expression of the ybjL gene was enhanced, production rate or yield of succinic acid is improved, and thus accomplished the present invention.
That is, the present invention provides the followings.
[1] A method for producing an acidic substance having a carboxyl group, which comprises culturing a microorganism which has an ability to produce an acidic substance having a carboxyl group and has been modified so that expression of the *ybjL* gene is enhanced by increasing copy number of the *ybjL* gene or modifying an expression control sequence of the *ybjL* gene, wherein the *ybjL* gene is a gene coding for a protein defined in the following (A) or (B):
   (A) a protein comprising the amino acid sequence of SEQ ID NO: 2, 4 or 87;
   (B) a protein comprising an amino acid sequence of SEQ ID NO: 2, 4 or 87, wherein one to 10 amino acid residues are substituted, deleted, inserted or added, and the protein improves an ability of the microorganism to produce an acidic substance having a carboxyl group when expression of the protein is enhanced in the microorganism;
      in a medium to produce and accumulate the acidic substance having a carboxyl group in the medium, and collecting the acidic substance having a carboxyl group from the medium,
      wherein the acidic substance consists of one or more kinds of organic acids selected from the group consisting of succinic acid, fumaric acid, malic acid, oxalacetic acid, citric acid, isocitric acid, α-ketoglutaric acid, L-glutamic acid, and L-aspartic acid.
[2] The method according to [1] above, wherein the *ybjL* gene is a gene coding for the protein of SEQ ID NO: 5 or 88.
[3] The method according to [1] or [2] above, wherein the microorganism is a bacterium belonging to the family *Enterobacteriaceae.*
[4] The method according to [3] above, wherein the microorganism is selected from the group consisting of bacteria belonging to the genera *Escherichia, Enterobacter, Raoultella, Pantoea,* and *Klebsiella.*
[5] The method according to [1] or [2] above,, wherein the microorganism is a rumen bacterium.
[6] The method according to [5] above, wherein the microorganism is *Mannheimia succiniciproducens.*
[7] A method for producing an acidic substance having a carboxyl group, which comprises allowing a microorganism which has an ability to produce an acidic substance having a carboxyl group and has been modified so that expression of the *ybjL* gene is enhanced by increasing copy number of the *ybjL* gene or modifying an expression control sequence of the *ybjL* gene, wherein the *ybjL* gene is a gene coding for a protein defined in the following (A) or (B):
   (A) a protein comprising the amino acid sequence of SEQ ID NO: 2, 4 or 87;
   (B) a protein comprising an amino acid sequence of SEQ ID NO: 2, 4 or 87, wherein one to 10 amino acid residues are substituted, deleted, inserted or added, and the protein improves an ability of the microorganism to produce an acidic substance having a carboxyl group when expression of the protein is enhanced in the microorganism;
      or a product obtained by processing the microorganism to act on an organic raw material in a reaction mixture containing carbonate ions, bicarbonate ions, or carbon dioxide gas to produce the acidic substance having a carboxyl group, and collecting the acidic substance having a carboxyl group.
[8] The method according to [7] above, wherein the acidic substance consists of one or more kinds of organic acids selected from the group consisting of succinic acid, fumaric acid, malic acid, oxalacetic acid, citric acid, isocitric acid, and α-ketoglutaric acid.
[9] A method for producing a succinic acid-containing polymer, which comprises the step of producing succinic acid by the method according to any one of [1] to [6] and [8] above, and the step of polymerizing the obtained succinic acid.

### Brief Explanation of the Drawings

Fig. 1 shows structure of helper plasmid RSF-Red-TER.
Fig. 2 shows construction of helper plasmid RSF-Red-TER.
Fig. 3 shows growth of *ybjL*-amplified strain in the presence of high concentration L-glutamic acid.
Fig. 4 shows accumulation of succinic acid obtained with *ybjL*-amplified strain of *Escherichia* bacterium.
Fig. 5 shows accumulation of succinic acid obtained with *ybjL*-amplified strain of *Enterobacter* bacterium.

### Best Mode for Carrying out the Invention

Hereinafter, the present invention will be explained in detail.

### <1> Microorganism used in the present invention having ability to produce acidic substance having a carboxyl group

The microorganism used in the present invention is a microorganism which has an ability to produce an acidic substance having a carboxyl group and has been modified so that expression of the *ybjL* gene is enhanced. The term "ability to produce an acidic substance having a carboxyl group" used herein means an ability of the microorganism used in the present invention to produce and accumulate an acidic substance having a carboxyl group in a medium or cells of the microorganism used in the present invention to such a degree that the acidic substance having a carboxyl group can be collected from the cells or medium when the microorganism used in the present invention is cultured in the medium. The microorganism having an ability to produce an acidic substance having a carboxyl group may be a microorganism originally having the ability to produce an acidic substance having a carboxyl group, or may be a microorganism obtained by modifying a microorganism such as those described below using mutation techniques or recombinant DNA techniques so that the microorganism has an ability to produce an acidic substance having a carboxyl group, or a microorganism imparted with or an ability to produce an acidic substance having a carboxyl group or microorganism in which ability to produce an acidic substance having a carboxyl group is enhanced by introduction of the gene used in the present invention.

In the present invention, the "acidic substance having a carboxyl group" means L-glutamic acid, L-aspartic acid, succinic acid, fumaric acid, malic acid, oxalacetic acid, citric acid, isocitric acid, and α-ketoglutaric acid.

Although parent strain of the microorganism which can be used for the present invention is not particularly limited, bacteria are preferred. As the bacteria, bacteria belonging to the family *Enterobacteriaceae,* bacteria classified into rumen bacteria, and coryneform bacteria are preferred.

The family *Enterobacteriaceae* encompasses bacteria belonging to the genera *Escherichia, Enterobacter, Erwinia, Klebsiella, Pantoea, Photorhabdus, Providencia, Raoultella, Salmonella, Serratia, Shigella, Morganella, Yersinia,* and the like. In particular, bacteria classified into the family *Enterobacteriaceae* according to the taxonomy used by the NCBI (National Center for Biotechnology Information) database (http://www.ncbi.nlm.nih.gov/Taxonomy/Browser/wwwtax.cgi?i d=91347) are preferred. Among these, bacteria belonging to the genus *Escherichia, Enterobacter, Raoultella, Pantoea, Klebsiella,* or Serratia are especially preferred.

A "bacterium belonging to the genus *Escherichia"* means that the bacterium is classified into the genus *Escherichia* according to the classification known to a person skilled in the art of microbiology, although the bacterium is not particularly limited. Examples of the bacterium belonging to the genus *Escherichia* used in the present invention include, but are not limited to, *Escherichia coli (E. coli).*

The bacterium belonging to the genus *Escherichia* that can be used in the present invention is not particularly limited. However, examples include, for example, the bacteria of the phyletic groups described in the work of Neidhardt et al. (Neidhardt F.C. Ed., 1996, Escherichia coli and Salmonella: Cellular and Molecular Biology/Second Edition, pp.2477-2483, Table 1, American Society for Microbiology Press, Washington, D.C.). Specific examples include the *Escherichia coli* W3110 (ATCC 27325), *Escherichia coli* MG1655 (ATCC 47076) and the like derived from the prototype wild-type strain, K12 strain.

These strains are available from, for example, the American Type Culture Collection (Address: 12301 10801 University Boulevard, Manassas, VA 20108, United States of America). That is, registration numbers are given to each of the strains, and the strains can be ordered by using these numbers. The registration numbers of the strains are listed in the catalogue of the American Type Culture Collection.

*Pantoea* bacteria, *Erwinia* bacteria, and *Enterobacter* bacteria are bacteria classified as γ-proteobacteria, and they are taxonomically very close to one another (J. Gen. Appl. Microbiol., 1997, 43, 355-361; Int. J. Syst. Bacteriol., 1997, 43, 1061-1067). In recent years, some bacteria belonging to the genus *Enterobacter* were reclassified as *Pantoea agglomerans, Pantoea dispersa,* or the like, on the basis of DNA-DNA hybridization experiments etc. (Int. J. Syst. Bacteriol., 1989, 39:337-345). Furthermore, some bacteria belonging to the genus *Erwinia* were reclassified as *Pantoea ananas* or *Pantoea stewartii* (refer to Int. J. Syst. Bacteriol., 1993, 43:162-173).

Examples of the *Enterobacter bacteria* include *Enterobacter agglomerans, Enterobacter aerogenes,* and the like. Specifically, the strains exemplified in European Patent Application Laid-open No. 952221 can be used. Typical strains of the genus *Enterobacter* include *Enterobacter agglomerans* ATCC 12287 strain, *Enterobacter aerogenes* ATCC 13048 strain, *Enterobacter aerogenes* NBRC 12010 strain (Biotechnol Bioeng., 2007, Mar. 27;98(2):340-348), and *Enterobacter aerogenes* AJ110637 strain (FERM ABP-10955), and the like. The AJ110637 strain was obtained from soil of seashore at Susuki Kaigan, Makinohara-shi, Shizuoka-ken on March, 2006 by cumulative liquid culture using glycerol as the carbon source. The full length 16S rDNA sequence was then determined, and a homology of 99.9% to that of the *Enterobacter aerogenes* NCTC 10006 strain was obtained. Moreover, also in a physiological test using an API kit, the strain showed results similar to the prototype species of *Enterobacter aerogenes,* and therefore it was identified as *Enterobacter aerogenes.* This strain was deposited at International Patent Organism Depository, Agency of Industrial Science and Technology (Central 6, 1-1, Higashi 1-Chome, Tsukuba-shi, Ibaraki-ken, 305-8566, Japan) on August 22, 2007, and assigned an accession number of FERM P-21348. Then, the deposition was converted to an international deposit based on the Budapest Treaty on March 13, 2008, and assigned an accession number of FERM ABP-10955.

Typical strains of the *Pantoea* bacteria include *Pantoea ananatis, Pantoea stewartii, Pantoea agglomerans,* and *Pantoea citrea.* Specific examples include the following strains:
*Pantoea ananatis* AJ13355 (FERM BP-6614, European Patent Laid-open No. 0952221)
*Pantoea ananatis* AJ13356 (FERM BP-6615, European Patent Laid-open No. 0952221)

Although these strains are described as *Enterobacter agglomerans* in European Patent Laid-open No. 0952221, they are currently classified as *Pantoea ananatis* on the basis of nucleotide sequence analysis of the 16S rRNA etc., as described above.

Examples of the *Erwinia* bacteria include *Erwinia amylovora* and *Erwinia carotovora,* examples of the *Klebsiella* bacteria include *Klebsiella planticola,* and examples of the *Raoultella* bacteria include *Raoultella planticola.* Specific examples include the following strains:
*Erwinia amylovora* ATCC 15580 strain
*Erwinia carotovora* ATCC 15713 strain
*Klebsiella planticola* AJ13399 strain (FERM BP-6600, European Patent Laid-open No. 955368)
*Klebsiella planticola* AJ13410 strain (FERM BP-6617, European Patent Laid-open No. 955368).
*Raoultella planticola* ATCC 33531 strain

Although the AJ13399 strain and the AJ13410 strain were classified as *Klebsiella planticola* at the time of the deposition, *Klebsiella planticola* is currently classified into *Raoultella planticola* (Drancourt, M., 2001, Int. J. Syst. Evol. Microbiol, 51:925-32).

Examples of the rumen bacteria include *Mannheimia* bacteria, *Actinobacillus* bacteria, *Anaerobiospirillum* bacteria, *Pyrobacterium* bacteria, and *Selenomonas* bacteria. Bacteria including *Mannheimia succiniciproducens, Actinobacillus succinogenes, Selenomonas ruminantium, Veillonella parvula, Wolnella succinogenes, Anaerobiospirillum succiniciproducens,* and the like can be used, and it is particularly desirable to use *Mannheimia succiniciproducens.* Specific strains include *Mannheimia* sp. 55E strain (KCTC0769BP strain, U.S. Patent Published Application No. 20030113885, International Patent Publication WO2005/052135).

### <1-1> Impartation of ability to produce acidic substance having a carboxyl group

Hereinafter, methods for imparting an ability to produce an acidic substance having a carboxyl group to bacteria or methods for enhancing an ability to produce an acidic substance having a carboxyl group of bacteria are described.

To impart an ability to produce an acidic substance having a carboxyl group, methods conventionally employed in the breeding of bacteria for producing substances by fermentation (see "Amino Acid Fermentation", Japan Scientific Societies Press, 1st Edition, published May 30, 1986, pp. 77-100) can be applied. Such methods include acquisition of an auxotrophic mutant, an analogue-resistant strain, or a metabolic regulation mutant, or construction of a recombinant strain having enhanced expression of an enzyme involved in biosynthesis of acidic substance having a carboxyl group. In the breeding of bacteria which produce an acidic substance having a carboxyl group, one or two or more properties, such as auxotrophic mutation, analogue resistance, or metabolic regulation mutation, may be imparted. The expression of one or two or more enzymes involved in biosynthesis of acidic substance having a carboxyl group can be enhanced. Furthermore, impartation of properties such as auxotrophic mutation, analogue resistance, or metabolic regulation mutation may be combined with enhancement of the biosynthetic enzymes.

An auxotrophic mutant strain for an acidic substance having a carboxyl group, a strain resistant to an analogue of an acidic substance having a carboxyl group, or a metabolic regulation mutant strain can be obtained by subjecting a parent or wild-type strain to a conventional mutagenesis, such as exposure to X-rays or UV irradiation, or treatment with a mutagen such as N-methyl-N'-nitro-N-nitrosoguanidine, and then selecting those which exhibit an autotrophy, analogue resistance, or metabolic regulation mutation and which also have an ability to produce an acidic substance having a carboxyl group.

Methods for imparting an amino acid- or organic acid-producing ability to microorganisms, and amino acid- or organic acid-producing bacteria will be specifically exemplified below.

### <L-Glutamic acid-producing bacteria>

Examples of the method of modifying a microorganism for imparting L-glutamic acid-producing ability to the microorganism include, for example, modifying a microorganism so that expression of a gene coding for an enzyme involved in the L-glutamic acid biosynthesis is enhanced. Examples of the enzyme involved in L-glutamic acid biosynthesis include glutamate dehydrogenase (hereinafter also referred to as "GDH")(*gdh*), glutamine synthetase (*glnA*), glutamate synthetase (*gltAB*), isocitrate dehydrogenase (*icdA*), aconitate hydratase (*acnA, acnB*), citrate synthase (hereinafter also referred to as "CS")(*gltA*), methylcitrate synthase (hereinafter also referred to as "PRPC" (*prpC*), phosphoenolpyruvate carboxylase (hereinafter also referred to as "PEPC") (*ppc*), pyruvate carboxylase (*pyc*), pyruvate dehydrogenase (*aceEF, lpdA*), pyruvate kinase (*pykA, pykf*), phosphoenolpyruvate synthase (*ppsA*), enolase (*eno*), phosphoglyceromutase (*pgmA, pgmI*), phosphoglycerate kinase (*pgk*), glyceraldehyde-3-phophate dehydrogenase (*gapA*), triose phosphate isomerase (*tpiA*), fructose bisphosphate aldolase (*fbp*), phosphofructokinase (*pfkA, pfkB*), and glucose phosphate isomerase (*pgi*), and the like. The abbreviations in parentheses after the enzyme names are the gene names corresponding to the enzymes. Among these enzymes, one or more of CS or PRPC, PEPC and GDH are preferred, and three enzymes are more preferred.

Methods for modifying a microorganism to increase expression of a target gene will be explained below.

The first method is to increase the copy number of a target gene by cloning the target gene on an appropriate plasmid and transforming a host bacterium with the obtained plasmid. For example, when the target gene is the gene coding for CS (*gltA* gene), the gene coding for PEPC (*ppc* gene) or the gene coding for GDH (*gdhA* gene), nucleotide sequences of these genes from *Escherichia* bacteria and *Corynebacterium* bacteria have already been elucidated (Ner, S. et al., 1983, Biochemistry, 22:5243-5249; Fujita, N. et al., 1984, J. Biochem., 95:909-916; Valle, F. et al., 1984, Gene, 27:193-199; Microbiology, 140:1817-1828, 1994; Eikmanns, B.J. et al., 1989, Mol. Gen. Genet., 218:330-339; Bormann, E.R. et al., 1992, Molecular Microbiology, 6:317-326), and therefore they can be obtained by synthesizing primers based on their respective nucleotide sequences, and performing PCR using chromosomal DNA as the template.

Examples of the plasmid used for transformation include a plasmid which autonomously replicates in the host bacterium belonging to the family *Enterobacteriaceae,* such as pUC19, pUC18, pBR322, RSF1010, pHSG299, pHSG298, pHSG399, pHSG398, pSTV28, pSTV29 (pHSG and pSTV are available from Takara Bio), pMW119, pMW118, pMW219, pMW218 (pMW vectors are available from Nippon Gene), and the like. Moreover, a phage DNA may also be used as the vector instead of a plasmid. Examples of plasmid for simultaneously enhancing activities of CS or PRPC, PEPC and GDH as described above include RSFCPG which has been incorporated with the *gltA* gene, *ppc* gene and *gdhA* gene (refer to European Patent Laid-open No. 0952221), and RSFPPG corresponding to RSFCPG in which the *gltA* gene is replaced with the *prpC* gene.

Examples of transformation methods include treating recipient cells with calcium chloride so to increase permeability for DNA, which has been reported for *Escherichia coli* K-12 (Mandel, M. and Higa, A., 1970, J. Mol. Biol., 53:159-162), and preparing competent cells from cells which are in the growth phase, followed by transformation with DNA, which has been reported for *Bacillus subtilis* (Duncan, C.H., et al., 1977, Gene, 1:153-167). Alternatively, a method of making DNA-recipient cells into protoplasts or spheroplasts, which can easily take up recombinant DNA, followed by introducing the recombinant DNA into the cells, which is known to be applicable to *Bacillus subtilis,* actinomycetes and yeasts (Chang, S. and Choen, S.N., 1979, Molec. Gen. Genet., 168:111-115; Bibb, M.J. et al., 1978, Nature, 274:398-400; Hinnen, A., et al., 1978, Proc. Natl. Sci., USA, 75:1929-1933) can also be employed. In addition, microorganisms can also be transformed by the electric pulse method (Japanese Patent Laid-open No. 2-207791).

The copy number of a target gene can also be increased by introducing multiple copies of the gene into the chromosomal DNA of the microorganism. Multiple copies of a gene can be introduced into the chromosomal DNA by homologous recombination (Experiments in Molecular Genetics, 1972, Cold Spring Harbor Laboratory) using multiple copies of a sequence as targets in the chromosomal DNA. As the sequence present in multiple copies on the chromosomal DNA, repetitive DNAs, and inverted repeats present at the end of a transposable element can be used. Also, as disclosed in Japanese Patent Laid-open No. 2-109985, it is possible to incorporate a target gene into a transposon, and allow it to be transferred to introduce multiple copies of the gene into the chromosomal DNA. The target gene can also be introduced into the bacterial chromosome by using Mu phage (Japanese Patent Laid-open No. 2-109985).

The second method is to increase expression of the target gene by replacing an expression regulatory sequence of the target gene, such as promoter, on the chromosomal DNA or plasmid with a stronger promoter. For example, the lac promoter, trp promoter, trc promoter, etc. are known as strong promoters. Moreover, it is also possible to introduce nucleotide substitution for several nucleotides into a promoter region of a gene, so that the promoter is modified to be stronger, or modify the SD sequence as disclosed in International Patent Publication WO00/18935. Examples of methods for evaluating strength of promoters and strong promoters are described in an article of Goldstein et al. (Goldstein, M.A., and Doi, R.H., 1995, Biotechnol. Annu. Rev., 1:105-128), etc.

Substitution of an expression regulatory sequence can be performed, for example, in the same manner as in gene substitution using a temperature-sensitive plasmid. Examples of vectors having a temperature-sensitive replication origin and usable for *Escherichia coli* and *Pantoea ananatis* include, for example, the pMAN997 plasmid described in International Publication WO99/03988, and the like. Furthermore, substitution of an expression regulatory sequence can also be performed by the method called "Red-driven integration" using Red recombinase of λ phage (Datsenko, K.A. and Wanner, B.L., 2000, Proc. Natl. Acad. Sci. USA. 97:6640-6645), the method combining the Red-driven integration method and the λ phage excisive system (Cho, E.H., et al., 2002, J. Bacteriol., 184:5200-5203) (WO2005/010175), and the like. Modification of an expression regulatory sequence can be combined with increasing gene copy number.

As shown in Reference Example 1, a strain resistant to a λ Red gene product, for example, the *Pantoea ananatis* SC17(0) strain, can be preferably used for the Red driven integration. The SC17(0) strain was deposited at the Russian National Collection of Industrial Microorganisms (VKPM), GNII Genetika (Russia, 117545 Moscow 1, Dorozhny proezd. 1) on September 21, 2005 under an accession number of VKPM B-9246.

Examples of microorganisms modified by the method described above so that expression of citrate synthase gene, methylcitrate synthase gene, phosphoenolpyruvate carboxylase gene and/or glutamate dehydrogenase gene are enhanced include the microorganisms disclosed in Japanese Patent Laid-open Nos. 2001-333769, 2000-106869, 2000-189169, 2000-333769, 2006-129840, WO2006/051660, and the like.

Furthermore, L-glutamic acid-producing ability can also be imparted by enhancing the 6-phosphogluconate dehydratase activity, 2-keto-3-deoxy-6-phosphogluconate aldolase activity, or both these activities. Examples of the microorganism of which 6-phosphogluconate dehydratase activity and 2-keto-3-deoxy-6-phosphogluconate aldolase activity are increased include the microorganism disclosed in Japanese Patent Laid-open No. 2003-274988.

The modification for imparting L-glutamic acid-producing ability may also be attained by decreasing or eliminating activity of an enzyme that catalyzes a reaction branching off from the L-glutamic acid biosynthesis pathway and producing a compound other than L-glutamic acid. Examples of such an enzyme catalyzing a reaction branching off from the L-glutamic acid biosynthesis pathway and producing a compound other than L-glutamic acid include 2-oxoglutarate dehydrogenase (*sucA*), isocitrate lyase (*aceA*), phosphate acetyltransferase (*pta*), acetate kinase (*ack*), acetohydroxy acid synthase (*ilvG*), acetolactate synthase (*ilvI*), formate acetyltransferase (*pfl*), lactate dehydrogenase (*ldh*), glutamate decarboxylase (*gadAB*), 1-pyrroline-5-carboxylate dehydrogenase (*putA*), and the like. Gene names are indicated in the parentheses after the enzyme names. It is particularly preferable to decrease or eliminate activity of 2-oxoglutarate dehydrogenase among these enzymes.

In order to decrease or eliminate the activities of the aforementioned enzymes, methods similar to the method for decreasing or eliminating the activity of lactate dehydrogenase (LDH) described later can be used.

Decrease or deficiency of intracellular activity of the target enzyme and the degree by which the activity is decreased can be confirmed by measuring the enzyme activity of a cell extract or a purified fraction thereof obtained from a candidate strain and comparing it with that of a wild-type strain. For example, the 2-oxoglutarate dehydrogenase activity can be measured by the method of Reed et al. (Reed L.J. and Mukherjee B.B., 1969, Methods in Enzymology, 13, pp.55-61).

Methods of eliminating or decreasing the 2-oxoglutarate dehydrogenase activity in *Escherichia* bacteria are disclosed in Japanese Patent Laid-open Nos. 5-244970, 7-203980 and the like. A method of eliminating or decreasing the 2-oxoglutarate dehydrogenase activity in coryneform bacteria is disclosed in International Patent Publication WO95/34672. Furthermore, such a method for *Enterobacter* bacteria is disclosed in Japanese Patent Laid-open No. 2001-333769.

Specific examples of *Escherichia* bacteria which are deficient in the 2-oxoglutarate dehydrogenase activity or in which the 2-oxoglutarate dehydrogenase activity is decreased include the following strains (U.S. Patent Nos. 5,378,616 and 5,573,945).
*E. coli* W3110sucA::Kmr
*E. coli* AJ12624 (FERM BP-3853)
*E. coli* AJ12628 (FERM BP-3854)
*E. coli* AJ12949 (FERM BP-4881)
*E. coli* W3110sucA::Kmr is obtained by disrupting the 2-oxoglutarate dehydrogenase gene (sucA gene) of *Escherichia coli* W3110. This strain is completely deficient in the 2-oxoglutarate dehydrogenase.

Other specific examples of bacterium wherein the activity of 2-oxoglutarate dehydrogenase is deleted or decreased include the following strains:
*Pantoea* ananatis AJ13601 (FERM BP-7207, European Patent Laid-open No. 1078989)
*Pantoea ananatis* AJ13356 (FERM BP-6615, United States Patent No. 6,331,419)
*Pantoea ananatis* SC17sucA (FERM BP-8646, WO2005/085419)
*Klebsiella planticola* AJ13410 strain (FERM BP-6617, United States Patent No. 6,197,559)
*Brevibacterium lactofermentum* ΔS strain (refer to International Patent Publication WO95/34672)

The SC17sucA strain is obtained by obtaining a low phlegm production mutant strain (SC17) from the AJ13355 strain, which was isolated from nature as a strain that could proliferate in a medium containing L-glutamic acid and a carbon source at low pH, and disrupting the 2-oxoglutarate dehydrogenase gene (*sucA*) of the mutant strain. The AJ13601 strain is a strain obtained by introducing the plasmid RSFCPG containing the *gltA, ppc* and gdhA genes derived from *Escherichia coli* and the plasmid pSTVCB containing the *gltA* gene derived from *Brevibacterium lactofermentum* into the SC17sucA strain to obtain the SC17sucA/RSFCPG+pSTVCB strain, and selecting a high concentration L-glutamic acid resistant strain at a low pH, and a strain showing a high proliferation degree and a high L-glutamic acid-producing ability from the SC17sucA/RSFCPG+pSTVCB strain (European Patent Laid-open No. 0952221). The AJ13356 strain was obtained by deleting the αKGDH-E1 subunit gene (*sucA*) of the AJ13355 strain.

The SC17sucA strain was assigned a private number of AJ417, deposited in the International Patent Organism Depositary, National Institute of Advanced Industrial Science and Technology (Central 6, 1-1, Higashi 1-Chome, Tsukuba-shi, Ibaraki-ken, Japan, postal code: 305-8566) on February 26, 2004, and assigned an accession number of FERM BP-08646.

The AJ13410 strain was deposited in the International Patent Organism Depositary, National Institute of Advanced Industrial Science and Technology (Central 6, 1-1, Higashi 1-Chome, Tsukuba-shi, Ibaraki-ken, Japan, postal code: 305-8566) on February 19, 1998, and assigned an accession number of FERM P-16647. The deposition was then converted to an international deposition under the provisions of Budapest Treaty on January 11, 1999 and assigned an accession number of FERM BP-6617.

The aforementioned *Pantoea ananatis* AJ13355, AJ13356, and AJ13601 strains and *Klebsiella planticola* AJ13399 strain have an ability to accumulate L-glutamic acid in a concentration which exceeds the saturation concentration of L-glutamic acid in a liquid medium when it is cultured under an acid condition.

Furthermore, in order to improve L-glutamic acid-producing ability of *Enterobacteriaceae* bacteria, the method of deleting the *arcA* gene (U.S. Patent No. 7,090,998), and the method of amplifying the *yhfK* gene, which is a glutamic acid excretion gene (WO2005/085419) can also be used.

Other than the above, examples of coryneform bacteria having L-glutamic acid-producing ability include the following strains.

*Brevibacterium flavum* AJ11573 (FERM P-5492, refer to Japanese Patent Laid-open No. 56-151495)

*Brevibacterium flavum* AJ12210 (FERM P-8123, refer to Japanese Patent Laid-open No. 61-202694)

*Brevibacterium flavum* AJ12212 (FERM P-8123, refer to Japanese Patent Laid-open No. 61-202694)

*Brevibacterium flavum* AJ12418 (FERM-BP2205, refer to Japanese Patent Laid-open No. 2-186994)

*Brevibacterium flavum* DH18 (FERM P-11116, refer to Japanese Patent Laid-open No. 3-232497)

*Corynebacterium melassecola* DH344 (FERM P-11117, refer to Japanese Patent Laid-open No. 3-232497)

*Corynebacterium glutamicum* AJ11574 (FERM P-5493, refer to Japanese Patent Laid-open No. No. 56-151495)

Microorganisms having L-glutamic acid-producing ability include the *Brevibacterium lactofermentum* AJ13029 strain (FERM BP-5189, refer to WO96/06180), which was made to be able to produce L-glutamic acid in a medium containing excessive amount of biotin in the absence of biotin action inhibitor by introducing a mutation for imparting temperature sensitivity to a biotin action inhibitor such as surfactants. Examples further include microorganisms belonging to the genus *Alicyclobacillus* and resistant to L-glutamic acid antimetabolite (Japanese Patent Laid-open No. 11-262398).

Furthermore, the microorganism having L-glutamic acid producing ability may be a microorganism having a property of decreased L-glutamic acid degradation property, or a property that expression of maleate synthase (*aceB*)·isocitrate lyase (*aceA*)·isocitrate dehydrogenase kinase/phosphatase (*aceK*) operon (henceforth abbreviated as "ace operon") has become constitutive. Examples of microorganisms having such a property include, for example, the followings.
*Escherichia coli* AJ12628 (FERM BP-3854)
*Escherichia coli* AJ12624 (FERM BP-3853)

The former is a mutant strain having both of a property that 2-oxoglutarate dehydrogenase activity is decreased and a property that expression of the *ace* operon has become constitutive, and the latter is a mutant strain in which 2-oxoglutarate dehydrogenase activity is decreased and L-glutamic acid decomposition activity is decreased (refer to French Patent No. 2680178).

When a *Pantoea* bacterium is used, it preferably has such a mutation that it extracellularly produces less phlegm as compared to a wild-type strain when it is cultured in a medium containing a saccharide. In order to introduce a mutation providing less extracellular production of phlegm as compared to a wild-type strain, any of the method of screening for a strain not producing viscous materials on the solid medium described in Japanese Patent Laid-open No. 2001-333769, and a method of disrupting the *ams* operon involved in polysaccharide synthesis may be used. The nucleotide sequence of the *ams* operon involved in polysaccharide synthesis is shown in SEQ ID NO: 66, and the amino acid sequences of AmsH, I, A, C and B encoded by this operon are shown in SEQ ID NOS: 67, 68, 69, 70 and 71, respectively.

### <Succinic acid-producing bacteria>

As succinic acid-producing bacteria, strains deficient in abilities to form acetic acid, lactic acid, ethanol and formic acid can be used, and specific examples include the *Escherichia coli* SS373 strain (International Patent Publication WO99/06532).

Strains deficient in abilities to form acetic acid, lactic acid, ethanol and formic acid can be obtained by obtaining a strain which cannot assimilate acetic acid and lactic acid in a minimal medium, or by decreasing activities of the lactic acid biosynthesis genes and acetic acid biosynthesis enzymes described below (International Patent Publication WO2005/052135).

Moreover, such strains as described above can also be obtained by imparting monofluoroacetic acid resistance (U.S. Patent No. 5,521,075).

Other examples of the method for obtaining a strain in which succinic acid-producing ability is improved include culturing a strain which is deficient in both formate lyase and lactate dehydrogenase and cannot assimilate pyruvic acid in a glucose-enriched medium under anaerobic conditions, and isolating a mutant strain having an ability to assimilate pyruvic acid (International Patent Publication WO97/16528).

An ability to produce succinic acid can also be imparted by amplification of a gene of enzyme which is involved in the succinic acid biosynthesis system described below, or deletion of a gene of enzyme which catalyzes a reaction branching off from the succinic acid biosynthesis system to produce another compound.

An ability to produce succinic acid can also be imparted by modifying a microorganism so that the enzymatic activity of lactate dehydrogenase (LDH, hereafter abbreviations of enzyme names are indicated in parentheses), which is a lactic acid biosynthesis system enzyme, decreases (International Patent Publications WO2005/052135, WO2005/116227, U.S. Patent No. 5,770,435, U.S. Patent Published Application No. 20070054387, International Patent Publication WO99/53035, Alam, K.Y. and Clark, D.P., 1989, J. Bacteriol., 171:6213-6217). Some microorganisms may have L-lactate dehydrogenase and D-lactate dehydrogenase, and such microorganism may be modified so that activity of either one of the enzymes, preferably activities of both the enzymes are decreased.

An ability to produce succinic acid can also be imparted by modifying a microorganism so that the enzymatic activity of the formic acid biosynthesis system enzyme, pyruvate-formate lyase (PFL), is decreased (U.S. Patent Published Application No. 20070054387, International Patent Publications WO2005/116227, WO2005/52135, Donnelly, M.I. et al., 1998, Appl. Biochem. Biotechnol., 70-72:187-198).

An ability to produce succinic acid can also be imparted by modifying a microorganism so that the enzymatic activities of phosphate acetyltransferase (PTA), acetate kinase (ACK), pyruvate oxidase (POXB), acetyl-CoA synthetase (ACS) and acetyl-CoA hydrolase (ACH), which are acetic acid biosynthesis system enzymes, are decreased (U.S. Patent Published Application No. 20070054387, International Patent Publications WO2005/052135, WO99/53035, WO2006/031424, WO2005/113745, and WO2005/113744).

An ability to produce succinic acid can also be enhanced by modifying a microorganism so that the enzymatic activity of alcohol dehydrogenase (ADH), which is an ethanol biosynthesis system enzyme, is decreased (refer to International Patent Publication WO2006/031424).

An ability to produce succinic acid can also be enhanced by decreasing activities of pyruvate kinase, glucose PTS (*ptsG*), ArcA protein, IclR protein (*iclR*), glutamate dehydrogenase (*gdh*) and/or glutamine synthetase (*glnA*), and glutamate synthase (*gltBD*) (International Patent Publication WO2006/107127, WO2007007933, Japanese Patent Laid-open No. 2005-168401). Indicated here in the parentheses following the enzyme names are gene names.

An ability to produce succinic acid can also be imparted by enhancing a biosynthesis system enzyme involved in the succinic acid production.

An ability to produce succinic acid can also be enhanced by enhancing enzymatic activities of pyruvate carboxylase, malic enzyme, phosphoenolpyruvate carboxylase, fumarase, fumarate reductase, malate dehydrogenase and phosphoenolpyruvate carboxykinase (Japanese Patent Laid-open No. 11-196888, International Patent Publication WO99/53035, Hong, S.H., and S.Y. Lee, 2001, Biotechnol. Bioeng., 74:89-95, Millard, C.S. et al., 1996, Appl. Environ. Microbiol., 62:1808-1810, International Patent Publication WO2005/021770, Japanese Patent Laid-open No. 2006-320208, Kim, P. et al., 2004, Appl. Environ. Microbiol., 70:1238-1241). Enhancement of enzymatic activities of these target enzymes can be performed by referring to the methods for enhancing expression of a target gene described, and the methods for enhancing expression of *ybjL* gene described later.

Specific examples of succinic acid-producing bacteria belonging to the family *Enterobacteriaceae* include the following strains.

*Escherichia coli* SS373 strain (International Patent Publication WO99/06532)

*Escherichia coli* AFP111 strain (International Patent Publication WO97/16528)

*Escherichia coli* NZN111 strain (U.S. Patent No. 6,159,738)

*Escherichia coli* AFP184 strain (International Patent Publication WO2005/116227)

*Escherichia coli* SBS100MG strain, SBS110MG strain, SBS440MG strain, SBS550MG strain, and SBS660MG strain (International Patent Publication WO2006/031424)

Examples of succinic acid-producing bacteria belonging to coryneform bacteria include the following strains.

*Brevibacterium flavum* AB-41 strain (Japanese Patent Laid-open No. 11-113588)

*Brevibacterium flavum* AB-41 strain (PC-amplified strain, Japanese Patent Laid-open No. 11-196888)

*Corynebacterium glutamicum* AJ110655 strain (FERM BP-10951)

*Brevibacterium flavum* MJ233Δldh strain (International Patent Publication WO2005/021770)

*Brevibacterium lactofermentum* (*Corynebacterium glutamicum*) 2256Δ(ldh, ach, pta, ack) (International Patent Publication WO2005/113744)

*Brevibacterium lactofermentum* 2256Δ(ldh, pta, ack, poxB) (International Patent Publication WO2005/113745)

*Corynebacterium glutamicum* Rldh-/pCRB-1 PC strain (International Patent Publication WO2005/010182)

Examples of succinic acid-producing bacteria belonging to rumen bacteria include the following strains.

*Mannheimia succiniciproducens* LPK, LPK7 and LPK4 (International Patent Publication WO2005/052135)

*Actinobacillus succinogenes* 130Z (U.S. Patent No. 5,504,004)

*Anaerobiospirillum succiniciproducens* FZ10 (U.S. Patent No. 5,521,075)

*Anaerobiospirillum succiniciproducens* FZ53 (U.S. Patent No. 5,573,931)

### <1-2> Enhancement of expression of ybjL gene

The microorganism used in the present invention can be obtained by modifying a microorganism which has an ability to produce an acidic substance having a carboxyl group such as those described above so that expression of the *ybjL* gene is enhanced. However, such modification that expression of the *ybjL* gene is enhanced is performed first, and then an ability to produce an acidic substance having a carboxyl group may be imparted. Furthermore, the microorganism used in the present invention may be a microorganism which has been imparted with ability to produce an acidic substance having a carboxyl group or microorganism in which ability to produce an acidic substance having a carboxyl group is enhanced by amplification of the *ybjL* gene.

The "*ybjL* gene" used in the present invention refers to the *ybjL* gene of *Escherichia coli* or a homologue thereof, the *ybjL* gene of Pantoea *ananatis* or a homologue thereof, or the *ybjL* gene of *Enterobacter aerogenes* or a homologue thereof. Examples of the *ybjL* gene of *Escherichia coli* include a gene coding for a protein having the amino acid sequence of SEQ ID NO: 4, preferably a gene having the nucleotide sequence of the nucleotide numbers 101 to 1783 in SEQ ID NO: 3. Furthermore, examples of the *ybjL* gene derived from *Pantoea ananatis* include a gene coding for a protein having the amino acid sequence of SEQ ID NO: 2, preferably a gene having the nucleotide sequence of the nucleotide numbers 298 to 1986 in SEQ ID NO: 1. Furthermore, examples of the *ybjL* gene derived from the *Enterobacter aerogenes* AJ110673 strain include a gene coding for a protein having the amino acid sequence of SEQ ID NO: 87, preferably a gene having the nucleotide sequence of the nucleotide numbers 19 to 1704 in SEQ ID NO: 86. The method of the present invention uses the *ybjL* gene coding for a protein comprising the amino acid sequence of SEQ ID NO: 2, 4 or 87. Furthermore, examples of the *ybjL* gene include a gene coding for a protein having the amino acid sequence of SEQ ID NO: 25 (SEQ ID NO: 24) as the *ybjL* gene derived from *Salmonella typhimurium,* a gene coding for a protein having the amino acid sequence of SEQ ID NO: 27 (SEQ ID NO: 26) as the *ybjL* gene derived from *Yersinia pestis,* a gene coding for a protein having the amino acid sequence of SEQ ID NO: 29 (SEQ ID NO: 28) as the *ybjL* gene derived from *Erwinia* carotovora, a gene coding for a protein having the amino acid sequence of SEQ ID NO: 31 (SEQ ID NO: 30) as the *ybjL* gene derived from *Vibrio cholerae,* a gene coding for a protein having the amino acid sequence of SEQ ID NO: 33 (SEQ ID NO: 32) as the *ybjL* gene derived from *Aeromonas hydrophila,* a gene coding for a protein having the amino acid sequence of SEQ ID NO: 35 (SEQ ID NO: 34) as the *ybjL* gene derived from *Photobacterium profundum,* and the like. Furthermore, the *ybjL* gene may be one cloned from a coryneform bacterium such as *Corynebacterium glutamicum* and *Brevibacterium lactofermentum, Pseudomonas* bacterium such as *Pseudomonas aeruginosa, Mycobacterium* bacterium such as *Mycobacterium tuberculosis* or the like, on the basis of homology to the genes exemplified above. The amino acid sequences of the proteins encoded by the *ybjL* genes of *Salmonella typhimurium, Yersinia pestis, Erwinia carotovora, Vibrio cholerae, Aeromonas hydrophila* and *Photobacterium profundum* described above are 96%, 90%, 88%, 64%, 60% and 68% homologous to the amino acid sequence of SEQ ID NO: 4, respectively, and 86%, 90%, 84%, 63%, 60% and 67% homologous to the amino acid sequence of SEQ ID NO: 2, respectively. The amino acid sequences of SEQ ID NOS: 2 and 4 show a homology of 86%. The consensus sequence of SEQ ID NOS: 2 and 4 is shown in SEQ ID NO: 5. The amino acid sequences of SEQ ID NOS: 4 and 87 show a homology of 92%, and the amino acid sequences of SEQ ID NOS: 2 and 87 show a homology of 83%. The sequence of the ybjL gene of the *Enterobacter aerogenes* AJ110637 strain (SEQ ID NO: 86) and the amino acid sequence encoded thereby (SEQ ID NO: 87) are newly found by the inventors of the present invention. The consensus sequence of the amino acid sequences of SEQ ID NOS: 2, 4 and 87 is shown in SEQ ID NO: 88.

The *ybjL* gene homologue refers to a gene derived from a microorganism other than *Escherichia coli, Pantoea ananatis* and *Enterobacter aerogenes,* exhibiting high structural similarity to the *ybjL* gene of *Escherichia coli, Pantoea ananatis* or *Enterobacter aerogenes* and coding for a protein which improves an ability to produce an acidic substance having a carboxyl group of a microorganism when expression thereof is enhanced in the microorganism. Examples of *ybjL* gene homologues include genes coding for a protein showing a homology of 70% or more, preferably 80% or more, more preferably 90% or more, still more preferably 95% or more, particularly preferably 97% or more, to the entire amino acid sequence of SEQ ID NO: 2, 4 or 87 or the amino acid sequence of SEQ ID NO: 2, 4 or 87, and improving an ability to produce an acidic substance having a carboxyl group of a microorganism when expression thereof is enhanced in the microorganism. The *ybjL* gene homologues coding for a protein showing a homology of 70% or more, preferably 80% or more, more preferably 90% or more, still more preferably 95% or more, particularly preferably 97% or more, to any of the aforementioned amino acid sequences may have the consensus sequence of the SEQ ID NOS: 5 or 88, preferably SEQ ID NO: 5. Homology of amino acid sequences and nucleotide sequences can be determined by using, for example, the algorithm BLAST of Karlin and Altschul (Pro. Natl. Acad. Sci. USA, 90, 5873 (1993)) or FASTA (Methods Enzymol., 183, 63 (1990)). Programs called BLASTN and BLASTX have been developed on the basis of this algorithm BLAST (refer to www.ncbi.nlm.nih.gov). In this specification, the term "homology" may be used to refer to "identity".

The *ybjL* gene may be a gene having a conservative mutation such as artificially modified genes, so long as enhancement of expression thereof provides improvement in ability to produce a target substance of a microorganism. That is, the *ybjL* gene may be a gene coding for an amino acid sequence of an existing protein, for example, a protein having an amino acid sequence of SEQ ID NO: 2, 4, 25, 27, 29, 31, 33, 35 or 87 including a conservative mutation, specifically, substitution, deletion, insertion or addition of one or several amino acid residues at one or several positions. Although number meant by the term "several" used herein may differ depending on positions in the three-dimensional structure of the protein or types of amino acid residues, it is 1 to 10, preferably 1 to 5. Furthermore, the *ybjL* gene may be a gene coding for a protein showing a homology of 70% or more, preferably 80% or more, more preferably 90% or more, still more preferably 95% or more, particularly preferably 97% or more, to the entire amino acid sequence of SEQ ID NO: 2, 4, 25, 27, 29, 31, 33, 35 or 87, which improves an ability to produce a target substance of a microorganism when expression thereof is enhanced in the microorganism.

The aforementioned substitution is preferably a conservative substitution that is a neutral substitution not providing functional change. The conservative mutation is a mutation wherein substitution takes place mutually among Phe, Trp and Tyr, if the substitution site is an aromatic amino acid; among Leu, Ile and Val, if the substitution site is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having hydroxyl group. Specific examples of substitution considered conservative substitution include: substitution of Ser or Thr for Ala; substitution of Gln, His or Lys for Arg; substitution of Glu, Gln, Lys, His or Asp for Asn; substitution of Asn, Glu or Gln for Asp; substitution of Ser or Ala for Cys; substitution of Asn, Glu, Lys, His, Asp or Arg for Gln; substitution of Gly, Asn, Gln, Lys or Asp for Glu; substitution of Pro for Gly; substitution of Asn, Lys, Gln, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution of Trp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val.

Such a gene can be obtained by modifying the nucleotide sequence of the nucleotide numbers 298 to 1986 in SEQ ID NO: 1, the nucleotide sequence of the nucleotide numbers 101 to 1783 in SEQ ID NO: 3, the nucleotide sequence of the nucleotide numbers 19 to 1704 in SEQ ID NO: 86, or the nucleotide sequence of SEQ ID NO: 24, 26, 28, 30, 32 or 34 by, for example, site-specific mutagenesis, so that substitution, deletion, insertion or addition of an amino acid residue or residues is included at a specific site of the encoded protein. Furthermore, such a gene can also be obtained by a conventionally known mutation treatment. Examples of the mutation treatment include treating a gene having any of the nucleotide sequences described above with hydroxylamine or the like *in vitro,* and treating a microorganism, for example, an Escherichia bacterium, containing the gene with ultraviolet ray irradiation or a mutagen used in a usual mutation treatment such as N-methyl-N'-nitro-N-nitrosoguanidine (NTG) or EMS (ethyl methanesulfonate). The mutation for the substitutions, deletions, insertions, additions, inversions or the like of amino acid residues described above also includes a naturally occurring mutation based on individual difference, difference in species of microorganisms that contain the ybjL gene (mutant or variant) and the like.

The *ybjL* gene may also be a DNA hybridizable with a complementary strand of DNA having the nucleotide sequence of the nucleotide sequence of the nucleotide numbers 298 to 1986 in SEQ ID NO: 1, DNA having the nucleotide sequence of the nucleotide numbers 101 to 1783 in SEQ ID NO: 3, DNA having the nucleotide sequence of the nucleotide numbers 19 to 1704 in SEQ ID NO: 86, or DNA having the nucleotide sequence of SEQ ID NO: 1, 3, 24, 26, 28, 30, 32, 34 or 86, or a probe that can be prepared from DNAs having these sequences under stringent conditions and coding for a protein which improves an ability to produce a substance of a microorganism when expression thereof is enhanced in the microorganism.

The "stringent conditions" referred to herein is a condition under which a so-called specific hybrid is formed, and non-specific hybrid is not formed. It is difficult to clearly express this condition by using any numerical value. However, the stringent conditions includes, for example, conditions where DNAs showing high homology to each other, for example, DNAs showing a homology of, for example, not less than 80%, preferably not less than 90%, more preferably not less than 95%, particularly preferably not less than 97%, hybridize with each other, and DNAs having homology lower than the above level do not hybridize with each other, and conditions of washing in ordinary Southern hybridization, i.e., conditions of washing once, preferably twice or three times, at salt concentrations and temperature of 1 x SSC, 0.1% SDS at 60°C, preferably 0.1 x SSC, 0.1% SDS at 60°C, more preferably 0.1 x SSC, 0.1% SDS at 65°C, still more preferably 0.1 x SSC, 0.1% SDS at 68°C.

The probe may be a probe having a partial sequence of the *ybjL* gene. Such a probe can be produced by PCR using oligonucleotides prepared on the basis of the nucleotide sequence of the gene and a DNA fragment including the gene as the template and performed in a manner well known to those skilled in the art. When a DNA fragment having a length of about 300 bp is used as the probe, the washing condition after hybridization under the aforementioned condition can be exemplified by 2 x SSC, 0.1% SDS at 50°C.

The aforementioned descriptions concerning gene homologue and conservative mutation are similarly applied to the other enzyme genes described in this specification.

Enhancement of the expression of the *ybjL* gene can be attained by increasing copy number of the *ybjL* gene, or modifying an expression control sequence of the *ybjL* gene, using transformation or homologous recombination performed in the same manners as the methods for enhancing expression of a target gene described above for L-glutamic acid-producing bacteria.

In the microorganism used in the present invention, activity for excreting an acidic substance having a carboxyl group is preferably improved by enhancement of the expression of the *ybjL* gene. Whether "the activity for excreting an acidic substance having a carboxyl group is improved" can be confirmed by comparing amount of the acidic substance having a carboxyl group excreted into a medium in which the microorganism used in the present invention is cultured with the amount of the same obtained by culturing a control microorganism into which the *ybjL* gene is not introduced. That is, the "improvement in the activity for excreting the acidic substance having a carboxyl group" is observed as increase of the concentration of the acidic substance having a carboxyl group accumulated in the medium in which the microorganism used in the present invention is cultured as compared to the concentration obtained with a control microorganism. Furthermore, the "improvement in the activity for excreting an acidic substance having a carboxyl group" is also observed as decrease in intracellular concentration of the acidic substance having a carboxyl group in the microorganism used in the present invention. As for the improvement of the "activity for excreting an acidic substance having a carboxyl group", the intracellular concentration of the acidic substance having a carboxyl group preferably decreases by 10% or more, more preferably 20% or more, particularly preferably 30% or more, as compared to the concentration in a strain in which expression of the *ybjL* gene is not enhanced. The absolute "activity for excreting an acidic substance having a carboxyl group" of a microorganism can be detected by measuring a difference of intracellular and extracellular concentrations of the acidic substance having a carboxyl group. Furthermore, the "activity for excreting an acidic substance having a carboxyl group" can also be detected by measuring activity for taking up amino acids into cells using reverted membrane vesicles with a radioisotope (J. Biol. Chem., 2002, vol. 277, No. 51, pp.49841-49849). For example, the activity can be measured by preparing reverted membrane vesicles from cells in which the *ybjL* gene is expressed, adding a substrate serving as a driving force such as ATP, and measuring uptake activity for RI-labeled glutamic acid. Moreover, the activity can also be measured by detecting an exchange reaction rate of labeled acidic substance having a carboxyl group and unlabeled acidic substance having a carboxyl group in live bacteria.

The microorganism used in the present invention may be a microorganism having an ability to accumulate L-glutamic acid in a liquid medium in an amount more than amount providing the saturation concentration of L-glutamic acid when it is cultured under acidic conditions (this ability is also referred to as the "L-glutamic acid accumulation ability under acidic conditions" hereinafter). Such a microorganism may be a microorganism that has come to have the L-glutamic acid accumulation ability under acidic conditions by enhancement of the expression of the *ybjL* gene, or a microorganism that originally has the L-glutamic acid accumulation ability under acidic conditions.

Specific examples of microorganisms having the L-glutamic acid accumulation ability under acidic conditions include the aforementioned *Pantoea ananatis* AJ13355 strain (FERM BP-6614), AJ13356 strain (FERM BP-6615), AJ13601 strain (FERM BP-7207) (for these, refer to Japanese Patent Laid-open No. 2001-333769), and the like. The *Pantoea ananatis* AJ13355 and AJ13356 strains were deposited at the National Institute of Bioscience and Human-Technology, Agency of Industrial Science and Technology, Ministry of International Trade and Industry (currently, National Institute of Bioscience and Human-Technology, National Institute of Advanced Industrial Science and Technology, Address: Central 6, 1-1, Higashi 1-Chome, Tsukuba-shi, Ibaraki-ken, 305-8566, Japan) on February 19, 1998 and given accession numbers of FERM P-16644 and FERM P-16645. The depositions were then converted to international deposits under the provisions of Budapest Treaty on January 11, 1999 and given accession numbers of FERM BP-6614 and FERM BP-6615. The AJ13601 strain was deposited at the National Institute of Bioscience and Human-Technology, Agency of Industrial Science and Technology (currently, International Patent Organism Depository, National Institute of Advanced Industrial Science and Technology) on August 18, 1999 and given an accession number of FERM P-17516. The deposition was converted to an international deposit under the provisions of the Budapest Treaty on July 6, 2000 and given an accession number of FERM BP-7207. These strains were identified as *Enterobacter agglomerans* when they were isolated and deposited as *Enterobacter agglomerans* AJ13355, AJ13356 and AJ13601 strains. However, they were recently reclassified as *Pantoea ananatis* on the basis of nucleotide sequencing of 16S rRNA and the like.

When an organic acid, especially succinic acid, is produced by the method of the present invention, use of a microorganism which is modified so that activities of one or more enzymes among lactate dehydrogenase (LDH), alcohol dehydrogenase (ADH), and pyruvate formate lyase (PFL) are decreased in addition to that expression of the *ybjL* gene is increased is more effective.

The expression "modified so that lactate dehydrogenase activity is decreased" used herein means that the lactate dehydrogenase activity is decreased as compared to that of a lactate dehydrogenase-unmodified strain. The lactate dehydrogenase activity is preferably decreased to 10% per cell or lower as compared to that of a lactate dehydrogenase-unmodified strain. The lactate dehydrogenase activity may be completely deleted. Decrease of the lactate dehydrogenase activity can be confirmed by measuring the lactate dehydrogenase activity by a known method (Kanarek, L. and Hill, R.L., 1964, J. Biol. Chem., 239:4202). Specific examples of the method for producing a mutant strain of *Escherichia coli* in which the lactate dehydrogenase activity is decreased include the method described in Alam, K.Y., and Clark, D.P., 1989, J. Bacteriol., 171:6213-6217, and the like. The microorganism used in the present invention in which lactate dehydrogenase activity is decreased and expression of the *ybjL* gene is enhanced can be obtained by, for example, preparing a microorganism in which LDH gene is disrupted, and transforming this microorganism with a recombinant vector containing the *ybjL* gene, as described in Example 1 described later. However, either of the modification for decreasing the LDH activity and the modification for enhancing expression of the *ybjL* gene may be performed first. In *Escherichia coli,* LDH is encoded by the *ldhA* and *lldD* genes. The DNA sequence of the *ldhA* gene is shown in SEQ ID NO: 36, the amino acid sequence encoded thereby is shown in SEQ ID NO: 37, the DNA sequence of the *lldD* gene is shown in SEQ ID NO: 38, and the amino acid sequence encoded thereby is shown in SEQ ID NO: 39.

In order to decrease or delete activity of LDH, a mutation that decreases or deletes the intracellular activity of LDH can be introduced into the LDH gene on a chromosome by a usual mutagenesis method. For example, it can be attained by deleting the gene coding for LDH on a chromosome, or modifying an expression control sequence such as a promoter and the Shine-Dalgarno (SD) sequence by gene recombination. Furthermore, it can also be attained by introducing a mutation for amino acid substitution (missense mutation), or a stop codon (nonsense mutation), or a frame shift mutation that adds or deletes one or two nucleotides into a region coding for LDH on a chromosome, or by deleting the gene partially or entirely (Qiu Z. and Goodman M.F., 1997, J. Biol. Chem., 272:8611-8617). Furthermore, the LDH activity can also be decreased or deleted by gene disruption, for example, by constructing a gene coding for a mutant LDH of which coding region is deleted, and substituting the constructed gene for the normal LDH gene on a chromosome by homologous recombination or the like, or by introducing a transposon or IS factor into the gene.

In order to introduce a mutation for decreasing or deleting the LDH activity by genetic recombination, for example, the following methods are used. The LDH gene on a chromosome can be replaced with a mutant gene by preparing a mutant LDH gene in which a partial sequence of the LDH gene is modified so that it does not produce an enzyme that can normally function, and transforming a bacterium with a DNA containing the mutant gene to cause homologous recombination between the mutant gene and the gene on a chromosome. Such site-specific mutagenesis based on gene substitution utilizing homologous recombination has been already established, and there are a method called Red driven integration developed by Datsenko and Wanner (Datsenko, K.A, and Wanner, B.L., 2000, Proc. Natl. Acad. Sci. USA, 97:6640-6645), a method of using a linear DNA such as a method utilizing the Red driven integration in combination with an excision system derived from λ phage (Cho, E.H., et al., 2002, J. Bacteriol., 184:5200-5203), a method of using a plasmid containing a temperature sensitive replication origin (U.S. Patent No. 6,303,383, Japanese Patent Laid-open No. 05-007491, WO2005/010175), and the like. Such site-specific mutagenesis based on gene substitution using homologous recombination as described above can also be performed by using a plasmid not having ability to replicate in a host.

The expression "modified so that alcohol dehydrogenase activity is decreased" used herein means that the alcohol dehydrogenase activity is decreased as compared to that of an alcohol dehydrogenase-unmodified strain. The alcohol dehydrogenase activity is preferably decreased to 10% per cell or lower as compared to an alcohol dehydrogenase-unmodified strain. The alcohol dehydrogenase activity may be completely deleted. Decrease of the alcohol dehydrogenase activity can be confirmed by measuring the alcohol dehydrogenase activity by a known method (Lutstorf, U.M. et al., 1970, Eur. J. Biochem., 17:497-508). Specific examples of the method for producing a mutant strain of *Escherichia coli* in which the alcohol dehydrogenase activity is decreased include the method described in Sanchez A.M. et al., 2005, Biotechnol. Prog., 21:358-365, and the like. The microorganism used in the present invention in which alcohol dehydrogenase activity is decreased and expression of the *ybjL* gene is enhanced can be obtained by, for example, preparing a microorganism in which a gene coding for alcohol dehydrogenase (ADH) is disrupted, and transforming this microorganism with a recombinant vector containing the *ybjL* gene. However, either of the modification for decreasing the ADH activity and the modification for enhancing expression of the *ybjL* gene may be performed first. The alcohol dehydrogenase activity can be decreased by a method similar to the method for decreasing the lactate dehydrogenase activity described above. As the ADH gene (*adhE*) of *Enterobacter aerogenes,* the nucleotide sequence (partial sequence) of the ADH gene of the *Enterobacter aerogenes* AJ110637 strain (FERM ABP-10955) is shown in SEQ ID NO: 74. The entire nucleotide sequence of this gene can be determined by, for example, isolating the ADH gene (*adhE*) from the chromosomal DNA of *Enterobacter aerogenes* on the basis of such a partial sequence.

The expression "modified so that pyruvate formate lyase activity is decreased" used herein means that the pyruvate formate lyase activity is decreased as compared to that of a pyruvate formate lyase-unmodified strain. The pyruvate formate lyase activity is preferably decreased to 10% per cell or lower as compared to a pyruvate formate lyase-unmodified strain. The pyruvate formate lyase activity may be completely deleted. Decrease of the pyruvate formate lyase activity can be confirmed by measuring the pyruvate formate lyase activity by a known method (Knappe, J. and Blaschkowski, H.P., 1975, Meth. Enzymol., 41:508-518). The microorganism used in the presc t invention in which pyruvate formate lyase activity is decreased and expression of the *ybjL* gene is enhanced can be obtained by, for example, preparing a microorganism in which PFL gene is disrupted, and transforming this microorganism with a recombinant vector containing the *ybjL* gene. However, either of the modification for decreasing the PFL activity and the modification for enhancing expression of *ybjL* may be performed first. The pyruvate formate lyase activity can be decreased by a method similar to the method for decreasing the lactate dehydrogenase activity described above.

When an organic acid, especially succinic acid, is produced by the method of the present invention, a bacterium modified so that the pyruvate carboxylase (PC) activity is enhanced, in addition to that expression of the *ybjL* gene is enhanced, may also be used. Enhancement of the pyruvate carboxylase activity may be combined with decrease of lactate dehydrogenase activity, alcohol dehydrogenase activity, and/or pyruvate formate lyase activity. The expression "modified so that pyruvate carboxylase activity is enhanced" means that the pyruvate carboxylase activity is increased as compared to that of an unmodified strain such as a wild-type strain or parent strain. The pyruvate carboxylase activity can be measured by, for example, a method of measuring decrease of NADH as described later.

As the PC gene used in the method of the present invention, a gene having an already determined nucleotide sequence or a gene obtained by isolating a DNA fragment encoding a protein having the PC activity from a chromosome of a microorganism, animal, plant, or the like and determining the nucleotide sequence. After the nucleotide sequence is determined, a gene synthesized on the basis of that sequence may be used.

As the PC gene, a PC gene derived from a coryneform bacterium such as *Corynebacterium glutamicum* (Peters-Wendisch, P.G. et al., 1998, Microbiology, vol. 144:915-927) may be used. Furthermore, so long as functions of encoded PC, i.e., the properties of being involved in carbon dioxide fixation, are not substantially degraded, a part of nucleotides in the nucleotide sequence of the PC gene may be replaced with other nucleotides, deleted, or inserted with another or other nucleotides. Alternatively, a part of the nucleotide sequence may be transfered.

The PC genes obtained from any of bacteria other than *Corynebacterium glutamicum,* other microorganisms, animals and plants can also be used. In particular, sequences of the PC genes derived from microorganisms, animals and plants described below are known (references are indicated below), and they can be obtained by hybridization or amplification by PCR of the ORF portions performed in the same manner as described above.

Human [Biochem. Biophys. Res. Comm., 202, 1009-1014, (1994)]

Mouse [Proc. Natl. Acad. Sci. USA., 90, 1766-1779, (1993)]

Rat [GENE, 165, 331-332, (1995)]

Yeast: Saccharomyces cerevisiae [Mol. Gen. Genet., 229, 307-315, (1991)], *Schizosaccharomyces pombe* [DDBJ Accession No.; D78170]

*Bacillus stearothermophilus* [GENE, 191, 47-50, (1997)]

Rhizobium etli [J. Bacteriol., 178, 5960-5970, (1996)]

The PC gene can be enhanced in the same manner as those used for enhancing expression of a target gene described above for L-glutamic acid-producing bacteria and enhancing expression of the *ybjL* gene.

### <2> Method for producing acidic substance having a carboxyl group of the present invention

An acidic substance having a carboxyl group can be produced by culturing the microorganism used in the present invention in a medium to produce and accumulate an acidic substance having a carboxyl group in the medium and collecting the acidic substance having a carboxyl group from the medium. Furthermore, an acidic substance having a carboxyl group can also be produced by allowing the microorganism used in the present invention or a product obtained by processing the microorganism to act on an organic raw material in a reaction mixture containing carbonate ions, bicarbonate ions, or carbon dioxide gas to produce the acidic substance having a carboxyl group, and collecting the acidic substance having a carboxyl group. The former method is preferred when the acidic substance having a carboxyl group is an acidic amino acid. The latter method is preferred when the acidic substance having a carboxyl group is an organic acid. Hereinafter, preferred embodiments of production of an acidic amino acid and an organic acid as acidic substance having a carboxyl group will be exemplified.

### <2-1> Production of acidic amino acid

An acidic amino acid can be produced by culturing the microorganism used in the present invention in a medium to produce and accumulate an acidic amino acid in the medium and collecting the acidic amino acid from the medium.

As the medium used for the culture, a usual medium containing a carbon source, nitrogen source and inorganic salts as well as trace amount organic nutrients such as amino acids and vitamins as required can be used. Either a synthetic medium or natural medium may be used. The carbon source and nitrogen source used in the medium may be of any type so long as substances that can be utilized by a strain to be cultured are chosen.

As the carbon source, saccharides such as glucose, glycerol, fructose, sucrose, maltose, mannose, galactose, starch hydrolysate and molasses can be used. In addition, alcohols such as ethanol may be used independently or in combination with another carbon source. Furthermore, organic acids such as acetic acid and citric acid other than the target substance may also be used as the carbon source. As the nitrogen source, ammonia, ammonium salts such as ammonium sulfate, ammonium carbonate, ammonium chloride, ammonium phosphate and ammonium acetate, nitrates and the like can be used. As the trace amount organic nutrients, amino acids, vitamins, fatty acids, nucleic acids, those containing these substances such as peptone, casamino acid, yeast extract and soybean protein decomposition products can be used. When an auxotrophic mutant strain that requires an amino acid or the like for growth is used, the required nutrient is preferably supplemented. As mineral salts, phosphates, magnesium salts, calcium salts, iron salts, manganese salts and the like can be used.

The culture is preferably performed as aeration culture, while the fermentation temperature is controlled to be 20 to 45°C, and pH to be 3 to 9. When pH falls during the culture, the medium is neutralized by addition of, for example, calcium carbonate, or with an alkali such as ammonia gas. An acidic amino acid is accumulated in the culture broth preferably after 10 to 120 hours of culture under such conditions as described above.

When the acidic amino acid is L-glutamic acid, the culture can be performed with precipitating L-glutamic acid in the medium by using a liquid medium adjusted to have a condition under which L-glutamic acid is precipitated. The condition under which L-glutamic acid is precipitated is, for example, pH 5.0 to 4.0, preferably pH 4.5 to 4.0, more preferably pH 4.3 to 4.0, particularly preferably pH 4.0.

After completion of the culture, L-glutamic acid can be collected from the culture medium by known collection methods. For example, L-glutamic acid can be collected by, after cells are removed from the culture medium, concentrating the culture medium to crystallize it, or by ion exchange chromatography or the like. When the culture is performed under conditions for precipitating L-glutamic acid, L-glutamic acid precipitated in the medium can be collected by centrifugation, filtration or the like. In this case, it is also possible to crystallize L-glutamic acid dissolved in the medium and then collect L-glutamic acid precipitated in the culture broth together with the crystallized L-glutamic acid.

### <2-2> Production of organic acid

An organic acid can be produced by allowing the microorganism used in the present invention or a product obtained by processing the microorganism to act on an organic raw material in a reaction mixture containing carbonate ions, bicarbonate ions, or carbon dioxide gas to produce the organic acid, and collecting the organic acid.

In the first embodiment, by culturing the microorganism in a medium containing carbonate ions, bicarbonate ions, or carbon dioxide gas, and an organic raw material, proliferation of the microorganism and production of the organic acid are simultaneously attained. In this embodiment, the medium corresponds to the reaction mixture. Proliferation of the microorganism and production of the organic acid may be simultaneously attained, or there may be a culture period in which proliferation of the microorganism is mainly attained, and a culture period in which production of the organic acid is mainly attained.

In the second embodiment, by allowing cells proliferated by culture in a medium to coexist with a reaction mixture containing carbonate ions, bicarbonate ions, or carbon dioxide gas, and an organic raw material, and thereby allowing the microorganism to act on the organic raw material in the reaction mixture, an organic acid is produced. In this embodiment, a product obtained by processing cells of the microorganism can also be used. Examples of the product obtained by processing cells include, for example, immobilized cells obtained by immobilizing cells with acrylamide, carragheenan, or the like, disrupted product obtained by disrupting cells, centrifugation supernatant of the disrupted product, fraction obtained by partial purification of the supernatant by ammonium sulfate treatment or the like, and the like.

Although the bacteria used for the culture may be those obtained on a solid medium such as agar medium by slant culture, those cultured beforehand in a liquid medium (seed culture) are preferred.

As the medium used for the culture, a medium usually used for culture of microorganisms can be used. For example, a generally-used medium obtained by adding natural nutrients such as meat extract, yeast extract and peptone, to a composition comprising inorganic salts such as ammonium sulfate, potassium phosphate and magnesium sulfate can be used.

In the aforementioned first embodiment, the carbon source added to the medium also serves as the organic raw material for the production of the organic acid.

In the aforementioned second embodiment, the cells after the culture are collected by centrifugation, membrane separation, or the like, and used for the organic acid production reaction.

The organic raw material used in the method of the present invention is not particularly limited so long as a carbon source which the microorganism used in the present invention can assimilate to produce succinic acid is used. However, fermentable carbohydrates including carbohydrates such as galactose, lactose, glucose, fructose, glycerol, sucrose, saccharose, starch and cellulose, polyalcohols such as glycerin, mannitol, xylitol and ribitol, and the like are usually used. Among these, glucose, fructose and glycerol are preferred, and glucose is particularly preferred. When the organic acid is succinic acid, fumaric acid or the like may be added in order to efficiently produce succinic acid as described in Japanese Patent Laid-open No. 5-68576, and malic acid may be added instead of fumaric acid.

Furthermore, a saccharified starch solution, molasses, or the like containing the aforementioned fermentable carbohydrates may also be used. Those fermentable carbohydrates may be used independently or in combination. Although concentration of the aforementioned organic raw material is not particularly limited, it is more advantageous that the concentration is as high as possible within such a range that culture of the microorganism and production of the organic acid are not inhibited. In the aforementioned first embodiment, concentration of the organic raw material in the medium is generally in the range of 5 to 30% (w/v), preferably 10 to 20% (w/v). Furthermore, in the aforementioned second embodiment, the concentration of the organic raw material in the reaction mixture is generally in the range of 5 to 30% (w/v), preferably 10 to 20% (w/v). Furthermore, the organic raw material may be supplemented along with decrease of the organic raw material with progress of the culture or reaction.

The aforementioned reaction mixture containing carbonate ions, bicarbonate ions, or carbon dioxide gas and the organic raw material is not particularly limited, and it may be, for example, a medium for culturing microorganisms, or it may be a buffer such as phosphate buffer. The reaction mixture is preferably an aqueous solution containing a nitrogen source, inorganic salts, and the like. The nitrogen source is not particularly limited so long as a nitrogen source which the microorganism used in the present invention can assimilate to produce an organic acid is chosen, and specific examples include various organic or inorganic nitrogen compounds such as ammonium salts, nitrates, urea, soybean hydrolysate, casein degradation products, peptone, yeast extract, meat extract, and corn steep liquor. Examples of the inorganic salts include various phosphates, sulfates, and metallic salts such as those of magnesium, potassium, manganese, iron, and zinc. If necessary, growth-promoting factors including vitamins such as biotin, pantothenic acid, inositol, and nicotinic acid, nucleotides, amino acids and the like are added. In order to suppress foaming at the time of the reaction, it is desirable to add an appropriate amount of a commercially available antifoam to the medium.

pH of the reaction mixture can be adjusted by adding sodium carbonate, sodium bicarbonate, potassium carbonate, potassium bicarbonate, magnesium carbonate, sodium hydroxide, calcium hydroxide, magnesium hydroxide, or the like. Since pH for the reaction is usually 5 to 10, preferably 6 to 9.5, pH of the reaction mixture is adjusted to be within the aforementioned range with an alkaline substance, carbonate, urea, or the like even during the reaction, if needed.

As the reaction mixture used in the present invention, water, buffer, medium or the like is used, but a medium is most preferred. The medium can be made to contain, for example, the aforementioned organic raw material, and carbonate ions, bicarbonate ions, or carbon dioxide gas, and the reaction can be performed under anaerobic conditions. The carbonate or bicarbonate ions are supplied from magnesium carbonate, sodium carbonate, sodium bicarbonate, potassium carbonate, or potassium bicarbonate, which can also be used as a neutralizing agent. However, if necessary, carbonate or bicarbonate ions may also be supplied from carbonic acid or bicarbonic acid or salts thereof or carbon dioxide gas. Specific examples of the salts of carbonic acid or bicarbonic acid include, for example, magnesium carbonate, ammonium carbonate, sodium carbonate, potassium carbonate, ammonium bicarbonate, sodium bicarbonate, potassium bicarbonate, and the like. Carbonate ions or bicarbonate ions may be added at a concentration of 0.001 to 5 M, preferably 0.1 to 3 M, more preferably 1 to 2 M. When carbon dioxide gas is contained, carbon dioxide gas is contained in an amount of 50 mg to 25 g, preferably 100 mg to 15 g, more preferably 150 mg to 10 g, per litter of the solution.

The optimal growth temperature of the microorganism used for the reaction is generally in the range of 25 to 40°C. The reaction temperature is generally in the range of 25 to 40°C, preferably in the range of 30 to 37°C. Amount of bacterial cells in the reaction mixture is, although it is not particularly limited, 1 to 700 g/L, preferably 10 to 500 g/L, more preferably 20 to 400 g/L. The reaction time is preferably 1 to 168 hours, more preferably 3 to 72 hours. The reaction may be performed batchwise or in a column.

Culture of bacteria is preferably performed under aerobic conditions. On the other hand, the organic acid production reaction may be performed under aerobic conditions, microaerobic conditions or anaerobic conditions. For the reaction under microaerobic conditions or anaerobic conditions, a method of performing the reaction in a sealed reaction vessel without aeration, a method of performing the reaction with supplying an inert gas such as nitrogen gas to the reaction mixture, a method of performing the reaction with supplying an inert gas containing carbon dioxide gas to the reaction mixture, and the like can be used.

The organic acid accumulated in the reaction mixture (culture medium) may be separated and purified from the reaction mixture in a conventional manner. Specifically, solids such as bacterial cells can be removed by centrifugation, filtration, or the like, then the resultant solution can be desalted with an ion exchange resin or the like, and the organic acid can be separated and purified from the solution by crystallization or column chromatography.

Furthermore, in the present invention, when the target organic acid is succinic acid, after succinic acid is produced by the method of the present invention described above, a polymerization reaction can be carried out by using the obtained succinic acid as a raw material to produce a polymer containing succinic acid. In recent years, with increase of environmentally friendly industrial products, polymers prepared from raw materials of plant origin have been attracting attentions. Succinic acid produced in the present invention can be converted into polymers such as polyesters and polyamides and used (Japanese Patent Laid-open No. 4-189822). Specific examples of succinic acid-containing polymers include succinic acid polyesters obtainable by polymerizing a diol such as butanediol and ethylene glycol and succinic acid, succinic acid polyamides obtainable by polymerizing a diamine such as hexamethylenediamine and succinic acid, and the like. In addition, succinic acid and succinic acid-containing polymers obtained by the production method of the present invention, and compositions containing these can be used for food additives, pharmaceutical agents, cosmetics, and the like.

### Examples

Hereinafter, the present invention will be explained more specifically with reference to examples.

### Reference Example 1: Construction of Pantoea ananatis strain resistant to λ Red gene product

In order to perform *sdhA* gene disruption in *Pantoea ananatis,* a recipient strain for efficiently carrying out the method called "Red-driven integration" or "Red-mediated integration" (Datsenko, KA. and Wanner, B.L., 2000, Proc. Natl. Acad. Sci. USA., 97, 6640-6645) was constructed.

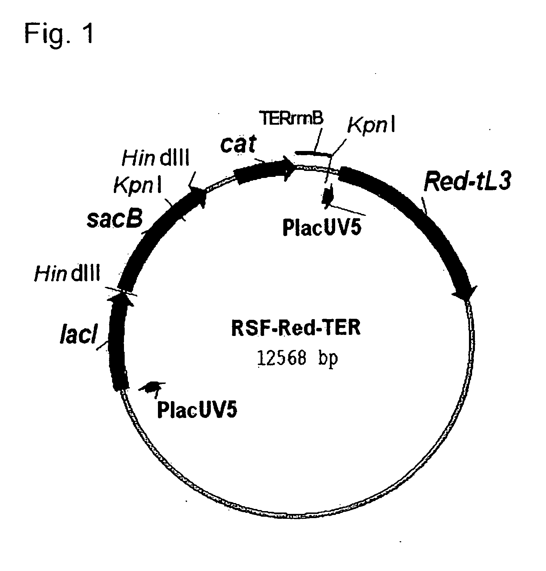

First, the novel helper plasmid RSF-Red-TER which expresses the *gam*, *bet* and exo genes of λ (henceforth referred to as "λ Red genes") was constructed (Fig. 1). The details thereof will be described in Reference Example 2.

This plasmid can be used in a wide range of hosts having different genetic backgrounds. This is because 1) this plasmid has the replicon of the RSF1010 wide host spectrum plasmid (Scholz, et al., 1989, Gene, 75:271-288; Buchanan-Wollaston et al., 1987, Nature, 328:172-175), which is stably maintained by many types of gram negative and gram positive bacteria, and even plant cells, 2) the λ Red genes, *gam*, *bet* and exo genes, are under the control of the PlacUV5 promoter, which is recognized by the RNA polymerases of many types of bacteria (for example, Brunschwig, E. and Darzins, A., 1992, Gene, 111, 1, 35-41; Dehio, M. et al, 1998, Gene, 215, 2, 223-229), and 3) the autoregulation factor PlacUV5-lacI and the p-non-dependent transcription terminator (TrrnB) of the *rrnB* operon of *Escherichia coli* lower the basal expression level of the λ Red genes (Skorokhodova, A.Y. et al, 2004, Biotekhnologiya (Rus), 5, 3-21). Furthermore, the RSF-Red-TER plasmid contains the levansucrase gene (*sacB*), and by using this gene, the plasmid can be collected from cells in a medium containing sucrose.

In *Escherichia coli,* the frequency of integration of a PCR-generated DNA fragment along with the short flanking region provided by the RSF-Red-TER plasmid is as high as the frequency obtained when using the pKD46 helper plasmid (Datsenko, K.A., Wanner, B.L., 2000, Proc. Natl. Acad. Sci. USA, 97, 6640-6695). However, expression of the λ Red genes is toxic to *Pantoea ananatis.* Cells transformed with the RSF-Red-TER helper plasmid grow extremely slowly in LB medium containing IPTG (isopropyl-β-D-thiogalactopyranoside, 1 mM) and an appropriate antibiotic (25 µg/ml of chloramphenicol or 40 µg/ml of kanamycin), and the efficiency of λ Red-mediated recombination is extremely low (10⁻⁸), if observed at all.

A variant strain of *Pantoea ananatis* which is resistant to expression of all three of the λ Red genes was selected. For this purpose, the RSF-Red-TER plasmid was introduced into the *Pantoea ananatis* SC17 strain (U.S. Patent No. 6,596,517) by electroporation. After an 18 hour culture, about 10⁶ transformants were obtained, and among these, 10 clones formed colonies of a large size, and all the remainder formed extremely small colonies. After an 18 hour culture, the large colonies were about 2 mm, and the small colonies were about 0.2 mm. Whereas the small colonies did not grow any more even when the culture was extended up to 24 hours, the large colonies continued to grow. One of the large colony *Pantoea ananatis* mutant strains which was resistant to expression of all three of the λ Red genes (*gam*, *bet*, and *exo*) was used for further analysis.

The RSF-Red-TER plasmid DNA was isolated from one clone of the large colony clones, and from several clones of the small colony clones, and transformed again into *Escherichia coli* MG1655 to examine the ability of the plasmid to synthesize an active Red gene product. By a control experiment for Red-dependent integration in the obtained transformants, it was demonstrated that only the plasmid isolated from the large colony clone induced expression of the λ Red genes required for the Red-dependent integration. In order to investigate whether the Red-mediated integration occurs in the selected large colony clone, electroporation was performed using a linear DNA fragment produced by PCR. This fragment was designed so that it contains a Km^{R} marker and a flanking region of 40 bp homologous to the *hisD* gene, and is integrated into the *hisD* gene of *Pantoea ananatis* at the SmaI recognition site. Two small colony clones were used as control. The nucleotide sequence of the *hisD* gene of *Pantoea ananatis* is shown in SEQ ID NO: 40. For PCR, the oligonucleotides of SEQ ID NOS: 41 and 42 were used as primers, and the pMW118-(λatt-Km^{r}-λatt) plasmid was used as the template. The two small colony clones which were not resistant to the λ Red genes were used as a control. Construction of the pMW118-(λattL-Kmr-λattR) plasmid will be explained in detail in Reference Example 3.

The RSF-Red-TER plasmid can induce expression of the Red genes by the *lacI* gene carried on the plasmid. Two kinds of induction conditions were investigated. In the first group, IPTG (1 mM) was added 1 hour before the electroporation, and in the second group, IPTG was added at the start of the culture to prepare cells in which electroporation is possible. The growth rate of the progeny of the SC17 strain harboring RSF-Red-TER derived from the large colony clone was not significantly lower than that of a strain not having the RSF-Red-TER plasmid. The addition of IPTG only slightly decreased the growth rate of these cultures. On the other hand, the RSF-Red-TER-introduced SC17 strain derived from the progeny of the small colony clones grew extremely slowly even without the addition of IPTG, and after induction, growth was substantially arrested. After electroporation of RSF-Red-TER isolated from the cells of the progeny of the large colony clone, many Km^{R} clones grew (18 clones after a short induction time, and about 100 clones after an extended induction time). All of the 100 clones that were investigated had a His⁻ phenotype, and about 20 clones were confirmed by PCR to have the expected structure of the chromosome in the cells. On the other hand, even when electroporation was performed with RSF-Red-TER isolated from cells of the progeny of the small colony clones, any integrated strain was not obtained.

The obtained large colony clone was grown on a plate containing 7% sucrose to eliminate the plasmid, and transformed again with RSF-Red-TER. The strain without the plasmid was designated SC17(0). This strain was deposited at the Russian National Collection of Industrial Microorganisms (VKPM), GNII Genetica (Address: 1 Dorozhny proezd., 1 Moscow 117545, Russia) on September 21, 2005, and assigned an accession number of VKPM B-9246.

All the clones which grew after the aforementioned re-transformation had a large size like the parent strain clone SC17(0). The Red-mediated integration experiment was performed in the SC17(0) strain re-transformed with the RSF-Red-TER plasmid. Three of the independent transformants obtained were investigated using the same DNA fragment as that used for the previous experiment. The short induction time (1 hour before electroporation) was employed. Km^{R} clones exceeding ten clones grew in each experiment. All the examined clones had the His⁻phenotype. In this way, a mutant strain designated SC17(0) which is resistant to the expression of the λ Red genes was selected. This strain can be used as a recipient strain suitable for the Red-dependent integration into the *Pantoea ananatis* chromosome.

### Reference Example 2: Construction of helper plasmid RSF-Red-TER

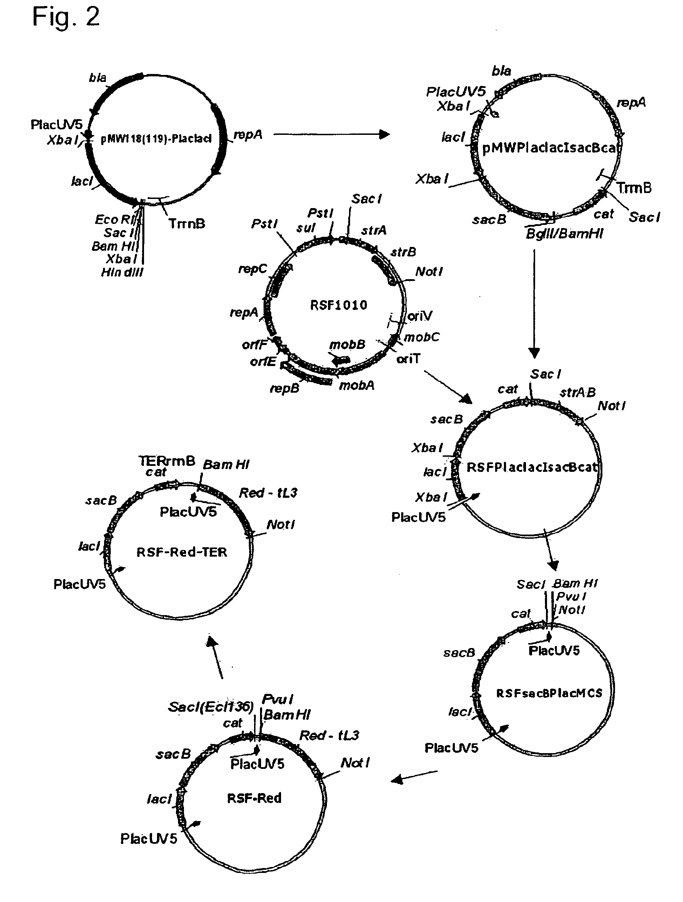

The scheme for constructing the helper plasmid RSF-Red-TER is shown in Fig. 2.

As the first step in the construction, an RSFsacBPlacMCS vector was designed. For this purpose, DNA fragments containing the cat gene of the pACYC184 plasmid and the structural region of the *sacB* gene of *Bacillus subtilis* were amplified by PCR using the oligonucleotides of SEQ ID NOS: 43 and 44, and 45 and 46, respectively. These oligonucleotides contained *Bgl*II, *Sac*I, *Xba*I and BamHI restriction enzyme sites, which are required and convenient for further cloning, in the 5' end regions, respectively. The obtained *sacB* fragment of 1.5 kb was cloned into the previously obtained pMW119-P_{lac}lacI vector at the XbaI-BamHI site. This vector was constructed in the same manner as that described for the pMW118-P_{lac}lacI vector (Skorokhodova, A.Y. et al, 2004, Biotekhnologiya (Rus), 5:3-21). However, this vector contained a polylinker moiety derived from pMW219 instead of the pMW218 plasmid.

Then, the aforementioned cat fragment of 1.0 kb was treated with *Bgl*II and *Sac*I, and cloned into the RSF-P_{lac}lacIsacB plasmid obtained in the previous step at the *Bam*HI-SacI site. The obtained plasmid pMW-P_{lac}lacIsacBcat contained the PlacUV5-lacI-*sac*B-cat fragment. In order to subclone this fragment into the RSF1010 vector, pMW-P_{lac}lacIsacBcat was digested with *Bgl*II*,* blunt-ended with DNA polymerase I Klenow fragment, and successively digested with *Sac*I. A 3.8 kb BglII-SacI fragment of the pMWP_{lac}lacIsacBcat plasmid was eluted from a 1% agarose gel, and ligated with the RSF1010 vector which had been treated with *Pst*I and *SacI. Escherichia coli* TG1 was transformed with the ligation mixture, and plated on the LB medium containing chloramphenicol (50 mg/L). The plasmids isolated from the grown clones were analyzed with restriction enzymes to obtain an RSFsacB plasmid. In order to construct an RSFsacBPlacMCS vector, a DNA fragment containing the PlacUV5 promoter was amplified by PCR using the oligonucleotides of SEQ ID NOS: 47 and 48 as primers and the pMW119-P_{lac}lacI plasmid as a template. The obtained fragment of 146 bp was digested with SacI and NotI, and ligated with the SacI-NotI large fragment of the RSFsacB plasmid. Then, by PCR using the oligonucleotides of SEQ ID NOS: 49 and 50 as primers, and the pKD46 plasmid (Datsenko, K.A., Wanner, B.L., 2000, Proc. Natl. Acad. Sci. USA, 97, 6640-6645) as a template, a DNA fragment of 2.3 kb containing the λRedαβγ genes and the transcription terminator tL3 was amplified. The obtained fragment was cloned into the RSFsacBPlacMCS vector at the PvuI-NotI site. In this way, the RSFRed plasmid was designed.

In order to eliminate read through transcription of the Red genes, a ρ-dependent transcription terminator of the rrnB operon of *Escherichia coli* was inserted at a position between the *cat* gene and the PlacUV5 promoter. For this purpose, a DNA fragment containing the PlacUV5 promoter and the TrrnB terminator was amplified by PCR using the oligonucleotides of SEQ ID NOS: 51 and 48 as primers and the chromosome of *Escherichia coli* BW3350 as a template. These obtained fragments were treated with KpnI and ligated. Then, the 0.5 kb fragment containing both PlacUV5 and TrrnB was amplified by PCR using the oligonucleotides of SEQ ID NOS: 48 and 52 as primers. The obtained DNA fragment was digested with EcoRI, blunt-ended by a treatment with DNA polymerase I Klenow fragment, digested with BamHI, and ligated with the Ec1136II-BamHI large fragment of the RSFsacBPlacMCS vector. The obtained plasmid was designated RSF-Red-TER.

### Reference Example 3: Construction of pMW118-(λattL-Km^{r}-λattR) plasmid

The pMW118-(λattL-Km^{r}-λattR) plasmid was constructed from the pMW118-attL-Tc-attR (WO2005/010175) plasmid by replacing the tetracycline resistance marker gene with the kanamycin resistance gene of the pUC4K plasmid. For that purpose, the EcoRI-HindIII large fragment from pMW118-attL-Tc-attR plasmid was ligated to two fragments from the pUC4K plasmid: HindIII-PstI fragment (676 bp) and EcoRI-HindIII fragment (585 bp). Basic pMW118-attL-Tc-attR was obtained by ligation of the following four fragments.
1) The *Bgl*II-*Eco*RI fragment (114 bp) including *attL* (SEQ ID NO: 55) which was obtained by PCR amplification of the region corresponding to *attL* of the *Escherichia coli* W3350 (containing λ prophage) chromosome using the primers P1 and P2 (SEQ ID NOS: 53 and 54) (these primers contained the subsidiary recognition sites for *Bgl*II and *Eco*RI).
2) The *Pst*I-*Hind*III fragment (182 bp) including attR (SEQ ID NO: 58) which was obtained by PCR amplification of the region corresponding to *attR* of the *Escherichia coli* W3350 (containing λ prophage) chromosome using the primers P3 and P4 (SEQ ID NOS: 56 and 57) (these primers contained the subsidiary recognition sites for *Pst*I and *Hind*III).
3) The *Bgl*II-*Hind*III large fragment (3916 bp) of pMW118-ter_rrnB. The plasmid pMW118-ter_rrnB was obtained by ligation of the following three DNA fragments:
   - The large DNA fragment (2359 bp) including the *Aat*II-*Eco*RI fragment of pMW118 that was obtained by digesting pMW118 with *Eco*RI, treating with DNA polymerase I Klenow fragment, and then digesting with *Aat*II;
   - The small *Aat*II-*Bgl*II fragment (1194 bp) of pUC19 including the *bla* gene for ampicillin resistance (ApR), which was obtained by PCR amplification of the corresponding region of the pUC19 plasmid using the primers P5 and P6 (SEQ ID NOS: 59 and 60) (these primers contained the subsidiary recognition sites for *Pst*I, *Aat*II and *Bgl*II);
   - The small *Bgl*II-*Pst*I fragment (363 bp) of the transcription terminator ter_rrnB, which was obtained by PCR amplification of the corresponding region of the *Escherichia coli* MG1655 chromosome using the primers P7 and P8 (SEQ ID NOS: 61 and 62) (these primers contained the subsidiary recognition sites for *Pst*I, *Bgl*II and *Pst*I).
4) The small *Eco*RI-*Pst*I fragment (1388 bp) (SEQ ID NO: 63) of pML-Tc-ter_thrL including the tetracycline resistance gene and the ter_thrL transcription terminator; the pML-Tc-ter_thrL plasmid was obtained by the following two steps:
   - the pML-ter_thrL plasmid was obtained by digesting the pML-MCS plasmid (Mashko, S.V. et al., 2001, Biotekhnologiya (in Russian), no. 5, 3-20) with *Xba*I and *Bam*HI, followed by ligation of the large fragment (3342 bp) with the *Xba*I-*Bam*HI fragment (68 bp) carrying ter_thrL terminator obtained by PCR amplification of the corresponding region of the *Escherichia coli* MG1655 chromosome using the primers P9 and P10 (SEQ ID NOS: 64 and 65) (these primers contained the subsidiary recognition sites for *Pst*I, *Xba*I and *Bam*HI);
   - the pML-Tc-ter_thrL plasmid was obtained by digesting the pML-ter thrL plasmid with *Kpn*I and *Xba*I followed by treatment with Klenow fragment of DNA polymerase I and ligated with the small EcoRI-Van91I fragment (1317 bp) of pBR322 including the tetracycline resistance gene (pBR322 was digested with *Eco*RI and Van91I and then treated with DNA polymerase I Klenow fragment).

### Example 1: Search of L-glutamic acid excretion gene

Search of L-glutamic acid excretion gene was performed as follows. Since L-glutamic acid is converted into an intermediate of the tricarboxylic acid cycle, 2-oxoglutarate, in one step by glutamate dehydrogenase, it is expected that L-glutamic acid is easily metabolized in many microorganisms having glutamate dehydrogenase or the tricarboxylic acid cycle. However, since a strain in which 2-oxoglutarate dehydrogenase is deleted cannot degrade L-glutamic acid, growth thereof is inhibited in the presence of glutamic acid of a high concentration. In this example, by using an extracellular polysaccharide biosynthesis system-deficient strain SC17sucAams derived from the *Pantoea ananatis* SC17sucA strain (refer to Japanese Patent Laid-open No. 2001-333769) as a 2-oxoglutarate dehydrogenase deficient strain, it was attempted to obtain an L-glutamic acid excretion gene utilizing resistance to L-glutamic acid of a high concentration as a marker.

Since the SC17sucA strain produced a marked amount of extracelluar polysaccharides when it grew on an agar medium containing a sugar source, handling thereof was extremely difficult. Therefore, by deleting the *ams* operon coding for extracellular polysaccharide biosynthesis system genes, production of extracellular polysaccharides was suppressed. PCR was performed by using pMW118-λattL-Km^{r}-λattR as a template and the primers of SEQ ID NOS: 6 and 7 to amplify a gene fragment containing a kanamycin resistance gene, *attL* and *attR* sequences of λ phage at the both ends of the resistance gene, and 50 bp upstream sequence of *amsI* and 50 bp downstream sequence of the *amsC* gene added to the outer ends of the λ phage sequences. This fragment was purified by using Wizard PCR Prep DNA Purification System (Promega).

Then, the SC17(0) strain was transformed with RSF-Red-TER to obtain an SC17(0)/RSF-Red-TER strain. This strain was cultured overnight in the L medium (medium containing 10 g of Bacto tryptone, 5 g of yeast extract and 5 g of NaCl in 1 L of pure water, pH 7.0) containing 25 mg/L of chloramphenicol, the culture medium after the overnight culture was inoculated in 1/100 volume into 100 mL of the L medium containing 25 mg/L of chloramphenicol and 1 mM isopropyl-β-D-thiogalactopyranoside, and culture was performed at 34°C for 3 hours. The cells prepared as described above were collected, washed three times with ice-cooled 10% glycerol, and finally suspended in 0.5 mL of 10% glycerol. The suspended cells were used as competent cells, and 100 ng of the PCR fragment prepared in the above section was introduced into the cells by using GENE PULSER II (BioRad) under the conditions of a field strength of 18 kV/cm, capacitor capacity of 25 µF and resistance of 200 Ω. Ice-cooled SOC medium (20 g/L of Bacto tryptone, 5 g/L of yeast extract, 0.5 g/L of NaCl, and 10 g/L of glucose) was added to the cell suspension, and culture was performed at 34°C for 2 hours with shaking. The culture was applied to a medium prepared by adding ingredients of minimal medium (medium containing 5 g of glucose, 2 mM magnesium sulfate, 3 g of monopotassium phosphate, 0.5 g of sodium chloride, 1 g of ammonium chloride and 6 g of disodium phosphate in 1 L) and 40 mg/L of kanamycin to the L medium (medium containing 10 g of Bacto tryptone, 5 g of yeast extract, 5 g of NaCl and 15 g of agar in 1 L of pure water, pH 7.0). The colonies which appeared were purified with the same medium, and then it was confirmed by PCR that the *ams* gene was replaced with the kanamycin resistance gene.

Chromosome was extracted from the *ams* gene-deficient strain using Bacterial Genomic DNA Purification Kit produced by Edge Biosystems. Separately, the SC17sucA strain was cultured overnight on an agar medium obtained by adding the ingredients of the minimal medium described above to the L medium. The cells were scraped with a loop, washed three times with ice-cooled 10% glycerol, and finally suspended in 10% glycerol so as to have a final volume of 500 µL. The suspended cells were used as competent cells, and 600 ng of the aforementioned chromosome DNA was introduced into the competent cells using GENE PULSER II (BioRad) under the conditions of a field strength of 17.5 kV/cm, capacitor capacity of 25 µF and resistance of 200 Ω. Ice-cooled SOC medium was added to the cell suspension, and culture was performed at 34°C for 2 hours with shaking. Then, the culture was applied to an agar medium prepared by adding ingredients of the minimal medium described above and 40 mg/L of kanamycin to the L medium. The colonies which appeared were purified with the same medium, and then it was confirmed by PCR that the *ams* gene was replaced with the kanamycin resistance gene. A strain confirmed so was designated as SC17sucAams strain.

Chromosomal DNA extracted from the *Pantoea ananatis* AJ13355 strain was partially digested with the restriction enzyme *Sau*3AI. Then, fragments of about 10 kb were collected and introduced into the *Bam*HI site of pSTV28 (Takara Bio) to prepare a plasmid library. This plasmid library was introduced into competent cells of the SC17sucAams strain prepared in a conventional manner by electroporation.

Selection was performed for the SC17sucAams strain introduced with the plasmid library on a plate of the L medium (medium containing 10 g of Bacto tryptone, 5 g of yeast extract, 5 g of NaCl and 15 g of agar in 1 L of pure water, pH 7.0) added with ingredients of minimal medium (medium containing 0.5 g of glucose, 2 mM magnesium sulfate, 3 g of monopotassium phosphate, 0.5 g of sodium chloride, 1 g of ammonium chloride and 6 g of disodium phosphate in 1 L of pure water) using chloramphenicol resistance as a marker to obtain transformants. These transformants were plated on a glucose minimal medium containing L-glutamic acid at a high concentration (medium comprising the glucose minimal medium mixed with 0.2 M L-glutamic acid, 100 mg/L each of lysine, methionine and diaminopimelic acid as final concentrations), in which SC17sucAams cannot form colonies.

The transformants were cultured at 34°C for 3 days, and 64 clones among the colonies that appeared were allowed to again form single colonies on the same plate. As a result, it was found that 11 clones among them formed colonies after 48 hours, and the remaining colonies formed colonies after 72 hours.

Then, the genes inserted into the vectors harbored by the transformants were analyzed. Plasmids were extracted from the transformants, and nucleotide sequences were determined. It was found that among 11 clones which formed colonies within 48 hours, 10 clones had the same loci, and all contained genes showing homology to *ybjL,* which was a gene of *Escherichia coli* of which function was unknown. In addition, this *ybjL* gene could not be obtained under the conditions that the glutamic acid excretion system gene described in WO2005/085419 *yhfK,* was obtained, and conversely, the *yhfK* gene could not be obtained under the condition of the selection used in this example.

In order to confirm that the factor which imparts glutamic acid resistance is *ybjL,* the *ybjL* gene was cloned. PCR was performed using the chromosomal DNA of AJ13355 strain as the template and oligonucleotides ybjL-F1 and ybjL-R2 shown in SEQ ID NOS: 8 and 9 to amplify the fragment of about 1.8 kb containing the *ybjL* gene of *P. ananatis.* This fragment was purified using Wizard PCR Prep DNA Purification System (Promega), and then treated with the restriction enzymes *Kpn*I and *Sph*I, and the product was ligated with pSTV28 (Takara Shuzo) treated with the same enzymes to obtain pSTV-PanybjL. The SC17sucA strain was transformed with this pSTV-PanybjL plasmid and the plasmid pSTV28 as a control for comparison (Takara Shuzo) to construct SC17sucA/pSTV-PanybjL strain and SC17sucA/pSTV28 strain.

The SC17sucA/pSTV-ybjL strain was plated on a glutamic acid-containing minimal medium (medium containing 0.5 g of glucose or sucrose, 2 mM magnesium sulfate, 3 g of monopotassium phosphate, 0.5 g of sodium chloride, 1 g of ammonium chloride, 6 g of disodium phosphate, 0.2 M sodium L-glutamate, 100 mg/L each of lysine, methionine and diaminopimelic acid and 15 g of agar in 1 L of pure water). Culture was performed at 34°C for 2 days. As a result, it was confirmed that whereas the control vector-introduced strain, SC17sucA/pSTV28 strain, could not form colonies, SC17sucA/pSTV-ybjL could form colonies on the minimal medium.

Then, the SC17sucA/pSTV-PanybjL strain was cultured in a glutamic acid-containing liquid minimal medium (medium containing 0.5 g of glucose, 2 mM magnesium sulfate, 3 g of monopotassium phosphate, 0.5 g of sodium chloride, 1 g of ammonium chloride, 6 g of disodium phosphate, 0.2 M sodium L-glutamate, 100 mg/L each of lysine, methionine and diaminopimelic acid in 1 L of pure water) to examine growth of the strain in the presence of high concentration L-glutamic acid.

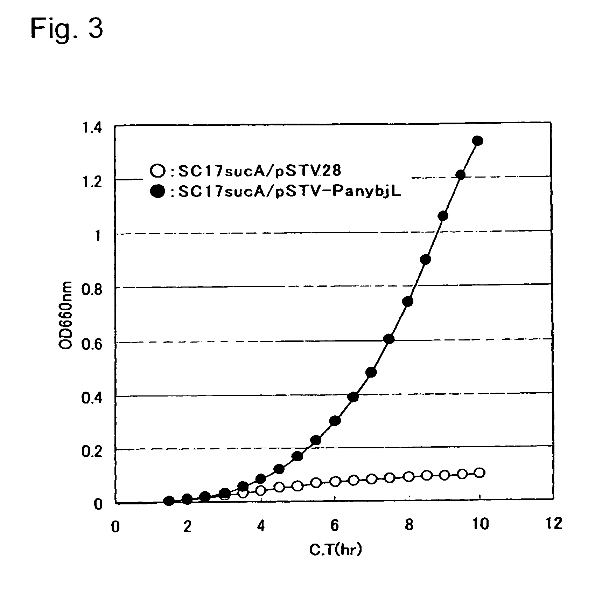

The results are shown in Fig. 3. It was found that growth of SC17sucA/pSTV-PanybjL in which *ybjL* gene was enhanced was markedly improved in the presence of high concentration L-glutamic acid as compared to the SC17sucA/pSTV28 strain used as a control strain. Accordingly, it was confirmed that *ybjL* is a factor which imparts resistance to L-glutamic acid.

### Example 2: Effect of ybjL gene amplification on L-glutamic acid production at neutral pH

Then, in order to examine effect of this gene on L-glutamic acid production, the plasmid for *ybjL* amplification, pSTV-PanybjL, was introduced into the L-glutamic acid producing bacterium SC17sucA/RSFCPG having the plasmid for L-glutamic acid production, RSFCPG, shown in SEQ ID NO: 10 (refer to Japanese Patent Laid-open No. 2001-333769), and L-glutamic acid productivity thereof was examined.

pSTV-PanybjL and the control plasmid for comparison, pSTV28 (Takara Shuzo), were each introduced into SC17sucA/RSFCPG by electroporation, and transformants were obtained using chloramphenicol resistance as a marker. After confirmation of the plasmids, the strain introduced with the plasmid for *ybjL* amplification was designated SC17sucA/RSFCPG+pSTV-PanybjL, and the pSTV29-introduced strain was designated SC17sucA/RSFCPG+pSTV28.

Then, SC17sucA/RSFCPG+pSTV-PanybjL and the control strain SC17sucA/RSFCPG+pSTV28 were cultured to examine the L-glutamic acid producing ability of the strains. The medium had the following composition.

### [Composition of culture medium]

### [section A]

| | |
|---|---|
| Sucrose | 30 g/L |
| MgSO₄·7H₂O | 0.5 g/L |

| [Group B] | |
|---|---|
| KH₂PO₄ | 2.0 g/L |
| Yeast Extract | 2.0 g/L |
| FeSO₄·7H₂O | 0.02 g/L |
| MnSO₄·5H₂O | 0.02 g/L |
| L-Lysine hydrochloride | 0.2 g/L |
| DL-Methionine | 0.2 g/L |
| Diaminopimelic acid | 0.2 g/L |
| (adjusted to pH 7.0 with KOH) | |

| [Group C] | |
|---|---|
| CaCO₃ | 20 g/L |

The ingredients of the groups A and B were sterilized at 115°C for 10 minutes by autoclaving, and the ingredient of the group C was sterilized at 180°C for 3 hours with dry heat. Then, the ingredients of the three groups were mixed, and 12.5 mg/L of tetracycline hydrochloride and 25 mg/L of chloramphenicol were added to the mixture.

SC17sucA/RSFCPG+pSTV29 and SC17sucA/RSFCPG+pSTV-PanybjL were each precultured on a medium obtained by adding ingredients of the minimal medium (medium containing 0.5 g of glucose, 2 mM magnesium sulfate, 3 g of monopotassium phosphate, 0.5 g of sodium chloride, 1 g of ammonium chloride and 6 g of disodium phosphate in 1 L of pure water), 25 mg/L of chloramphenicol and 12.5 mg/L tetracycline to the L medium (medium containing 10 g of Bacto tryptone, 5 g of yeast extract, 5 g of NaCl and 15 g of agar in 1 L of pure water, pH 7.0), and inoculated in an appropriate amount to 5 ml of the aforementioned medium contained in a test tube.

The cells were cultured for 17.5 hours, and then cell density, L-glutamic acid concentration and residual saccharide amount in the culture medium were measured. The cell density was examined by measuring turbidity at 620 nm of the medium diluted 51 times using a spectrophotometer (U-2000A, Hitachi). The L-glutamic acid concentration was measured in culture supernatant appropriately diluted with water by using Biotech Analyzer (AS-210, Sakera SI). The results are shown in Table 1. L-Glutamic acid accumulation was increased by about 3 g/L, and yield based on saccharide increased by about 9% in the *ybjL*-amplified strain, SC17sucA/RSFCPG+pSTV-PanybjL, as compared to the control strain, SC17sucA/RSFCPG+pSTV28.

**Table 1**

| | OD 620 nm (x 51) | L-Glutamic acid (g/L) | Yield based on saccharide (%) |
|---|---|---|---|
| SC17sucA/RSFCPG+pSTV28 | 0.385 | 15.0 | 44.5 |
| SC17sucA/RSFCPG+pSTV-PanybjL | 0.280 | 18.0 | 53.7 |

### Example 3: Effect of amplification of ybjL gene derived from Escherichia coli

Then, the *ybjL* gene of *Escherichia coli* was introduced into the *Pantoea ananatis* SC17sucA/RSFCPG strain, and effect thereof on glutamic acid production was examined.

PCR was performed using the oligonucleotides shown in SEQ ID NOS: 11 and 12 prepared on the basis of the sequence of the *ybjL* of *Escherichia coli* registered at GeneBank as AP009048 (SEQ ID NO: 3) and chromosome of *Escherichia coli* W3110 strain (ATCC 27325) as the template to obtain a fragment of about 1.7 kb containing the *ybjL* gene. This fragment was treated with *Sph*I and *Kpn*I, and ligated with pSTV28 (Takara Shuzo) at the corresponding site. The obtained plasmid for amplification of *ybjL* of *Escherichia coli* was designated as plasmid pSTV-EcoybjL for *ybjL* amplification.

The obtained plasmid pSTV-EcoybjL for *ybjL* amplification was introduced into the aforementioned SC17sucA/RSFCPG strain by electroporation, and a transformant was obtained using chloramphenicol resistance as a marker. The obtained *Escherichia coli ybjL* gene-amplified strain was designated as SC17sucA/RSFCPG+pSTV-EcoybjL.

Then, SC17sucA/RSFCPG+pSTV-EcoybjL and the control strain, SC17sucA/RSFCPG+pSTV28, were cultured to examine the L-glutamic acid producing ability of the strains. The medium had the following composition.

### [Composition of culture medium]

| [Group A] | |
|---|---|
| Sucrose | 30 g/L |
| MgSO₄·7H₂O | 0.5 g/L |

| [Group B] | |
|---|---|
| KH₂PO₄ | 2.0 g/L |
| Yeast Extract | 2.0 g/L |
| FeSO₄·7H₂O | 0.02 g/L |
| MnSO₄·5H₂O | 0.02 g/L |
| L-Lysine hydrochloride | 0.2 g/L |
| DL-Methionine | 0.2 g/L |
| Diaminopimelic acid | 0.2 g/L |
| (adjusted to pH 7.0 with KOH) | |

| [Group C] | |
|---|---|
| CaCO₃ | 20 g/L |

The ingredients of the groups A and B were sterilized at 115°C for 10 minutes by autoclaving, and the ingredient of the group C was sterilized at 180°C for 3 hours with dry heat. Then, the ingredients of the three groups were mixed, and 12.5 mg/L of tetracycline hydrochloride and 25 mg/L of chloramphenicol were added to the mixture.

SC17sucA/RSFCPG+pSTV28 and SC17sucA/RSFCPG+pSTV-EcoybjL were each precultured on a medium obtained by adding ingredients of the minimal medium (medium containing 0.5 g of glucose, 2 mM magnesium sulfate, 3 g of monopotassium phosphate, 0.5 g of sodium chloride, 1 g of ammonium chloride and 6 g of disodium phosphate in 1 L of pure water), 25 mg/L of chloramphenicol and 12.5 mg/L tetracycline to the L medium (medium containing 10 g of Bacto tryptone, 5 g of yeast extract, 5 g of NaCl and 15 g of agar in 1 L of pure water, pH 7.0), and inoculated in an appropriate amount to 5 ml of the aforementioned medium contained in a test tube.

The cells were cultured for 17.5 hours, and then cell density and L-glutamic acid concentration in the culture medium were measured in the same manners as those in Example 2. The results are shown in Table 2. L-Glutamic acid accumulation was increased by about 2 g/L, and yield based on saccharide increased by about 7% in the ybjL-amplified strain, SC17sucA/RSFCPG+pSTV-EcoybjL, as compared to the control strain, SC17sucA/RSFCPG+pSTV28.

**Table 2**

| | OD 620 nm (x 51) | L-Glutamic acid (g/L) | Yield based on saccharide (%) |
|---|---|---|---|
| SC17sucA/RSFCPG+pSTV28 | 0.385 | 15.0 | 44.5 |
| SC17sucA/RSFCPG+pSTV-EcoybjL | 0.348 | 17.2 | 51.2 |

### Example 4: Effect of amplification of ybjL gene on L-amino acid production in Escherichia coli

Then, the *ybjL* gene derived from *Pantoea ananatis* and the *ybjL* gene derived from *Escherichia coli* were each introduced into *Escherichia coli,* and effect of the amplification thereof was examined.

The aforementioned vector for amplification of *ybjL* gene derived from *Pantoea ananatis,* pSTV-PanybjL, vector for amplification of *ybjL* gene derived from *Escherichia coli,* pSTV-EcoybjL, and the control plasmid pSTV28 were each introduced into an *Escherichia coli* wild-type strain, W3110 strain, by electroporation to obtain transformants exhibiting chloramphenicol resistance. The strain in which *ybjL* derived from *Pantoea ananatis* was amplified was designated W3110/pSTV-PanybjL, the strain in which *ybjL* derived from *Escherichia coli* was amplified was designated W3110/pSTV-EcoybjL, and the pSTV28-introduced strain as a control was designated W3110/pSTV28.

Then, L-glutamic acid-producing abilities of the *ybjL*-amplified strains, W3110/pSTV-PanybjL and W3110/pSTV-EcoybjL, and W3110/pSTV28 as a control were examined. W3110/pSTV-PanybjL, W3110/pSTV-EcoybjL and W3110/pSTV28 as a control were each precultured on the L medium (medium containing 10 g of Bacto tryptone, 5 g of yeast extract, 5 g of NaCl and 15 g of agar in 1 L of pure water, pH 7.0) added with chloramphenicol, and one loop of cells were inoculated by using a 1-mL volume loop produced by Nunc to 5 mL of a medium having the following composition contained in a test tube, and cultured at 37°C for 11.5 hours with shaking. Cell density and L-glutamic acid concentration in the culture medium were measured in the same manners as those in Example 2. The results are shown in Table 3.

### [Composition of culture medium]

| [Group A] | |
|---|---|
| Glucose | 30 g/L |
| MgSO₄·7H₂O | 0.5 g/L |

| [Group B] | |
|---|---|
| (NH₄)₂SO₄ | 20 g/L |
| KH₂PO₄ | 2.0 g/L |
| Yeast Extract | 2.0 g/L |
| FeSO₄·7H₂O | 20 mg/L |
| MnSO₄·5H₂O | 20 mg/L |
| (adjusted to pH 7.0 with KOH) | |

| [Group C] | |
|---|---|
| Calcium carbonate | 20 g/L |

The ingredients of the groups A and B were sterilized at 115°C for 10 minutes by autoclaving, and the ingredient of the group C was sterilized at 180°C for 3 hours with dry heat. Then, the ingredients of the three groups were mixed, and 25 mg/L of chloramphenicol was added to the mixture.

**Table 3**

| | OD 620 nm (x 51) | L-Glutamic acid (g/L) | Yield based on saccharide (%) |
|---|---|---|---|
| W3110/pSTV28 | 0.341 | 1.2 | 6.5 |
| W3110/pSTV-PanybjL | 0.303 | 5.7 | 28.8 |
| W3110/pSTV-EcoybjL | 0.310 | 4.8 | 25.2 |

It was revealed that L-glutamic acid producing ability was markedly improved in the *Escherichia coli* W3110/pSTV-ybjL strain, which is a *Pantoea ananatis ybjL* gene-amplified strain, as compared to the vector-introduced strain, W3110/pSTV28 strain as a control.

Then, abilities to produce other amino acids of the *ybjL*-amplified strain were examined. W3110/pSTV-PanybjL, and W3110/pSTV28 as a control were precultured in the L medium (medium containing 10 g of Bacto tryptone, 5 g of yeast extract, 5 g of NaCl and 15 g of agar in 1 L of pure water, pH 7.0) added with chloramphenicol, and one loop of cells were inoculated by using a 5-mL volume loop produced by Nunc to 5 mL of a medium having the following composition contained in a test tube, and cultured at 37°C for 7 hours with shaking. Cell densities and L-amino acid concentrations in the culture medium were measured in the same manners as those in Example 2. The results are shown in Table 4.

### [Composition of culture medium]

| [Group A] | |
|---|---|
| Glucose | 40 g/L |
| MgSO₄·7H₂O | 0.5 g/L |

| [Group B] | |
|---|---|
| (NH₄)₂SO₄ | 20 g/L |
| KH₂PO₄ | 2.0 g/L |
| Yeast Extract | 2.0 g/L |
| FeSO₄·7H₂O | 20 mg/L |
| MnSO₄·5H₂O | 20 mg/L |
| (adjusted to pH 7.0 with KOH) | |

| [Group C] | |
|---|---|
| Calcium carbonate | 30 g/L |

The ingredients of the groups A and B were sterilized at 115°C for 10 minutes by autoclaving, and the ingredient of the group C was sterilized at 180°C for 3 hours with dry heat. Then, the ingredients of the three groups were mixed, and 25 mg/L of chloramphenicol was added to the mixture.

**Table 4**

| | W3110/pSTV28 | W3110/pSTV-PanybjL | MS medium* |
|---|---|---|---|
| Asp | 26.69 | 62.35 | 40.21 |
| Thr | 0.00 | 0.00 | 36.23 |
| Ser | 0.00 | 0.00 | 56.03 |
| Glu | 1258.75 | 3702.74 | 127.50 |
| Gly | 0.00 | 0.00 | 27.28 |
| Ala | 5.49 | 9.79 | 77.61 |
| (Cys)₂ | 16.49 | 13.72 | 10.13 |
| Val | 3.30 | 8.02 | 59.32 |
| Met | 0.00 | 0.00 | 85.06 |
| Ile | 0.00 | 0.00 | 50.51 |
| Leu | 0.00 | 0.00 | 80.02 |
| Tyr | 48.20 | 0.00 | 38.80 |
| Phe | 2.88 | 4.47 | 70.49 |
| Lys | 8.07 | 8.00 | 50.50 |
| His | 0.00 | 0.00 | 20.95 |
| Arg | 0.00 | 0.00 | 35.80 |
| OD 620 nm (x 51) | 0.245 | 0.197 | - |
| Residual glucose (g/L) | 28.9 | 28.3 | 40.0 |

| | | | |
|---|---|---|---|
| *: Blank inoculated with no cells | | | |

It was revealed that not only L-glutamic acid accumulation amount, but also L-aspartic acid accumulation amount was improved in the *Escherichia coli* W3110/pSTV-PanybjL, which is a *ybjL* gene-amplified strain, as compared to the vector-introduced control strain, W3110/pSTV28 strain.

### Example 5: Effect of amplification of ybjL gene on L-glutamic acid production in Klebsiella planticola

The *ybjL* gene derived from *Pantoea ananatis* was introduced into *Klebsiella planticola,* and effect of the amplification thereof was examined.

The aforementioned vector for amplification of *ybjL* gene derived from *Pantoea ananatis,* pSTV-PanybjL, and the control plasmid pSTV28 were each introduced into a *Klebsiella planticola* L-glutamic acid-producing strain, AJ13410 strain (Japanese Patent Application No. 11-68324), by electroporation to obtain transformants exhibiting chloramphenicol resistance. The strain in which *ybjL* derived from *Pantoea ananatis* was amplified was designated as AJ13410/pSTV-PanybjL, and the pSTV28-introduced strain as a control was designated as AJ13410/pSTV28.

Then, L-glutamic acid-producing abilities of the *ybjL*-amplified strain, AJ13410/pSTV-PanybjL, and AJ13410/pSTV28 as a control were examined. AJ13410/pSTV-PanybjL and AJ13410/pSTV28 were precultured on the L medium (medium containing 10 g of Bacto tryptone, 5 g of yeast extract, 5 g of NaCl and 15 g of agar in 1 L of pure water, pH 7.0) added with chloramphenicol, and one loop of cells were inoculated by using a 1-mL volume loop produced by Nunc to 5 mL of a medium having the following composition contained in a test tube, and cultured at 37°C for 17 hours with shaking. Cell density and L-glutamic acid concentration in the culture medium were measured in the same manners as those in Example 2. The results are shown in Table 5.

### [Composition of culture medium]

| [Group A] | |
|---|---|
| Sucrose | 30 g/L |
| MgSO₄·7H₂O | 0.5 g/L |

| [Group B] | |
|---|---|
| KH₂PO₄ | 2.0 g/L |
| Yeast Extract | 2.0 g/L |
| FeSO₄·7H₂O | 0.02 g/L |
| MnSO₄·5H₂O | 0.02 g/L |
| L-Lysine hydrochloride | 0.2 g/L |
| DL-Methionine | 0.2 g/L |
| Diaminopimelic acid | 0.2 g/L |
| (adjusted to pH 7.0 with KOH) | |

| [Group C] | |
|---|---|
| CaCO₃ | 20 g/L |

The ingredients of the groups A and B were sterilized at 115°C for 10 minutes by autoclaving, and the ingredient of the group C was sterilized at 180°C for 3 hours with dry heat. Then, the ingredients of the three groups were mixed, and 25 mg/L of chloramphenicol was added to the mixture.

**Table 5**

| | OD 620 nm (x 51) | L-Glutamic acid (g/L) | Yield based on saccharide (%) |
|---|---|---|---|
| AJ13410/pSTV28 | 0.236 | 5.3 | 23.3 |
| AJ13910/pSTV-PanybjL | 0.220 | 12.1 | 48.7 |

It was revealed that L-glutamic acid-producing ability was markedly improved in the *Klebsiella planticola* AJ13410/pSTV-PanybjL, which is a *Pantoea ananatis ybjL* gene-amplified strain, as compared to the vector-introduced strain, AJ13410/pSTV28 strain, as a control.

### Reference Example 4: Construction of L,D-lactate dehydrogenase gene-deficient Escherichia coli strain

This gene was deleted by using the method called "Red-driven integration" developed by Datsenko and Wanner (Datsenko, K.A. and Wanner, B.L., 2000, Proc. Natl. Acad. Sci. USA, vol. 97, No. 12, pp. 6640-6645), and the λ phage excision system (Cho, E.H., Gumport, R.I., and Gardner, J.F., 2002, J. Bacteriol., 184 (18):5200-5203). According to this method, it is possible to construct a gene-disrupted strain in a single step by using a PCR product obtained with synthetic oligonucleotide primers in which a part of the target gene is designed in the 5' end sequence and a part of an antibiotic resistance gene is designed in the 3' end sequence. Furthermore, by using the λ phage excision system in combination, the antibiotic resistance gene which is integrated into the gene-disrupted strain can be removed.

### <1-1> Construction of strain deficient in IdhA gene coding for D-lactate dehydrogenase

According to the description of WO2005/010175, PCR was performed by using synthetic oligonucleotides having sequences corresponding to parts of the *IdhA* gene in the 5' end sequences and sequences corresponding to both ends of *attL* and *attR* of λ phage at the 3' end sequences as primers and plasmid pMW118-attL-Cm-attR as the template. pMW118-attL-Cm-attR is a plasmid obtained by inserting *attL* and *attR* genes, which are the attachment sites of λ phage, and the cat gene, which is an antibiotic resistance gene, into pMW118 (Takara Bio), and the genes are inserted in the order of *attL-cat-attR.* The sequences of the synthetic oligonucleotides used as the primers are shown in SEQ ID Nos. 13 and 14. The amplified PCR product was purified on an agarose gel and introduced into *Escherichia coli* MG1655 strain containing the plasmid pKD46 capable of temperature-sensitive replication by eletroporation. Then, an ampicillin-sensitive strain not harboring pKD46 was obtained, and the deletion of the I*dhA* gene was confirmed by PCR. PCR was performed by using the synthetic oligonucleotides shown in SEQ ID NOS: 15 and 16 as primers. Whereas the PCR product obtained for the parent strain had a size of about 1.2 kb, the deficient strain showed a band for a size of about 1.9 kb.

To eliminate the att-cat gene introduced into the *IdhA* gene, the strain was transformed with a helper plasmid pMW-intxis-ts, and an ampicillin resistant strain was selected. The pMW-intxis-ts contains the λ phage integrase (Int) gene and excisionase (Xis) gene and shows temperature-sensitive replication. Then, the strain of which att-cat and pMW-intxis-ts were eliminated was confirmed by PCR on the basis of ampicillin sensitivity and chloramphenicol sensitivity. PCR was performed by using the synthetic oligonucleotides shown in SEQ ID NOS: 15 and 16 as primers. Whereas the PCR product obtained for the strain in which *att*-cat remained had a size of about 1.9 kb, a band of about 0.3 kb was observed for the strain in which *att*-cat was eliminated. The *IdhA* deficient strain obtained as described above was designated as MG1655ΔldhA strain.

### <1-2> Construction of strain deficient in lldD gene coding for L-lactate dehydrogenase

A strain was constructed in the same manner as that of the construction of the *ldhA* gene-deficient strain. PCR was performed using synthetic oligonucleotides having sequences corresponding to parts of the *lldD* gene in the 5' end sequences and sequences corresponding to both ends of *attL* and *attR* of λ phage in the 3' end sequences as primers and plasmid pMW118-attL-Cm-attR as the template. The sequences of the synthetic oligonucleotides used as the primers are shown in SEQ ID Nos. 17 and 18. The amplified PCR product was purified on an agarose gel and introduced into the *Escherichia coli* MG1655ΔldhA strain containing plasmid pKD46 capable of temperature-sensitive replication by eletroporation. Then, an ampicillin-sensitive strain not harboring pKD46 was obtained, and the deletion of the *lldD* gene was confirmed by PCR. PCR was performed by using the synthetic oligonucleotides shown in SEQ ID NOS: 19 and 20 as primers. Whereas the PCR product obtained for the parent strain had a size of about 1.4 kb, a band of about 1.9 kb was observed for the deficient strain.

To eliminate the *att*-cat gene introduced into the *lldD* gene, the strain was transformed with a helper plasmid pMW-intxis-ts, and an ampicillin resistant strain was selected. Then, the strain of which *att*-cat and pMW-intxis-ts were eliminated was obtained and confirmed by PCR on the basis of ampicillin sensitivity and chloramphenicol sensitivity. PCR was performed by using the synthetic oligonucleotides shown in SEQ ID NOS: 19 and 20 as primers. Whereas the PCR product obtained for the strain in which *att*-cat remained had a size of about 1.9 kb, a band of about 0.3 kb was observed for the strain in which *att*-cat was eliminated. The *lldD* deficient strain obtained as described above was designated as MG1655ΔldhAΔlldD strain.

### Example 6: Construction of ybjL gene-enhanced strain of succinic acid-producing bacterium

### <6-1> Construction of plasmid for gene amplification

In order to amplify the *ybjL* gene, plasmid pMW219::Pthr was used. This plasmid corresponds to the vector pMW219 (Nippon Gene) having the promoter region of the threonine operon (*thrLABC*) in the genome of the *Escherichia coli* shown in SEQ ID NO: 21 at the HindIII site and BamHI site, and enables amplification of a gene by cloning the gene at a podition in the plasmid downstream from that promoter.

### <6-2> Construction of plasmid for enhancing ybjL

PCR was performed by using the synthetic oligonucleotide having a BamHI site shown in SEQ ID NO: 22 as a 5' primer, the synthetic oligonucleotide having a BamHI site shown in SEQ ID NO: 23 as a 3' primer, which were prepared on the basis of the nucleotide sequence of the *ybjL* gene in the genome sequence of *Escherichia coli* (GenBank Accession No. U00096), and the genomic DNA of *Escherichia coli* MG1655 strain as the template, and the product was treated with the restriction enzyme BamHI to obtain a gene fragment containing the *ybjL* gene. The PCR product was purified and ligated with the vector pMW219::Pthr treated with *Bam*HI to construct plasmid pMW219::Pthr::ybjL for *ybjL* amplification.

### <6-3> Production of ybjL-amplified strain

pMW219::Pthr::ybjL obtained in <6-2> described above and pMW219 were used to transform the *Escherichia coli* MG1655ΔldhAΔlldD strain by the electric pulse method, and the transformants were applied to the LB agar medium containing 25 µg/ml of kanamycin, and cultured at 37°C for about 18 hours. The colonies which appeared were purified, and plasmids were extracted from them in a conventional manner to confirm introduction of the target plasmid. The obtained strains were designated as MG1655AldhAΔlldD/pMW219::Pthr::ybjL and MG1655ΔldhAΔlldD/pMW219, respectively. The *Enterobacter aerogenes* AJ110637 strain was also transformed with pMW219::Pthr::ybjL and pMW219 by the electric pulse method, and the transformants were applied to the LB agar medium containing 50 µg/ml of kanamycin, and cultured at 37°C for about 18 hours. The colonies which appeared were purified, and plasmids were extracted from them in a conventional manner to confirm introduction of the target plasmid. The obtained strains were designated as AJ110637/pMW219::Pthr::ybjL and AJ110637/pMW219, respectively.

### Example 7: Effect of ybjL amplification in succinic acid-producing strain of Escherichia bacterium

MG1655ΔldhAΔlldD/pMW219::Pthr::ybjL and MG16556ldhA6lldD/pMW219 were each uniformly applied to an LB plate containing 25 µg/ml of kanamycin, and cultured at 37°C for 16 hours. Then, each plate was incubated at 37° C for 16 hours under an anaerobic condition by using Anaeropack (Mitsubishi Gas Chemical). The obtained cells on the plate were washed with 0.8% brine, and then diluted 51 times, and thereby a cell suspension having OD = 1.0 (600 nm) was prepared. This cell suspension in a volume of 100 µl and a production medium (10 g/l of glucose, 10 g/l 2Na malate, 45.88 g/l of TES, 6 g/l of Na₂HPO₄, 3 g/l of KH₂PO₄, 1 g/l of NH₄Cl, adjusted to pH 7.3 with KOH and filtered) in a volume of 1.3 ml in which dissolving gases in the medium were replaced with nitrogen gas by bubbling nitrogen gas beforehand were put into 1.5-ml volume micro tubes, and the cells were cultured at 31.5°C for 10 hours by using a stirrer for micro tubes. After the culture, amount of succinic acid accumulated in the medium was analyzed by liquid chromatography. Two of Shim-pack SCR-102H (Shimadzu) connected in series were used as the column, and a sample was eluted at 50°C with 5 mM p-toluenesulfonic acid. The eluate was neutralized with 20 mM Bis-Tris aqueous solution containing 5 mM p-toluenesulfonic acid and 100 µM EDTA, and succinic acid was quantified by measuring electric conductivity with CDD-10AD (Shimadzu).

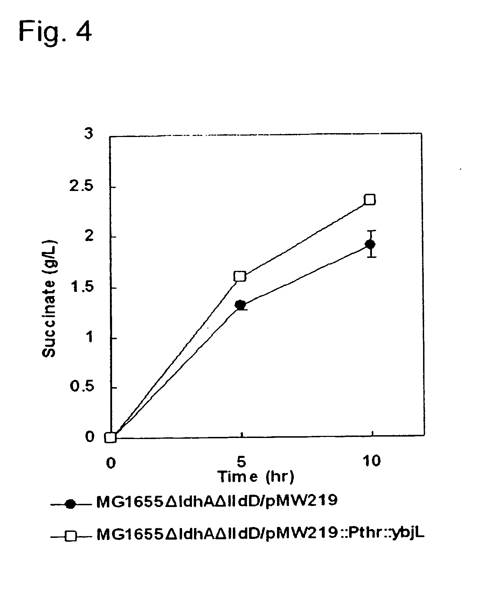

The amounts of succinic acid accumulated after 10 hours are shown in Table 6, and change of succinic acid accumulation is shown in Fig. 4.

Succinic acid accumulation was markedly increased in the *ybjL* gene-amplified strain MG1655ΔldhAΔlldD/pMW219::Pthr::ybjL as compared to MG1655ΔldhAΔlldD/pMW219 as the control.

**Table 6: Effect of ybjL amplification in succinic acid-producing strain, MG1655ΔldhAΔlldD**

| Strain | Succinate (g/L) |
|---|---|
| MG1655ΔldhAΔlldD/pMW219 | 1.91 (± 0.13) |
| MG1655ΔldhAΔlldD/pMW219::Pthr::ybjL | 2.34 (± 0.01) |

### Example 8: Effect of ybjL amplification in succinic acid-producing strain of Enterobacter bacterium

AJ110637/pMW219::Pthr::ybjL and AJ110637/pMW219 were each uniformly applied to an LB plate containing 50 µg/ml of kanamycin, and cultured at 37°C for 16 hours. Then, each plate was incubated at 37°C for 16 hours under an anaerobic condition by using Anaeropack (Mitsubishi Gas Chemical). The obtained cells on the plate were washed with 0.8% brine, and then diluted 51 times, and thereby a cell suspension having OD = 1.0 (600 nm) was prepared. This cell suspension in a volume of 100 µl and a production medium (10 g/l of glucose, 10 g/l 2Na malate, 45.88 g/l of TES, 6 g/l of Na₂HPO₄, 3 g/l of KH₂PO₄, 1 g/l of NH₄Cl, adjusted to pH 7.3 with KOH and filtered) in a volume of 1.3 ml in which dissolving gases in the medium were replaced with nitrogen gas by bubbling nitrogen gas beforehand were put into 1.5-ml volume micro tubes, and the cells were cultured at 31.5°C for 6 hours by using a stirrer for micro tubes. After the culture, amount of succinic acid accumulated in the medium was analyzed by liquid chromatography. Two of Shim-pack SCR-102H (Shimadzu) connected in series were used as the column, and a sample was eluted at 50°C with 5 mM p-toluenesulfonic acid. The eluate was neutralized with 20 mM Bis-Tris aqueous solution containing 5 mM p-toluenesulfonic acid and 100 µM EDTA, and succinic acid was quantified by measuring electric conductivity with CDD-10AD (Shimadzu).

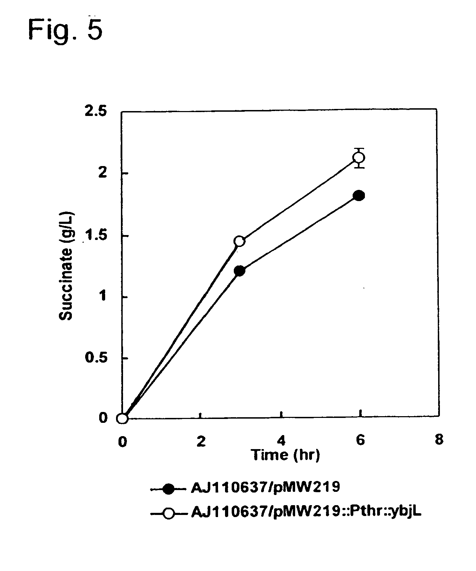

The amounts of succinic acid accumulated after 6 hours are shown in Table 7, and change of succinic acid accumulation is shown in Fig. 5.

Succinic acid accumulation was markedly increased in the *YbjL* gene-amplified strain AJ110637/pMW219::Pthr::ybjL as compared to AJ110637/pMW219 as the control.

**Table 7: Effect of ybjL amplification in AJ110637 strain**

| Strain | Succinate (g/L) |
|---|---|
| AJ110637/pMW219 | 1.81 (± 0.04) |
| AJ110637/pMW219::Pthr::ybjL | 2.11 (± 0.02) |

### Example 9

### <9-1> Construction of adhE-deficient strain of Enterobacter aerogenes AJ110637

When *Enterobacter aerogenes* AJ110637 is grown in a medium containing a sugar source under anaerobic conditions, it produces a marked amount of ethanol. Therefore, *adhE* coding for alcohol dehydrogenase was deleted to suppress generation of ethanol.

A gene fragment for deleting *adhE* was prepared by PCR using a plasmid pMW-attL-Tc-attR described in WO2005/010175 as the template and oligonucleotides of SEQ ID NOS: 72 and 73 as primers. pMW118-attL-Tc-attR is a plasmid obtained by inserting *attL* and *attR* genes, which are the attachment sites of λ phage, and the *Tc* gene, which is an antibiotic resistance gene, into pMW118 (Takara Bio), and the genes are inserted in the order of *attL-Tc-attR.* By PCR described above, a gene fragment containing a tetracycline resistance gene, *attL* and *attR* sequences of λ phage at the both ends of the resistance gene, and 60 bp upstream sequence and 59 bp downstream sequence of the *adhE* gene added to the outer ends of the λ phage sequences was amplified. This fragment was purified by using Wizard PCR Prep DNA Purification System (Promega).

Then, the *Enterobacter aerogenes* AJ110637 strain was transformed with RSF-Red-TER to obtain *Enterobacter aerogenes* AJ110637/RSF-Red-TER strain. This strain was cultured overnight in the LB medium containing 40 µg/L of chloramphenicol, the culture medium was inoculated in a 1/100 volume to 50 mL of the LB medium containing 40 µg/L of chloramphenicol and 0.4 mM isopropyl-β-D-thiogalactopyranoside, and culture was performed at 31°C for 4 hours. The cells were collected, washed three times with ice-cooled 10% glycerol, and finally suspended in 0.5 mL of 10% glycerol. The suspended cells were used as competent cells, and the PCR fragment prepared in the above section was introduced into the cells by using GENE PULSER II (BioRad) under the conditions of a field strength of 20 kV/cm, capacitor capacity of 25 µF and resistance of 200 Ω. Ice-cooled LB medium was added to the cell suspension, and culture was performed at 31°C for 2 hours with shaking. Then, the culture was applied to a medium prepared by adding 25 µg/L of tetracycline to the LB medium. The colonies that appeared were purified with the same medium, and then it was confirmed by PCR that the *adhE* gene was replaced with the tetracycline resistance gene.

### <9-2> Construction of pyruvate carboxylase gene-enhanced strain of AJ110637ΔadhE strain

An ability to produce succinic acid was imparted to the *Enterobacter aerogenes* AJ110637ΔadhE strain by amplifying *pyc* coding for pyruvate carboxylase derived from *Corynebacterium glutamicum* in that strain.

In order to express *pyc* derived from *Corynebacterium glutamicum* in the AJ110637ΔadhE strain, it was attempted to obtain a threonine operon promoter fragment of the *Escherichia coli* MG1655 strain. The total genomic sequence of *Escherichia coli* (*Escherichia coli* K-12 strain) has already been elucidated (Genbank Accession No.U00096, Science, 277, 1453-1474 (1997)). On the basis of this sequence, PCR amplification of the promoter region of the threonine operon (*thrLABC*) was performed. PCR was performed by using the synthetic oligonucleotide having an SacI site shown in SEQ ID NO: 75 as a 5' primer, the synthetic oligonucleotide shown in SEQ ID NO: 76 as a 3' primer, and the genomic DNA of *Escherichia coli* MG1655 strain (ATCC 47076, ATCC 700926) as a template to obtain a threonine operon promoter fragment (A) (SEQ ID NO: 77).

Furthermore, a *pyc* gene fragment derived from the *Corynebacterium glutamicum* 2256 strain (ATCC 13869) was obtained. PCR was performed by using the synthetic oligonucleotide shown in SEQ ID NO: 78 as a 5' primer, the synthetic oligonucleotide having an SacI site shown in SEQ ID NO: 79 as a 3' primer, and the genomic DNA of the *Corynebacterium glutamicum* 2256 strain (ATCC 13869) as a template to obtain a *pyc* gene fragment (B) (SEQ ID NO: 80).

PCR was performed by using the fragments (A) and (B) as templates, the primers of SEQ ID NOS: 75 and 79 to obtain a gene fragment (C) consisting of the fragments (A) and (B) ligated to each other. This gene fragment (C) was treated with the restriction enzyme SacI, and purified, and the product was ligated with the plasmid vector pSTV28 (Takara Bio) digested with the restriction enzyme SacI to construct a plasmid pSTV28::Pthr::pyc for *pyc* amplification.

The plasmid pSTV28::Pthr::pyc for *pyc* amplification was introduced into the aforementioned *Enterobacter aerogenes* AJ110637ΔadhE strain by electroporation to obtain a transformant exhibiting tetracycline and chloramphenicol resistance. This *pyc*-amplified strain of *Enterobacter aerogenes* AJ110637ΔadhE was designated as AJ110637ΔadhE/pSTV28::Pthr::pyc.

### <9-3> Construction of Enterobacter aerogenes ybjL gene-enhanced strain of AJ110637ΔadhE/pSTV28::Pthr::pyc

In the same manner as that described above, PCR amplification of the promoter region of the threonine operon (*thrLABC*) of *Escherichia coli (Escherichia coli* K-12 strain) was performed. PCR was performed by using the synthetic oligonucleotide shown in SEQ ID NO: 81 as a 5' primer, the synthetic oligonucleotide shown in SEQ ID NO: 82 as a 3' primer, and the genomic DNA of *Escherichia coli* MG1655 strain (ATCC 47076, ATCC 700926) as a template to obtain a threonine operon promoter fragment (A) (SEQ ID NO: 83).

Furthermore, in order to clone the *ybjL* gene of the *Enterobacter aerogenes* AJ110637 strain, PCR was performed by using the synthetic oligonucleotide shown in SEQ ID NO: 84 as a 5' primer, the synthetic oligonucleotide shown in SEQ ID. NO: 85 as a 3' primer, and the genomic DNA of the *Enterobacter aerogenes* AJ110637 strain as a template to obtain a *ybjL* gene fragment (B) (SEQ ID NO: 86).

PCR was performed by using the fragments (A) and (B) as templates, and the primers of SEQ ID NOS: 81 and 85 to obtain a gene fragment (C) consisting of the fragments (A) and (B) ligated to each other. This gene fragment (C) was blunt-ended by using TaKaRa BKL Kit (Takara Bio), and the 5' end was phosphorylated. Then, the fragment was digested with the restriction enzyme SmaI, and the product was ligated with the plasmid vector pMW218 dephosphorylated with alkaline phosphatase to construct a plasmid pMW218::Pthr::Ent-ybjL for *ybjL* amplification.

The aforementioned vector pMW218::Pthr::Ent-ybjL for amplification of the *ybjL* gene derived from the *Enterobacter aerogenes* AJ110637 strain, and the plasmid pMW218 for control were each introduced into the *Enterobacter aerogenes* AJ110637ΔadhE/pSTV28::Pthr::pyc strain by electroporation to obtain transformants exhibiting tetracycline, chloramphenicol and kanamycin resistance. The *ybjL*-amplified strain derived from the *Enterobacter aerogenes* AJ110637ΔadhE/pSTV28::Pthr::pyc strain was designated as AJ110637ΔadhE/pSTV28::Pthr::pyc/pMW218::Pthr::Ent-ybjL, and the pMW218-introduced strain as a control was designated as AJ110637ΔadhE/pSTV28::Pthr::pyc/pMW218.

### <9-4> Effect of amplification of ybjL derived from Enterobacter aerogenes in Enterobacter bacterium

AJ110637ΔadhE/pSTV28::Pthr::pyc/pMW218::Pthr::Ent-ybjL, and AJ110637ΔadhE/pSTV28::Pthr::pyc/pMW218 were each uniformly applied to an LB plate containing 50 µg/ml of kanamycin, 25 g/ml of tetracycline, and 40 µg/ml of chloramphenicol, and cultured at 31.5°C for 16 hours. Then, the cells were inoculated into 3 ml of a seed medium (20 g/l of Bacto tryptone, 10 g/l of yeast extract, 20 g/L of NaCl) contained in a test tube, and cultured at 31.5°C for 16 hours with shaking. A succinic acid production medium (100 g/l of glucose, 50 g/L of calcium carbonate subjected to hot air sterilization for 3 hours or more) in a volume of 3 ml was added to the medium obtained above, then the tube was sealed with a silicone stopper, and culture was performed at 31.5°C for 24 hours with shaking. After the culture, amount of succinic acid accumulated in the medium was analyzed by liquid chromatography. Two of Shim-pack SCR-102H (Shimadzu) connected in series were used as the column, and a sample was eluted at 50°C with 5 mM p-toluenesulfonic acid. The eluate was neutralized with 20 mM Bis-Tris aqueous solution containing 5 mM p-toluenesulfonic acid and 100 µM EDTA, and succinic acid was quantified by measuring electric conductivity with CDD-10AD (Shimadzu).

The amounts of succinic acid accumulated after 24 hours are shown in Table 8.

Succinic acid accumulation and succinic acid yield based on consumed saccharide were markedly increased in the *ybjL* gene-amplified strain AJ110637ΔadhE/pSTV28::Pthr::pyc/pMW218::Pthr::Ent-ybjL as compared to AJ110637ΔadhE/pSTV28::Pthr::pyc/pMW218 as the control.

**Table 8**

| | AJ110637 Δ adhE/pSTV28 ::Pthr::pyc/pMW218 | AJ110637ΔadhE/pSTV28:: Pthr::pyc/pMW218::Pthr: :Ent-ybjE |
|---|---|---|
| Consumed saccharide amount (g/L) | 9.53 (± 1.85) | 9.10 (± 0.66) |
| OD (600 nm) | 8.90 (± 0.18) | 8.72 (± 0.95) |
| Succinic acid accumulation (g/L) | 3.40 (± 0.56) | 6.37 (± 0.51) |
| Succinic acid yield based on consumed saccharide (%) | 35.80 (± 1.82) | 69.70 (± 1.79) |

### Explanation of Sequence Listing

SEQ ID NO: 1: *ybjL* gene of *Pantoea ananatis*
SEQ ID NO: 2: YbjL of *Pantoea ananatis*
SEQ ID NO: 3: *ybjL* gene of *Escherichia coli*
SEQ ID NO: 4: YbjL of *Escherichia coli*
SEQ ID NO: 5: Consensus sequence of YbjL of *Pantoea ananatis* and *Escherichia coli*
SEQ ID NO: 6: Primer for *ams* gene disruption
SEQ ID NO: 7: Primer for *ams* gene disruption
SEQ ID NO: 8: Primer for amplification of *ybjL* of *P. ananatis*
SEQ ID NO: 9: Primer for amplification of *ybjL* of *P. ananatis*
SEQ ID NO: 10: Sequence of RSFCPG plasmid
SEQ ID NO: 11: Primer for amplification of *ybjL* of *E. coli* W3110
SEQ ID NO: 12: Primer for amplification of *YbjL* of *E. coli* W3110
SEQ ID NO: 13: Primer for deletion of *ldhA*
SEQ ID NO: 14: Primer for deletion of *ldhA*
SEQ ID NO: 15: Primer for confirming deletion of *ldhA*
SEQ ID NO: 16: Primer for confirming deletion of *ldha*
SEQ ID NO: 17: Primer for deletion of *lldD*
SEQ ID NO: 18: Primer for deletion of *lldD*
SEQ ID NO: 19: Primer for confirming deletion of *lldD*
SEQ ID NO: 20: Primer for confirming deletion of *lldD*
SEQ ID NO: 21: Threonine promoter sequence
SEQ ID NO: 22: Primer for amplification of *ybjL* of *E. coli* MG1655
SEQ ID NO: 23: Primer for amplification of *ybjL* of *E. coli* MG1655
SEQ ID NO: 24: *ybjL* gene of *Salmonella typhimurium*
SEQ ID NO: 25: YbjL of *Salmonella typhimurium*
SEQ ID NO: 26: *ybjL* gene of *Yersinia pestis*
SEQ ID NO: 27: YbjL of *Yersinia pestis*
SEQ ID NO: 28: *ybjL* gene of *Erwinia carotovora*
SEQ ID NO: 29: YbjL of *Erwinia carotovora*
SEQ ID NO: 30: *ybjL* gene of *Vibrio cholerae*
SEQ ID NO: 31: YbjL of *Vibrio cholerae*
SEQ ID NO: 32: *ybjL* gene of *Aeromonas hydrophia*
SEQ ID NO: 33: YbjL of *Aeromonas hydrophia*
SEQ ID NO: 34: *ybjL gene* of *Photobacterium profundum*
SEQ ID NO: 35: YbjL of *Photobacterium profundum*
SEQ ID NO: 36: *Idha* gene of *Escherichia coli*
SEQ ID NO: 37: LdhA of *Escherichia coli*
SEQ ID NO: 38: *IldD* gene of *Escherichia coli*
SEQ ID NO: 39: LldD of *Escherichia coli*
SEQ ID NO: 40: Nucleotide sequence of *hisD* gene of *Pantoea ananatis*
SEQ ID NO: 41: Primer for amplification of fragment for incorporation of Km^{r} gene into *hisD* gene
SEQ ID NO: 42: Primer for amplification of fragment for incorporation of Km^{r} gene into *hisD* gene
SEQ ID NO: 43: Primer for amplification of *cat* gene
SEQ ID NO: 44: Primer for amplification of *cat* gene
SEQ ID NO: 45: Primer for amplification of *sacB* gene
SEQ ID NO: 46: Primer for amplification of *sacB* gene
SEQ ID NO: 47: Primer for amplification of DNA fragment containing PlacUV5 promoter
SEQ ID NO: 48: Primer for amplification of DNA fragment containing PlacUV5 promoter
SEQ ID NO: 49: Primer for amplification of DNA fragment containing λRedaβγ gene and tL3
SEQ ID NO: 50: Primer for amplification of DNA fragment containing λRedαβγ gene and tL3
SEQ ID NO: 51: Primer for amplification of DNA fragment containing PlacUV5 promoter and TrrnB
SEQ ID NO: 52: Primer for amplification of DNA fragment containing PlacUV5 promoter and TrrnB
SEQ ID NO: 53: Primer for amplification of *attL*
SEQ ID NO: 54: Primer for amplification of *attL*
SEQ ID NO: 55: Nucleotide sequence of *attL*
SEQ ID NO: 56: Primer for amplification of *attR*
SEQ ID NO: 57: Primer for amplification of *attR*
SEQ ID NO: 58: Nucleotide sequence of *attR*
SEQ ID NO: 59: Primer for amplification of DNA fragment containing *bla* gene
SEQ ID NO: 60: Primer for amplification of DNA fragment containing *bla* gene
SEQ ID NO: 61: Primer for amplification of DNA fragment containing *ter_rrnB*
SEQ ID NO: 62: Primer for amplification of DNA fragment containing *ter_rrnB*
SEQ ID NO: 63: Nucleotide sequence of the DNA fragment containing ter_thrL terminator
SEQ ID NO: 64: Primer for amplification of DNA fragment containing ter_thrL terminator
SEQ ID NO: 65: Primer for amplification of DNA fragment containing ter_thrL terminator
SEQ ID NO: 66: *ams* operon of *Pantoea ananatis*
SEQ ID NO: 67: AmsH of *Pantoea ananatis*
SEQ ID NO: 68: AmsI of *Pantoea ananatis*
SEQ ID NO: 69: AmsA of *Pantoea ananatis*
SEQ ID NO: 70: AmsC of *Pantoea ananatis*
SEQ ID NO: 71: AmsB of *Pantoea ananatis*
SEQ ID NO: 72: Nucleotide sequence of primer for deletion of *adhE*
SEQ ID NO: 73: Nucleotide sequence of primer for deletion of *adhE*
SEQ ID NO: 74: Nucleotide sequence of *adhE* of *Enterobacter aerogenes* AJ110637 (partial sequence)
SEQ ID NO: 75: Primer for amplification of threonine promoter
SEQ ID NO: 76: Primer for amplification of threonine promoter
SEQ ID NO: 77: Threonine promoter gene fragment
SEQ ID NO: 78: Primer for amplification of pyruvate carboxylase gene
SEQ ID NO: 79: Primer for amplification of pyruvate carboxylase gene
SEQ ID NO: 80: Pyruvate carboxylase gene fragment
SEQ ID NO: 81: Primer for amplification of threonine promoter
SEQ ID NO: 82: Primer for amplification of threonine promoter
SEQ ID NO: 83: Threonine promoter gene fragment
SEQ ID NO: 84: Nucleotide sequence of primer for amplification of *ybjL* of *Enterobacter aerogenes* AJ110637
SEQ ID NO: 85: Nucleotide sequence of primer for amplification of *ybjL* of *Enterobacter aerogenes* AJ110637
SEQ ID NO: 86: Nucleotide sequence of *ybjL* of *Enterobacter aerogenes* AJ110637
SEQ ID NO: 87: Amino acid sequence of YbjL of *Enterobacter aerogenes* AJ110637
SEQ ID NO: 88: Consensus sequence of YbjL of *Escherichia, Pantoea* and *Enterobacter*

### Industrial Applicability

Production efficiency of an acidic substance having a carboxyl group can be improved by using the microorganism used in the present invention.

## Claims

1. A method for producing an acidic substance having a carboxyl group, which comprises culturing a microorganism which has an ability to produce an acidic substance having a carboxyl group and has been modified so that expression of the *ybjL* gene is enhanced by increasing copy number of the *ybjL* gene or modifying an expression control sequence of the *ybjL* gene, wherein the *ybjL* gene is a gene coding for a protein defined in the following (A) or (B):
(A) a protein comprising the amino acid sequence of SEQ ID NO: 2, 4 or 87;
(B) a protein comprising an amino acid sequence of SEQ ID NO: 2, 4 or 87, wherein one to 10 amino acid residues are substituted, deleted, inserted or added, and the protein improves an ability of the microorganism to produce an acidic substance having a carboxyl group when expression of the protein is enhanced in the microorganism;
in a medium to produce and accumulate the acidic substance having a carboxyl group in the medium, and collecting the acidic substance having a carboxyl group from the medium,
wherein the acidic substance consists of one or more kinds of organic acids selected from the group consisting of succinic acid, fumaric acid, malic acid, oxalacetic acid, citric acid, isocitric acid, α-ketoglutaric acid, L-glutamic acid, and L-aspartic acid.

2. The method according to claim 1, wherein the *ybjL* gene is a gene coding for the protein of SEQ ID NO: 5 or 88.

3. The method according to claim 1 or 2, wherein the microorganism is a bacterium belonging to the family *Enterobacteriaceae.*

4. The method according to claim 3, wherein the microorganism is selected from the group consisting of bacteria belonging to the genera *Escherichia, Enterobacter, Raoultella, Pantoea,* and *Klebsiella.*

5. The method according to claim 1 or 2,, wherein the microorganism is a rumen bacterium.

6. The method according to claim 5, wherein the microorganism is *Mannheimia succiniciproducens.*

7. A method for producing an acidic substance having a carboxyl group, which comprises allowing a microorganism which has an ability to produce an acidic substance having a carboxyl group and has been modified so that expression of the *ybjL* gene is enhanced by increasing copy number of the *ybjL* gene or modifying an expression control sequence of the *ybjL* gene, wherein the *ybjL* gene is a gene coding for a protein defined in the following (A) or (B):
(A) a protein comprising the amino acid sequence of SEQ ID NO: 2, 4 or 87;
(B) a protein comprising an amino acid sequence of SEQ ID NO: 2, 4 or 87, wherein one to 10 amino acid residues are substituted, deleted, inserted or added, and the protein improves an ability of the microorganism to produce an acidic substance having a carboxyl group when expression of the protein is enhanced in the microorganism;
or a product obtained by processing the microorganism to act on an organic raw material in a reaction mixture containing carbonate ions, bicarbonate ions, or carbon dioxide gas to produce the acidic substance having a carboxyl group, and collecting the acidic substance having a carboxyl group.

8. The method according to claim 7, wherein the acidic substance consists of one or more kinds of organic acids selected from the group consisting of succinic acid, fumaric acid, malic acid, oxalacetic acid, citric acid, isocitric acid, and α-ketoglutaric acid.

9. A method for producing a succinic acid-containing polymer, which comprises the step of producing succinic acid by the method according to any one of claims 1 to 6 and 8, and the step of polymerizing the obtained succinic acid.

## Patentansprüche

1. Verfahren zum Herstellen einer sauren Substanz mit einer Carboxygruppe, welches das Kultvieren eines Mikroorganismus, der die Fähigkeit zur Produktion einer sauren Substanz mit einer Carboxygruppe hat und so modifiziert worden ist, dass dei Expression des ybjL-Gens durch Erhöhen der Kopienzahl des ybjL-Gens oder durch Modifizieren einer Expressionskontrollsequenz des ybjL-Gens erhöht ist, wobei das ybjL-Gen ein Gen ist, dass für ein in den folgenden (A) oder (B) definiertes Protein kodiert:
(A) ein Protein, das die Aminosäuresequenz der SEQ ID NO: 2, 4 oder 87 umfasst;
(B) ein Protein, das eine Aminosäuresequenz der SEQ ID NO: 2, 4 oder 87 umfasst, wobei ein bis 10 Aminosäurereste substituiert, deletiert, insertiert oder addiert sind, und das Protein die Fähigkeit des Mikroorganismus zur Produktion einer sauren Substanz mit einer Carboxygruppe verbessert, wenn die Expression des Proteins in dem Mikroorganismus erhöht ist;
in einem Medium zur Produktion und Anhäufung der sauren Substanz mit einer Carboxygruppe in dem Medium und das Gewinnen der sauren Substanz mit einer Carboxygruppe aus dem Medium umfasst, wobei die saure Substanz aus einer oder mehreren Arten von organischen Säuren ausgewählt ist, die aus der Gruppe ausgewählt sind, die aus Bernsteinsäure, Fumarsäure, Äpfelsäure, Oxalessigsäure, Zitronensäure, Isozitronensäure, α-Ketoglutarsäure, L-Glutaminsäure und L-Asparaginsäure besteht.

2. Verfahren nach Anspruch 1, wobei das ybjL-Gen ein Gen ist, das für das Protein der SEQ ID NO: 5 oder 88 kodiert.

3. Verfahren nach Anspruch 1 oder 2, wobei der Mikroorganismus ein Bakterium der Familie Enterobacteriaceae ist.

4. Verfahren nach Anspruch 3, wobei der Mikroorganismus aus der Gruppe ausgewählt ist, die aus Bakterien der Gattung Escherichia, Enterobacter, Raoultella, Pantoea und Klebsiella besteht.

5. Verfahren nach Anspruch 1 oder 2, wobei der Mikroorganismus ein Pansenbakterium ist.

6. Verfahren nach Anspruch 5, wobei der Mikroorganismus Mannheimia succiniciproducens ist.

7. Verfahren zum Herstellen einer sauren Substanz mit einer Carboxygruppe, welches das Zulassen des Umsetzens eines Mikroorganismus, der eine saure Substanz mit einer Carboxygruppe produzieren kann und so modifiziert worden ist, dass die Expression des ybjL-Gens durch Erhöhen der Kopienzahl des ybjL-Gens oder durch Modifizieren einer Expressionskontrollsequenz des ybjL-Gens erhöht ist, wobei das ybjL-Gen ein Gen ist, das für ein in den folgenden (A) oder (B) definiertes Protein kodiert:
(A) ein Protein, das die Aminosäuresequenz der SEQ ID NO: 2, 4 oder 87 umfasst;
(B) ein Protein, das eine Aminosäuresequenz der SEQ ID NO: 2, 4 oder 87 umfasst, wobei ein bis 10 Aminosäurereste substituiert, deletiert, insertiert oder addiert sind, und das Protein die Fähigkeit des Mikroorganismus zur Produktion einer sauren Substanz mit einer Carboxygruppe verbessert, wenn die Expression des Proteins in dem Mikroorganismus erhöht ist;
oder eines durch Verarbeiten des Mikroorganismus erhaltenen Produkts in einem Reaktionsgemisch, das Carbonationen, Bicarbonationen oder Kohlendioxidgas enthält, mit einem organischen Rohmaterial, wobei die saure Substanz mit einer Carboxygruppe produziert wird, und das Gewinnen der sauren Substanz mit einer Carboxygruppe umfasst.

8. Verfahren nach Anspruch 7, wobei die saure Substanz eine oder mehr Arten von organischen Säuren umfasst, die aus der Gruppe ausgewählt sind, die aus Bernsteinsäure, Fumarsäure, Äpfelsäure, Oxalessigsäure, Zitronensäure, Isozitronensäure und α-Ketoglutarsäure besteht.

9. Verfahren zum Herstellen eines Bernsteinsäure enthaltenden Polymers, welches die Stufe, in der Bernsteinsäure durch das Verfahren nach einem der Ansprüche 1 bis 6 und 8 produziert wird, und die Stufe umfasst, in der die erhaltene Bernsteinsäure polymerisiert wird.

## Revendications

1. Procédé pour produire une substance acide ayant un groupe carboxyle, qui comprend la culture d'un microorganisme qui a une aptitude à produire une substance acide ayant un groupe carboxyle et a été modifié de sorte que l'expression du gène *ybjL* est augmentée par augmentation du nombre de copies du gène *ybjL* ou modification d'une séquence de contrôle de l'expression du gène *ybjL,* où le gène *ybjL* est un gène codant une protéine définie dans (A) ou (B) suivant :
(A) une protéine comprenant la séquence d'aminoacides de SEQ ID NO:2, 4 ou 87 ;
(B) une protéine comprenant une séquence d'aminoacides de SEQ ID NO:2, 4 ou 87, où un à 10 résidus d'aminoacides sont substitués, délétés, insérés ou ajoutés, et la protéine améliore une aptitude du microorganisme à produire une substance acide ayant un groupe carboxyle quand l'expression de la protéine est augmentée dans le microorganisme ;
dans un milieu pour produire et accumuler la substance acide ayant un groupe carboxyle dans le milieu, et la collecte de la substance acide ayant un groupe carboxyle à partir du milieu,
où la substance acide consiste en un ou plusieurs types d'acides organiques choisis dans le groupe consistant en l'acide succinique, l'acide fumarique, l'acide malique, l'acide oxaloacétique, l'acide citrique, l'acide isocitrique, l'acide α-cétoglutarique, l'acide L-glutamique et l'acide L-aspartique.

2. Procédé selon la revendication 1, où le gène *ybjL* est un gène codant la protéine de SEQ ID NO:5 ou 88.

3. Procédé selon la revendication 1 ou 2, où le microorganisme est une bactérie appartenant à la famille des *Enterobacteriaceae.*

4. Procédé selon la revendication 3, où le microorganisme est choisi dans le groupe consistant en les bactéries appartenant aux genres *Escherichia, Enterobacter, Raoultella, Pantoea* et *Klebsiella.*

5. Procédé selon la revendication 1 ou 2, où le microorganisme est une bactérie de rumen.

6. Procédé selon la revendication 5, où le microorganisme est *Mannheimia succiniciproducens.*

7. Procédé pour produire une substance acide ayant un groupe carboxyle qui comprend le fait d'amener un microorganisme qui a une aptitude à produire une substance acide ayant un groupe carboxyle et a été modifié de sorte que l'expression du gène *ybjL* est augmentée par augmentation du nombre de copies du gène *ybjL* ou modification d'une séquence de contrôle de l'expression du gène *ybjL,* où le gène *ybjL* est un gène codant une protéine définie dans (A) ou (B) suivant :
(A) une protéine comprenant la séquence d'aminoacides de SEQ ID NO:2, 4 ou 87 ;
(B) une protéine comprenant une séquence d'aminoacides de SEQ ID NO:2, 4 ou 87, où un à 10 résidus d'aminoacides sont substitués, délétés, insérés ou ajoutés, et la protéine améliore une aptitude du microorganisme à produire une substance acide ayant un groupe carboxyle quand l'expression de la protéine est augmentée dans le microorganisme ;
ou un produit obtenu par traitement du microorganisme à agir sur une matière première organique dans un mélange réactionnel contenant des ions carbonate, des ions bicarbonate ou du gaz dioxyde de carbone pour produire la substance acide ayant un groupe carboxyle, et la collecte de la substance acide ayant un groupe carboxyle.

8. Procédé selon la revendication 7, où la substance acide consiste en un ou plusieurs types d'acides organiques choisis dans le groupe consistant en l'acide succinique, l'acide fumarique, l'acide malique, l'acide oxaloacétique, l'acide citrique, l'acide isocitrique et l'acide α-cétoglutarique.

9. Procédé pour produire un polymère contenant de l'acide succinique qui comprend l'étape de production d'acide succinique par le procédé selon l'une quelconque des revendications 1 à 6 et 8 et l'étape de polymérisation de l'acide succinique obtenu.
